# EUROPEAN PATENT SPECIFICATION

(11) **EP 2 281 060 B1**
(45) Date of publication and mention of the grant of the patent: **19.02.2014**
(21) Application number: 09731128.6
(22) Date of filing: 07.04.2009
(51) Int. Cl.: C12Q 1/68

(54) **ANDROGENETIC ALOPECIA**
ANDROGENETISCHER HAARAUSFALL
ALOPÉCIE ANDROGÉNÉTIQUE

(30) Priority: 07.04.2008 EP 08006933
(43) Date of publication of application: 09.02.2011
(73) Proprietor: Life & Brain GmbH, 53127 Bonn (DE); Heinrich-Heine-Universität Düsseldorf, 40225 Düsseldorf (DE); Rheinische Friedrich-Wilhelms-Universität, 53113 Bonn (DE)
(72) Inventor: NÖTHEN, Markus, M., 53127 Bonn (DE); BROCKSCHMIDT, Felix, F., 53127 Bonn (DE); KRUSE, Roland, 40225 Düsseldorf (DE); HILLMER, Axel, Singapore 138672 (SG)
(74) Representative: Vossius & Partner
(86) International application number: PCT/EP2009/002564
(87) International publication number: WO 2009/124720

(56) References cited:
- ELLIS JUSTINE A ET AL: "Polymorphism of the androgen receptor gene is associated with male pattern baldness" JOURNAL OF INVESTIGATIVE DERMATOLOGY, vol. 116, no. 3, March 2001 (2001-03), pages 452-455, XP002495449 ISSN: 0022-202X
- HILLMER AXEL M ET AL: "Genome-wide scan and fine-mapping linkage study of androgenetic alopecia reveals a locus on chromosome 3q26" AMERICAN JOURNAL OF HUMAN GENETICS, vol. 82, no. 3, March 2008 (2008-03), pages 737-743, XP002533378 ISSN: 0002-9297
- HILLMER AXEL M ET AL: "Susceptibility variants for male-pattern baldness on chromosome 20p11" NATURE GENETICS, vol. 40, no. 11, November 2008 (2008-11), pages 1279-1281, XP002533379 ISSN: 1061-4036
- RICHARDS J BRENT ET AL: "Male-pattern baldness susceptibility locus at 20p11" NATURE GENETICS, vol. 40, no. 11, November 2008 (2008-11), pages 1282-1284, XP002533380 ISSN: 1061-4036
- "rs2424407", SNP, [Online] XP002495450, Retrieved from the Internet: URL:http://www.ncbi.nlm.nih.gov/SNP/snp_ss .cgi?subsnp_id=3395026> [retrieved on 2001-09-21]
- MCGAUGHRAN J M ET AL: "Mutations in PAX1 may be associated with Klippel-Feil syndrome.", EUROPEAN JOURNAL OF HUMAN GENETICS, vol. 11, no. 6, June 2003 (2003-06), pages 468-474, ISSN: 1018-4813
- GIAMPIETRO P F ET AL: "An analysis of PAX1 in the development of vertebral malformations", CLINICAL GENETICS, vol. 68, no. 5, November 2005 (2005-11), pages 448-453, ISSN: 0009-9163

## Description

The present invention relates to methods for testing for a predisposition or presence of androgenetic alopecia comprising testing a sample obtained from a prospective patient or from a person suspected of carrying a predisposition for androgenetic alopecia as characterised in the appended claims.

Androgenetic alopecia (AGA, [MIM 109200]) is the most common form of hair loss in humans, occurring both in men and women. In men, this condition is commonly known as male pattern baldness (MPB), as hair is lost in a well-defined pattern, beginning above both temples. Over time, the hairline recedes and hair also thins at the crown (near the top of the head), often progressing to partial or complete baldness. In Caucasians, MBP is noticeable in about 20% of men aged 20, and increases steadily with age, so that a male in his 90s has a 90% chance of having some degree of MPB. In women, onset of androgenetic alopecia tends to occur later and in a milder form (Hanneken et al. (2003), Hautarzt 54:703-712). The pattern of hair loss in women also differs from male-pattern baldness. In women, the hair becomes thinner all over the head, and the hairline does not recede. Androgenetic alopecia in women rarely leads to total baldness.

The negative psychosocial effects associated with hair loss include decreased self-esteem, dissatisfaction with body image or appearance, self-consciousness, perception of aging, and often emotional stress. Thus, androgenetic alopecia has a considerable effect on the quality of life for many of those affected.

In addition to the psychosocial effects, recent studies indicate associations of male pattern baldness with: (1) benign prostatic hyperplasia (MIM 600082) (Hawk et al., (2000) B.I. Cancer Epidemiol Biomarkers Prev 9, 523-527); (2) coronary heart disease (Lotufo et al., (2000) J.E. Arch Intern Med 160, 165-171); (3) hyperinsulinemia; and (4) insulin-resistance-associated disorders, such as obesity (MIM 601665), hypertension (MIM 145500), and dyslipidemia (Matilainen et al, (2000) S. Lancet 356, 1165-1166). MBP is also a risk factor for clinical prostate cancer (MIM 176807) (Oh et al., (1998) Urology 51, 744-748).

The origin of androgenetic alopecia is genetic, with a high heritability of 81% (Nyholt DR, et al. (2003), J Invest Dermatol 121:1561-1564). So far, the androgen receptor gene (AR) located on the X chromosome is the only risk gene identified (Ellis, J.A., et al. (2001), J Invest Dermatol 116, 452-455; Ellis, J.A. et al. (2007), Hum Genet 121, 451-457; Hillmer et al. (2005) Am J Hum Genet 77, 140-148). Variations in the *AR* gene are assumed to lead to increased activity of androgen receptors in hair follicles, which can lead to a shorter cycle of hair growth and the growth of shorter and thinner strands of hair. Additionally, there is a delay in the growth of new hair to replace hair shafts that are shed.

The investigation of a large number of genetic variants covering the *AR* locus demonstrated that genetic variation in the AR gene is one prerequisite for the development of early-onset androgenetic alopecia, with an etiologic fraction of 0.46 (Hillmer et al. (2005) Am J Hum Genet 77, 140-148). However, the genetic variation in AR, which is located on the X chromosome, cannot explain the resemblance of fathers and sons with respect to androgenetic alopecia (Küster W and Happle R (1984), J Am Acad Dermatol 11, 921-926; Ellis, JA et al. (1998), J Invest Dermatol 110, 849-853), since sons always inherit the X chromosome from their mothers.

Hillmer et al. 2008 (American Journal of Human Genetics, 82: 737-743) describes a genomwide study which identified SNP linked to androgenetic alopecia. Whereas Hillmer *et al.* 2008 investigated SNP on chromosome 20, it does not disclose a linkage to androgenetic alopecia.

Thus, although androgen dependence is an important characteristic of androgenetic alopecia and genetic disposition and plays a substantial role in the development of androgenetic alopecia, androgenetic alopecia is nonetheless assumed to be polygenic, (Küster W and Happle R (1984), J Am Acad Dermatol 11, 921-926; Ellis, JA et al. (1998), J Invest Dermatol 110, 849-853; Nyholt, DR et al. (2003), J Invest Dermatol 121, 1561-1564). The psychosocial effects as well as the association of androgenetic alopecia with a variety of diseases underlines the value of understanding the molecular basis of this common form of hair loss.

The technical problem underlying the present invention was the provision of means and methods useful in the diagnosis of androgenetic alopecia or a predisposition thereto.

The solution to this technical problem is achieved by providing the embodiments characterised in the claims.

Accordingly, the present invention relates to a method for testing for a predisposition or presence of androgenetic alopecia comprising testing a sample obtained from a prospective patient or from a person suspected of carrying a predisposition for androgenetic alopecia, the method comprising determining (a) the presence and/or quantity of a nucleic acid molecule selected from the group consisting of: (i) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (ii) a nucleic acid molecule the complementary strand of which hybridises under stringent conditions to the nucleic acid molecule of (a)(i), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine; (iii) a nucleic acid molecule identical to any one of the nucleic acid molecules of (a)(i) or (a)(ii) wherein each thymidine is replaced by uridine; and/or (b) the presence and/or quantity of a nucleic acid molecule selected from the group consisting of: (i) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212. 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39. 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23. 26 to 28, 30 to 33, 36, 37, 41 to 47, 49, 52. 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; (ii) a nucleic acid molecule the complementary strand of which hybridises under stringent conditions to the nucleic acid molecule of (b)(i), wherein said nucleic acid molecule has at a position corresponding to position 201 of any one of SEQ ID NOs: SEQ ID NOs:78. 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine; (iii) a nucleic acid molecule identical to any one of the nucleic acid molecules of (b) (i) or (b)(ii) wherein each thymidine is replaced by uridine; and/or (c) the presence and/or quantity of a nucleic acid molecule which is complementary to the nucleic acid molecule of (a) or (b); or (d) the presence of a risk allele within the nucleic acid molecule of (a) as compared to the non-risk allele, wherein said risk allele is characterised in that it is an adenosine at position 201 of any one of SEQ ID NOs:1 to 77, a guanosine at position 201 of any one of SEQ ID NOs:78 to 149, a thymidine at position 201 of any one of SEQ !D NOs:150 to 219 or a cytidine at position 201 of any one of SEQ ID NOs:220 to 292; or (e) the presence of a risk allele within the nucleic acid molecule of (c) as compared to the non-risk allele, wherein said risk allele is characterised in that it is an thymidine at position 201 of any one of SEQ ID NOs:1 to 77, a cytidine at position 201 of any one of SEQ ID NOs:78 to 149, a adenosine at position 201 of any one of SEQ ID NOs:150 to 219 or a guanosine at position 201 of any one of SEQ ID NOs:220 to 292; or (f) the presence of a non-risk allele within the nucleic acid molecule of (b) as compared to the risk allele, wherein said non-risk allele is characterised in that it is an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6. 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 287, to 272, 276, 278, 280 to 283, 285 to 292; or (g) the presence of a non-risk allele within the nucleic acid molecule of (c) as compared to the risk allele, wherein said non-risk allele is characterised in that it is an thymidine at position 201 of any one of SEQ ID NOs:78, 79, 83, 86, 87, 90, 91, 93, 94, 96, 98 to 109, 111, 112, 116 to 123, 126, 128 to 142, 144 to 149, 151. 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a guanosine at position 201 of any one of SEQ ID NOs: 3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a cytidine at position 201 of any one of SEQ ID NOs: 1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a adenosine at position 201 of any one of SEQ 10 NOs:6, 12, 15, 17, 19, 24, 29, 40, 56, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; or (h) the presence of a risk allele of a genetic marker of androgenetic alopecia characterised in that the genetic marker is located in a chromosomal region of human chromosome 20p11 as characterised in the appended claims and wherein the genetic marker is selected from the group consisting of a single nucleotide polymorphism (SNP), variable number of tandem repeat (VNTR), microsatellites or short tandem repeats (STR) and wherein the presence of said nucleic acid molecule of (a) said genetic marker, or said risk allele is indicative of androgenetic alopecia or a predisposition for androgenetic alopecia and wherein the presence of said nucleic acid molecule of (b) said non-risk allele is indicative of the absence of androgenetic alopecia or a predisposition for androgenetic alopecia.

The term "androgenetic alopecia (AGA)" as used in accordance with the present invention refers to hair loss in humans. Androgenetic alopecia ([MIM 109200]) occurs both in men and women. In men, this condition is commonly known as male pattern baldness (MPB), as hair is lost in a well-defined pattern, beginning above both temples. In women, onset of androgenetic alopecia tends to occur later and in a milder form (Hanneken et al. (2003), Hautarzt 54:703-712). The pattern of hair loss in women also differs from male-pattern baldness. In women, the hair becomes thinner all over the head, and the hairline does not recede.

Nucleic acid molecules, in accordance with the present invention, have the same bearing as understood in the art. They include DNA, such as cDNA or genomic DNA, and RNA. It is understood that the term "RNA" as used herein comprises all forms of RNA including mRNA, ncRNA (non-coding RNA), tRNA und rRNA. The term "non-coding RNA" includes siRNA (small interfering RNA), miRNA (micro RNA), rasiRNA (repeat associated RNA), snoRNA (small nucleolar RNA), and snRNA (small nuclear RNA). At the same time, other forms of RNA, including the above mentioned specific forms, are deliberately envisaged in the respective embodiments. The term nucleic acid molecule is used interchangeably herein with the terms oligonucleotide or polynucleotide.

In accordance with the present invention, the nucleic acid molecules consist most preferably of at least 17 nucleotides, but may also consist of at least 19, 25, 50, 100, 150, 200 or more nucleotides. The present invention encompasses a large number of nucleic acid molecules, which all contain as a common denominator the respective nucleotide at position 201 of any one of SEQ ID NOs:1 to 292. For example, the 17-mer of the invention may contain as a common denominator the nucleotide at position 201 of any one of SEQ ID NOs:1 to 292, wherein said nucleotide at position 201 may be located within the 17-mer at position 1, 2, 3, 4, 5, 6, 7, 8, 9, 10, 11, 12, 13, 14, 15, 16 or 17 of the 17-mer if viewed from its 5' end.

The term "hybridises/hybridising" as used herein refers to a pairing of a nucleic acid molecule to a (partially) complementary strand of this nucleic acid molecule which thereby form a hybrid.

It is well known in the art how to perform hybridisation experiments with nucleic acid molecules, i.e. the person skilled in the art knows what hybridisation conditions s/he has to use in accordance with the present invention. Such hybridisation conditions are referred to in standard text books such as Molecular Cloning A Laboratory Manual, Cold Spring Harbor Laboratory (1989) N.Y. Preferred in accordance with the present invention are nucleic acid molecules capable of hybridising under stringent hybridisation conditions to the nucleic acid molecules of the invention that are associated with androgenetic alopecia, i.e. that do not cross hybridise to unrelated nucleic acid molecules.

Nucleic acid hybridisation will be affected by such conditions as salt concentration, temperature, or organic solvents, in addition to the base composition, length of the complementary strands and the number of nucleotide base mismatches between the hybridising nucleic acids, as will be readily appreciated by those skilled in the art. Stringent temperature conditions will generally include temperatures in excess of 30°C, typically 37°C, and preferably in excess of 45°C. Stringent salt conditions will ordinarily be less than 1000 mM, typically less than 500 mM and preferably less than 200 mM. However, the combination of parameters is much more important than the measure of any single parameter; see, e.g., Wetmur and Davidson, 1968. Probe sequences may also hybridise specifically to duplex DNA under certain conditions to form triplex or higher order DNA complexes. The preparation of such probes and suitable hybridisation conditions are well known in the art. Nucleic acid molecules capable of hybridising to the nucleic acid molecules of the invention are preferably at least 70%, more preferably at least 75%, at least 80%, at least 85%, at least 90%, at least 95% or 100% identical to the nucleic acid sequences of the invention.

Generally, stringent hybridisation conditions refer to conditions which comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization". Also contemplated are nucleic acid molecules that hybridize to the oligo- or polynucleotides of the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The embodiment recited herein above preferably refers to highly stringent conditions and alternatively to conditions of lower stringency.

In accordance with the present invention, the term "a nucleic acid molecule the complementary strand of which hybridises under stringent conditions to the nucleic acid molecule of ... " has the same bearing as understood in the art. It refers to a nucleic acid molecule being of the same strand as the nucleic acid molecule of the invention and having the above defined sequence identity with the nucleic acid molecule of the invention, thus enabling for its complementary strand to hybridise to the nucleic acid molecule of the invention. The nucleic acid molecule of this embodiment has the prescribed nucleotide in the position 201 of the corresponding nucleic acid molecule of the invention. Thus, whereas no 100% sequence identity over the entire length of said nucleic acid molecule to the nucleic acid molecule of the invention is required for its complementary strand to hybridise to the nucleic acid molecule of the invention, a 100% sequence identity is required in at least the nucleotide corresponding to the nucleotide in position 201 of the respective nucleic acid molecule of the invention.

The term "complementary nucleic acid molecule", as used herein, refers to a nucleic acid molecule the sequence of which is uniquely fitting to (e.g. hybridising to/complementary to preferably 100%) the sequence of the nucleic acid molecule of the invention. That is, the "complementary nucleic acid molecule" will be uniquely fitting to the nucleic acid molecule of the invention having the risk-allele, where said nucleic acid molecule of the invention has an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292, but will not be fitting to the sequences containing the non-risk allele as defined in SEQ ID NOs:1 to 292 at position 201. The risk and non-risk alleles at position 201 of the respective SEQ ID numbers are shown in table 1 below. Alternatively, the "complementary nucleic acid molecule" will be uniquely fitting to the nucleic acid molecule of the invention having the non-risk allele, where said nucleic acid molecule of the invention has an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; but will not be fitting to the sequences containing the risk allele as defined in SEQ ID NOs:1 to 292 at position 201. The risk and non-risk alleles at position 201 of the respective SEQ ID numbers are shown in table 1 below. The above definition also applies to the term "complementary strand".

As complementary nucleic acid molecules are also associated with androgenetic alopecia, they may be directly analysed for the presence of the respective complementary nucleotide at position 201 of the nucleic acid molecule of the invention, which is indicative of the presence or predisposition for androgenetic alopecia. In other words, a thymidine at position 201 of a complementary nucleic acid molecule to any one of SEQ ID NOs:1 to 77, a cytidine at position 201 of a complementary nucleic acid molecule to any one of SEQ ID NOs:78 to 149, an adenosine at position 201 of a complementary nucleic acid molecule to any one of SEQ ID NOs:150 to 219 or a guanosine at position 201 of a complementary nucleic acid molecule to any one of SEQ ID NOs:220 to 292 would be indicative of the presence or predisposition for androgenetic alopecia.

The same applies to complementary nucleic acid molecules of the nucleic acid molecule of the invention being indicative of a reduced risk for androgenetic alopecia or the absence of either androgenetic alopecia or a predisposition for this condition. Thus, a thymidine in a complementary nucleic acid molecule at a position corresponding to position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a guanosine in a complementary nucleic acid molecule at a position corresponding to position 201 of any one of SEQ ID NOs: 3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, an cytidine in a complementary nucleic acid molecule at a position corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or an adenosine in a complementary nucleic acid molecule at a position corresponding to position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 would be indicative for the absence of androgenetic alopecia or a predisposition therefore.

The nucleic acid molecules of the present invention are characterized as being associated with androgenetic alopecia. The sequences of any one of SEQ ID NOs:1 to 292 were uniformly chosen to contain the 200bp upstream and the 200bp downstream of the position of the respective risk alleles or non-risk allele at the genetic locus, hence said alleles are uniformly located at position 201 of any of SEQ ID NOs:1 to 292. The nucleic acid molecules of (a) contain the respective risk alleles associated with the presence or predisposition for androgenetic alopecia, whereas the nucleic acid molecules of (b) contain the respective non-risk alleles associated with the presence or predisposition for androgenetic alopecia.

The term "risk allele" in accordance with the present invention refers to the alleles with a higher frequency in the affected group than in the control group, whereas the term "non-risk allele" refers to the alleles with higher frequency in the non affected control groups. Both the risk alleles as well as the non-risk alleles can be unambiguously identified with information of position and the sequences contained in the SEQ ID NOs:1 to 292 as provided in the present application. Furthermore, table 1 details the individual risk alleles and non-risk alleles in the corresponding SEQ ID NOs as well as their position in the chromosomal region 20p11.

**Table 1: SNPs associated with androgenetic alopecia on chromosome 20p11**

| SEQ ID NO: | Name of SNP | Position of SNP | Position of sequence | Risk allele | Non-risk allele |
|---|---|---|---|---|---|
| 1 | rs2424407 | chr20:21693114 | chr20:21692914-21693314 | A | G |
| 2 | rs6035945 | chr20:21702301 | chr20:21702101-21702501 | A | G |
| 3 | rs6113363 | chr20:21705933 | chr20:21705733-21706133 | A | C |
| 4 | rs2208309 | chr20:21719761 | chr20:21719561-21719961 | A | G |
| 5 | rs12625990 | chr20:21726684 | chr20:21726484-21726884 | A | G |
| 6 | rs6035955 | chr20:21728253 | chr20:21728053-21728453 | A | T |
| 7 | rs6047633 | chr20:21729363 | chr20:21729163-21729563 | A | C |
| 8 | rs6082514 | chr20:21730156 | chr20:21729956-21730356 | A | G |
| 9 | rs6047636 | chr20:21732876 | chr20:21732676-21733076 | A | G |
| 10 | rs6047645 | chr20:21748445 | chr20:21748245-21748645 | A | G |
| 11 | rs6035959 | chr20:21749469 | chr20:21749269-21749669 | A | G |
| 12 | rs6082519 | chr20:21749501 | chr20:21749301-21749701 | A | T |
| 13 | rs2224367 | chr20:21749974 | chr20:21749774-21750174 | A | G |
| 14 | rs4281974 | chr20:21751581 | chr20:21751381-21751781 | A | G |
| 15 | rs6047649 | chr20:21755292 | chr20:21755092-21755492 | A | T |
| 16 | rs1535199 | chr20:21766308 | chr20:21766108-21766508 | A | G |
| 17 | rs4405823 | chr20:21772164 | chr20:21771964-21772364 | A | T |
| 18 | rs12479719 | chr20:21798786 | chr20:21798586-21798986 | A | G |
| 19 | rs997078 | chr20:21798850 | chr20:21798650-21799050 | A | T |
| 20 | rs6047684 | chr20:21801764 | chr20:21801564-21801964 | A | G |
| 21 | rs6075854 | chr20:21814377 | chr20:21814177-21814577 | A | G |
| 22 | rs4815081 | chr20:21825020 | chr20:21824820-21825220 | A | G |
| 23 | rs6137473 | chr20:21832693 | chr20:21832493-21832893 | A | G |
| 24 | rs6113418 | chr20:21837318 | chr20:21837118-21837518 | A | T |
| 25 | rs1884593 | chr20:21838817 | chr20:21838617-21839017 | A | C |
| 26 | rs6082558 | chr20:21839163 | chr20:21838963-21839363 | A | G |
| 27 | rs6047731 | chr20:21847141 | chr20:21846941-21847341 | A | G |
| 28 | rs6082560 | chr20:21850100 | chr20:21849900-21850300 | A | G |
| 29 | rs6515191 | chr20:21853732 | chr20:21853532-21853932 | A | T |
| 30 | rs6113424 | chr20:21854780 | chr20:21854580-21854980 | A | G |
| 31 | rs6113425 | chr20:21855562 | chr20:21855362-21855762 | A | G |
| 32 | rs2328682 | chr20:21866288 | chr20:21866088-21866488 | A | G |
| 33 | rs2208050 | chr20:21866427 | chr20:21866227-21866627 | A | G |
| 34 | rs6047745 | chr20:21867054 | chr20:21866854-21867254 | A | C |
| 35 | rs2424410 | chr20:21877057 | chr20:21876857-21877257 | A | C |
| 36 | rs6035995 | chr20:21879683 | chr20:21879483-21879883 | A | G |
| 37 | rs108275 | chr20:21884485 | chr20:21884285-21884685 | A | G |
| 38 | rs6047761 | chr20:21886282 | chr20:21886082-21886482 | A | C |
| 39 | rs4815086 | chr20:21886530 | chr20:21886330-21886730 | A | C |
| 40 | rs969921 | chr20:21895184 | chr20:21894984-21895384 | A | T |
| 41 | rs2207878 | chr20:21899847 | chr20:21899647-21900047 | A | G |
| 42 | rs6047768 | chr20:21901649 | chr20:21901449-21901849 | A | G |
| 43 | rs1555264 | chr20:21909410 | chr20:21909210-21909610 | A | G |
| 44 | rs2424427 | chr20:21931464 | chr20:21931264-21931664 | A | G |
| 45 | rs1980551 | chr20:21933781 | chr20:21933581-21933981 | A | G |
| 46 | rs201587 | chr20:21975674 | chr20:21975474-21975874 | A | G |
| 47 | rs201593 | chr20:21981819 | chr20:21981619-21982019 | A | G |
| 48 | rs913063 | chr20:21990418 | chr20:21990218-21990618 | A | C |
| 49 | rs1160312 | chr20:21998503 | chr20:21998303-21998703 | A | G |
| 50 | rs1887056 | chr20:21999811 | chr20:21999611-22000011 | A | C |
| 51 | rs6113491 | chr20:22005415 | chr20:22005215-22005615 | A | C |
| 52 | rs6113495 | chr20:22009487 | chr20:22009287-22009687 | A | G |
| 53 | rs11698943 | chr20:22013466 | chr20:22013266-22013666 | A | G |
| 54 | rs6036037 | chr20:22018525 | chr20:22018325-22018725 | A | C |
| 55 | rs6113508 | chr20:22024189 | chr20:22023989-22024389 | A | G |
| 56 | rs6036041 | chr20:22030448 | chr20:22030248-22030648 | A | G |
| 57 | rs201146 | chr20:22036650 | chr20:22036450-22036850 | A | C |
| 58 | rs201149 | chr20:22038387 | chr20:22038187-22038587 | A | T |
| 59 | rs201150 | chr20:22038581 | chr20:22038381-22038781 | A | T |
| 60 | rs6137547 | chr20:22057895 | chr20:22057695-22058095 | A | G |
| 61 | rs804531 | chr20:22076554 | chr20:22076354-22076754 | A | G |
| 62 | rs6113543 | chr20:22077323 | chr20:22077123-22077523 | A | G |
| 63 | rs804533 | chr20:22077932 | chr20:22077732-22078132 | A | G |
| 64 | rs6137561 | chr20:22092267 | chr20:22092067-22092467 | A | G |
| 65 | rs721353 | chr20:22096401 | chr20:22096201-22096601 | A | G |
| 66 | rs804571 | chr20:22100012 | chr20:22099812-22100212 | A | G |
| 67 | rs804580 | chr20:22108981 | chr20:22108781-22109181 | A | C |
| 68 | rs6137580 | chr20:22135693 | chr20:22135493-22135893 | A | G |
| 69 | rs804600 | chr20:22137917 | chr20:22137717-22138117 | A | G |
| 70 | rs6132492 | chr20:22141192 | chr20:22140992-22141392 | A | G |
| 71 | rs6137582 | chr20:22145211 | chr20:22145011-22145411 | A | G |
| 72 | rs804618 | chr20:22152465 | chr20:22152265-22152665 | A | G |
| 73 | rs1090293 | chr20:22155156 | chr20:22154956-22155356 | A | G |
| 74 | rs6106481 | chr20:22171640 | chr20:22171440-22171840 | A | G |
| 75 | rs6113592 | chr20:22177505 | chr20:22177305-22177705 | A | G |
| 76 | rs6047978 | chr20:22187448 | chr20:22187248-22187648 | A | C |
| 77 | rs804688 | chr20:22190336 | chr20:22190136-22190536 | A | G |
| 78 | rs6137432 | chr20:21701799 | chr20:21701599-21701999 | G | A |
| 79 | rs2328672 | chr20:21718398 | chr20:21718198-21718598 | G | A |
| 80 | rs1476396 | chr20:21718657 | chr20:21718457-21718857 | G | C |
| 81 | rs2208310 | chr20:21719845 | chr20:21719645-21720045 | G | T |
| 82 | rs2208311 | chr20:21721249 | chr20:21721049-21721449 | G | T |
| 83 | rs1476398 | chr20:21723783 | chr20:21723583-21723983 | G | A |
| 84 | rs2208313 | chr20:21724826 | chr20:21724626-21725026 | G | T |
| 85 | rs11698973 | chr20:21724952 | chr20:21724752-21725152 | G | T |
| 86 | rs2876622 | chr20:21725915 | chr20:21725715-21726115 | G | A |
| 87 | rs6035954 | chr20:21726074 | chr20:21725874-21726274 | G | A |
| 88 | rs6047635 | chr20:21729625 | chr20:21729425-21729825 | G | T |
| 89 | rs1540930 | chr20:21736175 | chr20:21735975-21736375 | G | C |
| 90 | rs6137445 | chr20:21737803 | chr20:21737603-21738003 | G | A |
| 91 | rs6137446 | chr20:21738861 | chr20:21738661-21739061 | G | A |
| 92 | rs6047641 | chr20:21739854 | chr20:21739654-21740054 | G | T |
| 93 | rs1540932 | chr20:21745520 | chr20:21745320-21745720 | G | A |
| 94 | rs2328640 | chr20:21751728 | chr20:21751528-21751928 | G | A |
| 95 | rs17738464 | chr20:21753589 | chr20:21753389-21753789 | G | C |
| 96 | rs2224260 | chr20:21756259 | chr20:21756059-21756459 | G | A |
| 97 | rs6113394 | chr20:21769149 | chr20:21768949-21769349 | G | T |
| 98 | rs6113395 | chr20:21769262 | chr20:21769062-21769462 | G | A |
| 99 | rs8122229 | chr20:21772282 | chr20:21772082-21772482 | G | A |
| 100 | rs2328647 | chr20:21778507 | chr20:21778307-21778707 | G | A |
| 101 | rs2064773 | chr20:21781737 | chr20:21781537-21781937 | G | A |
| 102 | rs1014883 | chr20:21792630 | chr20:21792430-21792830 | G | A |
| 103 | rs4815080 | chr20:21811397 | chr20:21811197-21811597 | G | A |
| 104 | rs6075852 | chr20:21814151 | chr20:21813951-21814351 | G | A |
| 105 | rs1998076 | chr20:21828045 | chr20:21827845-21828245 | G | A |
| 106 | rs6137476 | chr20:21845656 | chr20:21845456-21845856 | G | A |
| 107 | rs2208054 | chr20:21847708 | chr20:21847508-21847908 | G | A |
| 108 | rs2208055 | chr20:21847864 | chr20:21847664-21848064 | G | A |
| 109 | rs4390829 | chr20:21848117 | chr20:21847917-21848317 | G | A |
| 110 | rs2281526 | chr20:21856332 | chr20:21856132-21856532 | G | C |
| 111 | rs2024885 | chr20:21865393 | chr20:21865193-21865593 | G | A |
| 112 | rs2208051 | chr20:21866471 | chr20:21866271-21866671 | G | A |
| 113 | rs2328684 | chr20:21866642 | chr20:21866442-21866842 | G | T |
| 114 | rs6113435 | chr20:21868688 | chr20:21868488-21868888 | G | C |
| 115 | rs4815087 | chr20:21886603 | chr20:21886403-21886803 | G | C |
| 116 | rs201543 | chr20:21894928 | chr20:21894728-21895128 | G | A |
| 117 | rs6106434 | chr20:21897593 | chr20:21897393-21897793 | G | A |
| 118 | rs6047769 | chr20:21906049 | chr20:21905849-21906249 | G | A |
| 119 | rs6036003 | chr20:21909964 | chr20:21909764-21910164 | G | A |
| 120 | rs2424425 | chr20:21927109 | chr20:21926909-21927309 | G | A |
| 121 | rs6047798 | chr20:21933498 | chr20:21933298-21933698 | G | A |
| 122 | rs127747 | chr20:21936830 | chr20:21936630-21937030 | G | A |
| 123 | rs201564 | chr20:21955602 | chr20:21955402-21955802 | G | A |
| 124 | rs4815090 | chr20:21983416 | chr20:21983216-21983616 | G | T |
| 125 | rs6113483 | chr20:21984524 | chr20:21984324-21984724 | G | C |
| 128 | rs4813445 | chr20:21995177 | chr20:21994977-21995377 | G | A |
| 127 | rs6113496 | chr20:22009632 | chr20:22009432-22009832 | G | T |
| 128 | rs6113501 | chr20:22012973 | chr20:22012773-22013173 | G | A |
| 129 | rs6113509 | chr20:22025352 | chr20:22025152-22025552 | G | A |
| 130 | rs1883751 | chr20:22028540 | chr20:22028340-22028740 | G | A |
| 131 | rs201145 | chr20:22035670 | chr20:22035470-22035870 | G | A |
| 132 | rs6047917 | chr20:22052421 | chr20:22052221-22052621 | G | A |
| 133 | rs11698068 | chr20:22054230 | chr20:22054030-22054430 | G | A |
| 134 | rs804516 | chr20:22063729 | chr20:22063529-22063929 | G | A |
| 135 | rs804520 | chr20:22067141 | chr20:22066941-22067341 | G | A |
| 138 | rs973106 | chr20:22067832 | chr20:22067632-22068032 | G | A |
| 137 | rs6113539 | chr20:22071129 | chr20:22070929-22071329 | G | A |
| 138 | rs6047929 | chr20:22084786 | chr20:22084586-22084986 | G | A |
| 139 | rs804573 | chr20:22100230 | chr20:22100030-22100430 | G | A |
| 140 | rs6137568 | chr20:22111056 | chr20:22110856-22111256 | G | A |
| 141 | rs804596 | chr20:22134593 | chr20:22134393-22134793 | G | A |
| 142 | rs804598 | chr20:22136311 | chr20:22136111-22136511 | G | A |
| 143 | rs12329414 | chr20:22139825 | chr20:22139625-22140025 | G | T |
| 144 | rs961157 | chr20:22141523 | chr20:22141323-22141723 | G | A |
| 145 | rs804610 | chr20:22142770 | chr20:22142570-22142970 | G | A |
| 146 | rs804614 | chr20:22148414 | chr20:22148214-22148614 | G | A |
| 147 | rs709007 | chr20:22151787 | chr20:22151587-22151987 | G | A |
| 148 | rs4815098 | chr20:22157538 | chr20:22157338-22157738 | G | A |
| 149 | rs1337907 | chr20:22162901 | chr20:22162701-22163101 | G | A |
| 150 | rs6137433 | chr20:21703750 | chr20:21703550-21703950 | T | C |
| 151 | rs6035946 | chr20:21706674 | chr20:21706474-21706874 | T | A |
| 152 | rs975165 | chr20:21712736 | chr20:21712536-21712936 | T | C |
| 153 | rs2208308 | chr20:21718908 | chr20:21718708-21719108 | T | G |
| 154 | rs6047632 | chr20:21728197 | chr20:21727997-21728397 | T | C |
| 155 | rs11906825 | chr20:21732105 | chr20:21731905-21732305 | T | A |
| 156 | rs6047637 | chr20:21733073 | chr20:21732873-21733273 | T | C |
| 157 | rs6137444 | chr20:21733639 | chr20:21733439-21733839 | T | C |
| 158 | rs6075840 | chr20:21736459 | chr20:21736259-21736659 | T | A |
| 159 | rs6137448 | chr20:21739008 | chr20:21738808-21739208 | T | A |
| 160 | rs6047643 | chr20:21741400 | chr20:21741200-21741600 | T | A |
| 161 | rs2180700 | chr20:21744855 | chr20:21744655-21745055 | T | G |
| 162 | rs2328673 | chr20:21747692 | chr20:21747492-21747892 | T | A |
| 163 | rs6047646 | chr20:21748510 | chr20:21748310-21748710 | T | G |
| 164 | rs6035957 | chr20:21748585 | chr20:21748385-21748785 | T | C |
| 165 | rs16983003 | chr20:21751314 | chr20:21751114-21751514 | T | A |
| 166 | rs17665823 | chr20:21752341 | chr20:21752141-21752541 | T | C |
| 167 | rs12624315 | chr20:21753128 | chr20:21752928-21753328 | T | G |
| 168 | rs6082520 | chr20:21760118 | chr20:21759918-21760318 | T | C |
| 169 | rs6113393 | chr20:21763192 | chr20:21762992-21763392 | T | C |
| 170 | rs6106421 | chr20:21769217 | chr20:21769017-21769417 | T | C |
| 171 | rs6047663 | chr20:21769910 | chr20:21769710-21770110 | T | C |
| 172 | rs6047666 | chr20:21774762 | chr20:21774562-21774962 | T | C |
| 173 | rs6047676 | chr20:21790889 | chr20:21790689-21791089 | T | C |
| 174 | rs1014885 | chr20:21792827 | chr20:21792627-21793027 | T | C |
| 175 | rs2180439 | chr20:21801100 | chr20:21800900-21801300 | T | C |
| 176 | rs6047685 | chr20:21803904 | chr20:21803704-21804104 | T | A |
| 177 | rs6035971 | chr20:21804148 | chr20:21803948-21804348 | T | A |
| 178 | rs6035979 | chr20:21823172 | chr20:21822972-21823372 | T | G |
| 179 | rs4544515 | chr20:21833311 | chr20:21833111-21833511 | T | C |
| 180 | rs1884588 | chr20:21833500 | chr20:21833300-21833700 | T | C |
| 181 | rs6113416 | chr20:21834362 | chr20:21834162-21834562 | T | C |
| 182 | rs6047715 | chr20:21835266 | chr20:21835066-21835466 | T | A |
| 183 | rs1884592 | chr20:21838690 | chr20:21838490-21838890 | T | C |
| 184 | rs6113431 | chr20:21864356 | chr20:21864156-21864556 | T | A |
| 185 | rs2328680 | chr20:21866106 | chr20:21865906-21866306 | T | C |
| 186 | rs1007169 | chr20:21866310 | chr20:21866110-21866510 | T | G |
| 187 | rs6047744 | chr20:21866898 | chr20:21866698-21867098 | T | G |
| 188 | rs2009652 | chr20:21920581 | chr20:21920381-21920781 | T | C |
| 189 | rs6036007 | chr20:21922590 | chr20:21922390-21922790 | T | C |
| 190 | rs201548 | chr20:21922835 | chr20:21922635-21923035 | T | C |
| 191 | rs6106438 | chr20:21923026 | chr20:21922826-21923226 | T | C |
| 192 | rs6113456 | chr20:21925094 | chr20:21924894-21925294 | T | G |
| 193 | rs2424426 | chr20:21928725 | chr20:21928525-21928925 | T | C |
| 194 | rs201562 | chr20:21948174 | chr20:21947974-21948374 | T | G |
| 195 | rs201563 | chr20:21948281 | chr20:21948081-21948481 | T | C |
| 196 | rs201565 | chr20:21957335 | chr20:21957135-21957535 | T | C |
| 197 | rs201569 | chr20:21960862 | chr20:21960662-21961062 | T | C |
| 198 | rs201571 | chr20:21961514 | chr20:21961314-21961714 | T | C |
| 199 | rs201572 | chr20:21962754 | chr20:21962554-21962954 | T | C |
| 200 | rs201594 | chr20:21982671 | chr20:21982471-21982871 | T | C |
| 201 | rs6047844 | chr20:21985575 | chr20:21985375-21985775 | T | C |
| 202 | rs6036026 | chr20:21992205 | chr20:21992005-21992405 | T | C |
| 203 | rs4813446 | chr20:21997759 | chr20:21997559-21997959 | T | C |
| 204 | rs6113524 | chr20:22042192 | chr20:22041992-22042392 | T | A |
| 205 | rs1303872 | chr20:22059034 | chr20:22058834-22059234 | T | C |
| 206 | rs6113548 | chr20:22084839 | chr20:22084639-22085039 | T | C |
| 207 | rs6113551 | chr20:22087934 | chr20:22087734-22088134 | T | A |
| 208 | rs6137567 | chr20:22110856 | chr20:22110656-22111056 | T | C |
| 209 | rs767457 | chr20:22133332 | chr20:22133132-22133532 | T | C |
| 210 | rs804609 | chr20:22142475 | chr20:22142275-22142675 | T | C |
| 211 | rs6113583 | chr20:22143065 | chr20:22142865-22143265 | T | C |
| 212 | rs2876633 | chr20:22145686 | chr20:22145486-22145886 | T | A |
| 213 | rs804616 | chr20:22149839 | chr20:22149639-22150039 | T | C |
| 214 | rs804617 | chr20:22151480 | chr20:22151280-22151680 | T | G |
| 215 | rs1090292 | chr20:22154969 | chr20:22154769-22155169 | T | C |
| 216 | rs1415801 | chr20:22159414 | chr20:22159214-22159614 | T | G |
| 217 | rs1337908 | chr20:22189466 | chr20:22189266-22189666 | T | A |
| 218 | rs10485630 | chr20:22193867 | chr20:22193667-22194067 | T | G |
| 219 | rs6047982 | chr20:22194450 | chr20:22194250-22194650 | T | G |
| 220 | rs6047613 | chr20:21694305 | chr20:21694105-21694505 | C | G |
| 221 | rs6082508 | chr20:21717042 | chr20:21716842-21717242 | C | A |
| 222 | rs2224366 | chr20:21719909 | chr20:21719709-21720109 | C | G |
| 223 | rs6047628 | chr20:21722338 | chr20:21722138-21722538 | C | G |
| 224 | rs6047630 | chr20:21722842 | chr20:21722642-21723042 | C | G |
| 225 | rs2208312 | chr20:21724735 | chr20:21724535-21724935 | C | T |
| 226 | rs2208314 | chr20:21724875 | chr20:21724675-21725075 | C | A |
| 227 | rs6082512 | chr20:21728905 | chr20:21728705-21729105 | C | T |
| 228 | rs6082513 | chr20:21728972 | chr20:21728772-21729172 | C | A |
| 229 | rs6113378 | chr20:21729032 | chr20:21728832-21729232 | C | T |
| 230 | rs873137 | chr20:21732131 | chr20:21731931-21732331 | C | A |
| 231 | rs6113381 | chr20:21732950 | chr20:21732750-21733150 | C | T |
| 232 | rs6113382 | chr20:21735568 | chr20:21735368-21735768 | C | A |
| 233 | rs4815066 | chr20:21738075 | chr20:21737875-21738275 | C | A |
| 234 | rs16982990 | chr20:21738982 | chr20:21738782-21739182 | C | T |
| 235 | rs1540931 | chr20:21745349 | chr20:21745149-21745549 | C | A |
| 236 | rs2328642 | chr20:21754161 | chr20:21753961-21754361 | C | G |
| 237 | rs11696327 | chr20:21757409 | chr20:21757209-21757609 | C | T |
| 238 | rs2224261 | chr20:21762035 | chr20:21761835-21762235 | C | T |
| 239 | rs6047657 | chr20:21762130 | chr20:21761930-21762330 | C | A |
| 240 | rs1014884 | chr20:21792694 | chr20:21792494-21792894 | C | T |
| 241 | rs6047677 | chr20:21795479 | chr20:21795279-21795679 | C | G |
| 242 | rs987050 | chr20:21800049 | chr20:21799849-21800249 | C | A |
| 243 | rs6047683 | chr20:21801193 | chr20:21800993-21801393 | C | A |
| 244 | rs1012184 | chr20:21804914 | chr20:21804714-21805114 | C | T |
| 245 | rs6047688 | chr20:21807207 | chr20:21807007-21807407 | C | G |
| 246 | rs4815077 | chr20:21807643 | chr20:21807443-21807843 | C | T |
| 247 | rs6113404 | chr20:21811825 | chr20:21811625-21812025 | C | T |
| 248 | rs6075855 | chr20:21814547 | chr20:21814347-21814747 | C | T |
| 249 | rs6035978 | chr20:21823139 | chr20:21822939-21823339 | C | T |
| 250 | rs6047705 | chr20:21823655 | chr20:21823455-21823855 | C | T |
| 251 | rs1884589 | chr20:21833619 | chr20:21833419-21833819 | C | A |
| 252 | rs6047732 | chr20:21847248 | chr20:21847048-21847448 | C | G |
| 253 | rs3818179 | chr20:21849123 | chr20:21848923-21849323 | C | A |
| 254 | rs3818182 | chr20:21849398 | chr20:21849198-21849598 | C | A |
| 255 | rs927059 | chr20:21862194 | chr20:21861994-21862394 | C | T |
| 256 | rs2328681 | chr20:21866213 | chr20:21866013-21866413 | C | T |
| 257 | rs2328683 | chr20:21866447 | chr20:21866247-2186647 | C | T |
| 258 | rs4813442 | chr20:21887656 | chr20:21887456-21887856 | C | T |
| 259 | rs970616 | chr20:21900054 | chr20:21899854-21900254 | C | A |
| 260 | rs1555265 | chr20:21909472 | chr20:21909272-21909672 | C | T |
| 261 | rs169311 | chr20:21910333 | chr20:21910133-21910533 | C | A |
| 262 | rs6047790 | chr20:21926495 | chr20:21926295-21926695 | C | T |
| 263 | rs1980552 | chr20:21934005 | chr20:21933805-21934205 | C | T |
| 264 | rs6047799 | chr20:21934312 | chr20:21934112-21934512 | C | A |
| 265 | rs201559 | chr20:21945115 | chr20:21944915-21945315 | C | T |
| 266 | rs201561 | chr20:21947213 | chr20:21947013-21947413 | C | G |
| 267 | rs201574 | chr20:21963564 | chr20:21963364-21963764 | C | T |
| 268 | rs6137526 | chr20:21986726 | chr20:21986526-21986926 | C | T |
| 269 | rs6132477 | chr20:21988422 | chr20:21988222-21988622 | C | T |
| 270 | rs6137527 | chr20:21991921 | chr20:21991721-21992121 | C | T |
| 271 | rs6036029 | chr20:21997177 | chr20:21996977-21997377 | C | T |
| 272 | rs6137533 | chr20:22018663 | chr20:22018463-22018863 | C | T |
| 273 | rs4815092 | chr20:22029333 | chr20:22029133-22029533 | C | G |
| 274 | rs6113534 | chr20:22065778 | ch20:22065578-22065978 | C | G |
| 275 | rs708996 | chr20:22070158 | chr20:22069958-22070358 | C | G |
| 276 | rs804524 | chr20:22070810 | chr20:22070610-22071010 | C | T |
| 277 | rs1415798 | chr20:22078529 | chr20:22078329-22078729 | C | G |
| 278 | rs1415799 | chr20:22079194 | chr20:22078994-22079394 | C | T |
| 279 | rs2328701 | chr20:22084001 | chr20:22083801-22084201 | C | G |
| 280 | rs804553 | chr20:22092775 | chr20:22092575-22092975 | C | T |
| 281 | rs6106474 | chr20:22136798 | chr20:22136598-22136998 | C | T |
| 282 | rs804605 | chr20:22140057 | chr20:22139857-22140257 | C | T |
| 283 | rs804606 | chr20:22140446 | chr20:22140246-22140646 | C | T |
| 284 | rs6082642 | chr20:22144420 | chr20:22144220-22144620 | C | G |
| 285 | rs742655 | chr20:22147503 | chr20:22147303-22147703 | C | T |
| 286 | rs804615 | chr20:22149175 | chr20:22148975-22149375 | C | T |
| 287 | rs709006 | chr20:22151740 | chr20:22151540-22151940 | C | T |
| 288 | rs12625314 | chr20:22160946 | chr20:22160746-22161146 | C | T |
| 289 | rs12625315 | chr20:22160962 | chr20:22160762-22161162 | C | T |
| 290 | rs1337906 | chr20:22162821 | chr20:22162621-22163021 | C | T |
| 291 | rs804684 | chr20:22182736 | chr20:22182536-22182936 | C | T |
| 292 | rs6113604 | chr20:22200275 | chr20:22200075-22200475 | C | T |

The term "genetic marker" in accordance with the present invention refers to a DNA sequence at a genomic locus that varies due to mutation and that is associated with a disease. A genetic marker may be a short DNA sequence, such as a sequence surrounding a single base-pair change (single nucleotide polymorphism), or a long one, like for example microsatellites.

The term single nucleotide polymorphism (SNP) in accordance with the present invention refers to a DNA sequence variation occurring when a single nucleotide - A, T, C, or G - in the genome differs between paired chromosomes in an individual or between members of a species. Almost all common SNPs have only two alleles. For a variation to be considered a SNP, it must occur in at least 1% of the population. Within a population, SNPs can be assigned a minor allele frequency, which is the lowest allele frequency at a locus that is observed in a particular population. Single nucleotide polymorphisms may fall within coding sequences of genes, non-coding regions of genes, or in the intergenic regions between genes. SNPs within a coding sequence will not necessarily change the amino acid sequence of the protein that is produced, due to degeneracy of the genetic code. A SNP in which both forms lead to the same polypeptide sequence is termed *synonymous* or a silent mutation. If a different polypeptide sequence is produced they are *non-synonymous.* SNPs that are not in protein-coding regions nonetheless can affect gene splicing, transcription factor binding, or the sequence of non-coding RNA.

The term "variable number of tandem repeats (VNTR)" refers to short nucleotide sequences generally ranging from 14 to 100 nucleotides organised into clusters of tandem repeats that are usually repeated in the range of between 4 and 40 times per occurrence. Clusters of such repeats are scattered on chromosomes and each variant constitutes an allele.

The term "microsatellites" or "short tandem repeats" (STR) relates to polymorphic loci present in nuclear and organellar DNA consisting typically of repeating units of 1 to 10 base pairs in length. The repeated sequences are directly adjacent to each other and in the same orientation. Microsatellites and STRs are characterised by a high allelic variability due to an increased rate of mutation compared to other neutral regions of DNA.

Using a genome-wide association study (GWAS) in 296 individuals with androgenetic alopecia (male pattern baldness) and 347 controls, the genetic basis of androgenetic alopecia was investigated in a genome-wide context. The chromosome 20p11 locus was found to have a strong effect on the development of early onset androgenetic alopecia. Analysis of two of the associated SNPs on chromosome 20p11 (rs2180439 and rs6113491) for interaction with the primary associated SNP at the AR locus (rs1041668) did not provide evidence for an interaction with the AR locus (see Example 5). The chromosome 20p11 locus, therefore, shows no evident connection to the androgen pathway. Thus, the present inventors identified a chromosomal region associated with androgenetic alopecia. Within this region, SNPs have been shown to be associated with androgenetic alopecia (P < 0.05). These SNPs are located at position 201 of any one of SEQ ID NOs: 1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, 156, 157, 166, 175, 183, 198, 200, 208, 215, 255, 257, 258, 259, 270, 278, 286, 289, 290, and 292. The human genome has been analyzed in detail by the Human HapMap Project and the correlation between the alleles of two SNPs (at high correlation two SNPs are in linkage disequilibrium) in a genomic region is well known and the information is publicly available at www.hapmap.org. By combining the association data with the publicly available linkage disequilibrium data the inventors defined 240 additionally SNPs which are in linkage disequilibrium with at least one of the 52 associated SNPs. The risk alleles of the 240 SNPs are clearly defined by the risk alleles of the 52 associated SNPs and can therefore be used as a substitute of these. Any of the genetic markers within the chromosomal region can thus be used by the skilled person to determine the presence or predisposition for androgenetic alopecia.

Due to the abundance of established methods for assessing the genotypes of SNPs, it is now possible to conveniently diagnose the presence or a genetic predisposition for androgenetic alopecia in a short amount of time, at low cost, with high accuracy and without significant trouble for the person under investigation. Detection of the non-risk allele may also conveniently be used to demonstrate that a person does not have androgenetic alopecia or a predisposition therefore.

Methods for testing a sample for the presence of the nucleic acid molecule of the invention, for the presence of said genetic marker or for the presence of said risk or non-risk allele include, but are not limited to, nucleic acid amplification and oligonucleotide extension, sequencing or hybridization assays.

Examples for nucleic acid amplification assays and means to perform such include without limitation PCR, (including nested PCR, RT-PCR, PCR extension assays, Nucleic Acid Sequence Base Amplification (NASBA), single-strand confirmation polymorphism (SSCP) PCR), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissard et al., Clin. Chem. 48 (2002) 769-772; Blondal et al., Nucleic Acids Res 31 (2003) e155; Steemers et al., Nature Meth. 3 (2006) 31-33; Kakavas et al., J. Clin. Lab. Anal. 20 (2006) 1-7.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA. Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on lllumina's^{®} technology, assays based on the BeadArray^{®} technology, see, for example, Bames et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

In a preferred embodiment said determining of the presence of said genetic marker, said nucleic acid molecule or the presence of said risk or non-risk allele comprises PCR based techniques, RFLP-or AFLP based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, and 5'-nuclease assay-based techniques. Said techniques are well known to the person skilled in the art.

In a preferred embodiment, the method further comprises detecting an alteration in the expression of a nucleic acid molecule encoding a polypeptide having PAX1 function, wherein said nucleic acid molecule comprises (a) a nucleic acid molecule encoding a polypeptide having the amino acid sequence of SEQ ID NO:293; (b) a nucleic acid molecule having the DNA sequence of SEQ ID NO:294; (c) a nucleic acid molecule having the sequence of SEQ ID NO:294, wherein each thymidine is replaced by uridine; wherein the alteration of expression as compared to the amount of nucleic acid molecule expression in the control sample is indicative of androgenetic alopecia or a predisposition for androgenetic alopecia.

The transcriptional activator PAX1 (paired box 1) is a member of the paired box-containing class of developmental control genes and is located in the chromosomal region 20p11.2. PAX1 is involved in the generation of the vertebral column.

The term "polypeptide" as used herein has the same bearing as understood in the art. It describes molecules having linear molecular chains of amino acids and containing more than 20 amino acids. Polypeptides may further form oligomers consisting of at least two identical or different molecules. The corresponding higher order structures of such multimers are, correspondingly, termed homo- or heterodimers, homo- or heterotrimers etc. Also encompassed by the term "polypeptide" are proteins as well as fragments of proteins, wherein the term "fragment of proteins" refers to a portion of a protein comprising at least the amino acid residues necessary to maintain the biological activity of the protein.

The term "a polypeptide having PAX1 function" in accordance with the present invention refers to a polypeptide having or essentially having PAX1 function, wherein said function can be determined e.g. by its trans-activating capacity in reporter gene assays or by its activation of NKX3-2 (BAPX1), a downstream gene of PAX1.

The expression levels of a nucleic acid molecule encoding a polypeptide having PAX1 function can be measured by any method known in the art that can provide quantitative information regarding the levels to be measured. The methods preferably are highly sensitize and provide reproducible results. In particular, methods based upon the polymerase chain reaction (real-time PCR) and related amplification technologies, such as NASBA and other isothermal amplification technologies, may be used. A suitable approach may be, for example, real-time PCR employing the relative quantification approach using GAPDH or β-actin as housekeeping gene.

Amounts of purified protein in solution can be determined by physical methods, e.g. photometry. Methods of quantifying a particular protein in a mixture rely on specific binding, e.g of antibodies. Specific detection and quantitation methods exploiting the specficity of antibodies comprise immunohistochemistry (in situ) and surface plasmon resonance. Western blotting combines separation of a mixture of proteins by electrophoresis and specific detection with antibodies.

The present inventors found that *PAX1* is located in proximity to the locus identified to be associated with androgenetic alopecia. Furthermore, as shown in Example 6, *PAX1* was found to be expressed at considerable levels in the scalp. These expression data are compatible with a role of this gene in the development of androgenetic alopecia.

In a further preferred embodiment, the sample is blood, serum, plasma, fetal tissue, saliva, urine, mucosal tissue, mucus, vaginal tissue, fetal tissue obtained from the vagina, skin, hair, hair follicle or another human tissue.

Also described herein is a genetic marker for androgenetic alopecia characterised in that the genetic marker is located in a chromosomal region of human chromosome 20p11 and wherein the genetic marker is selected from the group consisting of a single nucleotide polymorphism (SNP), variable number of tandem repeat (VNTR), microsatellites or short tandem repeats (STR).

As described above, any of the genetic markers within the chromosomal region can thus be used for determining the presence of or predisposition for androgenetic alopecia. Thus, described herein is that the genetic marker is for use in the diagnosis of androgenetic alopecia.

In accordance with the present invention the chromosomal region of human chromosome 20p11 consists of the region having the sequence of SEQ ID NO:1 at the 5' end and the sequence of SEQ ID NO:292 at the 3' end.

Thus, the chromosomal region is enclosed between the sequence as defined by SEQ ID NO:1 at the 5' end and the sequence as defined by SEQ ID NO:292 at the 3' end. In other terms, the chromosomal region spans from position chr20:21692914 to position chr20:22200475 (the genomic positions are based on NCBI build 36).

In a more preferred embodiment, the chromosomal region of human chromosome 20p11 comprises at least one of SEQ ID NOs:1 to 292.

In a further preferred embodiment, the genetic marker is a SNP selected from the group consisting of the SNPs comprised in any one of SEQ ID NOs:1 to 292.

SEQ ID NOs:1 to 292 and the respective SNPs have been summarised in table 1 above. Table 1 further details the position of the respective SNPs in the chromosomal region 20p11 and the corresponding alleles.

Further described herein is a nucleic acid molecule selected from the group consisting of (a) a nucleic acid molecule consisting of at least 17 consecutive nucleotides of any of SEQ ID NOs:1 to 292; wherein the at least 17 consecutive nucleotide contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine; (c) a nucleic acid molecule identical to the nucleic acid molecule of (a) or (b) wherein each thymidine is replaced by uridine.

These nucleic acid molecules contain the respective risk alleles associated with the presence or predisposition for androgenetic alopecia. Thus, the described nucleic acid molecule is for use in the diagnosis of androgenetic alopecia.

Further described herein is a nucleic acid molecule selected from the group consisting of (a) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs: SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs: 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine; and (c) a nucleic acid molecule identical to any one of the nucleic acid molecules of (a) or (b) wherein each thymidine is replaced by uridine.

These nucleic acid molecules contain the respective non-risk alleles associated with the absence of androgenetic alopecia or the absence of a predisposition for androgenetic alopecia. Thus, the detection of these nucleic acid molecules is diagnostic of an absence of androgenetic alopecia or the absence of a predisposition for androgenetic alopecia. This described nucleic acid molecule is for use in the diagnosis of androgenetic alopecia.

Performing a genome-wide association analysis the present inventors found that 52 SNPs at the chromosome 20p11 locus showed association with androgenetic alopecia (P < 0.05). These SNPs are located at position 201 of any one of SEQ ID NOs;1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, 156, 157 , 166, 175, 183, 198, 200, 208, 215, 255, 257, 258, 259, 270, 278, 286, 289, 290, and 292. The human genome has been analyzed in detail by the Human HapMap Project and the linkage disequilibrium between two SNPs in a genomic region is well known and the information is publicly available at www.hapmap.org. By combining the association data with the publicly available linkage disequilibrium data the inventors defined 240 additionally SNPs which are in linkage disequilibrium with at least one of the 52 associated SNPs. The risk alleles of the 240 SNPs are clearly defined by the risk alleles of the 52 associated SNPs and can therefore be used as a substitute of these. Any of these 292 genetic markers within the chromosomal region can thus be used by the skilled person to determine the presence or predisposition for androgenetic alopecia.

In a preferred embodiment the genetic marker is a SNP selected from the group consisting of the SNPs defined by any one of SEQ ID NOs:1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, 156, 157, 166, 175, 183, 198, 200, 208, 215, 255, 257, 258, 259, 270, 278, 286, 289, 290 or 292. In another preferred embodiment of the method of the invention, the nucleic acid molecule consists of at least 17 consecutive nucleotides from any of SEQ ID NOs:1, 7, 23, 27, 30, 36, 39, 41, 42, 43, 51, 53, 60, 61, 62, 74, 77, 86, 92, 103, 104, 105, 108, 111, 116, 117, 118, 121, 132, 136, 141, 145, 153, 156, 157, 166, 175, 183, 198, 200, 208, 215, 255, 257, 258, 259, 270, 278, 286, 289, 290 or 292.

In a more preferred embodiment, the genetic marker is a SNP selected from the group consisting of the SNPs defined by any one of SEQ ID NOs: 51, 105, 157, 175 or 198. In another more preferred embodiment of the method of the invention, the nucleic acid molecule consists of at least 17 consecutive nucleotides from any of SEQ ID NOs: 51, 105, 157,175 or 198.

In a preferred embodiment of the method of the invention, the nucleic acid molecule is genomic DNA.

This preferred embodiment of the invention reflects the fact that usually the analysis would be carried out on the basis of genomic DNA from body fluid, cells or tissue isolated from the person under investigation.

Also described herein is a nucleic acid molecule which is complementary to the nucleic acid molecule of the invention.

Complementary nucleic acid molecules are for example useful in the analysis of the genetic setup in the recited positions in hybridisation assays, such as in Southern blot analysis of DNA preparations, but also in PCRs or primer extension protocols respectively. Thus, for example, a 17mer exactly complementary either to a sequence having the non-risk allele or to a sequence having the risk allele associated with androgenetic alopecia may be used to differentiate between the polymorphic variants. This is because a nucleic acid molecule labelled with a detectable label not exactly complementary to the DNA in the analyzed sample will not give rise to a detectable signal, if appropriate hybridization and washing conditions are chosen. Preferably, these conditions are highly stringent conditions as described above.

In this regard, it is important to note that the nucleic acid molecule described herein as well as the complementary nucleic acid molecule may be detectably labelled. Detectable labels include radioactive labels such as ³H, or ³²P or fluorescent labels. Labelling of nucleic acids is well understood in the art and described, for example, in Sambrook and Russel "Molecular Cloning, A Laboratory Manual", Cold Spring Harbor Laboratory, N.Y. (2001).

Furthermore, as complementary nucleic acid molecules are also associated with androgenetic alopecia, they may, as described above, be directly analysed for the presence of the respective complementary nucleotide at position 201 of the nucleic acid molecule of the invention, which is indicative of the presence or predisposition for androgenetic alopecia.

Further described herein is to a vector comprising the nucleic acid molecule as described above.

The term "vector" in accordance with the present invention refers to a vector that is a plasmid, cosmid, virus, bacteriophage or another vector conventionally used e.g. in genetic engineering. Incorporation of the nucleic acid into a vector offers the possibility of introducing the nucleic acid molecule efficiency into the cells and preferably the DNA of a recipient The recipient may be a single cell such as cell from a cell line. Such a measure renders it possible to express, when expression vectors are chosen, the respective nucleic acid molecule in the recipient Thus, incorporation of the nucleic acid molecule into an expression vector opens up the way to a permanently elevated level of the encoded protein in any cell or a subset of selected cells of the recipient Furthermore, chromosomal loci may be modified when using specific targeting vectors comprising a nucleic acid molecule to be replaced for the homologous chromosomal sequence of the host cell. Typical targeting vectors contain a positive selectable marker and the replacement construct, with two regions of homology to the target sequence located on either side. When transfected into cells, a double crossover event will replace the target sequences with the replacement construct sequences.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109).

The polynucleotide referred to above may also be inserted into vectors such that a translational fusion with another polynucleotide is generated. The other polynucleotide may encode a protein which may e.g. increase the solubility and/or facilitate the purifcation of the fusion protein. Non-limiting examples include pET32, pET41, pET43. The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see Sambrook and Russel ("Molecular Cloning, A Laboratory Manual". Cold Spring Harbor Laboratory, N.Y. (2001)). Generally, vectors can contain one or more origin of replication (ori) and inheritance systems for cloning or expression, one or more markers for selection in the host, e. g., antibiotic resistance, and one or more expression cassettes.
The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens Proc. Natl. Acad. Sci. USA 2001, 98: 1471) and optionally poly-A signals ensuring termination of transcription and stabilization of the transcript Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dhfr, gpt, neomycin, hygromycin, ampicillin and kanamycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:169). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture.

Specifically-designed vectors allow the shuttling of DNA between different hosts, such as bacteria- fungal cells or bacteria-animal cells (e.g. the Gateway® system available at Invitrogen).

The nucleic acid molecules as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention.

Also described herein is a host genetically engineered with the nucleic acid molecule or with the vector comprising the nucleic acid molecule as described above.

Preferably, introduction of the described nucleic acid molecule or the described vector comprising the nucleic acid molecule leads to integration of the nucleic acid molecule into the genome of the host. It is particularly envisaged that the described nucleic acid molecules may be introduced into the genome of the host by homologous recombination using techniques well known to the person skilled in the art. By integration of the described nucleic acid molecules into the genome, regulatory effects of the particular SNPs, such as for example transcriptional regulation of neighbouring genes, may be Investigated in suitable hosts.

The host cell may be any prokaryote or eukaryotic cell. Suitable prokaryotes/bacteria are those generally used for cloning like *E*. *coli* (e.g., *E coli* strains BL21(DE3), HB101, DH5α, XL1 Blue, Y1090 and JM101), *Salmonella typhimurium, Serratia marcescens, Pseudomonas putida, Pseudomonas fluorescens, Streptomyces lividans, Lactococcus lactis, Mycobacterium smegmatis* or *Bacillus subtilis.* A suitable eukaryotic host cell may be an animal cell such as a mammalian cell, an amphibian cell, a fish cell, an insect cell such as Drosophila S2 and Spodoptera Sf9 cells, a fungal cell or a plant cell. Mammalian host cells that could be used include, human Hela, HEK293, H9, Bowes melanoma cells and Jurkat cells, mouse NIH3T3 and C127 cells, Cos 1, Cos 7 and CV1, quail QC1-3 cells, mouse L cells, Chinese hamster ovary (CHO) cells or non-human embryonic stem cells. Also within the scope of the present invention are primary mammalian cells such as the immortalised keratinocyte cell line HaCaT, primary keratinocytes, primary hair folllicle cells or mouse embryonic fibroblasts (MEF). Appropriate culture mediums and conditions for the above-described host cells are well known in the art.

A method for the production of a transgenic non-human animal, for example a transgenic mouse, comprises introduction of the aforementioned nucleic acid molecule or targeting vector into a germ cell, an embryonic cell, stem cell or an egg or a cell derived thereof. The non-human animal can be used in accordance with the invention in a method for identifying potential roles of the risk alleles of the nucleic acid molecule of the invention. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., PCR amplification and sequencing or Southern blots with an appropriate probe; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), and the AK-7 line (Zhuang et al., Cell 77:875-884 (1994)). The success of generating a mouse line from ES cells bearing a specific targeted mutation depends on the pluripotence of the ES cells (i.e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are bom, e.g. as chimeric mice. The resultant transgenic mice are backcrossed and screened for the presence of the correctly targeted transgene(s), usually by PCR or Southern blot analysis on tail biopsy DNA of offspring so as to identify transgenic mice.

The transgenic non-human animals may, for example, be transgenic mice, rats, hamsters, dogs, monkeys, rabbits, pigs, or cows. Preferably, said transgenic non-human animals are mice.

Further described herein is a diagnostic composition for the detection of androgenetic alopecia or a predisposition for androgenetic alopecia comprising the nucleic acid molecules, the complementary nucleic acid molecules, the vector or the host cell described herein.

The term "diagnostic composition" relates to compositions for diagnosing a predisposition of individual patients for androgenetic alopecia. The described diagnostic composition comprises at least one of the nucleic acid molecules, the complementary nucleic acid molecules, the vector or the host cell recited above. Said composition may further comprise appropriate buffer(s), and enzymes such as reverse transcriptase, thermostable polymerases etc. The described diagnostic composition may be used to test for the presence of the described nucleic acid molecule using methods well known in the art. Such methods have been described above and are also described, e. g. in Sambrook and Russel "Molecular Cloning, A Laboratory Manual", Cold Spring Harbor Laboratory, N.Y. (2001).

Also described herein is a kit comprising the nucleic acid molecules, the complementary nucleic acid molecules, the vector containing the nucleic acid molecule or the host described herein.

The various components of the kit may be packaged In one or more containers such as one or more vials. The vials may, in addition to the components, comprise preservatives or buffers for storage.

The figures show:

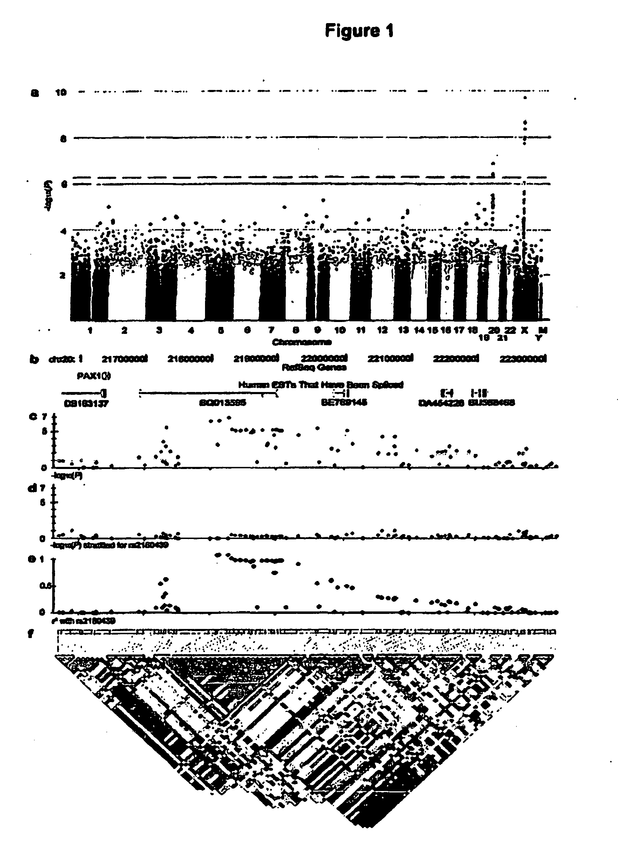

**Figure 1****: Genome-wide scan for association with AGA and details of the region 21,571,171 - 22,321,597 on chromosome 20p11.** (a) Negative log₁₀ of the Cochran-Armitage trend test *P* for the autosomes and of the allele frequency difference test *P* of chromosomes X, Y and the mitochondrion for quality-control-positive SNPs. Chromosomes are shown in alternating colours, with SNPs selected for the replication step highlighted in red. Genome-wide significance level is indicated by dashed red line. (b) Transcript information of the chromosome 20 locus (UCSC genome browser). (c) Negative log₁₀ association *P* are displayed for the GWAS (with rs2180439 indicated in red) and for an analysis stratified for rs2180439 (d). (e) The LD measured by r² between each SNP genotyped in the region and rs2180439 is shown. (f) LD displayed by GOLD heatmap and haplotype blocks indicated by black triangles were analyzed using Haploview software. High D' is indicated in red, low D' is indicated in blue.

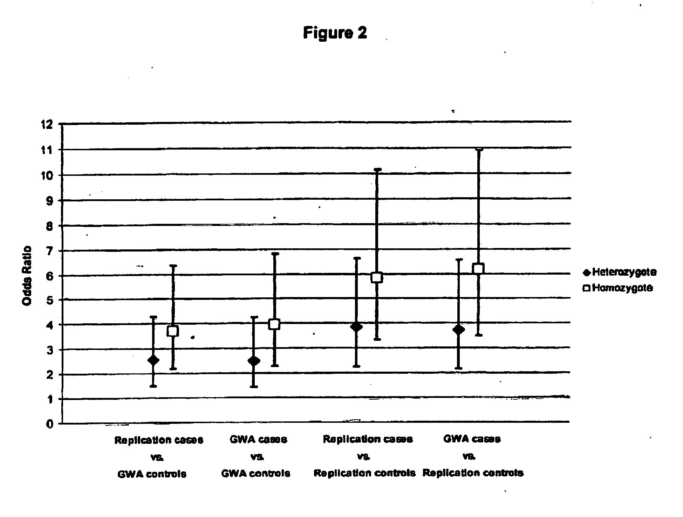

**Figure 2****: Genotype odds ratios of rs2180439 with increasing phenotypic divergence between cases and controls.** All cases had AGA that was representative of the most severely affected 10% of the distribution for the respective age class. Thereof, cases used in the GWAS were the most severely affecteds and cases used in the replication step were less extreme. Controls of the GWAS were population based where males with extreme AGA had been excluded. Controls of the replication step represented the least-affected 20% of the distribution for their age class. ORs for heterozygotes (C/T) are indicated by filled diamond, ORs for homozygotes (T/T) are indicated by open squares, 95% Cls are indicated by bars.

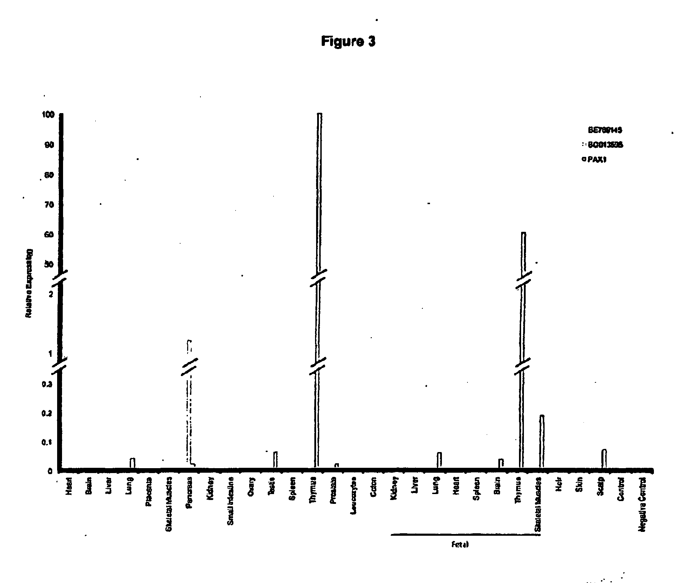

**Figure 3****: Expression of BQ013595,** BE789145**, and *PAX1* in human tissues.** Expression levels were determined by real time PCR, normalized by cyclophilin and displayed relative to the strongest expression (*PAX1* in adult thymus). *PAX1* Expression is detectable in scalp.

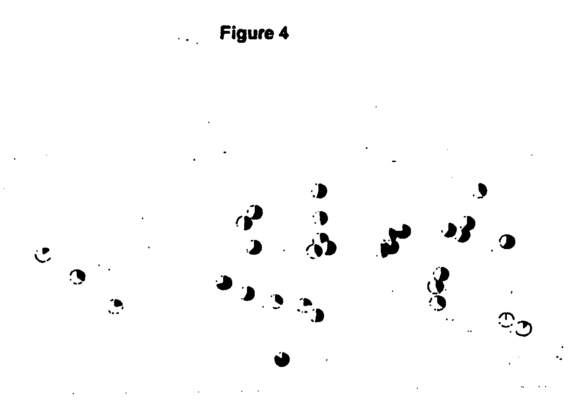

**Figure 4****: Global distribution of the rs2180439 risk allele.** Genotype data of the HGDP-CEPH Diversity Panel was extracted from the NGDP-CEPH Diversity Panel Database Supplement 2 at http://www.cephb.fr/hgdp-cephdb/. Worldwide distribution of the AGA risk allele T is indicated by the proportion of black shading in pie charts. Lower risk allele frequencies were observed in Oceanians and Native Americans

The examples illustrate the invention:

### Example 1: Materials and Methods

### German sample

Families and unrelated AGA and non-AGA individuals were recruited through various sources including press reports, and advertisements in magazines, newspapers and placards. Blood samples were drawn from 754 affected males who were <30 years of age having AGA of grades IV - VII, based on the classification of Hamilton JB (1951), Ann N Y Acad Sci 53:708-728 and modified by Norwood OT (1975), South Med J 68:1359-1365, or <40 years having AGA of grades V - VII. Based on these criteria, the AGA sample was representative of the most severely affected 10% of the distribution for the respective age class (Hamilton JB (1951), Ann N Y Acad Sci 53:708-728; Norwood OT (1975), South Med J 68:1359-1365). Of the affecteds, the 296 unrelated individuals with the strongest hair loss (mean±SD age [in years] 33.3±4.9, Hamilton/Norwood grades: median VI, lower quartile V, upper quartile VII) were selected for GWAS and 319 independent unrelated affecteds (mean±SD age [in years] 33.4±5.1, Hamilton/Norwood grades: median V, lower quartile V, upper quartile VI) were selected for the replication step. 234 non-affected males were >60 years of age and without AGA (mean±SD age [in years] 67.6±5.9), representing the least affected 20% of the distribution for this age class. 491 of the 754 affecteds were part of 352 nuclear families comprising of parents with one to four affected sons. As controls, we investigated 383 individuals (mean±SD age [in years] 61.1±5.3) from the population-based Heinz Nixdorf RECALL cohort (Schmermund A, et al. (2002), Am Heart J 144:212-218) consisting of 182 male and 201 female probands (mean±SD age [in years] 61.1±5.3) who were randomly selected from the general population. Of 129 male controls, hair status information was available: 16 individuals with grade I (Hamilton/Norwood classification), 21 individuals with grade II, one individual with grade IIa, eight individuals with grade III, 21 individuals with grade III vertex, four individuals with grade IIIa, ten individuals with grade IV, one individual with grade IVa, eleven individuals with grade V, 27 individuals with grade VI, and nine individuals with grade VII. Thirty six males with AGA grade VI and VII, respectively, were excluded from GWAS to increase phenotypic stratification between cases and controls. Females were included in the GWAS control sample as they were considered to show the representative AGA risk allele frequencies for population controls. The study was approved by the Ethics Committees of the Universities of Bonn, Düsseldorf and Essen, and informed consent was obtained from all participating individuals. All participants were of German descent

### Australian sample

As part of a population based twin study (Nyholt DR, et al. (2003), J Invest Dermatol 121:1561-1564), hair status was documented in 291 unrelated male individuals for whom DNA was available. Measures of hair loss were obtained in the course of an extensive semi-structured telephone interview with respondent booklet, designed to assess physical, psychological and social manifestations of alcoholism and related disorders, conducted with 6265 twins born 1964-71 from the volunteer based Australian Twin Registry. All males (45% of the sample) were asked to rate their degree of hair loss, if any, using the Hamilton-Norwood Baldness scale (Hamilton JB (1951), Ann N Y Acad Sci 53:708-728; Norwood OT (1975), South Med J 68:1359-1365), which was printed in the respondent booklet. This data collection scheme was validated in a study by Ellis JA, et al. (1998), J Invest Dermatol 110:849-853. Individuals with hair loss Hamilton/Norwood grade ≥ IIa were classified as AGA affecteds and individuals with Hamilton/Norwood grade I were classified as controls. The study was approved by the Human Research Ethics Committee of the Queensland Institute of Medical Research, and informed consent was obtained from all participating individuals.

### DNA extraction

EDTA anticoagulated venous blood samples were collected from all individuals. Lymphocyte DNA from German AGA affecteds, non-affecteds, families, and Australian samples was isolated by salting out with saturated NaCl solution (Miller SA, et al. (1988), Nucleic Acids Res 16:1215) and DNA from German population-based controls was isolated using a Chemagic Magnetic Separation Module I (Chemagen, Baesweiler, Germany) according to the manufacturer recommendations. Genomic DNAs were diluted to working concentrations of 50 ng/µl for genome-wide genotyping and 2.5 ng/µl for the replication step.

### Genotyping

Genome-wide genotyping of 317,503 SNPs in 150 AGA affecteds using the Illumina HumanHap300 BeadChip and of 561,466 SNPs in 146 affecteds and 383 population based controls using the Illumina HumanHap550 BeadChip was conducted according to the Infinium II protocol from Illumina (Illumina, San Diego, USA). A DNA sample was deemed to have failed if it generated genotypes at fewer than 95% of loci of the respective BeadChip. A SNP was deemed to have failed if fewer than 95% of case or control samples generated a genotype at the locus. SNPs were excluded with a minor allele frequency (MAF) < 0.01 in controls and a Hardy-Weinberg-Equilibrium (HWE) *P* < 10⁻⁷ in controls. 531,695 SNPs passed quality criteria and were used for association analysis.
For the replication study, the best SNPs were selected based on the P values of the Cochran-Armitage trend test for the autosomes and the pseudoautosomal region and based on the allele frequency difference test for chromosomes X, Y, and the mitochondrion. To maximize efficiency in the replication step, four tagging SNPs of chromosome 20p11 were selected using the pairwise tagging algorithm of Haploview software (Barrett JC, et al. (2005), Bioinformatics 21:263-265) (parameters: r² ≥ 0.8 and LOD ≥ 3) and CEU HapMap data (Frazer KA, et al. (2007), Nature 449:851-861). The genome-wide best SNP, rs19998076 (Supplementary Table 1), was forced to be included and rs2180439 which was in perfect LD with rs1998076 was added for additional confidence in the most significant SNP. The genome-wide next best 30 SNPs excluding the *AR* locus (chromosome X 65.5 - 67.2 Mb, build 36) were selected for replication analysis. Visual inspection of genotype clusters of selected SNPs revealed for rs4976846 a shifted genotype cluster for a subset of samples and the SNP was excluded from the genome-wide data set. Of the 34 selected SNPs four had to be excluded: rs11655206 and rs4805229 could not be included in the two plexes for MassArray genotyping, rs13079866 was a genotyping failure, and rs1139488 showed fuzzy genotype clusters, significant deviation from HWE, and 19 Mendel errors, respectively, in the family.data. Genotyping for the replication step in the German and Australian samples was performed using the MassArray system on a Sequenorn Compact MALDI-TOF device (Sequenom Inc., San Diego, CA, USA). Primer sequences and PCR/assay conditions can be obtained from the authors upon request. SNP call rates were >95.0% and samples with call rates <95% were excluded from the analysis.

### Statistical analysis

Genome-wide association analysis at single marker level was performed by Cochran-Armitage trend test for the autosomes and the pseudoautosomal region and by the allele frequency difference test for chromosomes X, Y, and the mitochondrion. *P* < 5 x 10⁻⁷ were assumed to be genome-wide significant as suggested by The Wellcome Trust Case Control Consortium (2007), Nature 447:661-678. Haplotype and family based analysis of the chromosome 20 locus was conducted with FAMHAP software (Becker T, et al. (2005), Ann Hum Genet 69:747-756). For case-control haplotype analysis, *P* values were calculated from the _{X}² distribution with n-1 degrees of freedom of the respective likelihood-ratio test (n = number of different haplotypes). The family data were analyzed with the permutation-based association test for nuclear families (Zhao H, et al. (2000), Am J Hum Genet 67:936-946; Knapp M, Becker T (2003), Hum Hered 56:2-9). For each marker (single locus analysis) and each marker combination (haplotype analysis), we used 10¹⁰ permutation replicates. As rs2180439 was the most associated marker in the German combined sample, we carried out further statistical analysis based on that marker in the compound data set. We used logistic regression as suggested by Cordell HJ, Clayton DG (2002), Am J Hum Genet 70:124-141. First, we tested for deviation from a multiplicative model on the OR scale by comparing a 2-degree of freedom (DF) model with parameter β₁ for a multiplicative SNP-effect and parameter β₂ for a dominance effect versus a 1-DF model with just β₁. Secondly, we tested for independent effects of the remaining four SNPs of the region. Therefore, we tested a 3-DF model with parameters β₁, β₂ and β₃ (parameter for multiplicative effect of additional SNP) versus the 2-DF model for rs2180439 (β₁, β₂). If this test was significant, we further tested for additional dominance at the new SNP (parameter β₄) by comparing the 4-DF model to the 3-DF model.
In order to test for interaction with the X chromosome, we considered the initial GWAS case sample (n = 296) versus the male controls (n = 146). For this data set, the model with β₁, β₃ and β₄ had a *P* value of 5.89 x 10⁻⁶. Introduction of the X-chromosomal SNP rs1041668 improved the model-fit significantly (*P* = 1.32 x 10⁻⁸), resulting in a 4-DF model with *P* = 1.17 x 10⁻¹³. On top of this model, neither the interaction terms with rs2180439 (*P* = 0.975) nor with rs6113491 (*P* = 0.984) yielded significant improvement of the model fit.

### Expression analysis

To test for tissue specific expression of BQ013595, BE789145 and *PAX1*, we used the Human Multiple-Tissue cDNA (MTC) Panels I, II and the Human Fetal MTC Panel (Takara Bio Europe/Clontech, Saint-Germain-en-Laye, France). In addition, total RNA from hair follicles, skin and scalp was extracted (RNeasy Micro Kit, Qiagen). Quality and quantity of the RNA was analyzed on a NanoDrop ND-1000 spectrophotometer (Peqlab Biotechnologie, Erlangen, Germany) and reverse transcription was performed using SuperScript First Strand Synthesis System for RT-PCR (Invitrogen, Karlsruhe, Germany) with oligo dT primers. cDNAs were adjusted in an initial real-time PCR experiment using the TaqMan Endogenous Controls Human Cyc (Cyclophilin, 4326316E; Applied Biosystems, Darmstadt, Germany) in which 5 µl of the MTC-cDNAs were set as reference. Relative quantifications in real-time experiments were performed in 384-well plates with the adjusted amounts of cDNAs per well in a total volume of 20 µl on the ABI Prism 7900HT Fast Real-Time PCR System (Applied Biosystems, Darmstadt, Germany) using Custom TaqMan Gene Expression Assays for BQ013595 (forward primer AGACATACTTTTCTATTCACCTTCCAATGATG, reverse primer GGTTCTGACCCTTATTATTGGAACTCA, probe AAGTCCCAACAAACTCCA), BE789145 (forward primer ACCTGACATCAACGTACAAAGATTCA, reverse primer GCGGCTTCTCCTCTGCAT, probe CATGGCCACAAGAGTTA), and *PAX1* (forward primer GGCTAGAGAAACCTGCCTTAGAG, reverse primer CGCCCACGGCAGAGA, probe ACACTCAGTCGGCCTCC). Each sample was normalized by TaqMan Endogenous Controls Human Cyc, and CT values were transformed by 100,000,000/(2^{CT}). Values were calculated relative to the expression of *PAX1* in thymus which was set as 100%.

### Example 2: Genome-wide association study

We conducted a genome-wide association study (GWAS) to identify predisposing genetic factors in AGA. The analysis was performed on 296 males with early-onset AGA (Hamilton/Norwood hair loss grades: median VI, lower quartile V, upper quartile VII) and 383 population based controls (201 females and 182 males), all of German descent, were genotyped with Illumina BeadChips (HumanHap300/HumanHap550). For the analysis, 36 male controls with strong AGA were excluded resulting in 347 controls. Following quality control, we performed association analysis on 531,695 single nucleotide polymorphisms (SNPs) with a minor allele frequency (MAF) of > 1% in controls. The SNP call rate was 99.78%.

We found highly significant association in a 1.7 Mb region flanking the previously implicated *AR* locus (Fig. 1). The best SNP (rs1998076, *P* = 1.3 x 10⁻⁷) located outside the *AR* locus was on chromosome 20, a locus which was supported by 17 additional SNPs with *P* < 10⁻⁵.

**Table 2: Genome-wide association results for AGA (excluding AR locus).**

| SNP | Chr. | Position [bp]^{a} | MAF cases | MAF controls | *P* | OR (95% Cl) |
|---|---|---|---|---|---|---|
| rs1998078 | 20 | 21,828,045 | 0.282 (A) | 0.427 (A) | 1.30 x 10⁻⁷ | 1.90 (1.50-2.41) |
| rs6075852 | 20 | 21,814,151 | 0.292 (A) | 0.429 (A) | 3.50 x 10⁻⁷ | 1.83 (1.45 - 2.31) |
| rs2180439 | 20 | 21,801,100 | 0.292 (C) | 0.429 (C) | 3.85 x 10⁻⁷ | 1.82 (1.45 - 2.30) |
| rs6137444 | 20 | 21,733,639 | 0.264 (C) | 0.383 (C) | 3.11 x 10⁻⁶ | 1.74 (1.37 - 2.21) |
| rs201571 | 20 | 21,961,514 | 0.289 (C) | 0.411 (C) | 4.31 x 10⁻⁶ | 1.72 (1.36 - 2.17) |
| rs10992241 | 9 | 93,931,599 | 0.156 (C) | 0.262 (C) | 5.31 x 10⁻⁶ | 1.92 (1.44 - 2.54) |
| rs6137473 | 20 | 21,832,693 | 0.321 (G) | 0.445 (G) | 5.58 x 10⁻⁶ | 1.70 (1.35 - 2.13) |
| rs6047768 | 20 | 21,901,649 | 0.331 (G) | 0.457 (G) | 6.09 x 10⁻⁶ | 1.70 (1.35 - 2.13) |
| rs2207878 | 20 | 21,899,847 | 0.331(G) | 0.457 (G) | 6.19 x 10⁻⁶ | 1.70 (1.35 - 2.13) |
| rs6035995 | 20 | 21,879,683 | 0.331 (G) | 0.455 (G) | 6.97 x 10⁻⁶ | 1.69 (1.35 - 2.13) |
| rs6113424 | 20 | 21,854,780 | 0.331 (G) | 0.455 (G) | 7.05 x 10⁻⁶ | 1.69 (1.35 - 2.12) |
| rs2024885 | 20 | 21,865,393 | 0.331 (A) | 0.455 (A) | 7.05 x 10⁻⁶ | 1.69 (1.35 - 2.12) |
| rs201543 | 20 | 21,894,928 | 0.331 (A) | 0.455 (A) | 7.05 x 10⁻⁶ | 1.69 (1.35 - 2.12) |
| rs17817969 | 18 | 70,473,226 | 0.223 (T) | 0.117 (T) | 7.16 x 10⁻⁶ | 2.17 (1.51 - 3.11) |
| rs6047769 | 20 | 21,906,049 | 0.333 (A) | 0.457 (A) | 8.47 x 10⁻⁶ | 1.68 (1.34 - 2.12) |
| rs1884592 | 20 | 21,838,690 | 0.331 (C) | 0.454 (C) | 8.58 x 10⁻⁶ | 1.68 (1.34 - 2.11) |
| rs6137478 | 20 | 21,845,856 | 0.331 (A) | 0.454 (A) | 8.58 x 10⁻⁶ | 1.68 (1.34 - 2.11) |
| rs6047731 | 20 | 21,847,141 | 0.331 (G) | 0.454 (G) | 8.58 x 10⁻⁶ | 1.68 (1.34 - 2.11) |
| rs6113491 | 20 | 22,005,415 | 0.359 (C) | 0.483 (C) | 8.63 x 10⁻⁶ | 1.66 (1.33 - 2.08) |
| rs1555264 | 20 | 21,909,410 | 0.332 (G) | 0.455 (G) | 8.64 x 10⁻⁶ | 1.68 (1.34 - 2.11) |
| rs4658627 | 1 | 242,577,799 | 0.172 (A) | 0.308 (A) | 9.60 x 10⁻⁶ | 2.14 (1.51 - 3.02) |
| rs11975187 | 7 | 153,863,909 | 0.490 (A) | 0.359 (G) | 1.02 x 10⁻⁵ | 1.86 (1.41 - 2.46) |
| rs4805229 | 19 | 33,692,043 | 0.380 (T) | 0.268 (T) | 1.34 x 10⁻⁵ | 1.67 (1.32 - 2.12) |
| rs9300398 | 13 | 96,690,316 | 0.363 (T) | 0.252 (T) | 1.46 x 10⁻⁵ | 1.69 (1.33 - 2.15) |
| rs4771987 | 13 | 96,699,391 | 0.429 (G) | 0.315 (G) | 1.88 x 10⁻⁵ | 1.63 (1.30 - 2.05) |
| rs3786343 | 18 | 59,798,875 | 0.458 (T) | 0.340 (T) | 2.27 x 10⁻⁵ | 1.64 (1.31 - 2.05) |
| rs6986303 | 8 | 134,547,711 | 0.309 (A) | 0.205 (A) | 2.33 x 10⁻⁵ | 1.74 (1.35 - 2.24) |
| rs9856948 | 3 | 189,240,095 | 0.419 (C) | 0.304 (C) | 2.51 x 10⁻⁵ | 1.65 (1.31 - 2.08) |
| rs1139488 | 9 | 115,193,721 | 0.308 (C) | 0.418 (C) | 2.77 x 10⁻⁵ | 1.63 (1.29 - 2.05) |
| rs13079866 | 3 | 196,641,541 | 0.061 (G) | 0.131 (G) | 2.84 x 10⁻⁵ | 2.33 (1.56 - 3.49) |
| rs7319347 | 13 | 66,977,210 | 0.072 (C) | 0.173 (C) | 3.00 x 10⁻⁵ | 2.70 (1.66 - 4.38) |
| rs17818946 | 18 | 70,519,734 | 0.170 (G) | 0.082 (G) | 3.06 x 10⁻⁵ | 2.30 (1.52 - 3.47) |
| rs6804475 | 3 | 113,152,321 | 0.246 (A) | 0.352 (A) | 3.22 x 10⁻⁵ | 1.66 (1.30 - 2.12) |
| rs500629 | 11 | 113,550,770 | 0.348 (G) | 0.241 (G) | 3.62 x 10⁻⁵ | 1.68 (1.30 - 2.12) |
| rs10495747 | 2 | 24,368,500 | 0.149 (C) | 0.075 (C) | 3.78 x 10⁻⁵ | 2.16 (1.50 - 3.10) |
| rs1464766 | 5 | 111,641,241 | 0.243 (A) | 0.372 (A) | 4.14 x 10⁻⁵ | 1.84 (1.35 - 2.51) |
| rs2551182 | 2 | 24,293,921 | 0.128 (T) | 0.061 (T) | 4.75 x 10⁻⁵ | 2.29 (1.54 - 3.39) |
| rs300639 | 9 | 32,776,553 | 0.067 (T) | 0.021 (T) | 4.83 x 10⁻⁵ | 3.45 (1.85 - 6.42) |
| rs10001582 | 4 | 124,578,020 | 0.364 (A) | 0.478 (A) | 4.91 x 10⁻⁵ | 1.60 (1.28 - 2.01) |
| rs11584662 | 1 | 184,847,447 | 0.417 (T) | 0.467 (G) | 4.92 x 10⁻⁵ | 1.60 (1.28 - 1.99) |
| rs1009070 | 11 | 29,484,237 | 0.106 (G) | 0.038 (G) | 5.20 x 10⁻⁵ | 3.05 (1.78 - 5.24) |
| rs880013 | 9 | 1,425,496 | 0.474 (T) | 0.415 (C) | 5.28 x 10⁻⁵ | 1.56 (1.25 - 1.95) |
| rs4896028 | 6 | 134,580,872 | 0.309 (G) | 0.209 (G) | 5.35 x 10⁻⁵ | 1.69 (1.32 - 2.18) |
| rs11725633 | 4 | 37,868,359 | 0.368 (T) | 0.483 (T) | 5.57 x 10⁻⁵ | 1.60 (1.28 - 2.00) |
| rs11655206 | 17 | 72,501,171 | 0.497 (T) | 0.361 (T) | 5.60 x 10⁻⁵ | 1.74 (1.32 - 2.30) |
| rs6550859 | 3 | 24,385,419 | 0.390 (G) | 0.468 (A) | 5.72 x 10⁻⁵ | 1.77 (1.34 - 2.34) |
| rs3786939 | 19 | 44,888,022 | 0.301 (A) | 0.184 (A) | 5.79 x 10⁻⁵ | 1.91 (1.39 - 2.61) |

| | | | | | | |
|---|---|---|---|---|---|---|
| (^{a} Build 36) | | | | | | |

Three SNPs at this locus reached genome-wide significance based on criteria suggested elsewhere (Consortium TWTCC (2007), Nature 447:661-678).

### Example 3: Replication study

To replicate the association findings, four tagging SNPs of the chromosome 20 region and the next best 29 SNPs (excluding the *AR* locus) were selected. In addition, rs2180439, which was in perfect LD with rs1998076, was included for the purpose of providing additional confidence in the most significant SNP. Selected markers were analyzed in an independent set of 319 affected and 234 unaffected individuals (males >60 years of age without AGA). The association with all five SNPs of the chromosome 20 locus was replicated (*P* = 8.13 x 10⁻¹⁰ for rs6113491).

**Table 3 Replication analysis in 319 German cases and 234 German controls**

| SNP | Chr. | Position [bp]^{a} | MAF cases | MAF controls | *P* | OR (95% Cl) |
|---|---|---|---|---|---|---|
| rs6113491 | 20 | 22,005,415 | 0.364 (C) | 0.447 (A) | 8.13 x 10⁻¹⁰ | 2.17 (1.7-2.77) |
| rs2180439 | 20 | 21,801,100 | 0.303 (C) | 0.485 (C) | 1.37 x 10⁻⁰⁹ | 2.17 (1.7-2.78) |
| rs1998076 | 20 | 21,828,045 | 0.301 (A) | 0.479 (A) | 3.69 x 10⁻⁰⁹ | 2.13 (1.66-2.73) |
| rs201571 | 20 | 21,961,514 | 0.313 (C) | 0.483 (C) | 2.21 x 10⁻⁰⁸ | 2.05 (1.6 - 2.62) |
| rs6137444 | 20 | 21,733,639 | 0.277 (C) | 0.404 (C) | 1.57 x 10⁻⁰⁵ | 1.76 (1.37 - 2.27) |
| rs10992241 | 9 | 91,971,333 | 0.289 (C) | 0.201 (C) | 8.68 x 10⁻⁰⁴ | 1.62 (1.22 - 2.15) |
| rs11725633 | 4 | 38,014,530 | 0.422 (T) | 0.479 (T) | 0.058 | 1.26 (0.99 - 1.6) |
| rs4771987 | 13 | 96,699,391 | 0.417 (G) | 0.363 (G) | 0.075 | 1.25 (0.98 - 1.6) |
| rs6804475 | 3 | 113,152,321 | 0.292 (A) | 0.340 (A) | 0.081 | 1.25 (0.97 - 1.62) |
| rs3786939 | 19 | 44,888,022 | 0.202 (A) | 0.162 (A) | 0.085 | 1.31 (0.96 - 1.79) |
| rs880013 | 9 | 1,425,496 | 0.431 (C) | 0.479 (C) | 0.123 | 1.21 (0.95 - 1.54) |
| rs500629 | 11 | 113,550,770 | 0.307 (G) | 0.276 (G) | 0.249 | 1.17 (0.9 - 1.52) |
| rs9300398 | 13 | 96,690,316 | 0.335 (T) | 0.303 (T) | 0.252 | 1.16 (0.9 - 1.5) |
| rs1009070 | 11 | 29,484,237 | 0.062 (G) | 0.076 (G) | 0.35 | 1.25 (0.78 - 2) |
| rs4896028 | 6 | 134,580,872 | 0.268 (G) | 0.244 (G) | 0.368 | 1.14 (0.86 - 1.5) |
| rs10495747 | 2 | 24,426,647 | 0.130 (C) | 0.115 (C) | 0.458 | 1.15 (0.8 - 1.65) |
| rs17817969 | 18 | 70,473,226 | 0.147 (T) | 0.132 (T) | 0.483 | 1.13 (0.8 - 1.6) |
| rs6986303 | 8 | 134,547,711 | 0.288 (A) | 0.269 (A) | 0.495 | 1.1 (0.84 - 1.43) |
| rs10001582 | 4 | 124,716,175 | 0.429 (A) | 0.409 (A) | 0.51 | 1.09 (0.85 - 1.38) |
| rs3786343 | 18 | 59,798,875 | 0.395 (T) | 0.376 (T) | 0.527 | 1.08 (0.85 - 1.38) |
| rs1464766 | 5 | 111,641,241 | 0.324 (A) | 0.342 (A) | 0.545 | 1.08 (0.84 - 1.39) |
| rs11975187 | 7 | 153,670,624 | 0.346 (G) | 0.363 (G) | 0.559 | 1.08 (0.84 - 1.38) |
| rs11584662 | 1 | 183,312,481 | 0.436 (T) | 0.453 (T) | 0.573 | 1.07 (0.84 - 1.36) |
| rs17818946 | 18 | 70,519,734 | 0.108 (G) | 0.098 (G) | 0.588 | 1.11 (0.75 - 1.65) |
| rs6550859 | 3 | 24,385,419 | 0.498 (A) | 0.483 (A) | 0.608 | 1.06 (0.84 - 1.35) |
| rs9856948 | 3 | 189,240,103 | 0.350 (C) | 0.340 (C) | 0.735 | 1.04 (0.81 - 1.34) |
| rs2551182 | 2 | 24,352,068 | 0.099 (T) | 0.096 (T) | 0.886 | 1.03 (0.69 - 1.54) |
| rs7319347 | 13 | 66,977,210 | 0.160 (C) | 0.162 (C) | 0.91 | 1.02 (0.74 - 1.41) |
| rs4658627 | 1 | 240,837,217 | 0.270 (A) | 0.274 (A) | 0.911 | 1.02 (0.78 - 1.33) |
| rs300639 | 9 | 32,776,553 | 0.023 (T) | 0.023 (T) | 1 | 1 (0.44 - 227) |

| | | | | | | |
|---|---|---|---|---|---|---|
| (^{a} Build 38) | | | | | | |

In addition, rs10992241 on chromosome 9 showed significance. However, the putative risk allele (C) in the replication study differed from that in the GWAS (T), and thus rs10992241 was not considered to be truly associated. After combining the samples from the genome-wide and replication analyses, rs2180439 showed the highest significance (*P* = 2.67 x 10⁻¹⁵, Table 4). Haplotype analysis did not significantly improve the association findings.

**Table 4 Association between the chromosome 20 locus and AGA in the German sample**

| | | | | MAF | | Genotypes | | | |
|---|---|---|---|---|---|---|---|---|---|
| SNP (position)^{a} | Sample | Cases^{b} | Controls^{c} | Cases^{b} | Controls^{c} | Cases^{b} | Controls^{c} | *P* | OR(95% Cl)^{d} |
| rs6137444 | GWAS | 296 | 347 | 0.264(C) | 0.383(C) | 14/128/154 | 49/168/130 | 3.11x10⁻⁴ | 1.74(1.37-2.21) |
| (21,733,639 bp) | Replication | 319 | 234 | 0.277(C) | 0.404(C) | 21/135/163 | 45/99/90 | 1.57x10⁻⁵ | 1.76(1.37-2.27) |
| | Combined^{e} | 605 | 579 | 0.269(C) | 0.391(C) | 35/255/315 | 93/267/219 | 2.20x10⁻¹⁰ | |
| rs2180439 | GWAS | 296 | 347 | 0.292(C) | 0.429(C) | 21/131/144 | 66/166/115 | 3.85x10⁻⁷ | 1.82(1.45-2.30) |
| (21,801,100 bp) | Replication | 319 | 234 | 0.303(C) | 0.485(C) | 23/147/149 | 62/103/69 | 1.37x10⁻⁹ | 2.17(1.70-2.78) |
| | Combines^{e} | 605 | 579 | 0.293(C) | 0.452(C) | 43/268/294 | 127/269/183 | 2.67x10⁻¹⁵ | |
| rs1998076 | GWAS | 296 | 347 | 0.282(A) | 0.427(A) | 20/120/144 | 65/163/115 | 1.30x10⁻⁷ | 1.90(1.50-7.41) |
| (21,828,045 bp) | Replication | 319 | 234 | 0.301(A) | 0.479(A) | 23/146/150 | 61/102/71 | 3.69x10⁻⁹ | 2.13(1.66-2.73) |
| | Combined^{e} | 605 | 579 | 0.292(A) | 0.448(A) | 43/267/295 | 126/267/186 | 7.73x10⁻¹⁵ | |
| rs201571 | GWAS | 296 | 347 | 0.289(C) | 0.411(C) | 17/137/142 | 61/163/123 | 4.31x10⁻⁶ | 1.72(1.36-2.17) |
| (21,961,514 bp) | Replication | 319 | 234 | 0.314(C) | 0.483(C) | 30/140/149 | 58/110/66 | 2.21x10⁻⁸ | 2.05(1.60-2.62) |
| | Combined^{e} | 605 | 579 | 0.298(C) | 0.44(C) | 46/269/290 | 119/272/188 | 1.21x10⁻¹² | |
| rs6113491 | GWAS | 296 | 347 | 0.359(C) | 0.483(C) | 29/154/112 | 88/159/100 | 8.63x10⁶ | 1.66(1.33-2.08) |
| (22,005,415 bp) | Replication | 319 | 234 | 0.364(C) | 0.447(A) | 38/156/125 | 77/105/52 | 8.13x10⁻¹⁰ | 2.17(1.70-2.77) |
| | Combined^{e} | 605 | 579 | 0.359(C) | 0.488(A) | 66/302/237 | 165/263/151 | 1.13x10⁻¹³ | |

| | | | | | | | | | |
|---|---|---|---|---|---|---|---|---|---|
| ^{a} NCBI build 36. ^{b} GWAS cases were the most extremely affected. ^{c} GWAS controls are population based, the most extreme AGA affected having been excluded; replication controls are males >60 years of age without AGA and representing the least affected 20%. ^{d} ORs were not calculated for the combined samples since the composition of the two control samples differed as regards the exclusion of AGA cases. ^{e} Twelve GWAS samples were excluded in the combined analysis due to a cell rate <95% for the reduced SNP set. | | | | | | | | | |

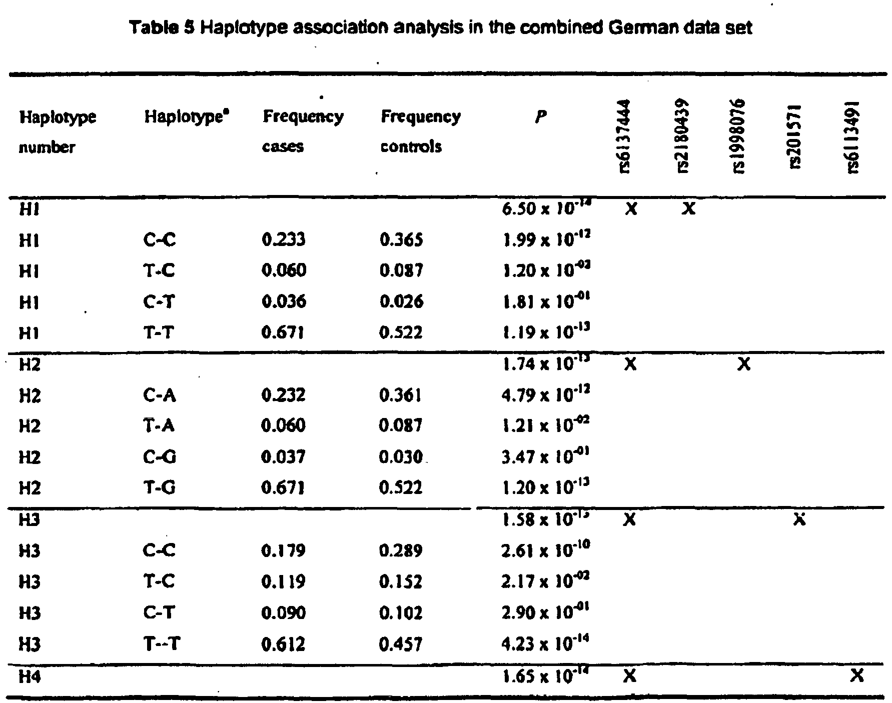

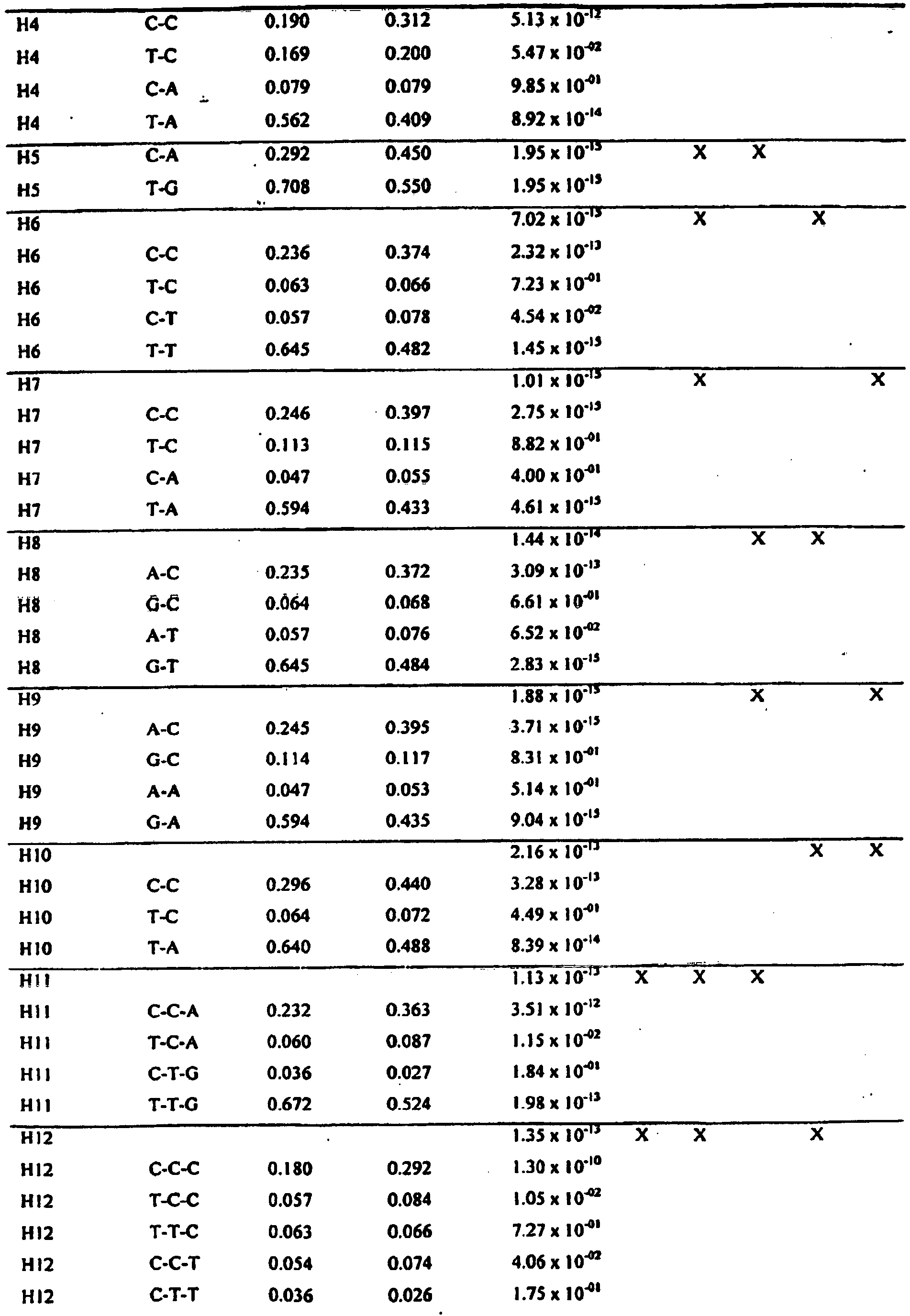

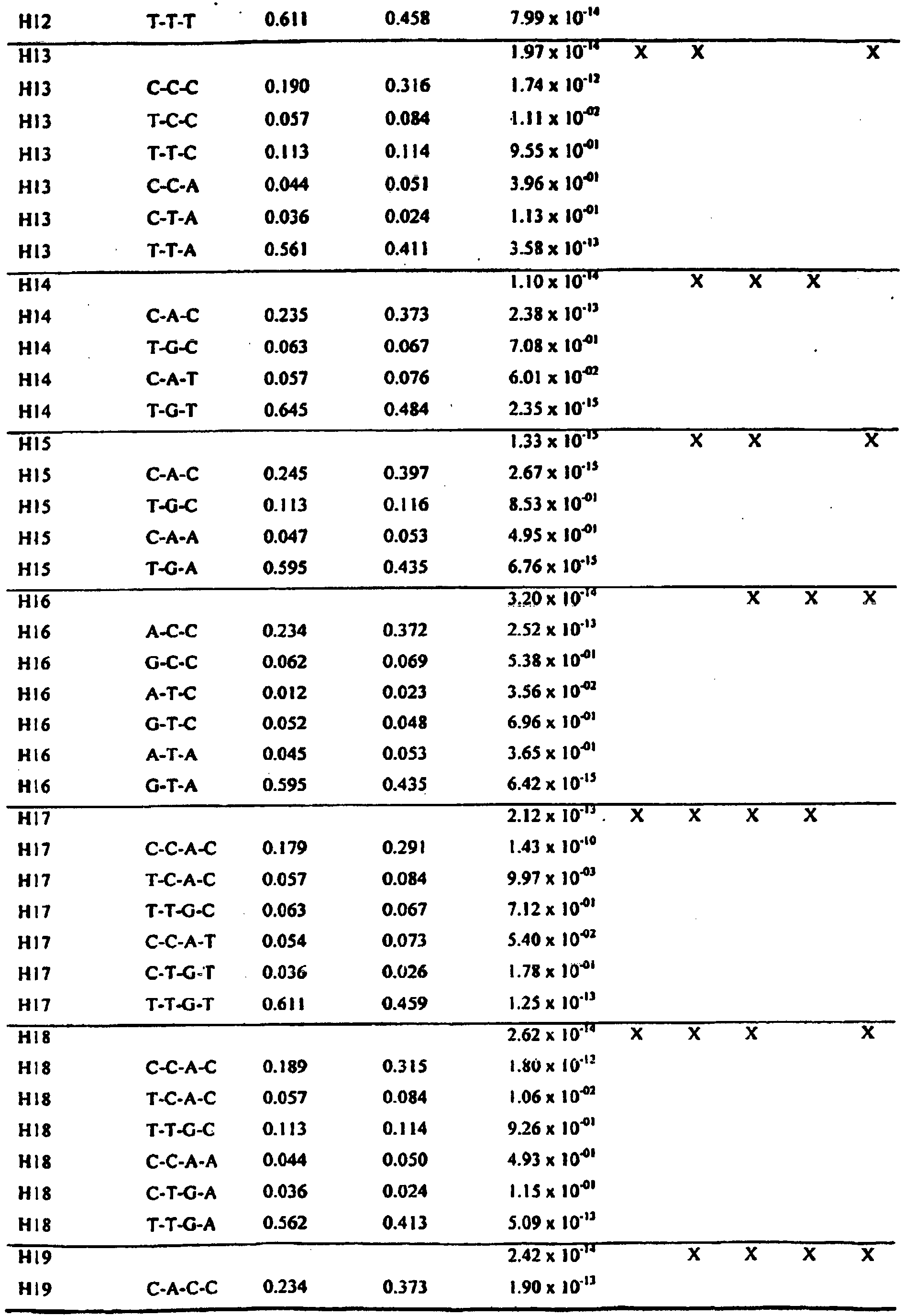

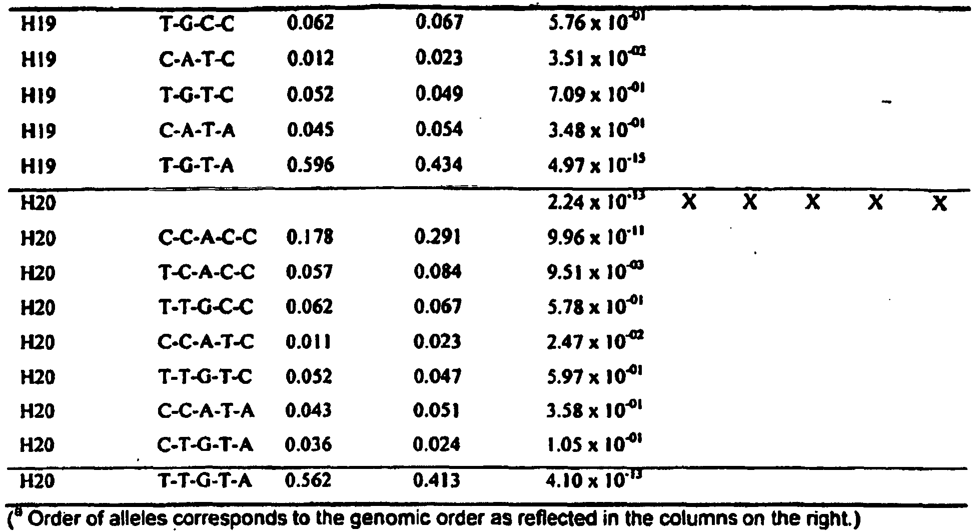

A subset of the affected sample were members of 352 nuclear families, and the results of a transmission disequillbrium test (TDT) were in accordance with the case-control findings which had shown association for the five chromosome 20 SNPs and the highest significance for rs2180439 (TDT *P* = 7.22 x 10⁻⁷).

**Table 6 Transmission disequilibrium test in 352 German nuclear families**

| SNP | Chr. | Position [bp]^{a} | Transmitted | Untransmitted | *P*^{b} | OR (95% Cl) |
|---|---|---|---|---|---|---|
| rs6137444 | 20 | 21,733,639 | 182 (C) | 255 (C) | 4.79 x 10⁻⁴ | 1.40 (1.16 - 1.69) |
| rs2180439 | 20 | 21,801,100 | 172 (C) | 277 (C) | 7.22 x 10⁻⁷ | 1.61 (1.33 - 1.95) |
| rs1998076 | 20 | 21,828,045 | 170 (A) | 270 (A) | 1.87 x 10⁻⁶ | 1.59 (1.31 - 1.92) |
| rs201571 | 20 | 21,961,514 | 168 (C) | 258 (C) | 1.30 x 10⁻⁵ | 1.54 (1.26 - 1.86) |
| rs6113491 | 20 | 22,005,415 | 193 (C) | 288 (C) | 1.48 x 10⁻⁵ | 1.49 (1.24 - 1.79) |

| | | | | | | |
|---|---|---|---|---|---|---|
| (^{a} Build 36; ^{b} Association P value) | | | | | | |

We also performed an initial step to investigate the importance of this locus in non-German populations by analyzing a modestly sized Australian sample for the five chromosome 20 SNPs. Again, rs2180439 showed significant association.

**Table 7 Replication analysis in Australian AGA sample**

| | | 141 cases^{a} vs. 150 controls | | | | 60 cases^{b} vs. 150 controls | | | |
|---|---|---|---|---|---|---|---|---|---|
| SNP | Minor allele | MAF cases | MAF controls | *P* | OR (95% Cl) | MAF cases | MAF controls | *P* | OR (95% Cl) |
| rs6137444 | C | 0.298 | 0.343 | 0.247 | 1.23 (0.86-1.74) | 0.275 | 0.343 | 0.171 | 1.37 (0.86-2.19) |
| rs2180439 | C | 0.340 | 0.420 | 0.041 | 1.4 (1-1.96) | 0.317 | 0.420 | 0.045 | 1.56 (1-2.44) |
| rs1998076 | A | 0.344 | 0.417 | 0.064 | 1.36 (0.97-1.91) | 0.317 | 0.417 | 0.053 | 1.54 (0.99-2.41) |
| rs201571 | C | 0.309 | 0.417 | 0.0069 | 1.6 (1.14-2.25) | 0.292 | 0.417 | 0.018 | 1.73 (1.1 -2.73) |
| rs6113491 | C | 0.401 | 0.477 | 0.066 | 1.36 (0.98-1.89) | 0.392 | 0.477 | 0.111 | 1.41 (0.92 - 2.17) |

| | | | | | | | | | |
|---|---|---|---|---|---|---|---|---|---|
| (^{a} AGA grade ≥ IIa; ^{b} AGA grade ≥ IV) | | | | | | | | | |

### Example 4: Phenotype

As regards phenotype, German GWAS cases (extreme) and controls (containing affecteds) differed from the replication cases (less extreme) and controls (unaffecteds). The genotype based ORs of rs2180439 in the four possible case-control comparisons showed an increase from the least extreme (replication cases/GWAS controls, OR = 3.72, 95% Cl 2.18-6.34) to the most extreme (GWAS cases/replication controls, OR = 6.16, 95% Cl 3.48-10.92) for the homozygotes (Fig. 2). Correlation of effect size and phenotypic difference supports an association between rs2180439 and AGA. However, this relation was less clear for the heterozygotes. We used logistic regression (Cordell HJ, Clayton DG (2002). Am J Hum Genet 70:124-141) and tested for deviation from a multiplicative model on the OR scale in the combined data set. The test showed a tendency toward a dominant model for rs2180439 (P = 0.023)

**Table 8 Test for deviation from a multiplicative model with two degrees of freedom versus one degree of freedom in the combined German data set**

| SNP | *P* |
|---|---|
| rs6137444 | 0.0608 |
| rs2180439 | 0.0225 |
| rs1998076 | 0.0203 |
| rs201571 | 0.0583 |
| rs6113491 | 0.0025 |

We also found evidence that rs2180439 and rs6113491 contribute independently to the risk of AGA, and that rs6113491 showed a dominant effect.

**Table 9 Tests for additional effects of further SNPs at the chromosome 20 locus in the combined German data set**

| | LD measure | *P* | |
|---|---|---|---|
| | r² with rs2180439 | Test for independence^{a} | Test for dominance^{b} |
| rs6137444 | 0.6 | 0.865 | n.d. |
| rs1998076 | 0.99 | 0.2634 | n.d. |
| rs201571 | 0.5 | 0.0367 | 0.3677 |
| rs6113491 | 0.44 | 0.0033 | 0.0192 |

| | | | |
|---|---|---|---|
| (^{a} 3 degrees of freedom versus 2 degrees of freedom; ^{b} 4 degrees of freedom versus 3 degrees of freedom) | | | |

To observe this effect, however, use of the combined sample was necessary since rs6113491 did not remain significant after stratifying the GWAS data for rs2180439 (Fig. 1).

### Example 5: Interaction

We tested chromosome 20p11 for interaction with the primary associated SNP at the *AR* locus (rs1041668). Introduction of rs1041668 improved the model-fit significantly. However, neither the interaction terms with rs2180439 (*P* = 0.975) nor with rs6113491 (*P* = 0.984) yielded significant improvement of the model-fit. Thus, our data provide no evidence for interaction effects between the two regions.

### Example 6: Gene contributing to the development of AGA

Two expressed sequence tags, BQ013595 and BE789145, map to the associated region on 20p11 (Fig. 1). Since a gene contributing to the development of AGA would be expected to be expressed in the human scalp, we quantified the expression of BQ013595 and BE789145 in various human tissues including skin, hair and scalp. In addition, we included the closest reference sequence gene, *PAX1* (paired box 1). BE789145 was not detectable in skin, hair or scalp and BQ013595 showed very low expression in hair and scalp. *PAX1* was found to be expressed at very low levels in hair and skin, but showed considerable expression in the scalp (Fig. 3). Although *PAX1* is located outside of the associated LD block, the expression data might suggest that *PAX1* confers the AGA relevant effect at this locus and that a regulatory variant within the associated LD block may modulate its expression.

### Example 7: Etiological fraction

The AGA risk allele T of rs2180439 has a frequency of 0.57 in our population based control sample of 383 individuals (before exclusion of the AGA affected controls for the GWAS step). The etiological fraction for rs2180439 is estimated at 0.32, underlining the importance of this locus in the development of AGA. The frequency of the risk allele varies world-wide from 0.03 to 0.86 (Fig. 4). This may account in part for population specific differences in AGA prevalence, but it does not explain the lower prevalence reported in South-East Asian populations (Paik, J.H., et al. (2001) N.I. Br J Dermatol 145, 95-99; Norwood OT (1975), South Med J 68, 1359-1365). It is of note that the chromosome 20 locus did not show linkage to AGA in a recent study (Hillmer, A.M. et al. (2008), Am J Hum Genet 82, 737-743). This supports the value of the association approach in detecting susceptibility genes for complex diseases (Risch, N., Merikangas, K. (1996), Science 273, 1516-1517).

## Claims

1. A method for testing for a predisposition or presence of androgenetic alopecia comprising testing a sample obtained from a prospective patient or from a person suspected of carrying a predisposition for androgenetic alopecia, the method comprising determining
(a) the presence and/or quantity of a nucleic acid molecule selected from the group consisting of:
(i) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292;
(ii) a nucleic acid molecule the complementary strand of which hybridises under stringent conditions to the nucleic acid molecule of (a)(i), wherein said nucleic acid molecule has at a position corresponding to position 201 of any one of SEQ ID NOs:1 to 77 an adenosine, corresponding to position 201 of, any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine;
(iii) a nucleic acid molecule identical to any one of the nucleic acid molecules of (a)(i) or (a)(ii) wherein each thymidine is replaced by uridine; and/or
(b) the presence and/or quantity of a nucleic acid molecule selected from the group consisting of:
(i) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:78, 79, 83, 88, 87, 90, 91, 93, 94, 96, 98 to 109, 111, 112, 118 to 123, 126, 128 to 142, 144 to 149, 151, 155, 158 to 160, 162, 165, 178, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 150, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 28 to 28, 30 to 33, 36, 37, 41 to 47, 49, 52, 53, 55, 58, 60 to 66, 68 to 75, 77, 153, 161, 163, 167, 178, 186, 187, 192, 194, 214, 218, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 248 to 250, 255, 256 to 258, 260, 262, 263, 265, 267 to 272, 276, 278, 280 to 283, 285 to 292;
(ii) a nucleic acid molecule the complementary strand of which hybridises under stringent conditions to the nucleic acid molecule of (b)(i), wherein said nucleic acid molecule has at a position corresponding to position 201 of any one of SEQ ID NOs: SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3. 7, 25, 34, 36, 38, 39, 48, 50, 51, 54, 57, 67, 76, 80, 89, 95, 110, 114, 115, 125, 150, 152, 154, 156, 157, 164, 168, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 18, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine;
(iii) a nucleic acid molecule identical to any one of the nucleic acid molecules of (b) (i) or (b)(ii) wherein each thymidine is replaced by uridine; and/or
(c) the presence and/or quantity of a nucleic acid molecule which is complementary to the nucleic acid molecule of (a) or (b); or
(d) the presence of a risk allele within the nucleic acid molecule of (a) as compared to the non-risk allele, wherein said risk allele is **characterised in that** it is an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; or
(e) the presence of a risk allele within the nucleic acid molecule of (c) as compared to the non-risk allele, wherein said risk allele is **characterised in that** it is an thymidine at position 201 of any one of SEQ ID NOs:1 to 77, a cytidine at position 201 of any one of SEQ ID NOs:78 to 149, a adenosine at position 201 of any one of SEQ ID NOs:150 to 219 or a guanosine at position 201 of any one of SEQ ID NOs:220 to 292; or
(f) the presence of a non-risk allele within the nucleic acid molecule of (b) as compared to the risk allele, wherein said non-risk allele is **characterised in that** it is an adenosine at position 201 of any one of SEQ ID NOs:78, 79, 83, 86, 87, 90, 91, 93, 94, 98, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 208, 208 to 211, 213 or 215, a guanosine at position 201 of any one of SEQ ID NOs:1, 2, 4, 5, 8 to 11, 13, 14, 18, 18, 20 to 23, 28 to 28, 30 to 33, 36, 37, 41 to 47, 49, 52, 53, 55, 58, 60 to 68, 68 to 75, 77, 153, 161, 163, 167, 178, 186, 187, 192, 194, 214, 218, 218 to 220, 222 to 224, 23*6, 241, 245, 252, 266, 273 to 275, 277, 279 or 284 or a thymidine at position 201 of any one of SEQ ID NOs:6, 12, 15, 17, 19, 24, 29, 40, 56, 59, 81, 82, 84, 85, 86, 92, 97, 113, 127, 143, 225, 227, 229, 231, 234, 237, 238, 240, 244, 246 to 250, 255, 256 to 258, 280, 282, 263, 265, 267 to 272, 278, 278, 280 to 283, 285 to 292; or
(g) the presence of a non-risk allele within the nucleic acid molecule of (c) as compared to the risk allele, wherein said non-risk allele is **characterised in that** it is an thymidine at position 201 of any one of SEQ ID NOs:78, 79, 83, 86, 87, 90, 91, 93, 94, 98, 98 to 109, 111, 112, 118 to 123, 128, 128 to 142, 144 to 149, 151, 155, 158 to 160, 162, 165, 178, 177, 162, 184, 204, 207, 212, 217, 221, 228, 228, 230, 232. 233, 235, 239, 242, 243, 251, 253, 254, 259, 261 or 264, a guanosine at position 201 of any one of SEQ ID NOs: 3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 196 to 203, 205, 206, 208 to 211, 213 or 215, a cytidine at position 201 of any one of SEQ ID NOs: 1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a adenosine at position 201 of any one of SEQ ID NOs:6, 12, 15, 17, 19, 24, 29, 40, 58, 59, 81, 82, 84, 85, 88, 92, 97, 113, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; or
(h) the presence of a risk allele of a genetic marker of androgenetic alopecia **characterised In that** the genetic marker is located in a chromosomal region of human chromosome 20p11; wherein the chromosomal region of human chromosome 20p11 consists of the region having the sequence of SEQ ID NO:1 at the 5' end and the sequence of SEQ ID NO:292 at the 3' end and wherein the genetic marker is selected from the group consisting of a single nucleotide polymorphism (SNP), variable number of tandem repeat (VNTR), microsatellites or short tandem repeats (STR)
and wherein the presence of said nucleic acid molecule of (a), said genetic marker, or said risk allele is indicative of androgenetic alopecia or a predisposition for androgenetic alopecia and wherein the presence of said nucleic acid molecule of (b) or said non-risk allele is indicative of the absence of androgenetic alopecia or a predisposition for androgenetic alopecia.

2. The method of claim 1, wherein said determining of the presence of said genetic marker, said nucleic acid molecule or the presence of said risk or non-risk allele comprises PCR based techniques, RPLP- or AFLP- based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, and 5'-nuclease assay-based techniques.

3. The method of claims 1 or 2, further comprising detecting an alteration in the expression of a nucleic acid molecule encoding a polypeptide having PAX1 function, wherein said nucleic acid molecule comprises:
(a) a nucleic acid molecule encoding a polypeptide having the amino acid sequence of SEQ ID NO:293;
(b) a nucleic acid molecule having the DNA sequence of SEQ ID NO:294;
(c) a nucleic acid molecule having the sequence of SEQ ID NO:294, wherein each thymidine is replaced by uridine;
wherein the alteration of expression as compared to the amount of nucleic acid molecule expression in the control sample is indicative of androgenetic alopecia or a predisposition for androgenetic alopecia.

4. The method according to any one of claims 1 to 3, wherein the sample is blood, serum, plasma, fetal tissue, saliva, urine, mucosal tissue, mucus, vaginal tissue, fetal tissue obtained from the vagina, skin, hair, hair follicle or another human tissue.

5. The method of claim 1, wherein the chromosomal region of human chromosome 20p11 comprises at least one of SEQ ID NOs:1 to 292.

6. The method of any one of claims 1 or 5, wherein the genetic marker is a SNP selected from the group consisting of the SNPs comprised in any one of SEQ ID NOs:1 to 292.

7. The method of claim 1, wherein the nucleic acid molecule is genomic DNA.

## Patentansprüche

1. Verfahren zum Testen auf eine Veranlagung für oder das Vorhandensein von Androgenetischer Alopezie, umfassend das Testen einer Probe, die von einem potentiellen Patienten oder von einer Person erhalten wurde, bei der eine Veranlagung für Androgenetische Alopezie vermutet wird, wobei das Verfahren die Bestimmung umfasst
(a) des Vorhandenseins und/oder der Menge eines Nukleinsäuremoleküls ausgewählt aus der Gruppe bestehend aus:
(i) einem Nukleinsäuremolekül bestehend aus mindestens 17 aufeinanderfolgenden Nukleotiden aus einer der SEQ ID NOs:1 bis 292, wobei die mindestens 17 aufeinanderfolgenden Nukleotide ein Adenosin an Position 201 einer der SEQ ID NOs:1 bis 77, ein Guanosin an Position 201 einer der SEQ ID NOs:78 bis 149, ein Thymidin an Position 201 einer der SEQ ID NOs:150 bis 219 oder ein Cytidin an Position 201 einer der SEQ ID NOs:220 bis 292 enthalten;
(ii) einem Nukleinsäuremolekül, dessen Komplementärstrang unter stringenten Bedingungen an das Nukleinsäuremolekül nach (a)(i) hybridisiert, wobei das Nukleinsäuremolekül an der Position, die Position 201 einer der SEQ ID NOs:1 bis 77 entspricht, ein Adenosin; an der Position, die Position 201 einer der SEQ ID NOs:78 bis 149 entspricht, ein Guanosin; an der Position, die Position 201 einer der SEQ ID NOs:150 bis 219 entspricht, ein Thymidin oder an der Position, die Position 201 einer der SEQ ID NOs:220 bis 292 entspricht, ein Cytidin hat;
(iii) einem Nukleinsäuremolekül, das identisch zu einem der Nukleinsäuremoleküle nach (a)(i) oder (a)(ii) ist, wobei jedes Thymidin durch Uridin ersetzt ist; und/oder
(b) des Vorhandenseins und/oder der Menge eines Nukleinsäuremoleküls ausgewählt aus der Gruppe bestehend aus:
(i) einem Nukleinsäuremolekül bestehend aus mindestens 17 aufeinanderfolgenden Nukleotiden aus einer der SEQ ID NOs:1 bis 292, wobei die mindestens 17 aufeinanderfolgenden Nukleotide ein Adenosin an Position 201 einer der SEQ ID NOs:78, 79, 83, 86, 87, 90, 91, 93, 94, 96, 98 bis 109, 111, 112, 116 bis 123, 126, 128 bis 142, 144 bis 149, 151, 155, 158 bis 160, 162, 165, 176, 177, 182, 184, 204, 207, 212, 217, 221, 226, 228, 230, 232, 233, 235, 239, 242, 243, 251, 253, 254, 259, 261 oder 264, ein Cytidin an Position 201 einer der SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 bis 175, 179 bis 181, 183, 185, 188 bis 191, 193, 195 bis 203, 205, 206, 208 bis 211, 213 oder 215, ein Guanosin an Position 201 einer der SEQ ID NOs: 1, 2, 4, 5, 8 bis 11, 13, 14, 16, 18, 20 bis 23, 26 bis 28, 30 bis 33, 36, 37, 41 bis 47, 49, 52, 53, 55, 56, 60 bis 66, 68 bis 75, 77, 153, 161, 163, 167, 178, 186, 187, 192, 194, 214, 216, 218 bis 220, 222 bis 224, 236, 241, 245, 252, 266, 273 bis 275, 277, 279 oder 284 oder ein Thymidin an Position 201 einer der SEQ ID NOs: 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 bis 250, 255, 256 bis 258, 260, 262, 263, 265, 267 bis 272, 276, 278, 280 bis 283, 285 bis 292 enthalten;
(ii) einem Nukleinsäuremolekül, dessen Komplementärstrang unter stringenten Bedingungen an das Nukleinsäuremolekül nach (b)(i) hybridisiert, wobei das Nukleinsäuremolekül an der Position, die Position 201 einer der SEQ ID NOs:78, 79, 83, 86, 87, 90, 91, 93, 94, 96, 98 bis 109, 111, 112, 116 bis 123, 126, 128 bis 142, 144 bis 149, 151, 155, 158 bis 160, 162, 165, 176, 177, 182, 184, 204, 207, 212, 217, 221, 226, 228, 230, 232, 233, 235, 239, 242, 243, 251, 253, 254, 259, 261 oder 264 entspricht, ein Adenosin; an der Position, die Position 201 einer der SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 bis 175, 179 bis 181, 183, 185, 188 bis 191, 193, 195 bis 203, 205, 206, 208 bis 211, 213 oder 215 entspricht, ein Cytidin; an der Position, die Position 201 einer der SEQ ID NOs: 1, 2, 4, 5, 8 bis 11, 13, 14, 16, 18, 20 bis 23, 26 bis 28, 30 bis 33, 36, 37, 41 bis 47, 49, 52, 53, 55, 56, 60 bis 66, 68 bis 75, 77, 153, 161, 163, 167, 178, 186, 187, 192, 194, 214, 216, 218 bis 220, 222 bis 224, 236, 241, 245, 252, 266, 273 bis 275, 277, 279 oder 284 entspricht, ein Guanosin oder an der Position, die Position 201 einer der SEQ ID NOs: 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 bis 250, 255, 256 bis 258, 260, 262, 263, 265, 267 bis 272, 276, 278, 280 bis 283, 285 bis 292 entspricht, ein Thymidin enthält;
(iii) einem Nukleinsäuremolekül, das identisch zu einem der Nukleinsäuremolekule nach (b)(i) oder (b)(ii) ist, wobei jedes Thymidin durch Uridin ersetzt ist; und/oder
(c) des Vorhandenseins und/oder der Menge eines Nukleinsäuremoleküls, das komplementär zu dem Nukleinsäuremolekül nach (a) oder (b) ist; oder
(d) des Vorhandenseins eines Risiko-Allels in dem Nukleinsäuremolekül nach (a) im Vergleich zum Nicht-Risiko-Allel, wobei das Risiko-Allel dadurch charakterisiert ist, dass es ein Adenosin an Position 201 einer der SEQ ID NOs:1 bis 77, ein Guanosin an Position 201 einer der SEQ ID NOs:78 bis 149, ein Thymidin an Position 201 einer der SEQ ID NOs:150 bis 219 oder ein Cytidin an Position 201 einer der SEQ ID NOs:220 bis 292 ist; oder
(e) des Vorhandenseins eines Risiko-Allels in dem Nukleinsäuremolekül nach (c) im Vergleich zum Nicht-Risiko-Allel, wobei das Risiko-Allel dadurch charakterisiert ist, dass es ein Thymidin an Position 201 einer der SEQ ID NOs:1 bis 77, ein Cytidin an Position 201 einer der SEQ ID NOs:78 bis 149, ein Adenosin an Position 201 einer der SEQ ID NOs:150 bis 219 oder ein Guanosin an Position 201 einer der SEQ ID NOs:220 bis 292 enthält; oder
(f) des Vorhandenseins eines Nicht-Risiko-Allels in dem Nukleinsäuremolekül nach (b) im Vergleich zum Risiko-Allel, wobei das Nicht-Risiko-Allel dadurch charakterisiert ist, dass es ein Adenosin an Position 201 einer der SEQ ID NOs:78, 79, 83, 86, 87, 90, 91, 93, 94, 96, 98 bis 109, 111, 112, 116 bis 123, 126, 128 bis 142, 144 bis 149, 151, 155, 158 bis 160, 162, 165, 176, 177, 182, 184, 204, 207, 212, 217, 221, 226, 228, 230, 232, 233, 235, 239, 242, 243, 251, 253, 254, 259, 261 oder 264, ein Cytidin an Position 201 einer der SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 bis 175, 179 bis 181, 183, 185, 188 bis 191, 193, 195 bis 203, 205, 206, 208 bis 211, 213 oder 215, ein Guanosin an Position 201 einer der SEQ ID NOs: 1, 2, 4, 5, 8 bis 11, 13, 14, 16, 18, 20 bis 23, 26 bis 28, 30 bis 33, 36, 37, 41 bis 47, 49, 52, 53, 55, 56, 60 bis 66, 68 bis 75, 77, 153, 161, 163, 167, 178, 186, 187, 192, 194, 214, 216, 218 bis 220, 222 bis 224, 236, 241, 245, 252, 266, 273 bis 275, 277, 279 oder 284 oder ein Thymidin an Position 201 einer der SEQ ID NOs: 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 bis 250, 255, 256 bis 258, 260, 262, 263, 265, 267 bis 272, 276, 278, 280 bis 283, 285 bis 292 enthält; oder
(g) des Vorhandenseins eines Nicht-Risiko-Allels in dem Nukleinsäuremolekül nach (c) im Vergleich zum Risiko-Allel, wobei das Nicht-Risiko-Allel dadurch charakterisiert ist, dass es ein Thymidin an Position 201 einer der SEQ ID NOs:78, 79, 83, 86, 87, 90, 91, 93, 94, 96, 98 bis 109, 111, 112, 116 bis 123, 126, 128 bis 142, 144 bis 149, 151, 155, 158 bis 160, 162, 165, 176, 177, 182, 184, 204, 207, 212, 217, 221, 226, 228, 230, 232, 233, 235, 239, 242, 243, 251, 253, 254, 259, 261 oder 264, ein Guanosin an Position 201 einer der SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 bis 175, 179 bis 181, 183, 185, 188 bis 191, 193, 195 bis 203, 205, 206, 208 bis 211, 213 oder 215, ein Cytidin an Position 201 einer der SEQ ID NOs: 1, 2, 4, 5, 8 bis 11, 13, 14, 16, 18, 20 bis 23, 26 bis 28, 30 bis 33, 36, 37, 41 bis 47, 49, 52, 53, 55, 56, 60 bis 66, 68 bis 75, 77, 153, 161, 163, 167, 178, 186, 187, 192, 194, 214, 216, 218 bis 220, 222 bis 224, 236, 241, 245, 252, 266, 273 bis 275, 277, 279 oder 284 oder ein Adenosin an Position 201 einer der SEQ ID NOs: 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 bis 250, 255, 256 bis 258, 260, 262, 263, 265, 267 bis 272, 276, 278, 280 bis 283, 285 bis 292 enthält; oder
(h) des Vorhandenseins eines Risiko-Allels eines genetischen Markers der Androgenetischen Alopezie, dadurch charakterisiert, dass der genetische Marker in einem chromosomalen Bereich des menschlichen Chromosoms 20p11 liegt, wobei der chromosomale Bereich des menschlichen Chromosoms 20p11 aus dem Bereich besteht, der die Sequenz der SEQ ID NO:1 am 5' Ende und die Sequenz der SEQ ID NO:292 am 3' Ende hat und wobei der genetische Marker ausgewählt ist aus der Gruppe bestehend aus einem Einzelnukleotidpolymorphismus (SNP), variabler Anzahl von Tandem-Repeat (VNTR), Mikrosatelliten oder kurzen Tandem-Repeats (STR)
und wobei das Vorhandensein des Nukleinsäuremoleküls nach (a), des genetischen Markers oder des Risiko-Allels Androgenetische Alopezie oder eine Veranlagung für Androgenetische Alopezie anzeigt und wobei das Vorhandensein des Nukleinsäuremoleküls nach (b) oder des Nicht-Risiko-Allels das Fehlen einer Androgenetische Alopezie oder einer Veranlagung für Androgenetische Alopezie anzeigt.

2. Verfahren nach Anspruch 1, wobei die Bestimmung des Vorhandenseins des genetischen Markers, des Nukleinsäuremoleküls oder des Vorhandenseins des Risiko- oder Nicht-Risiko-Allels PCR-basierte Techniken, RFLP- oder AFLP-basierte Techniken, DNA-Sequenzierungsbasierte Techniken, Hybridisierungs-basierte Techniken, Single-Strand Konformationspolymorphismenanalyse (SSCA), denaturierende Gradientengelelektrophorese (DGGE), Mismatch-Spaltungsnachweis, Heteroduplexanalyse, Techniken basierend auf Primerverlängerung und 5'-Nukleaseassay-basierte Techniken umfasst.

3. Verfahren nach Anspruch 1 oder 2, des weiteren umfassend das Detektieren einer Veränderung in der Expression eines Nukleinsäuremoleküls, das ein Polypeptid kodiert, das PAX1 Funktion hat, wobei das Nukleinsäuremolekül umfasst:
(a) ein Nukleinsäuremolekül, das ein Polypeptid kodiert, dass die Aminosäuresequenz der SEQ ID NO:293 hat;
(b) ein Nukleinsäuremolekül, das die DNA-Sequenz der SEQ ID NO:294 hat;
(c) ein Nukleinsäuremolekül, das die Sequenz der SEQ ID NO:294 hat, wobei jedes Thymidin durch Uridin ersetzt ist;
wobei die Veränderung der Expression im Vergleich zur Menge der Expression des Nukleinsäuremoleküls in einer Kontrollprobe Androgenetische Alopezie oder eine Veranlagung für Androgenetische Alopezie anzeigt.

4. Verfahren nach einem der Ansprüche 1 bis 3, wobei die Probe Blut, Serum, Plasma, fötales Gewebe, Speichel, Urin, Schleimhautgewebe, Schleim, Vaginalgewebe, fötales Gewebe von der Vagina, Haut, Haar, Haarfollikel oder anderes menschliches Gewebe ist.

5. Verfahren nach Anspruch 1, wobei der chromosomale Bereich des humanen Chromosoms 20p11 mindestens eine der SEQ ID NOs:1 bis 292 umfasst.

6. Verfahren nach einem der Ansprüche 1 oder 5, wobei der genetische Marker ein SNP ausgewählt aus der Gruppe bestehend aus den SNP ist, die in einer der SEQ ID NOs:1 bis 292 umfasst sind.

7. Verfahren nach Anspruch 1, wobei das Nukleinsäuremolekül genomische DNA ist.

## Revendications

1. Procédé d'analyse à la recherche d'une prédisposition ou de la présence d'une alopécie androgénétique comprenant l'analyse d'un échantillon obtenu à partir d'un patient prospectif ou à partir d'une personne suspectée de porter une prédisposition pour une alopécie androgénétique, le procédé comprenant la détermination
(a) de la présence et/ou de la quantité d'une molécule d'acide nucléique choisie dans le groupe consistant en :
(i) une molécule d'acide nucléique constitué d'au moins 17 nucléotides consécutifs de l'une quelconque de SEQ ID NO : 1 à 292 ; où les au moins 17 nucléotides consécutifs contiennent l'adénosine en position 201 de l'une quelconque de SEQ ID NO : 1 à 77, la guanosine en position 201 de l'une quelconque de SEQ ID NO : 78 à 149, la thymidine en position 201 de l'une quelconque de SEQ ID NO : 150 à 219 ou la cytidine composition 201 de l'une quelconque de SEQ ID NO : 220 à 292 ;
(ii) une molécule d'acide nucléique dont le brin complémentaire s'hybride dans des conditions stringentes à la molécule d'acide nucléique de (a)(i), où ladite molécule d'acide nucléique a à une position correspondant à la position 201 de l'une quelconque de SEQ ID NO : 1 à 77 une adénosine, correspondant à la position 201 de l'une quelconque de SEQ ID NO : 78 à 149 une guanosine, correspondant à la position 201 de l'une quelconque de SEQ ID NO : 150 à 219 une thymidine ou correspondant à la position 201 de l'une quelconque de SEQ ID NO : 220 à 292 une cytidine ;
(iii) une molécule d'acide nucléique identique à l'une quelconque des molécules d'acide nucléique de (a)(i) ou (a)(ii) où chaque thymidine est remplacée par une uridine ; et/ou
(b) de la présence et/ou de la quantité d'une molécule d'acide nucléique choisie dans le groupe consistant en :
(i) une molécule d'acide nucléique constitué d'au moins 17 nucléotides consécutifs de l'une quelconque de SEQ ID NO : 1 à 292 ; où les au moins 17 nucléotides consécutifs contiennent l'adénosine en position 201 de l'une quelconque de SEQ ID NO : 78, 79, 83, 86, 87, 90, 91, 93, 94, 96, 98 à 109, 111, 112, 116 à 123, 126, 128 à 142, 144 à 149, 151, 155, 158 à 160, 162, 165, 176, 177, 182, 184, 204, 207, 212, 217, 221, 226, 228, 230, 232, 233, 235, 239, 242, 243, 251, 253, 254, 259, 261 ou 264, la cytidine en position 201 de l'une quelconque de SEQ ID NO : 3, 7, 25, 34, 35, 38, 39, 48, 50, 51, 54, 57, 67, 76, 80, 89, 95, 110, 114, 115, 125, 150, 152, 154, 156, 157, 164, 166, 168 à 175, 179 à 181, 183, 185, 188 à 191, 193, 195 à 203, 205, 206, 208 à 211, 213 ou 215, la guanosine en position 201 de l'une quelconque de SEQ ID NO : 1, 2, 4, 5, 8 à 11, 13, 14, 16, 18, 20 à 23, 26 à 28, 30 à 33, 36, 37, 41 à 47, 49, 52, 53, 55, 56, 60 à 66, 68 à 75, 77, 153, 161, 163, 167, 178, 186, 187, 192, 194, 214, 216, 218 à 220, 222 à 224, 236, 241, 245, 252, 266, 273 à 275, 277, 279 ou 284 ou la thymidine en position 201 de l'une quelconque de SEQ ID NO : 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 à 250, 255, 256 à 258, 260, 262, 263, 265, 267 à 272, 276, 278, 280 à 283, 285 à 292 ;
(ii) une molécule d'acide nucléique dont le brin complémentaire s'hybride dans des conditions stringentes à la molécule d'acide nucléique de (b)(i), où ladite molécule d'acide nucléique a à une position correspondant à la position 201 de l'une quelconque de SEQ ID NO : 78, 79, 83, 86, 87, 90, 91, 93, 94, 96, 98 à 109, 111, 112, 116 à 123, 126, 128 à 142, 144 à 149, 151, 155, 158 à 160, 162, 165, 176, 177, 182, 184, 204, 207, 212, 217, 221, 226, 228, 230, 232, 233, 235, 239, 242, 243, 251, 253, 254, 259, 261 ou 264 une adénosine, correspondant à la position 201 de l'une quelconque de SEQ ID NO: 3, 7, 25, 34, 35, 38, 39, 48, 50, 51, 54, 57, 67, 76, 80, 89, 95, 110, 114, 115, 125, 150, 152, 154, 156, 157, 164, 166, 168 à 175, 179 à 181, 183, 185, 188 à 191, 193, 195 à 203, 205, 206, 208 à 211, 213 ou 215 une cytidine, correspondant à la position 201 de l'une quelconque de SEQ ID NO : 1, 2, 4, 5, 8 à 11, 13, 14, 16, 18, 20 à 23, 26 à 28, 30 à 33, 36, 37, 41 à 47, 49, 52, 53, 55, 56, 60 à 66, 68 à 75, 77, 153, 161, 163, 167, 178, 186, 187, 192, 194, 214, 216, 218 à 220, 222 à 224, 236, 241, 245, 252, 266, 273 à 275, 277, 279 ou 284 une guanosine ou correspondant à la position 201 de l'une quelconque de SEQ ID NO : 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 à 250, 255, 256 à 258, 260, 262, 263, 265, 267 à 272, 276, 278, 280 à 283, 285 à 292 une thymidine ;
(iii) une molécule d'acide nucléique identique à l'une quelconque des molécules d'acide nucléique de (b)(i) ou (b)(ii) où chaque thymidine est remplacée par une uridine ; et/ou
(c) de la présence et/ou de la quantité d'une molécule d'acide nucléique qui est complémentaire de la molécule d'acide nucléique de (a) ou (b) ; ou
(d) de la présence d'un allèle de risque au sein de la molécule d'acide nucléique de (a) comparativement à l'allèle de non risque, où ledit allèle de risque est **caractérisé en ce qu'**il s'agit d'une adénosine en position 201 de l'une quelconque de SEQ ID NO : 1 à 77, d'une guanosine en position 201 de l'une quelconque de SEQ ID NO : 78 à 149, d'une thymidine en position 201 de l'une quelconque de SEQ ID NO : 150 à 219 ou d'une cytidine en position 201 de l'une quelconque de SEQ ID NO : 220 à 292 ; ou
(e) de la présence d'un allèle de risque au sein de la molécule d'acide nucléique de (c) comparativement à l'allèle de non risque, où ledit allèle de risque est **caractérisé en ce qu'**il s'agit d'une thymidine en position 201 de l'une quelconque de SEQ ID NO : 1 à 77, d'une cytidine en position 201 de l'une quelconque de SEQ ID NO : 78 à 149, d'une adénosine en position 201 de l'une quelconque de SEQ ID NO : 150 à 219 ou d'une guanosine en position 201 de l'une quelconque de SEQ ID NO : 220 à 292 ; ou
(f) de la présence d'un allèle de non risque au sein de la molécule d'acide nucléique de (b) comparativement à l'allèle de risque, où ledit allèle de non risque est **caractérisé en ce qu'**il s'agit d'une adénosine en position 201 de l'une quelconque de SEQ ID NO : 78, 79, 83, 86, 87, 90, 91, 93, 94, 96, 98 à 109, 111, 112, 116 à 123, 126, 128 à 142, 144 à 149, 151, 155, 158 à 160, 162, 165, 176, 177, 182, 184, 204, 207, 212, 217, 221, 226, 228, 230, 232, 233, 235, 239, 242, 243, 251, 253, 254, 259, 261 ou 264, d'une cytidine en position 201 de l'une quelconque de SEQ ID NO : 3, 7, 25, 34, 35, 38, 39, 48, 50, 51, 54, 57, 67, 76, 80, 89, 95, 110, 114, 115, 125, 150, 152, 154, 156, 157, 164, 166, 168 à 175, 179 à 181, 183, 185, 188 à 191, 193, 195 à 203, 205, 206, 208 à 211, 213 ou 215, d'une guanosine en position 201 de l'une quelconque de SEQ ID NO : 1, 2, 4, 5, 8 à 11, 13, 14, 16, 18, 20 à 23, 26 à 28, 30 à 33, 36, 37, 41 à 47, 49, 52, 53, 55, 56, 60 à 66, 68 à 75, 77, 153, 161, 163, 167, 178, 186, 187, 192, 194, 214, 216, 218 à 220, 222 à 224, 236, 241, 245, 252, 266, 273 à 275, 277, 279 ou 284 ou d'une thymidine en position 201 de l'une quelconque de SEQ ID NO : 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 à 250, 255, 256 à 258, 260, 262, 263, 265, 267 à 272, 276, 278, 280 à 283, 285 à 292 ; ou
(g) de la présence d'un allèle de non risque au sein de la molécule d'acide nucléique de (c) comparativement à l'allèle de risque, où ledit allèle de non risque est **caractérisé en ce qu'**il s'agit d'une thymidine en position 201 de l'une quelconque de SEQ ID NO : 78, 79, 83, 86, 87, 90, 91, 93, 94, 96, 98 à 109, 111, 112, 116 à 123, 126, 128 à 142, 144 à 149, 151, 155, 158 à 160, 162, 165, 176, 177, 182, 184, 204, 207, 212, 217, 221, 226, 228, 230, 232, 233, 235, 239, 242, 243, 251, 253, 254, 259, 261 ou 264, d'une guanosine en position 201 de l'une quelconque de SEQ ID NO : 3, 7, 25, 34, 35, 38, 39, 48, 50, 51, 54, 57, 67, 76, 80, 89, 95, 110, 114, 115, 125, 150, 152, 154, 156, 157, 164, 166, 168 à 175, 179 à 181, 183, 185, 188 à 191, 193, 195 à 203, 205, 206, 208 à 211, 213 ou 215, d'une cytidine en position 201 de l'une quelconque de SEQ ID NO : 1, 2, 4, 5, 8 à 11, 13, 14, 16, 18, 20 à 23, 26 à 28, 30 à 33, 36, 37, 41 à 47, 49, 52, 53, 55, 56, 60 à 66, 68 à 75, 77, 153, 161, 163, 167, 178, 186, 187, 192, 194, 214, 216, 218 à 220, 222 à 224, 236, 241, 245, 252, 266, 273 à 275, 277, 279 ou 284 ou d'une adénosine en position 201 de l'une quelconque de SEQ ID NO : 6, 12, 15, 17, 19, 24, 29, 40, 58, 59, 81, 82, 84, 85, 88, 92, 97, 113, 127, 143, 225, 227, 229, 231, 234, 237, 238, 240, 244, 246 à 250, 255, 256 à 258, 260, 262, 263, 265, 267 à 272, 276, 278, 280 à 283, 285 à 292 ; ou
(h) de la présence d'un allèle de risque d'un marqueur génétique de l'alopécie androgénétique **caractérisé en ce que** le marqueur génétique est localisé dans une région chromosomique du chromosome humain 20p11 ; où la région chromosomique du chromosome humain 20p11 est constituée de la région ayant la séquence de SEQ ID NO : 1 à l'extrémité 5' et la séquence de SEQ ID NO : 292 à l'extrémité 3' et où le marqueur génétique est choisi dans le groupe constitué d'un polymorphisme d'un seul nucléotide (SNP), d'un nombre variable de répétitions en tandem (VNTR), de microsatellites ou répétitions courtes en tandem (STR)
et où la présence de ladite molécule d'acide nucléique de (a), dudit marqueur génétique ou dudit allèle de risque indique une alopécie androgénétique ou une prédisposition à une alopécie androgénétique et où la présence de ladite molécule d'acide nucléique de (b) ou dudit allèle de non risque indique l'absence d'alopécie androgénétique ou de prédisposition à une alopécie androgénétique.

2. Procédé selon la revendication 1, dans lequel ladite détermination de la présence dudit marqueur génétique ou de la présence dudit allèle de risque ou de non risque comprend des techniques basées sur la PCR, des techniques basées sur la RFLP ou l'AFLP, des techniques basées sur le séquençage d'ADN, des techniques basées sur l'hybridation, l'analyse d'un polymorphisme de conformation simple brin (SSCA), une électrophorèse sur gel dénaturant à gradient (DGGE), la détection du clivage des mésappariements, l'analyse des hétéroduplex, des techniques basées sur l'extension d'amorces, et des techniques basées sur un test avec des nucléases en 5'.

3. Procédé selon les revendications 1 ou 2, comprenant en outre la détection d'une modification de l'expression d'une molécule d'acide nucléique codant pour un polypeptide possédant une fonction PAX1, où ladite molécule d'acide nucléique comprend :
(a) une molécule d'acide nucléique codant pour un polypeptide ayant la séquence d'acides aminés de SEQ ID NO : 293 ;
(b) une molécule d'acide nucléique ayant la séquence d'ADN de SEQ ID NO : 294 ;
(c) une molécule d'acide nucléique ayant la séquence de SEQ ID NO : 294, où chaque thymidine est remplacée par une uridine ;
où la modification de l'expression comparativement à la quantité d'expression de la molécule d'acide nucléique dans l'échantillon contrôle indique une alopécie androgénétique ou une prédisposition à une alopécie androgénétique.

4. Procédé selon l'une quelconque des revendications 1 à 3, dans lequel l'échantillon est du sang, du sérum, du plasma, du tissu foetal, de la salive, de l'urine, du tissu mucosal, du mucus, du tissu vaginal, du tissu foetal obtenu à partir du vagin, de la peau, des cheveux, des follicules pileux ou d'un autre tissu humain.

5. Procédé selon la revendication 1, dans lequel la région chromosomique du chromosome humain 20p11 comprend au moins l'une de SEQ ID NO : 1 à 292.

6. Procédé selon l'une quelconque des revendications 1 ou 5, dans lequel le marqueur génétique est un SNP choisi dans le groupe constitué des SNP compris dans l'une quelconque de SEQ ID NO : 1 à 292.

7. Procédé selon la revendication 1, dans lequel la molécule d'acide nucléique est de l'ADN génomique.


### SEQUENCE LISTING

<110> Life & Brain GmbH et al.
<120> Androgenetic alopecia
<130> P1425 PCT
<150> EP 08 00 6933.9
   <151> 2008-04-07
<160> 294
<170> PatentIn version 3.4
<210> 1
   <211> 401
   <212> DNA
   <213> homo sapiens
<400> 1
   tgttgctctt cccatagagt tcccgacaca ccaataacct cctacctctc tacccatggg     60
   atttcggact gtttccagta ctccccatca ttgtagagtt tatttctaaa aagcccaccc    120
   acgctataga tgaaagctca aacacagagg ctgagaagtc cagggaccta cagctcataa    180
   atgacagggt caggacctca gcctggtcag ttgattcctg gccaaagttg tcgttggccc    240
   ttttttgaga agctaaatat aactttgctt acaagttcaa aggaaagtga ttcaaaactt    300
   gctccgtcaa atgtcttgat tttaggattc acaggtagaa ataaaatgag tgaaaaagca    360
   ctgaattcat gtgagaatgg gttccaaaaa atatagcaga t                        401
<210> 2
   <211> 401
   <212> DNA
   <213> homo sapiens
<400> 2
   tgaatattgg ataggcagtt agcatggttt gtcatgttcc gcaatttctt ttgctttggg 60
   agtcaaattt ctgaacacag aagatatttc tagcaatgtt aatagtgttt caggaactta 120
   tcatacttca ttttttaaaa tgaaaatctc actttgacag tggttttgag aattggatga 180
   ggcacatagg tgaaggacct gtaagacttc cctgacgtag tgactcaaat gatgtcaaac 240
   attcatcatc atgacttctg aaaaactgta ccactttcca gcttttgttt ccacttttcc 300
   cctttaaaaa atttgtagaa gtttaagagg tacaagtaca gttttgttcc atggctgtac 360
   tgcatagtgg tgaagtctgg actgttagtg taccccccac t 401
<210> 3
   <211> 401
   <212> DNA
   <213> homo sapiens
<400> 3
   gtgaaggacc tggccctggc tcagcaaggt tattgcattt aatcttcaca acaggtatta 60
   ttatcatttt ataggtaagg cagctctatt tttcttaggg ctagctctac aaataatttt 120
   ttttgtctta gaacaagctg atgtccaaaa tttgcttttt atggaaaatc tgtcgttgta 180
   aaaatatgac tctggagaca cattttctga gagcagtaaa taccagaagc ccagccaaaa 240
   aacttcagca ccctgttgag attgggaaaa ggcaacattg acttattttc acttaagttt 300
   gtgacttaga aaaaaactaa cagtgatggt tttcttcttc tctgttaggc ttgtggctgc 360
   aaattctttg catggccgtt tatttcactg aagcgaatga t 401
<210> 4
   <211> 401
   <212> DNA
   <213> homo sapiens
<220>
   <221> variation
   <222> 31
   <223> /replace="t"
<220>
   <221> variation
   <222> 285
   <223> /replace="t"
<220>
   <221> variation
   <222> 349
   <223> /replace="g"
<400> 4
   tcttccagaa aactgcctgc aagcaatttc cttaatcggg ttgctaaggg caaccccaaa      60
   gaaacaacat accaagctat ctgtctgtcc atccgaatcc caaaagcttt ccttgtgctt     120
   cctgctctga ccaaagaggc atttgaggca catgtgagag aacagctttc cagaaaccac     180
   acttgagtcc acagcagagc ggaaggggca ggaggcatca ggaccatttc cagatttctc     240
   tctggccctg attgtctaga tttgcatcca atcactgcaa gagtgtatac aaaagttgtt     300
   agaaagattt gggtttcaca cagagttatg aaaacaaggt agaagcctca tgtcttcatt     360
   tcccatgaaa tcttgtgggt gtaaatgaaa catgttttag t                         401
<210> 5
   <211> 401
   <212> DNA
   <213> homo sapiens
<400> 5
   cagaactggg aaaaagcaaa caataaatgt ccattgttta agccctccag tctgtggtat      60
   tttgttacgg cagcctgcgc taagacacca gccaattttg tttctgtttt ccctccctga     120
   agtcagtgag gatgggagct ctctaggtag cagtgtgagg gtctgaggga gaccaccgag     180
   ggacatgggt ggagagggac ggtcatgggt ctgagaggcc agcaggggcc agatcatgcc     240
   tgctttgtag gacacagtga ggggctgata cttcaagatc tgtgggaatg ggagcgggag     300
   cccagggagg gctgcaggga aggacttggc tctggctgct gtggagggag agcaggtggt     360
   gggaaggcag ggcaaaaagt gaggagtcca caagagacac t                         401
<210> 6
   <211> 401
   <212> DNA
   <213> homo sapiens
<220>
   <221> variation
   <222> 145
   <223> /replace="t"
<400> 6
   ttaggggagt tttgtgaact tacttcatgt gccattgaat ttctaatagg tgatatctaa      60
   ttttaaattg tagtttagag aaaaaatgtt gctattctat tgctgaaaca taattgatat     120
   ataaggagag tttggctttg aatacatttc tttctctgta tatatttatt ttagatttca     180
   tgagcgagta tttgattctt taaatattga ggacattgca aaacatgatt ttactggatt     240
   tttcagaatc gtaaagaaga ataattcttt tatagtttag taactatcta aaataattta     300
   gacacttttg accttatttt cttgatgttt ctttgagatg aggaggtagt aattgcaatt     360
   tcactttttc tattgaataa aaaactaaat gaagtgtatg a                         401
<210> 7
   <211> 401
   <212> DNA
   <213> homo sapiens
<220>
   <221> variation
   <222> 340
   <223> /replace="c"
<400> 7
   tttttcttga gcaaggtatg tataagtaag taaataaggg cataatgtca ggtaaactga    60
   atggctggaa agtggcaacc aggatttagt tatttggcat tatcacaggt ttttcagaaa   120
   cattcaattt ttagcaaaaa acaagtcccc tccaaaattc aatctactgc aattgatttg   180
   cacgtggttt aatgctgtta ctagtctata aataatggac tcaaatattt gaagttgtag   240
   atgctttcat actgttttaa acaaacataa cttatatgca aatggttaag aataccagaa   300
   acattgcaag aatgctcttt ttaaatcctg aagatattgg tttattttaa agcatcaagg   360
   atgatttaga tttttggaaa aacagaggtg gcatgaagat t                       401
<210> 8
   <211> 401
   <212> DNA
   <213> homo sapiens
<400> 8
   taggcgctga tttttgaaaa tgcaatcttt atccagagta tttgacaaat gctcagaatg    60
   ctcagaaatg tattaatata cagccagaca gctgtcaaag cagcaggctt ctcccctgga   120
   gagttcatta acctcatttc cacttgctga gtaaggagct ctgtcttgcc tgagagggac   180
   cacggtacca aaatgtacgg gtttcaagtg aggagtgaga aaaataactg aagtatgaga   240
   aaataaagat ttcttgataa ggcccaaaga ctagctcatt tcctagtatg tgttttaaat   300
   gcgcgttttc tgtttatttt acttacctat gcatttatct attcatctct tttcaaaatg   360
   ctttgaaggt gcaatgatga tatgctattt tatgaactaa t                       401
<210> 9
   <211> 401
   <212> DNA
   <213> homo sapiens
<220>
   <221> variation
   <222> 275
   <223> /replace="t"
<220>
   <221> variation
   <222> 398
   <223> /replace="t"
<400> 9
   tgaatgtgtg tgtgtatgtg taagtgcacg tgcacatgaa tgactacgta tgtttatgca      60
   cttgtgcatg catatacatg tacgtgtgct tgtatgcatg ggtgtgcatt atgtgcgtgt     120
   ttgcctgtgt gtgcatgcat gcgtgtgtgt gtgtgtgtgt tctctgtgga gggaggagac     180
   tggagattct cttccccaac gttcgaggcc agtttgccaa aaatcttggt gctaactgta     240
   tgtccaggtt acagatccac acgcaaatgc agacccaaca acactgaggc tcatcttttg     300
   ggtttctccc catcttcttc accatgactc agttctccaa gaaaaatgtt gttgctactt     360
   tcaggaacag tccaggatga gagcttaagg tagagtccgg a                         401
<210> 10
   <211> 401
   <212> DNA
   <213> homo sapiens
<220>
   <221> variation
   <222> 266
   <223> /replace="t"
<220>
   <221> variation
   <222> 341
   <223> /replace="t"
<400> 10
   gttcacaagg caaataaata acagatgttt gagattatgg atatgctaat tacccttacc     60
   tgatcactat gcattatatg tatcgaaaca tcactatgta ccccatgaat atgtcaatta    120
   aaaaaatagt ttcaccaaga cccactaaat caacagaatt ttgtggcaac attatgcaga    180
   ccccactgtt gagagtaaac gctatttgat atgatatcaa gttccatcag tttgcatcag    240
   atcatcagat gagagggcca taagcgtctt ctgttctcat aattatgcag atttattgtt    300
   ttgaaacaaa atcaacttct ctctaaaata agcaaggaca ctggggcaac caactattct    360
   cagagaagga gtgtttttaa tttttttcag gattcttagg a                        401
<210> 11
   <211> 401
   <212> DNA
   <213> homo sapiens
<220>
   <221> variation
   <222> 50
   <223> /replace="t"
<220>
   <221> variation
   <222> 105
   <223> /replace="g"
<220>
   <221> variation
   <222> 233
   <223> /replace="t"
<400> 11
   taatacacac acactagaaa cctaattggt tttgaatctt gagcaattgc ttagaaatgt      60
   tcatatgatg aaataaaatg ctttgtattg caaactttta tttaagaatg agagcttctt     120
   aattccgggt cgttggacaa tatccaaagt caaatggtag agacatatga aagatggtta     180
   gtaattaatc acgcctcacc ggtgtgccag gaaagtaaac tcaccaggag ccactctcag     240
   tgccttatca aattactaaa acttgacttg ctttcctgga ttagtaaagt gatacactgt     300
   tgattctgga aattcgtaac ttaagatgat tcctctgcat agtattcagc aaaccataag     360
   atggtatttt gataattttg agtttcatca aaatacagat t                         401
<210> 12
   <211> 401
   <212> DNA
   <213> homo sapiens
<220>
   <221> variation
   <222> 18
   <223> /replace="t"
<220>
   <221> variation
   <222> 73
   <223> /replace="g"
<220>
   <221> variation
   <222> 169
   <223> /replace="g"
<400> 12
   tgaatcttga gcaattgctt agaaatgttc atatgatgaa ataaaatgct ttgtattgca      60
   aacttttatt taagaatgag agcttcttaa ttccgggtcg ttggacaata tccaaagtca     120
   aatggtagag acatatgaaa gatggttagt aattaatcac gcctcaccag tgtgccagga     180
   aagtaaactc accaggagcc tctctcagtg ccttatcaaa ttactaaaac ttgacttgct     240
   ttcctggatt agtaaagtga tacactgttg attctggaaa ttcgtaactt aagatgattc     300
   ctctgcatag tattcagcaa accataagat ggtattttga taattttgag tttcatcaaa     360
   atacagatta ttatcctgcg tgagtccact gtaatcttag c                         401
<210> 13
   <211> 401
   <212> DNA
   <213> homo sapiens
<400> 13
   aggaatgagt tcttgatgac tccatgtgtg tcaacaagca gtttacgact tctcctttaa 60
   aacagaaaac gaacaagagt gtagttttga aaagtaaatt tatttattta tttattttta 120
   cttaggctta tataattttc tgttctgtaa gagagatctt ttagggccca gggaattatt 180
   ccataccttg gggacccggg gaaagactgc gtacctggcc aagactgatg atcaaccaag 240
   tgggatgctt tatttcctct ttagttagaa gtttcaggat caaagtgagc ctttgagcta 300
   tttgtcattt tccctctaaa tatttttgtc acacacacac aatttgtata gtggcttctt 360
   cagaagcctg tggcaatcct tcctggctgt gaaaacagat g 401
**<210>** 14
   <211> 401
   <212> DNA
   <213> homo sapiens
<220>
   <221> variation
   <222> 348
   <223> /replace="g"
<400> 14
   aacaacttct ggatatggat aatgttaatt cttgtccatg atacaattgg agaaattgag 60
   gcttaaagag atgaagtcac ttttcaagtg ctacatagat gctaagtgat aaagaagaaa 120
   ttcaaaccca ggactcttga ttgcaggccc ccagctctta tcacagacac tatacaaaca 180
   ggaatacttt gtgttaccct gtgttaatgg ttctcttatt ttgctgtgca tcagaatacc 240
   ctggaaggat gactaacata cagattgcca ggctcagcac ctgctgtgtc taagccagga 300
   tatctagggg catttctagt aagttccagg taatgctgat gctgctgatt tagggacaac 360
   actttgagaa atatggctgt gtattatggg tctatttgaa c 401
<210> 15
   <211> 401
   <212> DNA
   <213> homo sapiens
<400> 15
   ccctgaggcc ctcagagtca tacacagagg aagagcttgg gtcagacttt ggccttttct 60
   gcttgtttca tcaaaacaga aaccacaagc tttatcacag cctcaaaatg cattttctct 120
   cttctttggg gttaaaaaca aatttttctt caaagaaagc atattgagat ttttaagtca 180
   agttcacaat taaaaaaaaa ttttcttgaa agatgtgtgt attgtcttaa catgggttcc 240
   ctttgttaac acctcccagt ggatttattt ggcatgaggg atctaggggc aggaccctgg 300
   acttcttctt agacaaccta acctctttgg tccagaacta tctcaaggtt ctgctaccaa 360
   catgttagaa gcaaaaggcc caggaaacaa cccagtcact t 401
<210> 16
   <211> 401
   <212> DNA
   <213> homo sapiens
<220>
   <221> variation
   <222> 378
   <223> /replace="c"
<400> 16
   gaattgaata accatcataa ggaaacaata aaacatgaag tgactgagac cagatgatca 60
   ccactgggca cacgtggtct ctgtagcagg ctcagggagc ccagggttgg ctttcagttt 120
   cctgcaacac agagcagtcc cttcagcact ctgccaataa ggatcccatc ggttaatgag 180
   acgagcttca actaactggc ggaggctcgg agggtggggc acccacgtta cattttctcc 240
   ttgcccacaa ctctttgaat tttagtctgg ttgtcaattg ttctagcatc tcttccccct 300
   agtgtgctgt ttctcccctc ataaaaacag ctacagagct gaggtctgaa atatctggag 360
   tatataaaga tggtgaggat tggaggggtt catggtgctc t 401
<210> 17
   <211> 401
   <212> DNA
   <213> homo sapiens
<220>
   <221> variation
   <222> 319
   <223> /replace="g"
<400> 17
   cttgagtttt aaaagttgtc agaagaattc tgagaagatg atagagtagg aaataccaga 60
   aatccctctc cccatctaga caataatagc attggcaata tctgtctgac atgattattt 120
   tgggattctg gagtctattg aatatttgca acttctaggg gaaggcttgg atggtaaatt 180
   gtttaatttt gatcattttc tgctcctagc acagtagctg ctacctatct ttcacctctg 240
   gaccctcctc tcttgagcag gcagcatgca tgtgttcctg gagaagtttg cacacagctt 300
   gttggaggca gggtgggcaa aaggatcctg ccctccaaat atcagggatt tgtactctga 360
   ctgctgatgg ctgcttctga tcacaggggt gctggcaatg a 401
<210> 18
   <211> 401
   <212> DNA
   <213> homo sapiens
<220>
   <221> variation
   <222> 265
   <223> /replace="t"
<220>
   <221> variation
   <222> 361
   <223> /replace="g"
<220>
   <221> variation
   <222> 366
   <223> /replace="t"
<400> 18
   acagattaaa tccctatttg aaataaaagt agctgtttac tcatgtaaat gcattgtttt     60
   tccttgacta cttaaaggca aacagattaa aggacatgga ttgtctaaag aggctttggt    120
   gaagagtatg ttgaaattcc tttttttttc cctactgcaa tccttttcac caattcattt    180
   aggctttcta tggcttttaa gagatgagag agaaaagttt ttgtttctaa ttaacaaggt    240
   ttcacaagca ggcagaggca ttacagtttc catttaaaaa gaaagaaaac ctagaaaaat    300
   ggtgacatgt tcctccaccg gcaggatggg ggtaagggac aactttgggc atgccaagat    360
   actttcttca ctgagagatt tattttaaaa tattacttta t                        401
<210> 19
   <211> 401
   <212> DNA
   <213> homo sapiens
<220>
   <221> variation
   <222> 137
   <223> /replace="g"
<220>
   <221> variation
   <222> 297
   <223> /replace="g"
<220>
   <221> variation
   <222> 302
   <223> /replace="t"
<400> 19
   tgactactta aaggcaaaca gattaaagga catggattgt ctaaagaggc tttggtgaag     60
   agtatgttga aattcctttt tttttcccta ctgcaatcct tttcaccaat tcatttaggc    120
   tttctatggc ttttaaaaga tgagagagaa aagtttttgt ttctaattaa caaggtttca    180
   caagcaggca gaggcattac tgtttccatt taaaaagaaa gaaaacctag aaaaatggtg    240
   acatgttcct ccaccggcag gatgggggta agggacaact ttgggcatgc caagatactt    300
   tcttcactga gagatttatt ttaaaatatt actttataag caaaagggca atctatcatt    360
   ggattttcca acaccatgtt ggtgttttaa ttaaaatagc a                        401
<210> 20
   <211> 401
   <212> DNA
   <213> homo sapiens
<220>
   <221> variation
   <222> 378
   <223> /replace="g"
<400> 20
   ttgcctccaa gttctggcaa ttatgagtaa agctcctgta aacattaatg tgcaggtttt    60
   tgtgtggact taagttttca actcctctgg gtaaatacca ggagcaccac tgctagattg   120
   tatggtaaga ctatgtttac ctttgtaaga aactgccaaa ctctcttgca aagtgatggt   180
   attattttac atttctataa gcaatagatg agggttcttg ttcctcttca tcttgccagc   240
   atttggtgta gccagtgtct tggatttttt tccacagaat gatattttta catatccctg   300
   aacaagggat gatgtttaaa attcttagca atgagtatca ccagggcagg agctcacaga   360
   acagatgtgg atattcaagt gaagattggc tgatcactgg c                       401
<210> 21
   <211> 401
   <212> DNA
   <213> homo sapiens
<220>
   <221> variation
   <222> 23
   <223> /replace="t"
<220>
   <221> variation
   <222> 27
   <223> /replace="g"
<220>
   <221> variation
   <222> 282
   <223> /replace="g"
<220>
   <221> variation
   <222> 371
   <223> /replace="t"
<400> 21
   agtgctgctt gatgcctatt acctttatgt aaagacacag ccagctcacg tgagctctta      60
   ggaaggggga tagagaaata aataccctga cctcatcttc ctgccctctg ggctgctggc     120
   ctccaaacca gaagcagggc actggagctt accaacatgg tacacaaagg ctggcatcct     180
   ggagctcaga gcagaggaga ggaggtcaga cagtggatct agaggggcag acaaaaatat     240
   ccagaacaag tttcactctc catgggcctg aactagtggt tacaattgct ggaaggtaga     300
   aagtgttagg gggtgttcag gtatgggatc cagatgaagt gggagagaat tttagtatgc     360
   tttgtgaaga cttcccatga tttgaaattg tgtaaggact g                         401
<210> 22
   <211> 401
   <212> DNA
   <213> homo sapiens
<400> 22
   ccagagttcc catgattctg cacagaggaa ggttctcata gtaaggatct gatgctgtgt      60
   ctgcaggctt ggcttggcca tgggagtatg ctctgtccat atgcagaagt cactcctgga     120
   gcagctctca accagtgact gagagataaa taacctggct tcctcacccc tcaggtgagc     180
   tgtgttccac acagtctacc ggattcctcc agggggatcg agcctcagct gctcaattat     240
   tacctcagta ataacttact aattaatgca cctggtgttc actcctcttc tccatttcac     300
   ctctccgttt acctgcgtgc ttcctgggat tgcatctcaa aatgaactaa ggaagtgaaa     360
   tccttacctt agagcggaat tctgagggaa acccaaacta a                         401
<210> 23
   <211> 401
   <212> DNA
   <213> homo sapiens
<220>
   <221> variation
   <222> 173
   <223> /replace="t"
<220>
   <221> variation
   <222> 240
   <223> /replace="t"
<220>
   <221> variation
   <222> 280
   <223> /replace="g"
<400> 23
   tcctttgtaa gtcttttgct cattcatcac caaggttaga agtcatttct ctgtgaaccc 60
   ttccctatca gagcaggtct gagcatgtgg tgtccgttca gtgggggtaa tgtgtgggca 120
   gctgaagtct gattcgtacc ttccagacca aaagaaaggc actggttcca gcgtagtgtc 180
   ccatagcaga aacatggctc ggagctcagt ttaaaggtgt tccttgaatg aggccaatcc 240
   tttttggggg aggggaaggg gaaggagggt ctcccaaccc ccgccttttt tatttgagaa 300
   agaatctcat tctgtcaccc aggctggagt gaagtggtgc gatctcaact cactgcaacc 360
   tccacctcct gggttcaaga gattctcctg cctcagcctc c 401
<210> 24
   <211> 401
   <212> DNA
   <213> homo sapiens
<400> 24
   gtaatcactg tgatctggga gatgcgtttg gactggaccg gccagtgagg ggcctaccta 60
   cctgcctaga tgaaaactat cacttgcaca gaaatacatg gaaagatgaa gaggcataaa 120
   gttaatggag attaattgta ccagctttgt aggagcacag gaataataac catttatgga 180
   acaaatattt ctatgtattt tttttcaaac tgtaattaag ggtaccattg tccccaattc 240
   tttacccctc cctttatctc tatcctttgc caagtgactt gcagtccccc acctctatgg 300
   gaggagtata tgtcctttat ccttgactct gggcgtgacc ttgtgatttg ctttggccaa 360
   tgacaccaag gggacagtga cagtgttcca gttctgatcc t 401
<210> 25
   <211> 401
   <212> DNA
   <213> homo sapiens
<220>
   <221> variation
   <222> 74
   <223> /replace="t"
<220>
   <221> variation
   <222> 232
   <223> /replace="g"
<400> 25
   ttttatgtat caacaattta agttctactg ctttaagatt atatggacat taaatgtaca      60
   gaaaaataga aaccaacaaa agataaataa attaaataca tttttaaaaa agtctcgcga     120
   gttgtggtgg cttaatattg ccgtgagcta atgccttgag ggctactctc tctggagtgg     180
   gaatggagct ctcagtaatt cgacagtttt ggccagtggc ttgtcacacg gattgtgcat     240
   tcacctggct ttaggaggaa gaggaggcat ggcagccctg ccttcatctt cctctttact     300
   ttgccctcac tatcactgtt aacttcgatt catggatgct tttcagggat ttttttttct      360
   aagccaccta gccaattcga ggggtttagt gtacttaaaa c                         401
<210> 26
   <211> 401
   <212> DNA
   <213> homo sapiens
<220>
   <221> variation
   <222> 237
   <223> /replace="c"
<400> 26
   ggattttttt ttctaagcca cctagccaat tcgaggggtt tagtgtactt aaaaccagat      60
   agtataatcc ataaacccac taaattgggc acatgtgagc tgcaatatgt cacaagatgc     120
   agaaaaccag ccctgagtga gggcactgcc ttccgcagct gtcctctgga tctttcctgt     180
   aatgtcagtg acacatgacc gctggcatgc agcctgctaa gggacggtgt ccatctgcat     240
   atcaaccaag acaaaagcct ggctcatcat ttatttagag aaagcaaaca taaatggaag     300
   aaattatatt ttcctcctat tccatagttt atcatgtctt gccccacctg ggtagtgtca     360
   cttgtcctga agatggtagg agtgagggaa aagaggtgag c                         401
<210> 27
   <211> 401
   <212> DNA
   <213> homo sapiens
<220>
   <221> variation
   <222> 89
   <223> /replace="g"
<220>
   <221> variation
   <222> 308
   <223> /replace="c"
<400> 27
   aatggatgta tggagatgtt tctctccagc aaagataagg aaatggctgg aagactgcga      60
   aagaatagat gtgccacttt ggaagttgac ataaaaatga atgatatttt tactttttgg     120
   aaagccaagc aggtcccaga gtcgtggggc agtacttggc ctcatctctg aggccaccca     180
   ttctgctgct ggcagccaca gtgatgtacc cttggcacta tgtcgctccc cctacttggc     240
   atcctcagtt tttctaaact catgggtgag agacgaatga cgtcagaggc ccaaaccttt     300
   ggatgctgat ctaaaagcat caaccaaaac ccaacacccg agcttcgtaa gttcccattt     360
   gaagcaggag cagtttttat agcaacagac aaaggattgc t                         401
<210> 28
   <211> 401
   <212> DNA
   <213> homo sapiens
<220>
   <221> variation
   <222> 24
   <223> /replace="c" /replace="g" /replace="t"
<220>
   <221> variation
   <222> 153
   <223> /replace="g"
<400> 28
   ctaagacagt cttttacaat ctaacaaaaa cctttctgct gtggaagttc tgtggaaagg      60
   gtcacagatc atgtgcagtc acacaacact ctttggatta tcactgtcat gaaggagcaa     120
   tgggggagac caactcctcc acgcagggct caagatgtgt gcatggtagg ccaagagggc     180
   aagtgggtgg ggaggtggaa ggggtcagga aggaggcaca gacaccgtca aagcttaagg     240
   agaacggacc gttgacacat ggaaatggat actgtgacac ttagatttat cgtcaggcaa     300
   actgttcacc acaaacccaa agttcctcta aacaatgcct aggaaaatct tccagaagtt     360
   ttgaggatct ggtgaccata ttttcagaat caaaagttgg g                         401
<210> 29
   <211> 401
   <212> DNA
   <213> homo sapiens
<220>
   <221> variation
   <222> 137
   <223> /replace="c"
<400> 29
   taggtaaata tttacaaact gaaggtcaaa cacaaatgtc tactttacaa ctactatact      60
   gcattcataa tttctctcct cattttatac tctgatgaaa taccaacttg gtcatttctg     120
   tgcattgtca agagggtcga agtcgaagca gaagcaagca gctggctggc ttagcttatg 180
   gttcctctgg cacaggaaag tggtgagagt cattattctt attatccagg agttaacgga 240
   tttttgcatg actgggctga gtgtatgttt attttctggc ccttgataat tactgtattc 300
   ccattttcaa tatgaatttg tttctatcca atggatggtc aacagtagcc actgtaattc 360
   tttttgaggg gctatatatg caccattatt tttcaaactt g 401
<210> 30
   <211> 401
   <212> DNA
   <213> homo sapiens
<220>
   <221> variation
   <222> 93
   <223> /replace="t"
<220>
   <221> variation
   <222> 260
   <223> /replace="g"
<220>
   <221> variation
   <222> 274
   <223> /replace="t"
<400> 30
   ttacataaag tcaggctgac atgggttcaa attctggctc ttcacaataa ccctataagg 60
   caaatactat tcccgtttta cagctgaaaa acctgaagtc aggaggtcag gctaagttaa 120
   tatgtctatg gtcttatagc tagtgggtgg gagatgagaa tgaatccagt ctggctgaag 180
   agtccatact tctggcctcc gtcatagcct gggcttccca aagcttgtgc ttttgttcct 240
   tattggcaac aggccatcaa ctgcattcgt tacgtaaggt tattatgagg agtaagggag 300
   ttattaaaag tgtctagcat ggttcctagg acatagtagg tactcaaata tttttccctt 360
   tctgttacta ttaacacttt ctttataaaa taaaaattgg c 401
<210> 31
   <211> 401
   <212> DNA
   <213> homo sapiens
<400> 31
   ctaacactga gaaactccag acagatcctg aagttaacaa aacatgaatc tattatctac 60
   ttaaaatgac tgcaataaag gcttcttgat ctataactct agttttgcat gaagatcaac 120
   aagctttcac atggtaggtg gagtaaatgt gaaggtgaac agtgtcagat gtgaaagata 180
   cagtgggatt cttgccatac gctcttcagt cttaaacctt ctgcaggtga tccaactaca 240
   ccccaggaat taagaagcag attataaacc tcaggccttt aaagggcaaa aaggtctgaa 300
   ttagtccaaa agttcagggc aaaactctaa atgactttta gttacaacgg aaacccactt 360
   ggaaagttgc ttttcttctc caatagcttc ccaagaaagg g 401
<210> 32
   <211> 401
   <212> DNA
   <213> homo sapiens
<220>
   <221> variation
   <222> 19
   <223> /replace="t"
<220>
   <221> variation
   <222> 70
   <223> /replace="t"
<220>
   <221> variation
   <222> 115
   <223> /replace="t"
<220>
   <221> variation
   <222> 126
   <223> /replace="t"
<220>
   <221> variation
   <222> 147
   <223> /replace="t"
<220>
   <221> variation
   <222> 223
   <223> /replace="c"
<220>
   <221> variation
   <222> 340
   <223> /replace="g"
<220>
   <221> variation
   <222> 360
   <223> /replace="t"
<220>
   <221> variation
   <222> 384
   <223> /replace="g"
<400> 32
   taataataat agtgaaggcc tggactgggt agaggaagaa actgatccaa aacaaacgtg 60
   caatgaggcc tcagccaatt cctggggggc agatctggct gtgtacagca gttcctgaat 120
   aatgtcggtt cattcgatgt tctttcctta taacgttgat gagaaaacaa ttgatttgca 180
   cctggggcca ccatctgtgt gtatttgcac aacctcccca taactgcatg ggttttctct 240
   gagtactcta gtttgctctc tcattccaaa gtcatgcaca ttaggttaat tggcatgtct 300
   acatggactc agtgtgagtg aatgtgagca tgggtgtgaa tgtgctctgc catgagagac 360
   tgtcctgttc agggctggtt cccacctcac tccgtgagct g 401
<210> 33
   <211> 401
   <212> DNA
   <213> homo sapiens
<220>
   <221> variation
   <222> 8
   <223> /replace="t"
<220>
   <221> variation
   <222> 62
   <223> /replace="g"
<220>
   <221> variation
   <222> 84
   <223> /replace="c"
<220>
   <221> variation
   <222> 221
   <223> /replace="t"
<220>
   <221> variation
   <222> 245
   <223> /replace="g"
<400> 33
   ttctttcctt ataacgttga tgagaaaaca attgatttgc acctggggcc accatctgtg 60
   tatatttgca caacctcccc ataactgcat gggttttctc tgagtactct agtttgctct 120
   ctcattccaa agtcatgcac attaggttaa ttggcatgtc tacatggact cagtgtgagt 180
   gaatgtgagc atgggtgtga gtgtgctctg ccatgagaga ctgtcctgtt cagggctggt 240
   tcccacctca ctccgtgagc tgctgggatg ggctctggcc acccatgacc atgaactgga 300
   aataagcagg taaagaatta tcttacttgg ttttattaat ctttcttaaa tgtatgtata 360
   gttcacattt atttcaatgt ttaatattag aaatgttttg c 401
<210> 34
   <211> 401
   <212> DNA
   <213> homo sapiens
<220>
   <221> variation
   <222> 45
   <223> /replace="t"
<400> 34
   gtgtcattag aggtacagaa acacactttt tttttctatc tgtggtgtct caaattgtta 60
   tggtggtgtt attttcttct aaatgtcttt ttaaggaaag aaaaataaaa atttaaatta 120
   ttagcaacga tttttgccat tcatctttat attatgcatt gctaatttta aacacaagtg 180
   tgtttaatta acttgtatgg cgactcacca aaatatacat tttgtatttt attactcatg 240
   cgtgtatagg tttttgttct aacaagaaga gtaaaaacac tgcacaaaac taactcaaat 300
   gattttattt cacttattga tatgtgcata ttctgccaat gttctctacc tttggcatac 360
   tggtgactaa cagagaactg aaaggaaatg taactatctg a 401
<210> 35
   <211> 401
   <212> DNA
   <213> homo sapiens
<220>
   <221> variation
   <222> 50
   <223> /replace="t"
<220>
   <221> variation
   <222> 195
   <223> /replace="c"
<220>
   <221> variation
   <222> 259
   <223> /replace="c"
<400> 35
   aaaaaagttt caaaggcagt ttttaaaatg cttagttaaa ggctaacatc agtatagttt      60
   aaaaggtaat tgatctatag atgaactaac aactaccaac agcatctctt ttgtcagtct     120
   atagctgaca gatccctcct tcttatgcct acaagtctga catctactga agtttcatag     180
   attggctgaa agcagtagtc cggaaaactt tagtcccagt tccagagatt ttcattctct     240
   ataccatcag ctctttggaa actggtgcct tttaggctct gaattgacat gggttcaagg     300
   gcatctcact ctgttgacct tgtaccttga atttcctcaa cattttcttt gtgatttgat     360
   atcaatatta gttctctagc tacctgcatt acaatagtcc t                         401
<210> 36
   <211> 401
   <212> DNA
   <213> homo sapiens
<400> 36
   atatagggtc ctccatagta tttgcaaata attttaaaat ttccatattt gtaatttgcc      60
   attttaattt ttctaatggt gttaacttat atcttctctt tttattttaa aatttgtatt     120
   gatacataat atttatacat atttatggta ttcatgtgat attttgttac atgcttaaaa     180
   tgtgtaatga tcaagtcatg gtttttaggg tatccattac ctcatgtatt tatcattttt     240
   atgtattgtg aacatttcaa gtcctctttt ctggtatttt gaagtatgaa tatagtgttg     300
   ttaactatag ttaccctact ctgatatcaa acattagaac ttatttcttc tatctaactg     360
   tatgttcgtg cccattaacc aacctcccac cacacaccct t                         401
<210> 37
   <211> 401
   <212> DNA
   <213> homo sapiens
<220>
   <221> variation
   <222> 28
   <223> /replace="t"
<220>
   <221> variation
   <222> 80
   <223> /replace="t"
<220>
   <221> variation
   <222> 400
   <223> /replace="g"
<400> 37
   cctggatgtc taagtctctt gctggacctg agaagttttc agctattatt ttgttaagta      60
   gattttctat gcctttgacc ttctcttcat tttctgtaac attcaaaaat caaatattgg     120
   tcactttatg gtatcccata tgctatctag gcattattcg ttctttttta tttagttttt     180
   aattcatttt tgtctgattt gatgatttca aaagacctgt cttcaagttg tatgattttt     240
   tcttctgttc gatcttatct cttgttgaag actttgtatt tttaatttga ttcaagaaat     300
   tccttagttt caggattttt ctctggttct tttttataat acctatctct ttggtaaatt     360
   ttcattaata tcttgtgttg ctttttctga cttctttgta t                         401
<210> 38
   <211> 401
   <212> DNA
   <213> homo sapiens
<220>
   <221> variation
   <222> 160
   <223> /replace="t"
<400> 38
   atggtgtgct gtagctgctt aaagcccaca gcttgtgtgg gactcagcag gaacttctgt      60
   tctgatgtaa tgctatcgtg tgatgcccag acatcttctt ctgttactct cagggcttga     120
   gagggtagat gggctctctc atggctagga ttgctagagc tgcagtggaa atgtggacca     180
   ctggggctct ctcacttata ctttctccac ttggggagct cctctgggat cccagcttat     240
   cccagctgag caggctactt cactttgctc ctctaccttg ccttaggtgt tccctgtcac     300
   ttctctgttg aattatagtg ttctttctta gatgatctat tcaaagtgtg gttatctact     360
   cactattttt gtttttctct gtgcaggagg taccagatgc c                         401
<210> 39
   <211> 401
   <212> DNA
   <213> homo sapiens
<220>
   <221> variation
   <222> 274
   <223> /replace="c"
<400> 39
   agcaggctac ttcactttgc tcctctacct tgccttaggt gttccctgtc acttctctgt      60
   tgaattatag tgttctttct tagatgatct attcaaagtg tggttatcta ctcactattt     120
   ttgtttttct ctgtgcagga ggtaccagat gcctctagtc agccgtctta tagcccctcc 180
   tatatcgttt taatttttgt cttggtcaaa cttgcaaaag gttgtctact ttgttgatgt 240
   taaaattttt tcgttttatc gaattttgat tttgtaattc acacttcagg cttttatatt 300
   tatccattct gtccttgtag cttagttgga tttattttgt tgttacttcc tgtgcttctt 360
   tgtttgacgt attagtttac ttatttacat ccccaccagc a 401
<210> 40
   <211> 401
   <212> DNA
   <213> homo sapiens
<220>
   <221> variation
   <222> 247
   <223> /replace="g"
<220>
   <221> variation
   <222> 290
   <223> /replace="g"
<220>
   <221> variation
   <222> 339
   <223> /replace="g"
<220>
   <221> variation
   <222> 400
   <223> /replace="c"
<400> 40
   ttgagttgtt gggtaccaga actgataggt gtcatgtatt taagcccatt tgaaaaatac 60
   atttcaataa tggagtgaga ctctgtctca aaaaaatttc aaatgtcttt tctcaattga 120
   aaaaacctat tatttctaga tagataagta aataaccttt gcaaactcga gccacacttt 180
   tcccctttct tttaaaattc tgcttaatat aattgaaaac tgaagtctga ctctccaagc 240
   ctataaagga taaaagagag acaggataga tagaaataac tggcaacgaa gagttactgg 300
   gctttggctc catgggagac aatagtggtt tcatagaaag caatggtgga ctcttagtca 360
   actgaagctt gggtggaact tagctgcata attaagaaga t 401
<210> 41
   <211> 401
   <212> DNA
   <213> homo sapiens
<220>
   <221> variation
   <222> 121
   <223> /replace="g"
<400> 41
   gtttctggcc tgtgcttcta caaaacacac agcacatatt ttctaaccat aattgaaacc 60
   attttaaaat agtgtttttg gaggagtgaa tctatgtgaa acttctgtaa gctatttgta 120
   agacagccgt gaaaatcctc catgttctat gcattggatt ttttttcctc cttactcaca 180
   aggatcatct tcaggttaat gtcacagaaa ttgggacaac ttagtgttcg agaaatagag 240
   aaatagctca aggagctatt agataccaac ccataccatg ttgttttctg agggtgggag 300
   aaactagaag aaagtttgac attactctca ttccatgaga caacctcaag aacacaaacc 360
   caaactatcc aggtaggact tttagtttag ggagaccacc a 401
<210> 42
   <211> 401
   <212> DNA
   <213> homo sapiens
<400> 42
   ttaagatgac gtatcctgta ggatggacta aataatgctg cagcaaggaa tacttcccaa 60
   attgtcatgg cttgcgataa gtcttatgtt tttctcatgt tttatgtcca ttatggatct 120
   tgccctggta cccagcctga tgcagcagct gctctctgga tcatgcctca tcgctgtggc 180
   agagcttatt gcaaatgagt gactctacct gggagtgaca cataactcca ctcacaattc 240
   attgtccaaa ataagtcaat gaccacacct aacttcagtg ggacaaagaa acactatcct 300
   actctgtgct tagaaggaag aaagatgaaa atatttggtg atggctctat gactaccatg 360
   gatagctttt attttaatat tgtacattaa tagcgctctc t 401
<210> 43
   <211> 401
   <212> DNA
   <213> homo sapiens
<220>
   <221> variation
   <222> 103
   <223> /replace="g"
<220>
   <221> variation
   <222> 263
   <223> /replace="t"
<400> 43
   gagtatgcaa gtacatgcat gtgtgtactt cgcacactgt tatctatttt aggctcaaat 60
   agatgtatct atggttgaga aattcccatt aaatattggg gcaaggttca gatgctgtag 120
   atatttgttt acattttatc aaatgctttc ttacatgtga acccctcaat ctctgagaaa 180
   tcacctaagg gaagccatgg gtctgcctcc tccaggtttc attattgcat ggaatgtgag 240
   gggatttctg tcttaattaa tgcataagcc agctcttctg agtttgggtg gatatccttg 300
   aggagaaagg tagctaaaat tagcagcctc tactctcttc tgtgttcctt ataaaaaaaa 360
   agagaaactg tctcatcttt aagaatggac cagttatgaa c 401
<210> 44
   <211> 401
   <212> DNA
   <213> homo sapiens
<220>
   <221> variation
   <222> 90
   <223> /replace="g"
<400> 44
   ttgttctcac caaaactcat gttgaagttt gatttctcat tgtggcagtg ttgaaaggtg 60
   gaagctgatg ggaggtgttt gggtcatgaa ggctctgtcc tcattgatgg attggtgcca 120
   ttctcacagt agtgacttct ccctctggca agactaggta agttcctaca ggaatagatt 180
   agtttctgaa agagtgtgtc gttataaagg cagcacgctc ctcagctttt ctctcttagc 240
   acgcgtccat ttcccttttg accttctctg ccatattaga ggcaacatgg aaaccctcac 300
   cagaagtcag ggctttgccc ttgaactttc caccctgcag aaccatgagc taaataaacc 360
   cattttcttt ataaatcact cagttctggt attctgttat a 401
<210> 45
   <211> 401
   <212> DNA
   <213> homo sapiens
<220>
   <221> variation
   <222> 45
   <223> /replace="c"
<400> 45
   tatcatgagt atatctaacc ctatgtttac tgtatttgtg ctatgatatg aatatatttt 60
   gatttagtat ttgatcagtc tttactgatt gttgagaatc tactaactca gtgcttctca 120
   aagtggtaaa gggctagttt ttcaaaattt cttatctctc ctggattggt gcatagtctt 180
   accacatatg actcatctcc ggcttgcacc acttgctaat caccacgtaa gtgtggcagc 240
   atctaaacaa atctgtcttc atttcaagga agagaacgct gataatgcac atggatgttg 300
   caacaatgtc aaattgctat gaaaatttct aaatgaattt tctgctcaat tgcctacctt 360
   gttgcaaatt aataacaggc agttaacata actggtagtg t 401
<210> 46
   <211> 401
   <212> DNA
   <213> homo sapiens
<220
   <221> variation
   <222> 314
   <223> /replace="t"
<400> 46
   acagggtcag gtgaggaatg tgaatacttt taggatgggg cctttcctcc tagatgtttc 60
   taggtggtgg tttccttatg aaatgacgac accccagagt catgaccaaa ggctcatcac 120
   agtcagcttg aaggatgccc tgtgtcagct cctacttctg ccccactgtt gggctggcac 180
   ctacttcttc tgaaagacac gcctcgggtg tcccctctgc ggtggcacaa agtggctctc 240
   agtgaacctt acaataataa attataaatt aataagtaat tgatagattg aataaatgaa 300
   tgtttggaga gttcgtcttt ggttagatac attttcaata tgggtttatc tgttgatcat 360
   tcagaaataa aagcgatatt taactgtgtc tcttatttgg a 401
<210> 47
   <211> 401
   <212> DNA
   <213> homo sapiens
<220>
   <221> variation
   <222> 296
   <223> /replace="g"
<220>
   <221> variation
   <222> 302
   <223> /replace="g"
<400> 47
   aggggaaaat gattaagtac tgggttattt gagaaaccat gaagaccaga aggaagtgac 60
   acaagttttc aaatgctgaa agaaaagtct tatcaaacca ggattttata ttcaacaaaa 120
   aattccttca ggaatgaagg attaacaaag atatagtcaa atgagggaaa actaagagag 180
   tttattgcca tcagacctgt gctaaaagaa ttgcttaaaa aaattcttca gacagaaggg 240
   aaatgataag aaatggaact tggaacttca aaaataaaag aagatcaaca gaaatagcca 300
   cataaataga gtagactatt tttctccttt tgagttcttt tttccttttt tctatttgtg 360
   taaatttagg gagtacaagc acagttttgt tacatggata c 401
<210> 48
   <211> 401
   <212> DNA
   <213> homo sapiens
<400> 48
   tagagtgtcc caatttgaat gtcagatctg gttacagata cattgagata atggtccctg 60
   aaccctgggc aactgggcat gttctggggt tgctggagtc cttgttctgg tcacttccag 120
   ccatataaac atactttttt ctttgctgag gaactgatct gtgctgagga atgccacatc 180
   tgggctctgt tgaagaaggt cggcaaacag aagagagaga tggggcattt cttcctccac 240
   tccctccctg ccttggggtc acaattccag cagcagttgt acccttccct tctggtgggc 300
   acttcccctg cagccctctt ccttagctcc tagtatctcc tagggcagta atagctccaa 360
   actgctccaa actgctgtta gtctttgggg gcctcccaac c 401
<210> 49
   <211> 401
   <212> DNA
   <213> homo sapiens
<220>
   <221> variation
   <222> 124
   <223> /replace="g"
<220>
   <221> variation
   <222> 153
   <223> /replace="g"
<220>
   <221> variation
   <222> 187
   <223> /replace="t"
<220>
   <221> variation
   <222> 315
   <223> /replace="t"
<400> 49
   ttcatctgtt caagaaacaa caatgaaaaa aaatggtgat taaatgcaac cacacctaaa 60
   taaggtagcc aggaggtaga accagcttct ccatgaacag gccacatacc atctcccggg 120
   gctacctcca gtgtccagcc tggctgtgct caagccctca gggcttgctg gactctcctg 180
   gcagtggggg gtcaggactc gagtaggttg gatgctgata gcagagagtg tgattctccg 240
   gcctgatgac ccagaatcac tcccaggact ggaatcccag ctctcccagg aggaaggagt 300
   cgtccctggt ctcccgcatc ccccaaatgc tgattctgtg tctgcatgtg gaggggtaac 360
   aaacaaacaa caaaggacca tctcaattaa cctgccacac a 401
<210> 50
   <211> 401
   <212> DNA
   <213> homo sapiens
<220>
   <221> variation
   <222> 91
   <223> /replace="c"
<400> 50
   ggatatcaga aaagtgttag aaagaggaaa gtagagtaaa tccaaacctc tctctctaca 60
   ctctccaaac ctgtccccca cacccacccc acccccttgt tgccaagttg taagatcttg 120
   ggctgacttc caagttccca aaattcaagt gtggaaaaag ccaactaatc tcctggaaaa 180
   atctcctgat gtgtattttt ctttaacaat ctgggctgat tttaaactta gcccacagta 240
   aactgtgctc aaccggcatt atcattatgg acacttgctg ctgtattttc caaggatctc 300
   ccctcccccg ctggtattgc taccagatac agaatggaat ttgtcaggac aagccatgta 360
   ttactactaa accattaatt cagttagtgc attaggagtg g 401
<210> 51
   <211> 401
   <212> DNA
   <213> homo sapiens
<400> 51
   ctgcagacat gtatgcacta gcttcccttt ggacactctt tttttaaaaa aatgaggtga 60
   catggacata atttaaaagt aaccatttta atgtgaacaa tttagtggca ttttgtacat 120
   tcataatgtt gtgcaacaac cacccttact aattttaaaa cattttcctc accctaaaag 180
   gaaaacctgt acccattaag caattgctcc ctatttctcc cacctctggc accaccaatt 240
   ttatgttctg tatcctggat ttacctattc tggccacttc acataaatat aactacacaa 300
   tacataacct gttgtgtctg gcttttttta cttagcataa tattttcaag actcatccac 360
   attgtatcag gcatcagtac tttgactgga taatattcca t 401
<210> 52
   <211> 401
   <212> DNA
   <213> homo sapiens
<220>
   <221> variation
   <222> 346
   <223> /replace="t"
<400> 52
   agaatatgta ttgggtggag tattctgtgt gttaggtcta gttgttcaag tcttctattt 60
   tcttattcgt tttctgttta tattttatcc atgatttaaa gtggggtatt gaagtctcta 120
   ttactgcaga actatctatt tctccttcaa ttttgtcagt gtttgcttca tatacttgag 180
   gtgttctgtg aaagggctgt gtatacaaca tgatctccaa atgcagattg agccaagaaa 240
   tgaaaaaatg agtcagacaa atccagtttg tcagtaaatg gtgatttatt agaggactta 300
   cagacagaaa tgtggtcttg ggtggctgca agccaggaag aattcggcag tgcaaccctc 360
   taggcccagg gcttatatat cttgggtggg gggtaagtac a 401
<210> 53
   <211> 401
   <212> DNA
   <213> homo sapiens
<400> 53
   agaaaaccca atgcaaggca aaagagcttg gttgagtcac cccaagtgac aaagtcatac 60
   ttaagcttct ggtttcagtc agtcatcttt gctgagaggt tggagaacac atactagtta 120
   agacaaataa atagcaattt aaactttgga catgctttct tctcttagct atgggaggga 180
   cacatgtttg gagaaaaatc gaagcatgtc aggagggatg taaaaacatt tttatttcta 240
   atcaacaagt ttataatgct ttaaatgctt ttggtttgta acaagttaaa aatgaatgtc 300
   tatttgtttt ttttaaaatc tgtcttcttt gcagtatgat ttccttggtg ttagaagaaa 360
   aaaaactgtg ccttatgaaa atatagatta aaaatgtcta a 401
<210> 54
   <211> 401
   <212> DNA
   <213> homo sapiens
<220>
   <221> variation
   <222> 176
   <223> /replace="c"
<220>
   <221> variation
   <222> 339
   <223> /replace="t"
<400> 54
   cagcattctt aatcaaaaaa ttcaaaattg aaaatgcccc agtgaagcat tcctttgagc 60
   atcatgtcag tgttcaaaac actttggatt ttaaagcatt ttagatttca gattttcaga 120
   ttaggtatac tcaacctgta ttatctttga tgcaatgtta tcattatact ttatgacttt 180
   tatcatgctt tctcttagtt ctttgaacat aattataatg gctattttga agtctttttc 240
   tcttaaatct gatgttttgt cattctcaca gacaaattta tgttttctgc tttttctggt 300
   atatggctca tatttttctg tttcattgca tgcctcatca ctttcttttg gaagttggac 360
   aatttagatc atatgtattg cagcaactgt gggtagtagc c 401
<210> 55
   <211> 401
   <212> DNA
   <213> homo sapiens
<220>
   <221> variation
   <222> 258
   <223> /replace="t"
<220>
   <221> variation
   <222> 297
   <223> /replace="t"
<220>
   <221> variation
   <222> 344
   <223> /replace="g"
<400> 55
   attgcatcct tgtttttttc ctataataat gtgtctcaag ttcattttgt acaattcctt 60
   tccagtcttg cattcatttt ccaaaggaga acttgttcct ttcaatggga aatgatattt 120
   aaagatcaca gcttgggtga cattatttct agttcttttc actataaagt aaaatgcttt 180
   tttcacttta cattttcacc gctaggaaat gattttttaa aaaagataaa atacatcatg 240
   aggtcatact ggaaaacctt attcccacaa acgccaacat aacaacttgt tttatcctac 300
   aaaacacaca cagcaccatt gttactacat ataataagat aatagaaaaa cctatataca 360
   aatgaaaaag agccttaaac gttgttttac agttcttctt g 401
<210> 56
   <211> 401
   <212> DNA
   <213> homo sapiens
<220>
   <221> variation
   <222> 23
   <223> /replace="g"
<220>
   <221> variation
   <222> 121
   <223> /replace="g"
<400> 56
   gccttgaggt ctgtatagag acaaatagcc tgagagagga agaacctatt tgtgtaagca 60
   tcaggggtaa tgatgtggag atgtctgtgt gtctgtgttt gtgtgtgggt gtgtgtaaca 120
   agatagcgag agagtagaca gagagagaca gagagacaaa gagagactga gagagagagg 180
   cagggagaaa cagaatgtgt gtgtgtaaga gagagagtca gagagaggag agagagggag 240
   agagacagag gcagagagag acaaagtgtg catgtgtgtg tgtgtgtgag agagagagag 300
   agagagaaag agggagagag acagagacaa agaccaagat aaagacagag acagagagag 360
   aatggaaaga gagagcgaga gagaagatga tgaactggag g 401
<210> 57
   <211> 401
   <212> DNA
   <213> homo sapiens
<220>
   <221> variation
   <222> 68
   <223> /replace="g"
<220>
   <221> variation
   <222> 70
   <223> /replace="c"
<220>
   <221> variation
   <222> 112
   <223> /replace="t"
<220>
   <221> variation
   <222> 338
   <223> /replace="g"
<400> 57
   gtgaactgaa gttgcagaga aatacatgcc accttatgat gaggaataca acaaaatcta 60
   acaattgaaa gtatccaagg atggtttagg atgatctgga gagggaagtt ggaggatagg 120
   tatgtgtgac caaattatat gaccttaatg tcccatatat cttgagattc tatgatttaa 180
   agatgccttt agtatttaat ccatggcttg acccaatcca tagtcaactt tttaatctat 240
   aacattttaa aagataagca aataaagcca agttggttag attctcccac ccaaatccaa 300
   gcctaaactt atgtaattga cattggattt attagtcaca aaaattatgt aacacatggg 360
   aaatatatat ttgtaaaaaa aaatgagagt tttttaaggt c 401
<210> 58
   <211> 401
   <212> DNA
   <213> homo sapiens
<220>
   <221> variation
   <222> 395
   <223> /replace="t"
<400> 58
   cattatgaaa tgaaaagaat ccacaatgga atgaaactgg taaagtattc aagctgcgta 60
   agtcctggtt ttatcaggtc acaaaaatta attctcatat cattctgttg ttttgcaaac 120
   aaactgcaag attgcagagg gttttattta ttttagacag cctgaaaaca gcaaggtgta 180
   caataacagg aataaccagg tcatttgaac aggatcgtga cagctattga tgctacctgt 240
   ccagcctgga catgatttgg cctgaccaag cacccaagaa ggctcagtgg gttgtgagca 300
   caccctgagc caaaagcgtt atgggctgct ccaaaccaag tgtactttta acgtggatta 360
   ataaaaacct gatattcggg ttcagaaata tgacagttgc a 401
<210> 59
   <211> 401
   <212> DNA
   <213> homo sapiens
<220>
   <221> variation
   <222> 7
   <223> /replace="t"
<400> 59
   accaggacat ttgaacagga tcgtgacagc tattgatgct acctgtccag cctggacatg 60
   atttggcctg accaagcacc caagaaggct cagtgggttg tgagcacacc ctgagccaaa 120
   agcgttatgg gctgctccaa accaagtgta cttttaacgt ggattaataa aaacctgata 180
   ttcgggttca gaaatatgac tgttgcattg taaggctaca tctagagtag tgcctttctt 240
   ctgggctcca catttagaaa ggacagggac aaagtaaaga atttcagatg aagaggaaca 300
   aaatgggaaa ggccatgaaa ttgatttgtt atacatattt gttcagggaa agaaaggaca 360
   gaggcagaat gataagagga gcaaggaaca caggacagag g 401
<210> 60
   <211> 401
   <212> DNA
   <213> homo sapiens
<400> 60
   aagggataaa aaaagatatc ctaggcaaat aaaagaagcc aaaaaacagc aggaatagct 60
   atagttatat caggtaaaat aaacttcaag gtaaaaacca tgaaacaaga caaataaggt 120
   cactatacaa tgaaaaatgt tcaattcatc aagaggatat aacaattgta aatgggtcca 180
   acatcaaagc aactgaatat gtaaagaaaa tagtaataga tatgaaggga gagatagact 240
   ataatacaat aatagtaggg gacttcaaaa ctcatcaatg gacagatcat ccagacagaa 300
   aaccaataag gagacattga acttaaacta taatttagac caaatggact tgagtgacat 360
   atacataaca ttctacctat cagcagcagc agcagcagat t 401
<210> 61
   <211> 401
   <212> DNA
   <213> homo sapiens
<220>
   <221> variation
   <222> 117
   <223> /replace="t"
<220>
   <221> variation
   <222> 275
   <223> /replace="c"
<220>
   <221> variation
   <222> 349
   <223> /replace="9"
<400> 61
   ctttgcccat gcttccttag taggaagaaa aagagaagga aaataatggc tagtaagtgg 60
   ataatagcaa gttagaggat cctttgcaat cctggctggc tctgtcctgt gcgcctcctt 120
   tccttatcct taaaggtttg tgaacttctt gtcaccagca gacaattgag attcaaacat 180
   atgtcagtgt cattctgaac gaaaaatgga agtgtgatcc ttaagaaaaa tggctctgca 240
   tattatcata gcttcttaga tttggaaaat aaccatggtg tctggaaaaa attgagagaa 300
   tcttactttc aggttaaatg ccatatccct ttcaactcca aatacattag tatttctaaa 360
   gtaagaataa atgattctga gactccgtta gtgaaaacta a 401
<210> 62
   <211> 401
   <212> DNA
   <213> homo sapiens
<220>
   <221> variation
   <222> 98
   <223> /replace="t"
<400> 62
   ctgccaggaa agaaaacgtg aactgacatg taactagccc tgtctggcac ccaccacttt 60
   acttattagg tggcatctgg tccctatacc cacgtttcgg tgtcagtttc ttcatcttta 120
   tgaatgatct gaggctggga gagggttggc cccaggtctc actcattgaa gagatttgaa 180
   tccaggcgca tctcattcca gcacccatat ttatccagtg cattctctag ggcaagatat 240
   ttcccttaca tgttttcctc ttaactgctt aaaaagttct tttggcacag tattcctgta 300
   ttctagtaac aaactacaat gttatgtgtc agtattccta ttccataaat aagaacctgg 360
   aggatgtgaa ataagtgatg tgtaaatgcg tgaaagatag c 401
<210> 63
   <211> 401
   <212> DNA
   <213> homo sapiens
<400> 63
   tcttactccc catccctcct tgaacctggg cttgaccaat agaagacaat gggtttggtg 60
   ctttgcaacc tctgctcctg gtcctgagag gtcttgcagc ctccttctgg gtgctggcaa 120
   ctctttcttg gaaagttcac cctggtgcaa gccggcaact atgaacaaag gtaaacaatt 180
   ctcagaccac cacgctgtga gaagcccaaa tgtagggaag ggtatcagac atgagagtga 240
   agaaattacc ctggaagttg gtcctctagc cccagccacc ctggtggatc agaaagaaac 300
   cgcccaaccc agaaaaccat aagaaaaata aatggttatc agaagtcact aacttctgag 360
   gtggtttgtt acagggcaag aaattactgg gacatatatt c 401
<210> 64
   <211> 401
   <212> DNA
   <213> homo sapiens
<220>
   <221> variation
   <222> 40
   <223> /replace="t"
<220>
   <221> variation
   <222> 49
   <223> /replace="t"
<220>
   <221> variation
   <222> 394
   <223> /replace="t"
<400> 64
   ttaaagtaat ccacacatga atatagagaa caaaaagaac gtaaaaatga aaatgtattt 60
   tttaatttga gaaacatgtt ttatcaatac atatacacaa agatggcaat taaaaaggaa 120
   aacagactta ctgtgaataa aacacaaatt ttgtctacat acactagaaa attttcctca 180
   gatccttgga acccgttgat gtatttttat agaatagccg tgatctaatt tttggcaggg 240
   gtgtgttcac atgtgtgcgt atccattctt agaaatgtgt aaaatagtat accaaagtta 300
   aagtaaatag aatgaaacaa tttttctcta cttagtaaca tttttcagta actctcctga 360
   aacaatttcc atctgcaact ttacctttaa aagctttaat t 401
<210> 65
   <211> 401
   <212> DNA
   <213> homo sapiens
<220>
   <221> variation
   <222> 18
   <223> /replace="t"
<220>
   <221> variation
   <222> 47
   <223> /replace="t"
<220>
   <221> variation
   <222> 175
   <223> /replace="g"
<220>
   <221> variation
   <222> 226
   <223> /replace="t"
<220>
   <221> variation
   <222> 276
   <223> /replace="t"
<400> 65
   tttactcttg atcaaatcag cattttggca atgaaagtca gaagacgtgt gttttacaaa 60
   gtcaaaatat tgttttattt tattataatt tgaaattaca ttttaaccag tctaatggct 120
   atttctgagc cttaactgac atgaaactgt ctgtattggt gcaattataa tgcaagatat 180
   ttgtagcttc atatgctgct gcagaggcta aacgcagagg atttagcctc ctttgacact 240
   caatcattgt acatttatct tggaaaggga aaaatgtaag tggctgataa ctaattttaa 300
   ttgacatcta gaaaaaataa ataaaccaag caaaccaaat gtgttttctt aaaactatgt 360
   tcttcttagc tattagcctc tctttagcta cagaaagaaa t 401
<210> 66
   <211> 401
   <212> DNA
   <213> homo sapiens
<220>
   <221> variation
   <222> 5
   <223> /replace="g"
<220>
   <221> variation
   <222> 360
   <223> /replace="t"
<400> 66
   ataaagagta gagatcttct gctaactgta aatggaaata tggattagag agaaacagac    60
   atgcactgtt ttaagccact aaacagtagc cactacccat tggttaatac agataagaaa   120
   actctcacaa aataaaagtg agggggaaaa tagggtaaaa aatgtgtcct gaatgtatga   180
   aatcaatttt gtgtgtgagg ggaaaataat gtaacaaata tacacacaag gaaaccccct   240
   cccccaaacc caccagaata ttgtttttac ctctgggtgt tgagactata gagttttaat   300
   ttctttatat attcttctgt ttttctagaa taaatgcata ttattttata attacaaagg   360
   ctacttttta actcttcata atcataactt ataatggtaa a                       401
<210> 67
   <211> 401
   <212> DNA
   <213> homo sapiens
<220>
   <221> variation
   <222> 249
   <223> /replace="t"
<400> 67
   taggcaatga gaagaaaatc gtaattaagg ctgagagaat ggcattgcat cgttgcagag    60
   gtaagaaaca gcactgggac tttaggaagt attttcacta ggatcaagta taatttgcac   120
   aagggaaggg gaaacaggag tagaaaagta ggcaggagcc agagaataat atgccttgca   180
   tgccttacta agaatatgga ctttactcca ttctttgggg agttatggac agataactga   240
   tatttccagc cacaatcaat ccagaaacac ccctgttttg acaatacccc agcctcctct   300
   agggggagcc acctttgatt ttcatggcag ggtgggatcc agaaagctga aagtctcttt   360
   ccaaacttga tttggagaca tgaatttcac acaaatacaa c                       401

<210> 68
   <211> 401
   <212> DNA
   <213> homo sapiens
<400> 68
   tgctgcaatt gtatgcacat gaccatgagg catgatttcc tgaactgggt gggagatagg    60
   ggcacagaat ttttgactca gttccattgc tttaacctgg aaaaaaaatt ccaacatcct   120
   tggaactctg attttctgct tgtaatacat ggagtgtaac agctgagcca caggctgttt   180
   tgagaacaga acaaaaagct gtaaatatgt cagtcaataa aactctacca agcaaagagc   240
   ctttataaat tcaaaacact attgatggtt ttaaattatt ttctctgaaa aaaacataac   300
   atcagaggtt atttaacttt tctattattt ataatacagt caccaattta gagataggag   360
   acttaatttt ctcttttgtc caaaatcaac tcaaattatg c                        401
<210> 69
   <211> 401
   <212> DNA
   <213> homo sapiens
<220>
   <221> variation
   <222> 152
   <223> /replace="t"
<220>
   <221> variation
   <222> 223
   <223> /replace="t"
<400> 69
   acatcctctg caacatctgt tgtttcctga ctttttaatg attgccattc taactggtat    60
   ggattttggt atctcattgt gcttttgatt tgcatttctc taatgaccag tgatgatgag   120
   ctttttttca tatgtttgtt ggctgcataa acgtcttctt ttgaaaagtg tctgttcata   180
   tccttcaccc actttttgac gagggtgttt gtttttctct tgaaaatttg tttaagttct   240
   ttgtagattc tggatattag ccctttgtca gatgaagaga ttgcaaacat tttctcccat   300
   tctgtaggtt gcctgttcac tctgatgatc ttgaaaatgg tcatattgcc caaagtaatt   360
   tatagattca gtgctatccc catcaagcta ccattgactt t                        401
<210> 70
   <211> 401
   <212> DNA
   <213> homo sapiens
<220>
   <221> variation
   <222> 63
   <223> /replace="c"
<400> 70
   aacctttcag tggcttcttc cttttggtta ttacgaataa tgctgctatg aacatgggag 60
   tgaaaatgtc tgttcaagtt cccgttttta attatttcag gtatatgcca agaagtggag 120
   atgctggatc atctggtaat ttaacattta atttttgaag acccactata ctgttttccg 180
   cagcgaccgt accatattac gtttcctacc agtaatatga gaaggttcta ctttctccac 240
   atcctcacca ctatttgtct ttttatgctt ttgttttaat actagacatc ctaatgggtt 300
   tgaggtggta tcttgtgatt ttaatttgca tttccctaat gattagtgac actgagcatc 360
   ttttcatgtg cttattggcc atttgtatat ctgctttggg g 401
<210> 71
   <211> 401
   <212> DNA
   <213> homo sapiens
<400> 71
   tgaaataatc caactgcagg aaccaactca atagggggag gattgcctaa ttttcaagca 60
   tttctgattc attatgtcac cattagcata gtattcccaa aacgcacttc gtgacatgca 120
   aagatcccaa gataaaaatt tcacctatgt gtacgtctgc aaaagagaaa aacaattgtc 180
   agaatgagca ctaaaccttc gttgatggtt ataggaggga gtggaattat aggacagact 240
   taattttatg ctttctcttc tccagtgatt tacttttgta cagaaaataa gccatattta 300
   taattagaaa aagaggaaaa cgtaactgat tcaaaactac atgtacaata tgaattttag 360
   cttacaaatt tatacataga ggtaaacatc aaaaacggtc a 401
<210> 72
   <211> 401
   <212> DNA
   <213> homo sapiens
<220>
   <221> variation
   <222> 83
   <223> /replace="t"
<400> 72
   agttggagaa tctactgact aaaagttgaa atggaaaatt ttaagactac aaataaagat 60
   cagactaaag gcaaggcaga gccgccctgg gagtacagtg aatggggcat ctgtgtccca 120
   ctcgggcgcc gatcgccttg tccagctact gaacagttac tttgagtgca tcaggaggga 180
   gaaaattaca ggcacagctg gcatgaatcc actccccatc catggccagt gcaaaggaga 240
   caagacactg tctcttcact tttatacact tacttcattt tgtgaaactt ctcttgccct 300
   tactatatgt gcatattgtt caagtgaata cctgccagtt ttagaagaga aagggttagt 360
   ctgttcttgg ctgctacatc aaaataccat aacctggggg c 401
<210> 73
   <211> 401
   <212> DNA
   <213> homo sapiens
<220>
   <221> variation
   <222> 14
   <223> /replace="t"
<400> 73
   ataataacac cagcaaatgt ttagctaaaa aatttagcca tacatgccac acattgtgct     60
   acacattgag ctctttggtc ctcacatcaa cgctgaatta ggagctatga ttatccccat    120
   tctttagatt agtaagctgg gtatcaccaa ggagaagtac ttgccccaaa gtcacatggc    180
   tcatatgtgt tggaagcaag gtttgtgaca gatcttccat gttaaaccat tatgttatcc    240
   tccttaggag agagtatggt tttatgaata tactgctcca attggctgag atgacaatac    300
   tagtttttaa tcggcggctg tgacatattg agggcttata ttaagaatgc gagctgctcc    360
   aaaatgtccc tgtcctgcag ttgtacaatc acatattttt a                        401
<210> 74
   <211> 401
   <212> DNA
   <213> homo sapiens
<220>
   <221> variation
   <222> 24
   <223> /replace="t"
<220>
   <221> variation
   <222> 51
   <223> /replace="c"
<220>
   <221> variation
   <222> 172
   <223> /replace="t"
<220>
   <221> variation
   <222> 330
   <223> /replace="t"
<400> 74
   caaaaacttt gaagttagat aagctgattc aaatcctaaa gccagccctt gagtgctgtg 60
   tgaccttggg taagttatag aacctttctg ggcctcggtc tctttacctt ttaaatgagg 120
   ccaagtgtgt acatgatgct aattaagtga gacagtccat gtgaaggctt agcacagtct 180
   ctggcattca ataagaacac ggtgagtgag ttgtttttct tcctgtcatt ttaggggttg 240
   tagcaatagt gtttttgatt gcagtgggtt gttgactgcc aagtgaatga ttatacatgg 300
   acatggaagt tgagatatga aaaaagccac gtgggtcaga gtctcatggt cagctaccat 360
   ggcaatcctg aaaagctcct ctacctgaat ggcctgtcct g 401
<210> 75
   <211> 401
   <212> DNA
   <213> homo sapiens
<220>
   <221> variation
   <222> 12
   <223> /replace="g"
<220>
   <221> variation
   <222> 237
   <223> /replace="t"
<400> 75
   tgaaggggtg gatcaggcag agggtcttag gccactgctg gtaaatgcca aactaactct 60
   gaggtcagaa gtccttgatg ctacatcccc aggttcaaaa gcctaagaag ccctcatttg 120
   gactgggcag cgtccctcca cctgagagaa attaataaca gcaagcttct gaataattgc 180
   tgaaatccac acctgaaccc ggatggacca gagcagccac aaagtcctca aagcacctga 240
   ccagccccct cagtgggcta tttttgtaga aaaagaatga gtcctggaaa acaaccaaat 300
   acccattgtt tggctgcgtt tattttccat caagagtgat gcagatagct tgcccagtag 360
   ggacccactc agcgtctaaa gactaggcaa atggaggtgg g 401
<210> 76
   <211> 401
   <212> DNA
   <213> homo sapiens
<220>
   <221> variation
   <222> 57
   <223> /replace="c"
<400> 76
   cattcaaata caggaataag aacttcattt tgtaacaaaa acaataatta tataacactt 60
   cttaatatta gctatcactt cctaatattt tcactgtcac actgagctaa attttaatat 120
   gaattatctc actcaatcct taaaccaatc tgtataaggc aggcaacata acttttccaa 180
   agttgtacag ggaacaagaa ccaaaactag gacccaaatc aaggattctc tgatgatctc 240
   catgcttgta atgttctctt tctttttccc ccaccaatat gtggaccacg gtcttcttaa 300
   caaaatagct gcaatatgtg gtattaatca ttatgtacac acacagacac atacacaaat 360
   acaaaggaga tttgctatta acttagattg tacaattaaa a 401
<210> 77
   <211> 401
   <212> DNA
   <213> homo sapiens
<220>
   <221> variation
   <222> 240
   <223> /replace="c"
<400> 77
   cttgattcaa aacggaatgt catgttggtc agcaatatat gcttccacta aataaccaaa 60
   tacacaaatt gaaataaagt gaaattcttt ttccagagtt aatgaaaggt tcctgtttgt 120
   gaatttgggg ctttgaagtc caaggcttcc aaacagcacc aggaatttac tggtgattta 180
   ttactatttg gacaagtggc gaacagagca ttactctacg gaaagtccac attccctaca 240
   tgtcaaggga gccttcctga aatggggaca ccagctcccc ttgctgcaac tcctcttagg 300
   gctgtgcctc aggtggggtc cacatagtct ggtgtggtca tctctcctgg gccagcgccc 360
   tccccctctc tctgctctag cccctgccct atcctgtgtc g 401
<210> 78
   <211> 401
   <212> DNA
   <213> homo sapiens
<400> 78
   atggaaatat aagctgtaaa ctgaaataat atattttcta taataattat gaattcagct 60
   ggatggaaca gaaaacttaa atgacagcag ctattctttc ccatacagca agaaggtgag 120
   tggtggatgg ctgagggcca gcgcagggct cctgatgtta taaatgtcca ggttgcctcc 180
   ctcttcaggc tcggcaccct aagttggaga cttttgctct cagtatcaca ggatgggttt 240
   ccaggcgttg agactgcatt ccaggtgggg aaaagaggaa agagcaaagg taaaaattca 300
   gctgaatcta cactgcttat ctggagaaaa atacttcccc agaagctcca tttggtacat 360
   ttctgcttat gtctaattgg acagaactga gtcacatggc c 401
<210> 79
   <211> 401
   <212> DNA
   <213> homo sapiens
<220>
   <221> variation
   <222> 274
   <223> /replace="t"
<400> 79
   ttaatgcttc cttttgtggg aagaacccct gaggtaatca gtggggttca ctgaggtccc      60
   ctcctctttt cctctacacc actgttttat gcgtcctgtc tagcctcgtg ttgaaagtgc     120
   ctatttctgg gctccaatta ggctcattct ccctcttttc tttgaattat catgaactga     180
   attgatctgt cttttaaaaa attatccttg taaattgggt ctatctttta agtattaacc     240
   ttatttccct caattcaact tatttccaaa taactattag gcctgcccat taggtgataa     300
   gtttttttaa aaataatttg tcactgtaga aatattcaaa catatacaaa aatagagagt     360
   atagcctgtg aactcctaca tccctagcac ccagctttgt c                         401
<210> 80
   <211> 401
   <212> DNA
   <213> homo sapiens
<220>
   <221> variation
   <222> 15
   <223> /replace="t"
<400> 80
   ttatttccaa ataactatta ggcctgccca ttaggtgata agttttttta aaaataattt      60
   gtcactgtag aaatattcaa acatatacaa aaatagagag tatagcctgt gaactcctac     120
   atccctagca cccagctttg tcactcctcc atattatgtt gaagtaaatc tcagatatct     180
   ttctactata aatatttcac catattcttt taacagctaa ggaaacttaa aaaataacca     240
   tcatacaatt tattacttaa atttattaat acatttttaa tatcagatgg taagttacag     300
   gatggagctg tgacaccctt gtcatgaaca agcctacagt tgtagactta tgctcacaaa     360
   gggtgctcag ctccctatta atgaattgat tgatgaatca a                         401
<210> 81
   <211> 401
   <212> DNA
   <213> homo sapiens
<220>
   <221> variation
   <222> 117
   <223> /replace="g"
<220>
   <221> variation
   <222> 265
   <223> /replace="c"
<400> 81
   ctgtccatcc gaatcccaaa agctttcctt gtgcttcctg ctctgaccaa agaggcattt 60
   gaggcacatg tgagagaaca gctttccaga aaccacactt gagtccacag cagagcagaa 120
   ggggcaggag gcatcaggac catttccaga tttctctctg gccctgattg tctagatttg 180
   catccaatca ctgcaagagt ttatacaaaa gttgttagaa agatttgggt ttcacacaga 240
   gttatgaaaa caaggtagaa gcctgatgtc ttcatttccc atgaaatctt gtgggtgtaa 300
   atgaaacatg ttttagtctc aaatcctgga gctgtggggt tcacaatgga gaaatatcca 360
   ggtctcttga acaagactgg acgataacca gggacagttc c 401
<210> 82
   <211> 401
   <212> DNA
   <213> homo sapiens
<400> 82
   tctcactcca tgtcaagggc tattcttctt ctctctctct gtctctaaca atattgcaac 60
   gatattctga atttagttgt aaatttagtt ggaatttgat ataagctatt tgattttcct 120
   tcacaaattt gtttacagca atttcctccc agaacacact gataaagaga agtagatgac 180
   agttattgat gaaccctgct tctggcagtt attaatcagc cattgacttg atgaagcagc 240
   catggatgcc acacctgatg gtgaggcatg tgacctgggg tccagctgcc atgggtcatc 300
   catgtggctt tgggagtgtt gggaagctac tgcttcagca tctccaagga acaagcgtcc 360
   tgtgagagtg gaagtgagag gtcagtaaag acagacccgt t 401
<210> 83
   <211> 401
   <212> DNA
   <213> homo sapiens
<220>
   <221> variation
   <222> 101
   <223> /replace="g"
<220>
   <221> variation
   <222> 153
   <223> /replace="t"
<220>
   <221> variation
   <222> 189
   <223> /replace="g"
<220>
   <221> variation
   <222> 224
   <223> /replace="g"
<220>
   <221> variation
   <222> 308
   <223> /replace="g"
<400> 83
   catatgtcaa tgcatagaaa ttatttcaaa taaagtatgt ttctgtgaac ataaaacatt     60
   tacttgttat gacactgtaa tcagggcgtc acagattcta agatatgaaa tgctaagcac    120
   cccatctcta ccaaaataaa aaaaatttag cccggcgtgg tggaacacac tgtagtctca    180
   gcttctcaag aagcaggggt aggaggacaa cttgagcctg ggaagctgca gtgagctgtg    240
   gttgtgtcac tgcactccag tctgggtgac agagggagac tatgtctcaa caaacaaaca    300
   aacaaacaaa caataaataa atagaaagaa atactaagta gacatttgat tatctcatgc    360
   ccaggttgta tctggagata agggtatgct ggaaagaatt c                        401
<210> 84
   <211> 401
   <212> DNA
   <213> homo sapiens
<220>
   <221> variation
   <222> 110
   <223> /replace="t"
<220>
   <221> variation
   <222> 250
   <223> /replace="c"
<220>
   <221> variation
   <222> 327
   <223> /replace="t"
<400> 84
   agaatcttag tggtgactgt gcaaagccat ttggtctaat gattttacca agagcatctc     60
   agaacactag tattggtagt atgtagcctt gttaataaat atatacaagc acatgttgaa    120
   atgacttgtt attatatggc tagagcaaac ccacgtcatc ctcatgatgt cttgctgtca    180
   caatctcatt ctctcttggg ttctgacctg actcctcaag caagagaaca agggttggca    240
   gagctctcca ggaatgcaca gccccaggtg cccagcagag ggcatcaagt atgacagata    300
   ttggaatgac atctgttgtg tgtgcagagc agactgactg agtggaacca tgaccaaaga    360
   gaactcaggt agactcaagt gcttctggga aatttgagaa a                        401
<210> 85
   <211> 401
   <212> DNA
   <213> homo sapiens
<220>
   <221> variation
   <222> 75
   <223> /replace="t"
<220>
   <221> variation
   <222> 124
   <223> /replace="c"
<220>
   <221> variation
   <222> 376
   <223> /replace="t"
<400> 85
   tgttattata tggctagagc aaacccacgt catcctcatg atgtcttgct gtcacaatct      60
   cattctctct tggggtctga cctgactcct caagcaagag aacaagggtt ggcagagctc     120
   tccaggaatg cacagcccca ggtgcccagc agagggcatc aagtatgaca gatattggaa     180
   tgacatctgt tgtgtgtgca tagcagactg actgagtgga accatgacca aagagaactc     240
   aggtagactc aagtgcttct gggaaatttg agaaacttcc ctgacagaga atttacttca     300
   agtcagatac ctccccaata attgaaaatc ctgccaataa tgaggaaaga gaagtggacc     360
   caaattccta tttccctgaa tctgctcttc agtttgttca t                         401
<210> 86
   <211> 401
   <212> DNA
   <213> homo sapiens
<220>
   <221> variation
   <222> 91
   <223> /replace="g"
<220>
   <221> variation
   <222> 284
   <223> /replace="g"
<220>
   <221> variation
   <222> 360
   <223> /replace="g"
<400> 86
   agggtagggg agtgtagagg ttcaggagtg gtgttttagt ttggtgttgt gcccagggca     60
   gaccctcaga taaaatgcct ttaagacaag atcctgtgga atggagggag gacctaagga    120
   ggcaatgcag atgtctggta acggagggtc ccaattagaa aacacaaaag atcagaaaac    180
   ctaaggcctg agtcagggtc agtacctgaa gtggggctga gcagggtgtt ggggacgagt    240
   tgaggacaga gagggcctga aggtgagatc agttatcaat cagattaggc cttgtgaaga    300
   ctttggctct tactctggat gggaagtaga ggtctggcat tctctgcctt tcatttggaa    360
   gggaaccctc aggagtggaa ggggagcaaa gctgggaaaa g                         401
<210> 87
   <211> 401
   <212> DNA
   <213> homo sapiens
<220>
   <221> variation
   <222> 42
   <223> /replace="g"
<220>
   <221> variation
   <222> 125
   <223> /replace="g"
<220>
   <221> variation
   <222> 272
   <223> /replace="g"
<400> 87
   aaacacaaaa gatcagaaaa cctaaggcct gagtcagggt cagtacctga agtggggctg     60
   agcagggtgt tggggacgag ttgaggacag agagggcctg aaggtgagat cagttatcaa    120
   tcagattagg ccttgtgaag actttggctc ttactctgga tgggaagtag aggtctggca    180
   ttctctgcct ttcatttgga agggaaccct caggagtgga aggggagcaa agctgggaaa    240
   agtagcaata aagtacctgc aaatgtttta tatagatttt gttcacatct ccaaaaaaag    300
   tgcaaaaact cccctagctc tgcattttcc agccactgtg gactgaattg tgcccctccc    360
   caccaagtta atatgcagaa gcccgaactc ccaatgtgac a                         401
<210> 88
   <211> 401
   <212> DNA
   <213> homo sapiens
<220>
   <221> variation
   <222> 78
   <223> /replace="c"
<400> 88
   aaacataact tatatgcaaa tggttaagaa taccagaaac attgcaagaa tgctcttttt     60
   aaatcctgaa gatattggtt tattttaaag catcaaggat gatttagatt tttggaaaaa    120
   cagaggtggc atgaagattt ctgaactcgc tcacacacaa acccgtcaca gccgctgtga    180
   tctataagcc aaatttgtgt tagaactttt cttttttgaa taacaacaaa acaacaaaaa    240
   cgctcctaac tgaaactggg aaaggactag aaactttgtt ttcttactaa tgtgaggttg    300
   gaaacatatt ggctttcaag tgaattggcc gtgaccacag cagcacttgg atctgaggga    360
   gccgagattg caggcagatg tgagtttggc acttgcatgc c                         401
<210> 89
   <211> 401
   <212> DNA
   <213> homo sapiens
<220>
   <221> variation
   <222> 352
   <223> /replace="t"
<400> 89
   taacatagta acagcttaca tttttatcct ttggttttat tgaaggatcc tgtaaatgaa     60
   taaaaccata agaaaacata tggaagaaca gtcaaatagg ttataaatat aatcaacact    120
   gttttaacag catcacagag caagcatgga gggaatgata ttttattgat tatttagagc    180
   ctaagccagg ccttgtgcaa cgcatttctc atgtataatc ttatctgacc tcatgaagga    240
   ggattttcct taaactgggg agtctgaggc ttagcaaatg aatcgcacaa ccaggatttg    300
   aactaaagtt taccttcatc taaatacgcg tttttctttc tctctctctc tctttttttt 360
   tttaccccta tcgcatcata gccacatctg tggccacaca a 401
<210> 90
   <211> 401
   <212> DNA
   <213> homo sapiens
<400> 90
   tgcaaccaag cgactaaggg ggcaggaaag gggcttccgg taagagtcga agtgtgaatt     60
   ttaatttcgg tggttctagg gatggcaaga tccaaggtgt agccaagtga aagagttgcc    120
   aaggtgggat ggagagggag gtcgctggag gtgagcagcc caggacctgc agatgagaag    180
   tgggattagt ggtccccaga aatgccgtgt tccaagggga agccggcggt gggcaaagga    240
   ctagcagagg gttcagtccg aagccgccac cagggggcgc gagagagcag caagccgtaa    300
   cagctcgcgt aaaaggcggg agctggggct tcctgggggt tccctagaag cggacctcga    360
   gacaaggtcc tgggtgcaag cggtttattt gggggatggc t                         401
<210> 91
   <211> 401
   <212> DNA
   <213> homo sapiens
<220>
   <221> variation
   <222> 202
   <223> /replace="t"
<220>
   <221> variation
   <222> 322
   <223> /replace="t"
<220>
   <221> variation
   <222> 348
   <223> /replace="t"
<400> 91
   cgagcgggat gctgtggatg acaagggagc tgctctcacc tccccgcccc gagacagatg      60
   aggcactcga ggacctgcag caggcctcag agagggctag gaccagggag gggctgcgga     120
   tggagagcag agtgacggcc tagaaaggag gttcccgagg gctctgggga ggcggaagag     180
   gggacgcggt gtgttgagat acggggagaa gttcagagca gcatgggcat gcaggtgcct     240
   gcagaggggc ccatctgggc tgcaccccag caggacaggg ccatggcttg gtgtgggttc     300
   ggctggctgc acagcttctt gcaaagtttg tccccagggt ccttgggagt agaggtcttt     360
   cggaaggcac catctccaga gcgtgtttct gagtaactct g                         401
<210> 92
   <211> 401
   <212> DNA
   <213> homo sapiens
<220>
   <221> variation
   <222> 7
   <223> /replace="g"
<400> 92
   tgctgaagac tgcccatggg tagcatcatg tggagtgtgc gttgtgttca ttgggcctgt      60
   gtgtcatgaa gacactgtgt actgttcata cagagaccct ttgatgagac accacaagag     120
   aaactccttg taggagcctc tggcagaaga aaaatggttt tcttttgagc taccagatta     180
   aaaaaaaatt gtggttcccc tgaatcctat aacagcattt gtctgcctgc aaagctcagc     240
   tccaggtttg taggccaccc aacttaagcc tttgtgaaca catttttggg aactgatgct     300
   accctggact tcactgaact atcttacttc atttttcctt tactcgtctt tatttaatag     360
   ttttattgaa ttatttatgt aaatctcacc tcatcatgct t                         401
<210> 93
   <211> 401
   <212> DNA
   <213> homo sapiens
<220>
   <221> variation
   <222> 30
   <223> /replace="c"
<400> 93
   caaggaatgc cctagaacaa acgcctggca catagtaagt tctacccaag tatttattat      60
   tataataatt gtataaacat ttaacgactt ccttaaattg tttcctatta cttgctattt     120
   tgaatagtgt ttcaattaat atccttgtga tatggttttg ctgtgtcccc aaccaaatct     180
   tatcttgagc tgtaattccc acagttccca tgtatcgtgg gagaaaccta gtgggaggtg     240
   atagaattat gggggcgggt ctttcctgtg ctgttcttgt gatagtgaat gagtctcatg     300
   agatctgatg gctttaaaaa tggaagtttc tccacacaag ctgtctcttt gcctgctgcc     360
   atccatgtaa gctgtgactt gttcctcctg gccttctgcc a                         401
<210> 94
   <211> 401
   <212> DNA
   <213> homo sapiens
<220>
   <221> variation
   <222> 54
   <223> /replace="g"
<220>
   <221> variation
   <222> 356
   <223> /replace="t"
<400> 94
   cccccagctc ttatcacaga cactatacaa acaggaatac tttgtgttac cctatgttaa      60
   tggttctctt attttgctgt gcatcagaat accctggaag gatgactaac atacagattg     120
   ccaggctcag cacctgctgt gtctaagcca ggatatctag gggcatttct agtaagttcc      180
   aggtaatgct gatgctgctg atttagggac aacactttga gaaatatggc tgtgtattat     240
   gggtctattt gaacattcga ggaaaataag taggataaaa gtgatttggg acaacagtct     300
   cattcttttg atatgattaa tacatatgac acaaatatct tctttggtaa tttttaaaaa     360
   aattagatat aatagaaatt tatttttctc ttacacaatg g                         401
<210> 95
   <211> 401
   <212> DNA
   <213> homo sapiens
<400> 95
   ctgtcactgt gattttcctt gcattgtcaa ttggctacag tattcttcat gatttctctt 60
   gactaacatg ccttctgaca atgttgtctg aagctgtggc ttcatttggg aaatttctgt 120
   gtgttttaga aaatgtaatg ctgtcatcac aatgggccat agacatttca gtctttctag 180
   gtgttctgaa ggggttggct caaaaataca gggcaggctt tcacgttggt ctacttttag 240
   tagtgacatc tttcagattt acctaaaata atggctaata tttgtctaaa aactttctag 300
   tggagaactg tactcatact aatggctagt aaaattaatg gcttactgtg tgtattgcat 360
   gctcgctgaa gtgttttctg tgtgtccact cagttactat g 401
<210> 96
   <211> 401
   <212> DNA
   <213> homo sapiens
<400> 96
   aagaagccac accttccatt tctcactgca aagagctgtg gtgacagaca tgtaagaatt 60
   ttatttcttt tttaaagtaa cttagattac agtagggctg ccatgagaaa ggtgacacag 120
   aagagcagac gagagggagc agaaaggtgt atgcagattc tcttgtgtcc atcagcctta 180
   agaacagggg ctgctgagcc aagtcagagg ctgaagccac tcctagggat aagatccccc 240
   catgtctcta ctctggggac attagctcca ctgtttagac tagcttctca ctttcaccca 300
   catttttagg ccagtggaaa cttccataaa tccaggtcat acattcacat tcaaccgatg 360
   tgtgtttacc cactacttcc gatctgaaat tttgggaagc t 401
<210> 97
   <211> 401
   <212> DNA
   <213> homo sapiens
<220>
   <221> variation
   <222> 48
   <223> /replace="g"
<220>
   <221> variation
   <222> 61
   <223> /replace="c"
<220>
   <221> variation
   <222> 124
   <223> /replace="c"
<220>
   <221> variation
   <222> 145
   <223> /replace="t"
<220>
   <221> variation
   <222> 269
   <223> /replace="t"
<220>
   <221> variation
   <222> 314
   <223> /replace="g"
<400> 97
   ttccccagtg ggacgactct gcggcatgtc tgcgctggct cccagggaac agcaccccag      60
   atgcccacac taataacctg gtctctcact catcctgact gggctgcctt cccttccctg     120
   tctgtcttcc cctttcttct ccctctcctc ctgggatcac ctcccataca aactcctcac     180
   cctcatttcc ttgtcccaag ttctactttt cagggaaccc aggatacttt cctcctggtc     240
   ctaagaaaac ccaggcaaac caagtttcct gctctcatac aatttacagc atgttgggtt     300
   gaacaggcaa cttaacaaac attaaaaacg aagtgtaacg agtgctgagg tcagggagca     360
   tagactggcg gaaggcacag agccacaggg gaccctaagc t                         401
<210> 98
   <211> 401
   <212> DNA
   <213> homo sapiens
<220>
   <221> variation
   <222> 11
   <223> /replace="c"
<220>
   <221> variation
   <222> 32
   <223> /replace="t"
<220>
   <221> variation
   <222> 88
   <223> /replace="t"
<220>
   <221> variation
   <222> 156
   <223> /replace="t"
<220>
   <221> variation
   <222> 305
   <223> /replace="t"
<400> 98
   ttccctgtct gtcttcccct ttcttctccc tctcctcctg ggatcacctc ccatacaaac      60
   tcctcaccct catttccttg tcccaaggtc tacttttcag ggaacccagg atactttcct     120
   cctggtccta agaaaaccca ggcaaaccaa gtttcctgct ctcatacaat ttacagcatg     180
   ttgggttgaa caggcaactt aacaaacatt aaaaacgaag tgtaacgagt gctgaggtca     240
   gggagcatag actggcggaa ggcacagagc cacaggggac cctaagctag cccaggagag     300
   gtcccagaag ctgagctggg agtgactggg gctccctgcc ttttcttccc cttcttgaac     360
   ttccactcct ctctgtttca tttcttctct tcctttcaga a                         401
<210> 99
   <211> 401
   <212> DNA
   <213> homo sapiens
<220>
   <221> variation
   <222> 83
   <223> /replace="t"
<220>
   <221> variation
   <222> 345
   <223> /replace="g"
<400> 99
   tttgggattc tggagtctat tgaatatttg caacttctag gggaaggctt ggatggtaaa      60
   ttgtttaatt ttgatcattt tcagctccta gcacagtagc tgctacctat ctttcacctc     120
   tggaccctcc tctcttgagc aggcagcatg catgtgttcc tggagaagtt tgcacacagc     180
   ttgttggagg cagggtgggc aaaaggatcc tgccctccaa atatcaggga tttgtactct     240
   gactgctgat ggctgcttct gatcacaggg gtgctggcaa tgaaaaagcc attattgttg     300
   ttgcatcttc ccacattatt gcaagcccgt ctacctctgg atgaactgag ttccacggta     360
   tttaaaaagc tggcaccttt gttacccact tcagttttct a                         401
<210> 100
   <211> 401
   <212> DNA
   <213> homo sapiens
<220>
   <221> variation
   <222> 26
   <223> /replace="g"
<220>
   <221> variation
   <222> 158
   <223> /replace="g"
<400> 100
   agggtaaaaa caaggacatt tcttaagaat ctatagcagt aatccaggaa aaaaataatg     60
   tggtcttaaa ttggaaagtg gtgggggtaa gatatcagat tcatgtgatc atgactaagc    120
   aactccagga ttttctgaca gaaagcatgt ggcatgaagg aaagagcaga gtcaaggagg    180
   gctctggaac ttgggccaga aaaactagaa ggataaataa ataataaaaa gtttagcagt    240
   caaattcata acagtaaata aataaattaa aaataataaa aattaatgga taaaaagttg    300
   ttatgtaaat tcagtgccct attccatctc ctaggttatt ttcactagaa gttaattaga    360
   actctccagt tgtcccagcc tagacagcct gctagcccct t                        401
<210> 101
   <211> 401 ..
   <212> DNA
   <213> homo sapiens
<220>
   <221> variation
   <222> 290
   <223> /replace="t"
<400> 101
   aatgttatga atatatttat aacttttata gcaaataaaa taataatgca ttcagatttt     60
   gacatggatg ctaaacaaac aatgaaaggt ttgtgtctga gagaatcagt attaataatt    120
   atttagtatt aagtaagcat tgttaagtaa ttaagtacta agaattgagt acaatatttt    180
   aaaatgtgac tttacagaga aagagagtac aaattgtata aaattcaaga acacataaaa    240
   ccaatctata ctgatgggca tcagaatagt ggctatcttt tgggaggtgc tgactaggca    300
   cacaagatgc cttcttgggg gttgtacatg gcctgcatct tgacttggtg gtgattgatt    360
   acagggttgt gaacatatgt aaaaacttca tcaaggtgta c                        401
<210> 102
   <211> 401
   <212> DNA
   <213> homo sapiens
<220>
   <221> variation
   <222> 265
   <223> /replace="t"
<220>
   <221> variation
   <222> 398
   <223> /replace="t"
<400> 102
   aatcaagtaa aagaatggtt tgaataataa catattgata acagataata caaacttttt     60
   tcattaagct tcagaagaat cttagtagaa atgcattgtt tggaatgcat atgctttggt    120
   ctcaataaaa atatccggtt gcagagatat ttcatgtggt ttgaactaac aggcaaaaaa    180
   ttaaaaattc attggtatac actaattcat gcaattgtat attttggagg taagtgtaat    240
   ggcataggtt tgctgtaatg gaaccgattg gaaaatgatc tcttggagaa aatttattgc    300
   tggtcataag atttctttgg tttagaaaaa tgctgggttt agtaaaatct gaggcactaa    360
   atgaaagtct gctttaataa aaatgtggat ctgagaactg t                        401
<210> 103
   <211> 401
   <212> DNA
   <213> homo sapiens
<220>
   <221> variation
   <222> 26
   <223> /replace="g"
<400> 103
   gctctaactc tggggacttg tattaagccc tgactttgtt ctctgtctcc ttgaagttag     60
   gaatccactg cactgagggt gccaaagtgc tcctcaatca ctggttccta cagtccaatg    120
   ggcaatatca gcaaaagtgt tttgtagtgt agtgacagtg ggattcattc tctttcacat    180
   atgtccacag cagtggtggc agcagctgca gcaaagtgct agtgggtgcc agggtgcttg    240
   cccctctgca ggtgttcagc accgtggtgg aggcaacaca gctgtggggg gcaggggccc    300
   ttgctgatga ctgtgtgcat ggtcatactg gaggtggtgt tggctcaggg gtggggcact    360
   ggcagttgta gttctgggtg ccttctctgt gccccacaag c                        401
<210> 104
   <211> 401
   <212> DNA
   <213> homo sapiens
<220>
   <221> variation
   <222> 249
   <223> /replace="t"
<220>
   <221> variation
   <222> 253
   <223> /replace="g"
<400> 104
   aattttgttt cctggaggca ctctgatacc tattactgta tcaatcagga tccaggcaga 60
   aagtagacat catgcttaaa ctgggtaact tgaagataat ataggaaaat agcaagcaat 120
   agtctagaat cctaaaagtc tgaatctatt atcaacctta ggtgtgaatg ggcaagggga 180
   gggagtggat tctgtcatac aacacatgag tgcaaaagag ctgtgtagtg ctgcttgatg 240
   cctattacct ttatgtaaag acacagccag ctcacgtgag ctcttaggaa gggggataga 300
   gaaataaata ccctgacctc atcttcctgc cctctgggct gctggcctcc aaaccagaag 360
   cagggcactg gagcttacca acatggtaca caaaggctgg c 401
<210> 105
   <211> 401
   <212> DNA
   <213> homo sapiens
<220>
   <221> variation
   <222> 85
   <223> /replace="g"
<220>
   <221> variation
   <222> 145
   <223> /replace="g"
<400> 105
   gtgacacatt gaattttatt actgactctg tgtttcaaaa cattttgggg actgaaaaac 60
   ccaccaggca aatctcaggc cttaaaaagc agttaaactg aattgcaaat aaaaggctta 120
   gaaagtcctt aaaatcataa acttagcatc tccaatagga caaatgaact ttgtcttctc 180
   atctgagtta acagatttgg aagaatcaaa atcttttctg ttgtatagac tacatgctac 240
   catttccaca ggcaagctgc ccctaaattt ggaaaacttt tccttctgaa accacccaat 300
   ctctttagct tgatttgggc aaccgaatct cttaggcttt tgcctctttg tgtccaaaac 360
   tagcactgtt cctgagagca ggctatcaat catttacata t 401
<210> 106
   <211> 401
   <212> DNA
   <213> homo sapiens
<220>
   <221> variation
   <222> 38
   <223> /replace="c"
<400> 106
   gcgtcgctca cgctgggagc tgtagactgg agctgttgct attcggccat cttggctcct 60
   ccccaagggg aagcttttta agctttttat tctaaggact attgaggtca tttcagtgat 120
   caaagtgaga tttattgttc actaatcggg ctcctatgtg atcttatatt gcatattagt 180
   gcagatttta tataatttct attcacaaca gctattgatg aaacaatgtt tcccacattt 240
   ttgtcctttt agtgctaaaa attaaaacca aagttagggg tatagaattt aggattatag 300
   aactagggat ttgttggtcc ttaaatagct gggtgctgtt tgttgaaaga gtgattctac 360
   tatgaaattg catagcatgt tgcaagttcc atcataatat a 401
<210> 107
   <211> 401
   <212> DNA
   <213> homo sapiens
<220>
   <221> variation
   <222> 198
   <223> /replace="g"
<220> variation
   <221> variation
   <222> 357
   <223> /replace="g"
<400> 107
   aactaacctc attctatgcc cacttaacct ttctaatagc tttagcactg aatgactcat 60
   atgaacccat tttacaggtg agaaaattga ggcctagaga agttttgagg cacatctgct 120
   tatagctagt gaaaagtgaa ttaaaactct gaactagtct caccttatag ccagagctat 180
   ccatcatggt agcacgcagc atctaggtga gtttgtgctc tggagagttc atgtttttca 240
   ctgctcacag ctcctgtgtt ctaggtggta cgtaatggtg aacagactgg gcccgagatt 300
   aagattacat gattggaagt gcaagttcat cacttttgga gcaccttggg caaatcactt 360
   gtccttcata gtcatttgtt tctgcataag taaaattggg a 401
<210> 108
   <211> 401
   <212> DNA
   <213> homo sapiens
<220>
   <221> variation
   <222> 42
   <223> /replace="g"
<220>
   <221> variation
   <222> 45
   <223> /replace="g"
<220>
   <221> variation
   <222> 392
   <223> /replace="g"
<400> 108
   gtctcacctt atagccagag ctatccatca tggtagcacg cagcatctag gtgagtttgt     60
   gctctggaga gttcatgttt ttcactgctc acagctcctg tgttctaggt ggtacgtaat    120
   ggtgaacaga ctgggcccga gattaagatt acatgattgg aagtgcaagt tcatcacttt    180
   tggagcacct tgggcaaatc acttgtcctt catagtcatt tgtttctgca taagtaaaat    240
   tgggataata ataatagtgg ataatagtgc tggctcatag agttattggg agttaattac    300
   atgagttaaa tcatgtaaag tgttgagggc agtgcctagg cacgtagtaa gaaatgtgag    360
   tgattactgt ttttataacc ttattgttaa tactaatttg c                        401
<210> 109
   <211> 401
   <212> DNA
   <213> homo sapiens
<220>
   <221> variation
   <222> 139
   <223> /replace="g"
<400> 109
<210> 110
   <211> 401
   <212> DNA
   <213> homo sapiens
<400> 110
   gccttggtct cccaaagtgc tgagactaca ggtgtgagcc actgtgcccg gccagttttt     60
   cttttattct ctgtctgctt ccttcatttc tcaaaggcat ctcaaactca gtgggcccaa    120
   aaccaaagtc aagctccatc agaacatcct gctacttcca actttaaaat atatcttaaa    180
   tcacaatttc ttgctacatg caaccctttg gttcaaacta ccatcatccc tcgcttgaac    240
   ttagaattga ttttcctgct tcagtatttt tttaaatgat gaaatatttc aaataaacca    300
   tgtgtggctt tatacttttt attacataaa aaggatgaaa atctgccggg tgcactggct    360
   cgcacttgta atcccagcac ccatcccagg ccgaggtgga t                        401
<210> 111
   <211> 401
   <212> DNA
   <213> homo sapiens
<220>
   <221> variation
   <222> 183
   <223> /replace="g"
<220>
   <221> variation
   <222> 200
   <223> /replace="t"
<220>
   <221> variation
   <222> 313
   <223> /replace="g"
<220>
   <221> variation
   <222> 332
   <223> /replace="t"
<220>
   <221> variation
   <222> 343
   <223> /replace="g"
<220>
   <221> variation
   <222> 390
   <223> /replace="t"
<400> 111
   tttattttct tcaaaacatt aaagggaaga actaaaaaca aaaaccagca aacaaaataa 60
   tatttgtgaa aagtgatttt caaaaatttc caaactgctg tggggtgatg agtgacacac 120
   atttccataa agtttctcat tctatctgtg tctttcagtg tgaacaaaaa atttctttct 180
   aaaatgtgaa gattttactc acttcactac tacctccttt agtggttgga agtcatttga 240
   atttttcttt acttttctag caagaattct ttgttttgtg ctatgactca cgtgaataat 300
   agctcctggc ttaatctgaa gctctgtgtt ggctctgcca gcatgagaca ggaagacctt 360
   ctccactttt gcccagtaat tttcattctc cccccacagg g 401
<210> 112
   <211> 401
   <212> DNA
   <213> homo sapiens
<220>
   <221> variation
   <222> 18
   <223> /replace="g"
<220>
   <221> variation
   <222> 40
   <223> /replace="c"
<220>
   <221> variation
   <222> 157
   <223> /replace="g"
<220>
   <221> variation
   <222> 177
   <223> /replace="t"
<220>
   <221> variation
   <222> 372
   <223> /replace="t"
<400> 112
   ggggccacca tctgtgtata tttgcacaac ctccccataa ctgcatgggt tttctctgag 60
   tactctagtt tgctctctca ttccaaagtc atgcacatta ggttaattgg catgtctaca 120
   tggactcagt gtgagtgaat gtgagcatgg gtgtgaatgt gctctgccat gagagactgt 180
   cctgttcagg gctggttccc acctcactcc gtgagctgct gggatgggct ctggccaccc 240
   atgaccatga actggaaata agcaggtaaa gaattatctt acttggtttt attaatcttt 300
   cttaaatgta tgtatagttc acatttattt caatgtttaa tattagaaat gttttgcctt 360
   tatttagaag tgtggtattt tgtgatcaga aatctgccat a 401
<210> 113
   <211> 401
   <212> DNA
   <213> homo sapiens
<220>
   <221> variation
   <222> 6
   <223> /replace="t"
<220>
   <221> variation
   <222> 30
   <223> /replace="g"
<400> 113
   agagactgtc ctgttcaggg ctggttccca cctcactccg tgagctgctg ggatgggctc 60
   tggccaccca tgaccatgaa ctggaaataa gcaggtaaag aattatctta cttggtttta 120
   ttaatctttc ttaaatgtat gtatagttca catttatttc aatgtttaat attagaaatg 180
   ttttgccttt atttagaagt ttggtatttt gtgatcagaa atctgccata ggaacttcat 240
   tatagtttat atcaattagc ctatggaaaa attgtttttg ttatatgtca ttttgcttaa 300
   agctgcagtt tccaggactc tatcaacaac attaagtgag gactttgtgt gatttgcggg 360
   gccaagtgca aaatgaaatt gtagggtacc ttgttcaaaa t 401
<210> 114
   <211> 401
   <212> DNA
   <213> homo sapiens
<400> 114
   cttaaagata acaaacatat atgtttaaca ttttgagttt ttaatctttt tttgtttaga 60
   acctctttat ggaaacatgt ctagcttact aaggtaaaat tgactcactc attgactcat 120
   ttagcaaatt atttattgat ctcctattta tcagacaggg tggaatatag aaaaggtctc 180
   tgcccttctg gagtttctaa cccagtgtgg aaaactgata atacataact aaataactca 240
   tataccagtg taattacagt ggataagtgc taggaagaaa atgattgtga tagtaggtaa 300
   ccaggagggg tgtacttgga taactatcac ctcttggatc ttgactcctg gcttgatctc 360
   cctcttagag ataggcaatg caagtggttt tttgtattag c 401
<210> 115
   <211> 401
   <212> DNA
   <213> homo sapiens
<220>
   <221> variation
   <222> 128
   <223> /replace="c"
<400> 115
   tctttcttag atgatctatt caaagtgtgg ttatctactc actatttttg tttttctctg 60
   tgcaggaggt accagatgcc tctagtcagc cgtcttatag cccctcctat atcgttttaa 120
   tttttgtatt ggtcaaactt gcaaaaggtt gtctactttg ttgatgttaa aattttttcg 180
   ttttatcgaa ttttgatttt ctaattcaca cttcaggctt ttatatttat ccattctgtc 240
   cttgtagctt agttggattt attttgttgt tacttcctgt gcttctttgt ttgacgtatt 300
   agtttactta tttacatccc caccagcaat gcacaaaagt tctaattccc ccacatccta 360
   ccaacacttg ttactttctg ttttgttttt tttaattata a 401
<210> 116
   <211> 401
   <212> DNA
   <213> homo sapiens
<400> 116
   aagggtgtat cacaacactg ggaagcatat tccttaacat taggagttta tgtgatttag 60
   ctgaagagag aacacctgta ttagttaaaa gggaatattt agcagcattt aaacaaaagc 120
   tatttcacat gtcactaagt atactataag aatttccaga aagaagtatc atcctttgtc 180
   taatagttca gaggatttct attagtaaat gaaaaatgac agcctcattt tatcctccaa 240
   atatgtcaaa attatcttga gttgttgggt accagaactg ataggtgtca tgtatttaag 300
   cccatttgaa aaatacattt caataatgga gtgagactct gtctcaaaaa aatttcaaat 360
   gtcttttctc aattgaaaaa acctattatt tctagataga t 401
<210> 117
   <211> 401
   <212> DNA
   <213> homo sapiens
<220>
   <221> variation
   <222> 39
   <223> /replace="t"
<400> 117
   cagtgccttg atcttggact gctcaacctc taaaactggg agaaataaat ttcattattt 60
   ataaattacc cagtctgggt atgtttttaa tagcagcatg gatggacaaa gacagtgtgt 120
   aacactcagt atcaacaaac tccaaggaaa tgggcaccct gatactctat tgtgtagtgt 180
   aaattcacac aaattttgga aagcaatttg gcaatatata tcaaaatctt aaaaaagttc 240
   taggcacaag aaccctgttt tagaaattaa gtctattgga gtagtctgca ggaagttgtg 300
   tctgtaaatt tgtttatcac agttctgttt atactggaag ataaccctca agaaatgtta 360
   tatatctaat aatagaggat tacaattaca tgatgaaatt t 401
<210> 118
   <211> 401
   <212> DNA
   <213> homo sapiens
<400> 118
   tttatttagt tctgctctga tctttgttat ttcttttctt ctgctgggtt tgggtttggt 60
   ttgttcttgt ttctctggtt ccttttcagt agttaggggt taaattaaac tcagaaatca 120
   gtctatgcat accctgacct cagagatgat gctgttccac cacacctttg ctgggcttgc 180
   attctacttt ggaattccac atcttgagaa ttatctcttt aatggcaaaa gtgccaataa 240
   gcctttccca attttctcca ttgtgaaagt aacatctctt atatcataat gtatataata 300
   tccatgtcaa tgtattttgg gtgtaaaggg tgaaagttag gaagtatcca gttggaattc 360
   tgtgtaaagt gcactgaatc tggggaggca gagaatgctc t 401
<210> 119
   <211> 401
   <212> DNA
   <213> homo sapiens
<220>
   <221> variation
   <222> 1
   <223> /replace="g"
<220>
   <221> variation
   <222> 76
   <223> /replace="t"
<220>
   <221> variation
   <222> 157
   <223> /replace="t"
<400> 119
   agtttattta ccttttctag gattttggga aaagagaagg gccttgcaga gcccctctta 60
   ttaataaccc ctgcccaacc cccctacttt tccagatcct ttccctttct aaggagaggt 120
   cctgcaggac gcctaccaga gccatgcatc agacagcggc ttctgcccag tcacggctgg 180
   gtgggtgtgg ccagcttatg agaaagttca cgtaagaggt tgatttaatc tctccagttt 240
   taaaatgtgg aatggtatgg tctgaatggc tttgaattag aataaggtct aaactgattt 300
   gagagcaagg cctggatttg aaagggagag tgatgaaatc aaagaatgtc agagccaagt 360
   aatgttagag atttaacaag aactgcatgt attccctgtt a 401
<210> 120
   <211> 401
   <212> DNA
   <213> homo sapiens
<400> 120
   atctggcaaa agaaagtcat tcagattaaa aaagaataaa ttaaattatc cctctttgat 60
   gatgacataa tcttacagct agaaaaacat aaagactcca ccaaaaaact cttggctccg 120
   ataaataaat tcagtatagt tgcaggatac aacatacaaa aatccataca tttctatata 180
   ccaataataa actagctaag aaaggaatca agaaggcaac ttcatttaca gtagcaacaa 240
   agaaaacaaa atacctagga ataaattaac caaggtggtg aaaatctcta aaagtaaaac 300
   tataacacat ggataaaagt aaccaaaaag cacatcaaca aatcaaaagt caccgcatgc 360
   tcatggatca gaagtattca tatcattaat gtttctatac t 401
<210> 121
   <211> 401
   <212> DNA
   <213> homo sapiens
<220>
   <221> variation
   <222> 328
   <223> /replace="c"
<400> 121
   ccaattgttt tctagcaatg aaaggaatca tgtaaaacca cactagttac cttaatccca 60
   gttagaaaac actagaggaa ttcgttatga aacccaaaga catgatgacc ttcacaattc 120
   tccaggcaag ttcttcatgt accatgacaa gtgggactga gagttttggg aaatgcagaa 180
   gaaatgtgtc attttgaaga attctatgtg gaagattggg attctagtgg cttaaaatga 240
   tgtggagtga aattgttttc aatgaaaata tttatcaaag ttatatcatg agtatatcta 300
   accctatgtt tactgtattt gtgctatgat atgaatatat tttgatttag tatttgatca 360
   gtctttactg attgttgaga atctactaac tcagtgcttc t 401
<210> 122
   <211> 401
   <212> DNA
   <213> homo sapiens
<220>
   <221> variation
   <222> 16
   <223> /replace="g"
<400> 122
   gaaaagtaaa gtggaagtag gcataaagat gacactgcag gtctcaattg gagattcaaa      60
   gtcagtcatt tccccagtta ggctctaaca caatgtaaca tgatgagggg agggatttgc     120
   taatcacaac cagagtcacc actgagtgaa ggcatgttct atgccaggct cccaaattaa     180
   ctaccctcat taatttccca atcttgcttt tggggagcca caccccacct cccttgatca     240
   cactagactg tagcaaagac agatgataac aataatggtt aatatttact gagagcacac     300
   caatgccagg cactgctcta agccttttgt gtgttttaac tcattttatt ccctacagtg     360
   accttctgag gaagtaccta ttatgatctt tatttttcag a                         401
<210> 123
   <211> 401
   <212> DNA
   <213> homo sapiens
<400> 123
   acctcaccca gctcccatgc aaccaaaaag gccagtctca ctcccactgt gcgcctgcca      60
   acagcaccaa gtttatttcc aggcagtggg taagcagtgc tgagaacttg ccccgggcta     120
   ccagcctccc agctgagaga gcaggcaagg cttttgcgct tccctgcctg ttgagtctgc     180
   ccacaagatt cacgccatcc actgagttct ggtcaggaag cttcatgttt ggttggaatt     240
   attaccaagt ttgtctagag gtttccttct ccctgtggtc ttttccaagt tcccctggta     300
   gccctcccca aggacgccta tgagacaagt cagaaatggc ttccctgggg acccagaaat     360
   cccacggggc ttttcctgct gcttcctcta ccctgtattt t                         401
<210> 124
   <211> 401
   <212> DNA
   <213> homo sapiens
<220> variation
   <221> variation
   <222> 182
   <223> /replace="t"
<220>
   <221> variation
   <222> 285
   <223> /replace="t"
<220>
   <221> variation
   <222> 325
   <223> /replace="g"
<400> 124
   aacagaacaa agacataaaa acagataatg gtatgagtac tgcagttgaa atgcaattag 60
   ggattcttta gcctacttgg tctttacacc tcttgccctg ttccccatcc ctatgtggca 120
   tcacaagaaa tcttacagcc tgaaaaatat ggaacataaa acactgttct gtcatttatc 180
   acacaaacag ggtcaatttc tagaataaaa gaagacattc ttaataaatt tattctggga 240
   aaaagtctta aacgggtgat accaggaaaa ttgggacata cggtcatcct ggttctatac 300
   cacaagagtt gaaatgagag gaaaatatgg ttaagtgaga agaaatggat tccagcccca 360
   tttctatcat ccacgagctg tgtattctta aaaagttact c 401
<210> 125
   <211> 401
   <212> DNA
   <213> homo sapiens
<220>
   <221> variation
   <222> 81
   <223> /replace="t"
<220>
   <221> variation
   <222> 212
   <223> /replace="t"
<400> 125
   atgactggca cccaaatagg cttgctgggt tggtgcactg tcaggtttcc aattctttcg 60
   atgtgtctgt agccttggtc aggccaggtc tgccttgtcc tcctggatgg acatttagga 120
   aatgcatcct aaccccatca gcacagtcat gcactggtct tcttttccca tatgtgtgtg 180
   ccgccatctg ggacaactta cccattgtct gctagggcct ttgatgcttg attataggga 240
   aagaggaaaa catcagctac aatagagcaa tctgttctgt ttgggacatc ttacaatctt 300
   ttttcctcct tttctttttt tcaatgcaca tatttttgat catcttcaca tagggatgta 360
   tattggtaat ggaatgtatt ctagaattct ttctcttgaa a 401
<210> 126
   <211> 401
   <212> DNA
   <213> homo sapiens
<220>
   <221> variation
   <222> 348
   <223> /replace="t"
<400> 126
   taaatattat atgctattaa ttctaaggtc ctgacataat actctgccat taagcttttc 60
   atctggaggt tgtatattgt tttgtaatta tttgctaatt aatcaggcac tagttctaga 120
   atttaggaaa caccagctca gtagcttctc aaccatgatg aagagtagac agtattagaa 180
   aaacacagta gtgggagagt agaagattgg gggaaggaaa agagagtccc taagcacaaa 240
   gtgtgcttct ctacctctcc tccttctcca ttctgtgctt ccctggcaga tcttctgggc 300
   gaggagccaa gagaaggttt gaaagtttgt ctgcagctag ataaacaact ttagtggaga 360
   gatggatttt ggcgtcactt aggaggtccc acctaatttt g 401
<210> 127
   <211> 401
   <212> DNA
   <213> homo sapiens
<220>
   <221> variation
   <222> 56
   <223> /replace="g"
<400> 127
   ttcaattttg tcagtgtttg cttcatatac ttgaggtgtt ctgtgaaagg gctgtatata 60
   caacatgatc tccaaatgca gattgagcca agaaatgaaa aaatgagtca gacaaatcca 120
   gtttgtcagt aaatggtgat ttattagagg acttacagac agaaatgtgg tcttgggtgg 180
   ctgcaagcca ggaagaattc tgcagtgcaa ccctctaggc ccagggctta tatatcttgg 240
   gtggggggta agtacatatc cctggaagaa atgtgtaggt gacttaaaga atactactgg 300
   catcacaacc tatgatgcat ccaacatcaa gggctgtttt ggaggaaagg tgaaacttac 360
   aataaatagg tatttctaca tagggagtaa tatatcaatg a 401
<210> 128
   <211> 401
   <212> DNA
   <213> homo sapiens
<400> 128
   actttcactt tactccattt ctaagtttga tattctggta gaattaaagt aagagttttc 60
   tttctttcat ctccttttta tctctctcat attgatttct tttaatggaa aattttttta 120
   tttattattt tatcgttccg atagactttg ccgagcatct ctcctcaaat ctgtatccag 180
   attatttcct caattgtctt aagttggctg tgctatatca ttagtttatg tctttttatc 240
   ttcccatgta aatactttac taattctggg accagaatag atatctttgt atgtgtttta 300
   gttagctatt gctgtataat tccagtatcc ataggcgcac tttgttaaag agtggctcat 360
   ccgtctatgg tcacatgaaa ggaagtacaa ttcagggttc t 401
<210> 129
   <211> 401
   <212> DNA
   <213> homo sapiens
<220>
   <221> variation
   <222> 157
   <223> /replace="t"
<400> 129
   aaacattgta atttaagaat ttcttttgaa atttttattt tcctctgcca gttaattcag      60
   tgttatttta ttttaccagt tgggatcaat aaatctttgt tacagtcatc aaccatcact     120
   atgataaatt ttgaatttca tgagctactc actcaacatt tttaaaatta tttcaaaatt     180
   ggacacttaa gttcatctat aagctgggta taggaaacag ttaagaatgt gtaggtatca     240
   gtgcaaactg tgtcttcctg tacctagaaa atcccctgaa tctttctccc attccaagga     300
   cagaatttct ttttcctact ttgcactttt tccttctctt taattccatt aaaatgaagt     360
   tgcaatctct agatggcaga tatttcttaa gcacaaaaac t                         401
<210> 130
   <211> 401
   <212> DNA
   <213> homo sapiens
<220>
   <221> variation
   <222> 387
   <223> /replace="g"
<400> 130
   ccacagcgtg tgatctcttt gcacatgctc acccccactt aaactttccg tcattagtga      60
   aaacatcatg aggccattac cagatgcagc tgcctaatct tggacttttc agccaccaga     120
   accatgagcc aaatatacct cttttcttta tatattaccc agcttcaggt attctgttgt     180
   agcaatacaa aatagactga aacagtgtgt gaatgtggaa aagtctggga atgtgcaagt     240
   aggcagtaag atgctgtcta accaaagcct gaactgaaag ggcagtgatg ggtatttctg     300
   ttttcccaaa gatttttcat atcatagtgt aattatatca acaagcaccc aaagttttca     360
   gaagtttcac aatatgccta cattaaagca taccagatta t                         401
<210> 131
   <211> 401
   <212> DNA
   <213> homo sapiens
<220>
   <221> variation
   <222> 46
   <223> /replace="t"
<220>
   <221> variation
   <222> 69
   <223> /replace="g"
<220>
   <221> variation
   <222> 108
   <223> /replace="t"
<220>
   <221> variation
   <222> 323
   <223> /replace="c"
<400> 131
   cttcaccttc tcaggtaata cctgatagtc caccaggcag acggggttgg cgatgcttcc      60
   agcactgaac actctctggg tttgctagaa ctacccaggc caacacccca ccactttttt     120
   ttcctctcat ttttgtaatg tggcttctta taaatttaaa cttgtttcag catttgcctt     180
   gttgcctgtc tggaaataac aagttgtttg ttttgcttta taatttgaga aacaggctac     240
   cattcccttc ccatcatgga aaacattctg caatgatctg tatcatcttc caatcatggt     300
   aatcactgac taacctgttt tcaccagaga ggttttttat ctaattggaa gtttgagcct     360
   gaaactcaga gaagataaat ggacaatttt ggtctcacgt g                         401
<210> 132
   <211> 401
   <212> DNA
   <213> homo sapiens
<220>
   <221> variation
   <222> 141
   <223> /replace="t"
<220>
   <221> variation
   <222> 337
   <223> /replace="t"
<220>
   <221> variation
   <222> 392
   <223> /replace="c"
<400> 132
   tttttactcc actcttgcta tcttacctcc tgcttttttt ttcagtgtat gtctcctctg      60
   gtcacatgat tatatttatg gggtgcactt gatcctctac tgtgttattt tctattccta     120
   catgatctta tttagtaccg gggctttaaa gaccatctgt tgatgactcc caagtcagta     180
   tttctggctc cattctcctc aaatggtaaa ctcaaactga atatctcctt ttggatatct     240
   gaaaggcatc tcaaatccaa catctcaaac attactctga gttctccacc ccatatgctc     300
   ctctaaaatc tttcccatgt tattaaatgg tcccacctta aacacacacc taagagcaat     360
   tcttcactct tctccttccc aaaagatcta catctaattt a                         401
<210> 133
   <211> 401
   <212> DNA
   <213> homo sapiens
<400> 133
   aaaaataaaa gacaaaagta attaagtggc aaataaaaag tagagtggat cgttaagact      60
   aaacactatt tttaaagacc agtataacag acaaatgttt tgatggtgaa agagagtgag     120
   agtgagagag agaaagtgag tgagaaataa tataaacaaa caatatcagg aaaaaataat     180
   ggagcacaaa catataatga agtattttag aaactatgaa ttgatatgaa caactgtatg     240
   ccagtaggtt aaaagcccat gcaatggaca attttctagg aaagtgtaaa tgccatgatt     300
   agtcttcaca accataaaac aagtattaat acaaattgaa atggtaatca gtatcttatt     360
   cccaaaggca tcagacccac atggtttttc cagtaacttt c                         401
<210> 134
   <211> 401
   <212> DNA
   <213> homo sapiens
<220>
   <221> variation
   <222> 5
   <223> /replace="t"
<220>
   <221> variation
   <222> 45
   <223> /replace="c"
<220>
   <221> variation
   <222> 266
   <223> /replace="g"
<220>
   <221> variation
   <222> 341
   <223> /replace="g"
<400> 134
   ggcactattc ccaatagcaa agacttggaa ccaacccaaa tgtcgaataa tgatagactg 60
   gattaagaaa aagtggcaca tatacactat ggaatactat gcagccataa aaaatgatga 120
   gttcatgtcc tttgttggga catggatgaa gctggaaacc attattctca gcaaagtacc 180
   acaaagacaa aaaatcaaac atcgcttgtt ctcactcata ggtaggaatt gaccagtgag 240
   aacacatgga cacaggaaag ggaccatcac acactgggga ctgttgtggg gtggagggag 300
   gggggaggga tagcattagg agatatacct aatgctaaat acgagttaat gggtgcaaca 360
   cagcaacatg gcacatgtat acatatgtaa caaacctgca c 401
<210> 135
   <211> 401
   <212> DNA
   <213> homo sapiens
<220>
   <221> variation
   <222> 323
   <223> /replace="g"
<400> 135
   gaatccggga ggcagaggtt gtggtgagct gagattgcgc cattgcactc cagcctgggc 60
   aaaaagagtg aaactccatc aaagaaagaa agagagagag agagagaagg agagaaggag 120
   ggaaatatta tgaaaaaaag tgagacagag aaaaccatac agaccagagt gaaacgcagt 180
   taaagctaat ccgtggtggt agaggtcaga atatagatac cttgaagact ggctggttaa 240
   ggaacccaag gaaacctttt gaatgtttct tgacatggtt gcagattaca tgagtatata 300
   cataagtaaa aattcattat gcattttatc gtatgtaaac tacacctcag tttaattgaa 360
   aaatagatct tatatatctt ttattatgtt tattgacttg a 401
<210> 136
   <211> 401
   <212> DNA
   <213> homo sapiens
<400> 136
   atttacaaaa tatatctttt cccttcttta aaaaaacttc atagtaaatt atgcataaag 60
   cttaccatca tatcattttt aagtgtacag tttactggtg ttaagtacat tcatgtccat 120
   cactgccact gatctccaga actctcttca tcttgcaaaa gtgaaactct atacctcatt 180
   cctccctcca tttatgccac atcaaaccac cattctattt tctgtctgta tgaatttgac 240
   tattctaggt atcttatata agatacctta tataaggtac cttatatctt atataatatc 300
   atctatatta tataagtggt atcttatata agattccact tatgtggaat catatagtat 360
   ttgtcctttt gtgactggct taattcacat agcataatgt t 401
<210> 137
   <211> 401
   <212> DNA
   <213> homo sapiens
<220>
   <221> variation
   <222> 114
   <223> /replace="t"

<220>
   <221> variation
   <222> 281
   <223> /replace="c"
<400> 137
   aaataagaag aaaaaaagga gtcatctctg ggactacacc ttgagaatct caagttttaa     60
   tggatggctt aggaattact caagttagga ggcagaaaga gccagcagag aagcaggagg    120
   aaaacaggaa agagctgtga cccagagtaa agggccatag aggtcagctg tatcagatgt    180
   gaccaagagc tcaagtccaa aaaggtctga ataataaccc ttgtgatggg gaggttatga    240
   ctgaccccca ggggctattt cattgaaata atgggatcag aggacagttt ggagatggtg    300
   gggtgtgact gggaaataag aaagttggaa tagcaagtat ggaggcctct tcccagagag    360
   gaagaggtgg catggcctct ggagccagac aatccaagtt t                        401
<210> 138
   <211> 401
   <212> DNA
   <213> homo sapiens
<220>
   <221> variation
   <222> 124
   <223> /replace="c"
<220>
   <221> variation
   <222> 254
   <223> /replace="t"
<220>
   <221> variation
   <222> 343
   <223> /replace="c"
<400> 138
   tgtccatatg ttcacagcag agtttttgat gtgtttgatt gtggggtgat accgcagacc 60
   ccactgaggg tataacacac accaggtgac ttcactaaaa tccccacggt gaaacacatt 120
   tgcggccctt ttccctgccc ctggattttg ctaaggatct ggggggttct gtggaagtct 180
   gaaggtcagt ggacaggacg agtttggaga tgcgcatttg gaaactgtac ccctcaagtg 240
   acaaggaaat gagcgtggat ttaagaagat aatgaccgtt ctgtgaagtt ttaatggaat 300
   ttttaagcaa gaaaaccctt accctgtgaa aacttcagtc tgcacagaag aattggtggg 360
   agaaggaaga ctttgcctaa gatcacttta tatagcacaa c 401
<210> 139
   <211> 401
   <212> DNA
   <213> homo sapiens
<220>
   <221> variation
   <222> 142
   <223> /replace="t"
<220>
   <221> variation
   <222> 374
   <223> /replace="g"
<220>
   <221> variation
   <222> 395
   <223> /replace="g"
<400> 139
   tatacacaca aggaaacccc ctcccccaaa cccaccagaa tattgttttt acctctgggt 60
   gttgagacta tagagtttta atttctttat atattcttct gtttttctag aataaatgca 120
   tattatttta taattacaaa ggctactttt taactcttca taatcataac ttataatggt 180
   aaaatttata taaaatttca atctgtgcat atgtaaataa attaactttc ttgttatgca 240
   tttaagttag ttataggttt ccactgtttt cttcagacaa tgattatcat tgtgtgtaat 300
   ttcaacctag taactcaatt gtttctgtag aattgatagt agaattaatt actcaatagt 360
   tatggatatt ttaagactac ataaatgcta ttcaacactc t 401
<210> 140
   <211> 401
   <212> DNA
   <213> homo sapiens
<220>
   <221> variation
   <222> 1
   <223> /replace="t"
<220>
   <221> variation
   <222> 160
   <223> /replace="g"
<400> 140
   cctagtgcac atgtattgca gtactagaat tgttaaccca tacccacatg ggaaaaagtt 60
   tatcaactag agtacagtgc ttatgtacaa tgttttttgc aatctgaggc tccactgatt 120
   tctaaaatta cttaggtcag catctttttt cttcacccta ttcagtgaag tgtttcatcc 180
   gtttgtaata cagttagatt atttgtaaca gtctgaattt cattttgaga tccctgacct 240
   gctaaattat acttttaatt ggcacatatt attgttccta ctattcactt ttttaatgaa 300
   gtgaacttca cagatatgaa attaaccatt tgaatgtgaa caatttagtg gcatttagga 360
   cattcacagt gttgtgtaac tacctctaac tagttccaga a 401
<210> 141
   <211> 401
   <212> DNA
   <213> homo sapiens
<220>
   <221> variation
   <222> 122
   <223> /replace="t"
<220>
   <221> variation
   <222> 212
   <223> /replace="c"
<220>
   <221> variation
   <222> 399
   <223> /replace="g"
<400> 141
   gttgctgtgt cttcgttttt ttcagataac ccccaaatca gtgaataaac tagttcagag 60
   tcacacggcc agtaagaaat agggtcaaga tctgagctca gcaaagtctg tctgcttttt 120
   tctacatcat accatgcttt taagcttaat acataagaca gattatttcc tgcttcattt 180
   agtcgtccta tttctgcctt atttagaaat agttccaagg cgatttagtt tcttgcactt 240
   ttctaccctg ttgctgctgt tttatttttc ttatacttca tttcacttga actcctcacc 300
   tccagcttca tttattctct tggggtccaa aaagaaaatg attataaacc aaggaaggtt 360
   gcaatgatcc tttcccccag ggaattttcc cagggctgaa g 401
<210> 142
   <211> 401
   <212> DNA
   <213> homo sapiens
<400> 142
   cttgctttga ccaatgggat gtgagtggac ataatatata ccagtccaag tagagaattt      60
   aacccaagtc aaacatgtgt ttggctcagt ctcctcactt ctgcccactg tcctaagagg     120
   agttttctcc aggtaaacac tactgcttca acctgggtcc cagaaagaca aacacatgct      180
   ctagatctgt gtcacctacc attgccagct gacccaacac agaggactta tgaaaaatac     240
   ataatagttg taagctactg agatttggag ttttttatta tagagcattt tcatagccaa      300
   agttgattaa ttcatacatt aaacatcttc ttaaccacaa gacttccagg gcattcagtt     360
   tgctgatgtc ctctgtagat ctcaaaatta attggcccta g                         401
<210> 143
   <211> 401
   <212> DNA
   <213> homo sapiens
<220>
   <221> variation
   <222> 3
   <223> /replace="t"
<220>
   <221> variation
   <222> 273
   <223> /replace="t"
<400> 143
   aggtggaatt ttgaatcaca tgggaagttt cagtgtttca aagcatgaag tagagcttcc      60
   ctaacccctg ctatgactac tctgctttca gtgttggaaa ggaaatgatc ttttaaaagg    120
   tggaatatgc aaaaggggaa aacagttctc ctctgtcttt tcctggggtc gggaggggga     180
   ttgaggcatt tccccaaaga tccctgctaa ctctgtgacc ttccaagatt cgttaactgt     240
   gccagagaag gaaatgtacc tgcaattgct cactggggta atatttaagt caaggctgga     300
   tttcaagctc aatttaagtg ctacattttg atttttttta agaagaaaga tttgcctgta      360
   aaaatgctac ttttcttata caagatctat aaacatgctt t                         401
<210> 144
   <211> 401
   <212> DNA
   <213> homo sapiens
<220>
   <221> variation
   <222> 217
   <223> /replace="g"
<220>
   <221> variation
   <222> 353
   <223> /replace="g"
<400> 144
   ttccctaatg attagtgaca ctgagcatct tttcatgtgc ttattggcca tttgtatatc 60
   tgctttgggg atatgtctaa gtcttttacc cattttcaaa attgatacgc agcagatgta 120
   catattttcg gaattcacat gacaatttga tacacttata taatcaaatc agattaattg 180
   gaatattcgt tatcttaaat acttttcttt atactaagaa cattcacaaa attatctttt 240
   agctattttg aaatatacaa tgaattaata tctgcagtca taccacctga atgcatctca 300
   tctcatctgc ccatttttta attgggctac ttatattttt gttgttgaat taagaaaaat 360
   tagattcata gcttaattat gatgaatgta tttatcatct c 401
<210> 145
   <211> 401
   <212> DNA
   <213> homo sapiens
<220>
   <221> variation
   <222> 256
   <223> /replace="t"
<400> 145
   gaatatactt ttatgctcaa acataaaatc tggtaataag gttattatta tatctatatc 60
   tcagcttcat ccaatttgct gtcgtctctc tttattatat acttgattga tggactgatg 120
   caccatggag gcattgctga attgaagaga agagacatcg gggtggaatt aatcatgcag 180
   cagattgcta cataactctt atttcccatg agatgagaat cacagtgata aaccatgaag 240
   acagctgtat gtggcctaaa actaagacca gaatgctcaa ataagttcca atttatcttg 300
   tttccagaga taaattagta ggaaggggtg aaaaggagaa atatttgtga atcatacagt 360
   agtcacagct attggttgat gtctaggata ttaaaatgtg a 401
<210> 146
   <211> 401
   <212> DNA
   <213> /replace="g"
<400> 146
   gctggcttct gagaggggag tacttattca gagaggaata gaacttaata taaacattcc 60
   tccatccaga aaaaaaaagt taactgacac tgggggcaaa agcctggtct ccagctgtct 120
   ataaaactgg ctgttccaat atctgaactc acagaatccc tcccccaact gggagttaat 180
   ttggagtttg agttgaaaac atgttcaaag ttttgcccta aatcagtcag ccaacaggta 240
   tttctgtgcc tgtccttcga gccctgtgct gtacacttaa attttatttc ttaggagcca 300
   aaaaaaaaaa aaaaaaaaaa tgaaatctat agaactgatt cataggtcgc aacaaaatgc 360
   acaggttggt gctcagtttc ttcctggctc cttatctttc t 401
<210> 147
   <211> 401
   <212> DNA
   <213> /replace="t"
<400> 147
   gctctaactc aacctttcat ttgtttcaag agggaggtat cagaaccaca acaaatggaa      60
   tgaaacctac cactaccacc tgcctggcac aaagaaataa tttacaattc acggggaatt     120
   tcctgtgttt cattttgtct gaagaactgt aaccggactt gaatgaacta agtgtctaat     180
   tgttgcatta tttttcttgt agagatttta gtgttttatt actttctcca gtttacttca     240
   atagtgaatt aggttccctc taccatctga aatgttacag ttcgataaaa taggtaaatt     300
   aataaagtct aaagtgcttc caagctgagc agagaccttt ctgtgcgctg tgcttttatt     360
   gctcttccca gctgatattt ataactgggc catatgtggt t                         401
<210> 148
   <211> 401
   <212> DNA
   <213> homo sapiens
<400> 148
   ggataaagtc ataggaatat ttttaaagcc tttcatggta aacagaagaa aaagaatact      60
   aaaagtacta taattattga gcattgtgag gaagaagcaa gcagaaagaa ggcattgaga     120
   tacctctcat agtgaaaaac caatggattt ttgtctacct ttgttaaaat tcaagaattt     180
   aatgagtcat ttgttaaact actggaagct tgagattggc catgatggga atatttacat     240
   cccagaaagt gaaaaaacaa acagattaga aagcaaccat gttaactaaa aacagtattg     300
   aaaagtgagg caggggcagg gaggagttag cagaaggaga ctgcctgttc ttttgttgct     360
   gagtatgtat ttttctatac atacgtaagt atttattacg t                         401
<210> 149
   <211> 401
   <212> DNA
   <213> homo sapiens
<220>
   <221> variation
   <222> 121
   <223> /replace="t"
<400> 149
   gataaacctt tatagttaga aagagtctaa atgttggtgt ctgtaagcac aataagtaaa 60
   tgagtttggt ttttccttcc aaaataactg aacgttcact ttaaatataa tgttagccag 120
   ctttgaatcc agaatattta catcttgcag actgtgtctt gcagcctctg aatcaactga 180
   ctgggagcat ccgtctctgc atgcagggct gctgaaaggt gggagcagag ataaaagaca 240
   ggagatggga agaaacaaag ggagccaaga gatttcatag aatgctgcaa gagacattct 300
   tttgatctat ttggactcct ccaaggcggg atccagagaa agaagagtgg caggttggag 360
   accacacccc tagattagct ctcctttgaa ctgggatggt g 401
<210> 150
   <211> 401
   <212> DNA
   <213> homo sapiens
<400> 150
   ttcaaaaaat gtctctgcat gtcttttgct cgctttttaa tggggttatt tgctttcttt 60
   tttttttttt tgagtgtttg agttccttgt aaattctgca tattagtccc ctgtcagatg 120
   gatagtttgc aaatattttc tcccactctg caagctgttt gttcactctg ttgattgttt 180
   cttttgctgt gaagcttctt cgtttaatta agtcccattt gtctactttt gttttgttgc 240
   atttgtttct gaggtattag tcatgaatcc tttgcctaga ccaatgtcca gaaatgattt 300
   ccctaggttt ctttctagta tttttatagt ttcaggtctt acatttaagt ctttaattca 360
   tcttgagtta gtttttgcct atggtgagag acagaggtct g 401
<210> 151
   <211> 401
   <212> DNA
   <213> homo sapiens
<220>
   <221> variation
   <222> 218
   <223> /replace="t"
<220>
   <221> variation
   <222> 344
   <223> /replace="g"
<400> 151
   attgatagtc tagaaacttg ttagagagaa aggaatttaa tcaaaggtag aagtaattta 60
   tagggtaagt ccgacatata catactttct ctaagccttg gcttgattgg ttttcatttt 120
   tgggatgtgt atttgacatt tgaaacaatt ttggtcaata atgctttcat gcgaggctga 180
   gagttctttg acctatacat agaaaatgag ggagagccaa gagaaatttg gtgctggtgg 240
   tgactgctga actttcagga tcattaaaat atactgctaa ctgcatgaaa taagcacatt 300
   tgactcaagt ttctccttgg ggatggtatt ggtcagtttc tcaagagaga agccctcttc 360
   ctctccctta ccaaagactc tgtctccagt taaacaggga g 401
<210> 152
   <211> 401
   <212> DNA
   <213> homo sapiens
<400> 152
   aatttcaaat gggtcttgta ttcttaatat aaaccttatt tggatataac atatgattct 60
   tttaatatat attgttgatt ttgacttgat agtattatac taaggttttt gggtgcatat 120
   tcactaaaga gtagcaccta ctattttgtt ttctggaatt gtctttccct gatttggata 180
   ttcttgtttt ttttaactag cgaaatgaat tagtaatttt ttgtcttttt ctgttcccct 240
   gagcaattcc tgtattagag ggtctgtttc tttaatgttc agtagtatct catgcctaaa 300
   tctgtgtggg cctagtgtcc ttttttccct ctctttttta ttgaaggact aaagctttga 360
   atgcttttgc agtttcttcc aaagtttatc atctactcag c 401
<210> 153
   <211> 401
   <212> DNA
   <213> homo sapiens
<400> 153
   attacttaaa tttattaata catttttaat atcagatggt aagttacagg atggagctgt 60
   gacacccttg tcatgaacaa gcctacagtt gtagacttat gctcacaaag ggtgctcagc 120
   tccctattaa tgaattgatt gatgaatcaa ttaaatgatg ttgtgattat tggctatcac 180
   tgacacttgc agaaggtagc gctctcttgt gctgatttca tgttaatctc atctaacttt 240
   aaggagctca tttcatgaca catggaattt aaaggccagt actctacaat tcatggacac 300
   tcttctaact caaagtagct attaaggaaa ccaatcttaa agtcaggatg acagggacct 360
   taaagtacca attccatttc cagccttata gactcaacca a 401
<210> 154
   <211> 401
   <212> DNA
   <213> homo sapiens
<220>
   <221> variation
   <222> 257
   <223> /replace="t"
<400> 154
   ccatacaata gatttcctac tttaactacc taaatttgtt tcaaaaccaa ccactattag      60
   gggagttttg tgaacttact tcatgtgcca ttgaatttct aataggtgat atctaatttt     120
   aaattgtagt ttagagaaaa aatgttgcta ttctattgct gaaacataat tgatatataa     180
   ggagagtttg gctttgaata catttctttc tctgtatata tttattttag atttcatgag     240
   cgagtatttg attcttaaaa tattgaggac attgcaaaac atgattttac tggatttttc     300
   agaatcgtaa agaagaataa ttcttttata gtttagtaac tatctaaaat aatttagaca     360
   cttttgacct tattttcttg atgtttcttt gagatgagga g                         401
<210> 155
   <211> 401
   <212> DNA
   <213> homo sapiens
<220>
   <221> variation
   <222> 227
   <223> /replace="t"
<400> 155
   tgtcctttct acctttgcct ggagacttag cagtgcctcc caccacagca gacagaaggt      60
   acatctcact ctaccctctg acatcagctc cctcatggaa cttcctttgg ccaatgagcc     120
   gagagcagag gcgtgagttg tgtctagcca gagggttcaa atgtgcttgc gtgatttggc     180
   ttggcttttt gagcttcagc attctccatg ggaaaatcat gccctgggta ggcattgctc     240
   cttcaacctg tgtcctggaa tgaagacaca tggaacagtc tggaacctaa aacaaagcct     300
   ggagttcagc tgagatcagc taaggtgtgg ctgggacctg tgcatatgga ataaatgttt     360
   gctcttatag gtaaatgcga tgtggaagtt gttcattact c                         401
<210> 156
   <211> 401
   <212> DNA
   <213> homo sapiens
<220>
   <221> variation
   <222> 4
   <223> /replace="g"
<220>
   <221> variation
   <222> 78
   <223> /replace="t"
<400> 156
   aacattcgag gccagtttgc caaaaatctt ggtgctaact gtatgtccag gttacagatc 60
   cacacgcaaa tgcagaccca acaacactga ggctcatctt ttgggtttct ccccatcttc 120
   ttcaccatga ctcagttctc caagaaaaat gttgttgcta ctttcaggaa cagtccagga 180
   tgagagctta aggtagagtc cggacatgtg catggaagca cagccatgat tctatctaat 240
   tggctcaaac aaaagtcttg gacagtattt tggctttttt tttttttttt ttttgagaca 300
   tgatcttact ctgtcaccta gcctggagtt cagtggtgcg atcataggtc actgcagcct 360
   tgatctccca ggctaaagca atcctcccac ttcagcccca t 401
<210> 157
   <211> 401
   <212> DNA
   <213> homo sapiens
<400> 157
   tgcctagcct atattttttg tgattggttt tctgagtcta taaagaaaaa aggatgttct 60
   tcagctattt ggggactttt ggctccaccc caggcccata agtgaatgca acaacaaggt 120
   ttaatgactg ttggacactg tgtttatcga gagacattct gcagggaagc agaaacaaca 180
   taattgccta taaaaacatc ctttaaaaag ttagcagtct aatccataaa aatgaatttt 240
   ggcaggtgca aatgtagcca gggccaaggc agggcgtgcg gtggatgggg gcgggaaatc 300
   cattcaggcg cggaggcttg cctgttgttg tccctggagt gcgccgtcct ctctcttgtt 360
   cctgcttctt ttttactctc ctttctcctc agcagccatc c 401
<210> 158
   <211> 401
   <212> DNA
   <213> homo sapiens
<220>
   <221> variation
   <222> 68
   <223> /replace="t"
<400> 158
   gcacaaccag gatttgaact aaagtttacc ttcatctaaa tacgcgtttt tctttctctc 60
   tctctctctt ttttttttta cccctatcgc atcatagcca catctgtggc cacacaagaa 120
   atactttccc aacactcagt agctaattat tcataaggtt ttagggtgac ggtgggccat 180
   gtgcagagat gctttgaaag acttccagga aaatcatgct ttatggctta gaatttgggg 240
   acatgactgt acctgaattg agatggacag gaaatcattt ctccctcctt cctgcttcag 300
   cacacccctc ttcccactca agagattctg gagacgcttc aaggccatct ccttgccaga 360
   tgcctctgtt ttagaaaagt catttcaagt gccccaagga g 401
<210> 159
   <211> 401
   <212> DNA
   <213> homo sapiens
<220>
   <221> variation
   <222> 54
   <223> /replace="g"
<220>
   <221> variation
   <222> 55
   <223> /replace="t"
<220>
   <221> variation
   <222> 175
   <223> /replace="t"
<400> 159
   gaggttcccg agggctctgg ggaggcggaa gaggggacgc ggtgtgttga gatacgggga 60
   gaagttcaga gcagcatggg catgcaggtg cctgcagagg ggcccatctg ggctgcaccc 120
   cagcaggaca gggccatggc ttggtgtggg ttcggctggc tgcacagctt cttgcaaagt 180
   ttgtccccag ggtccttggg agtagaggtc tttcggaagg caccatctcc agagcgtgtt 240
   tctgagtaac tctgaggatg ggttccgggt tatcactgtg tgttggaaac agcagcgccc 300
   aggctgccgg aggaggctgg tctccctggc aggtcctggc tgtgtctaga gttttgtggg 360
   tcctggagat aaggctccgg tttgaggccc ctggggctgg c 401
<210> 160
   <211> 401
   <212> DNA
   <213> homo sapiens
<400> 160
   gaagtgtctg tgtacacagc tcctgacagt tcctgcctgc ctggctccta gcccagtgct 60
   gttagaaatc tttcccaaac agctctacct ttttgaaagt ccagaccctt gacatccatc 120
   tctccctact gctcagaatt gtctgtatct atcttttacc aatgggggaa aggaaagaaa 180
   aggcagataa agagtgctgc accacagaac cttgggggat taaaaagaag caggctgtgc 240
   tgagagccta ggatcagaaa gaatctcact gcaccctcac cttctttaac ccaggcatag 300
   acttccctcc atccattcca atacctggag tgtctcattg gtgtagatga aaacacggca 360
   gtcaggtata agcaggtcct ggagctctga ctagctcatt t 401
<210> 161
   <211> 401
   <212> DNA
   <213> homo sapiens
<400> 161
   caaatacatt tgttttatga gtgtcaaagg ctctaatata ttaatattac taatatatta 60
   gtacaaaata ctaatataat actaatatac tacaatgtat tatgtctatt atatataata 120
   catcagtata ttagttatat taatatatta ctaatgcatt aataaagaaa aaagatgtta 180
   aactagacag gatccatcgt gtccttgtgg cattttgatg gaggccttca cctcaacagt 240
   gagttaagcc tagagtatga ggatgagttc tgtgcattag ccactttaaa gtttgtttgt 300
   cgttactcag tatacaacag agaaaacctc aaaatccctc ttcacttctc ttccaactct 360
   gcttttccca gtgtgagtag aatcaaacca tatgtactgt t 401
<210> 162
   <211> 401
   <212> DNA
   <213> homo sapiens
<400> 162
   catgtaagaa ggattttatt gttcttctat gcttagcatt ccttctgcta tgtggaatag 60
   tcctggagta acttgacaca cagaaagtac catattttaa acagaagcaa ttcattgtag 120
   tggcttctgc gagcaataga aacttgcaga acgtacaagg aattcaaaca tctcaacaga 180
   aaaaagccca ccaagtttcc acaaaaaact acaattagaa ctgccatatg atctggcaat 240
   cccatttctg ggcatttatc caaagaaaag gaaatcagta tatcaaagag acacctccaa 300
   accccatgtt cattgcagca ttattcacaa tagctaagat atgaaatcaa cctaggtgtc 360
   caacaacaaa tgaatggata aagaaaatgt ggtatatata c 401
<210> 163
   <211> 401
   <212> DNA
   <213> homo sapiens
<220>
   <221> variation
   <222> 136
   <223> /replace="g"
<220>
   <221> variation
   <222> 276
   <223> /replace="t"
<400> 163
   actatgcatt atatgtatcg aaacatcact atgtacccca tgaatatgtc aattaaaaaa     60
   atagtttcac caagacccac taaatcaaca gaattttgtg gcaacattat gcagacccca    120
   ctgttgagag taaacactat ttgatatgat atcaagttcc atcagtttgc atcagatcat    180
   cagatgagag ggccataagc gtcttctgtt ctcataatta tgcagattta ttgttttgaa    240
   acaaaatcaa cttctctcta aaataagcaa ggacactggg gcaaccaact attctcagag    300
   aaggagtgtt tttaattttt ttcaggattc ttaggaaaat caaatatcaa ataacataca    360
   ggaatgtaga gttcaatgac aaaaaggaaa tctcttgact g                        401
<210> 164
   <211> 401
   <212> DNA
   <213> homo sapiens
<220>
   <221> variation
   <222> 61
   <223> /replace="g"
<220>
   <221> variation
   <222> 126
   <223> /replace="t"
<400> 164
   cccactaaat caacagaatt ttgtggcaac attatgcaga ccccactgtt gagagtaaac     60
   actatttgat atgatatcaa gttccatcag tttgcatcag atcatcagat gagagggcca    120
   taagcgtctt ctgttctcat aattatgcag atttattgtt ttgaaacaaa atcaacttct    180
   ctctaaaata agcaaggaca ctggggcaac caactattct cagagaagga gtgtttttaa    240
   tttttttcag gattcttagg aaaatcaaat atcaaataac atacaggaat gtagagttca    300
   atgacaaaaa ggaaatctct tgactgtatg aatagtttca tgaagtcaca gcattggttt    360
   aatagcaaaa attttatttg gaaagaaact tacatttgga t                        401
<210> 165
   <211> 401
   <212> DNA
   <213> homo sapiens
<400> 165
   tgttacctat tatgattaat attgttttcc tttttttctt gcaaatagtt ttaagtctcc 60
   ttcagttcta ttcatttttt ttttctctaa agagcttccc cagcattcct ccattttctt 120
   gaaactgttt atcatgcttg ttttgtgtca agccgtgggg ctctgtagag catgaaacaa 180
   gaataagatt ctattactta accttcattt aatagtatac atttgccaat taagagcttt 240
   acacaaattt tcttattaag tcttttcaac aacttctgga tatggataat gttaattctt 300
   gtccatgata caattggaga aattgaggct taaagagatg aagtcacttt tcaagtgcta 360
   catagatgct aagtgataaa gaagaaattc aaacccagga c 401
<210> 166
   <211> 401
   <212> DNA
   <213> homo sapiens
<220>
   <221> variation
   <222> 45
   <223> /replace="g"
<400> 166
   cccttcttca ttttaaattt attttatttt tagttttgat taaaagaccc acacataaac 60
   ataaaattta ctatctttac catttttaaa tgtacagttc agtagtgtta catatagtca 120
   catcttgctg caatgtccct ccagaagatt tctgtcatct tctttggaaa attttaagaa 180
   actattacca aaaagtgtac cgagatatag ttttaaggct gatgtaccaa tgacaccacg 240
   tgaataaatt ctgtacattc acctgtaaag ctcctcagct tttgggcttt tttctcctta 300
   ttggctggtt ctaaataaat tttatgaatt cccaataaac ggcttttggg aaatgccagg 360
   tcatcattgt aataaaaagt ggtataaggg gaaaacaact c 401
<210> 167
   <211> 401
   <212> DNA
   <213> homo sapiens
<400> 167
   tgtatgacat cactgacagg gactaattta aaagatttaa ttattcccca attcacacat 60
   gtcttgattt atttttgata aagagtagca aatgggtacc ctaggattct atacaaataa 120
   gccagtgtaa tttgatggaa taaacactga tttggatgtt aagagatttt cagctaaccc 180
   tggctcttct gataactggc gttgttactt ggggaagtca gttaatctct cgtttgtaga 240
   atgatggggc taaactaact ggtctcttag aaattgtttc ttcaagacct aaccttctgt 300
   ggttctcctt aatgcctgga tgcaatgacc ttactactga tggaaagatt atcttgggaa 360
   atgttggcca aaactaaacc ttctccccac acatctgttc t 401
<210> 168
   <211> 401
   <212> DNA
   <213> homo sapiens
<400> 168
   acagaaatta gaagctaagc attgcaattt aaagaattat aggaacttag gaatcataac 60
   aattttcttt aaatatcagg ttatttccct taaataagtt atgtgcaagc acttcgtaaa 120
   ttctaaggtc ttaatcaaaa ttttgatttt attactatta ttattgggct atgtgaaaaa 180
   gagaagtgaa atactaagcc catgaaaaaa taagatttta tttaacataa tttaagacac 240
   agagcacttg ttcagactca agtctgttac attcttacat agagtaactc acattattgt 300
   tggaaccaga gctacagttc gtatctttct tgagccttcc tcagggtcca gagttctgag 360
   agaatttcat tatcaccatc tcccaagaaa aacaagcttc a 401
<210> 169
   <211> 401
   <212> DNA
   <213> homo sapiens
<400> 169
   tattttatgt atttcccatt gttctgctat tacaaatagt tgttcagtaa gcattcttgt 60
   acatttatct ttgtgcaatt gaagaccaat atttctctca cttatgtagt gccacccttg 120
   ctagtaggaa tgttccgtta aatgcaaaac ctgcagaact tggactcttc aacatgtttc 180
   tactttgcca aaactgtcta cgcctccctg tgcttgcttg ctgctccttg gctcttcttt 240
   gtctttaatg ttgcccttaa ctctaataaa agcctctgtc ttagtttagg tttccccaga 300
   aacacattta agacaaggat ttgaatgcaa gtggcttatt caagaggtgg cctcaagatg 360
   cacaggtagg ggagtggagg gtggggcgga acagggaagg c 401
<210> 170
   <211> 401
   <212> DNA
   <213> homo sapiens
<220>
   <221> variation
   <222> 56
   <223> /replace="c"
<220>
   <221> variation
   <222> 77
   <223> /replace="t"
<220>
   <221> variation
   <222> 133
   <223> /replace="t"
<220>
   <221> variation
   <222> 246
   <223> /replace="g"
<220>
   <221> variation
   <222> 350
   <223> /replace="t"
<400> 170
   actaataacc tggtctctca ctcatcctga ctgggctgcc ttcccttccc tgtctgtctt 60
   cccctttctt ctccctctcc tcctgggatc acctcccata caaactcctc accctcattt 120
   ccttgtccca aggtctactt ttcagggaac ccaggatact ttcctcctgg tcctaagaaa 180
   acccaggcaa accaagtttc ctgctctcat acaatttaca gcatgttggg ttgaacaggc 240
   aacttaacaa acattaaaaa cgaagtgtaa cgagtgctga ggtcagggag catagactgg 300
   cggaaggcac agagccacag gggaccctaa gctagcccag gagaggtccc agaagctgag 360
   ctgggagtga ctggggctcc ctgccttttc ttccccttct t 401
<210> 171
   <211> 401
   <212> DNA
   <213> homo sapiens
<400> 171
   ggttaaatgc ctttcagggt agctgaaaac tacttaccaa tcagcagctg gcagaaatgt 60
   gtaccaagag ctctcatctg gaagtgggca gctgactcat gttggagttg tcatccctac 120
   tccagcagcc ttctgggtga aaagttcttc aaagcacatg catgtgaggc cagaccattt 180
   ctcttctgtt ttctatgctc ctcaccaaaa aagaatgagt catggagctc tctaattcag 240
   gatccagtag agcatcagac agaatgcagg ctgttgtttg aacatccgtc tccagataga 300
   ctgcagtgtc atttaaacaa agatgtatgc atttgaatat ggtgccatac aattcttgtg 360
   ccgaactggc atatcttctg agggaagatt ttatagaaat a 401
<210> 172
   <211> 401
   <212> DNA
   <213> homo sapiens
<220>
   <221> variation
   <222> 27
   <223> /replace="g"
<400> 172 .
   ggaaacaaaa aggattataa gaaagtaata taagcaactg tacaccaaca aattggataa 60
   ctgaaatgaa atggacaatt tctagaaata cacagtgtat caaaactgaa caacaaagaa 120
   aataaaaaat ttgaatagac ctataactag taagaaaatc tcctgatata gagaagccct 180
   gggctccatg gctttactgg ctaattctac caaacattaa aatacaaatt aatgccaatc 240
   atcccaaaaa ttttccaaaa aattgaagag gaatgaatgc ttcttaactt attctataaa 300
   gccattacct taatactaaa gccagacaaa gacattacaa aaaagaccaa tattccttat 360
   aaacattgat gtgaaaactc tcaagaaaat actagcaaat t 401
<210> 173
   <211> 401
   <212> DNA
   <213> homo sapiens
<400> 173
   aaagaaagat atttctagca catatgagtt tatggatgga gactcatgcc tgccacactg 60
   tgaaaacatt cttcccttca aaatccctat cagtgtaatt tgaagtgaat attctagtac 120
   cacatgcttt tggactctaa ggaagagata taatgtacga aaatgtggta tctgaatgca 180
   gcaaattgaa aaataccagt ccagatactt tggggccatg cagagagttt cccagtcagg 240
   tgtaagtgtg ggtggtggtg gtttgaatac tgaagggatg gacaggtggg ggtgacaact 300
   aagagctgcc acctggggag aatcctaggc agagtctggg cctggacaac ccaaccagta 360
   gtgaccgtgg gccggacgag cctctgggct ctgcagctcc g 401
<210> 174
   <211> 401
   <212> DNA
   <213> homo sapiens
<220>
   <221> variation
   <222> 4
   <223> /replace="g"
<220>
   <221> variation
   <222> 68
   <223> /replace="t"
<400> 174
   tacactaatt catgcaattg tatattttgg aggtaagtgt aatggcatag gtttgctgta 60
   atggaaccga ttggaaaatg atctcttgga gaaaatttat tgctggtcat aagatttctt 120
   tggtttagaa aaatgctggg tttagtaaaa tctgaggcac taaatgaaag tctgctttaa 180
   taaaaatgtg gatctgagaa ctgtggtttt tagtaagtgg gcatttgatc cattaaaatt 240
   gtattggttt tattctttac gttggctttt cctgaaatcc atcagtttga agcatgaagt 300
   aagcacttgc cagatattgc tccaataatg aaacattgaa agcaaggccc tgaataactg 360
   ttaaaacaag tcctattaat cagctgtgac ttgtcccttt g 401
<210> 175
   <211> 401
   <212> DNA
   <213> homo sapiens
<220>
   <221> variation
   <222> 106
   <223> /replace="c"
<220>
   <221> variation
   <222> 294
   <223> /replace="c"
<400> 175
   atccaatcaa ccatcacaga aagaaagcat gaagtccaga tgactggacc aaaagaacac 60
   cctaggttgg atggatgctg gatggccaac tggatgggaa aaaagagggt cttgataaca 120
   ttctaattag ccccaaaccc tctattccca tgagccagtc tcttctcttt aagttgatgc 180
   tagctgccgt tttgtgttat ctgttacaga ctaatacaat ttgcaataat tgaagatgca 240
   atatttattg atatattatt atcaattgaa gtccatagct tacattagga ttcactctta 300
   gttttgtatg ttctgtggat tttgacatat gtaaaatgac ttgtgcccac cattacagta 360
   tcacatagaa tggtttcact gccccaaaat accctctgtt c 401
<210> 176
   <211> 401
   <212> DNA
   <213> homo sapiens
<400> 176 .
   aaatggactt aagccattac ctttggctgt cctagcaggg gagggtttca gactttcatt      60
   tctttcccag cctatggtgt gaggggaaaa gttgtcttgg catggagata cactgtcagt     120
   gcagactcct caaagatgaa tggatgtgca tttaagaaca gatagctgag aacatctctg     180
   aacatcagtt tctgcatata aaaaatagag ccaataaact cttcctcacc agtttcttga     240
   gaaaattaag tgatataaca tgtgggaaat gcctgcctta ggagacatct agaccaacac     300
   tgttgcaatg atggacgtct tctatgttct gtactgtcca acagaatagc caccagccac     360
   atgtggccac tgaacacttg aaacatgatt aatgctatga a                        401
<210> 177
   <211> 401
   <212> DNA
   <213> homo sapiens
<220>
   <221> variation
   <222> 280
   <223> /replace="t"
<400> 177
   attaagtgat ataacatgtg ggaaatgcct gccttaggag acatctagac caacactgtt      60
   gcaatgatgg acgtcttcta tgttctgtac tgtccaacag aatagccacc agccacatgt     120
   ggccactgaa cacttgaaac atgattaatg ctatgaagga aattaatttt tacttttgta     180
   tgactttagt taatttaagt atgagagcca catgtgcctg gtggctacca tgttggatgg     240
   ctcaggtcta gacaatatgt gtaatgttga tcatgatgac gatcatatta gttctgcttc     300
   ctcaagttgg agtcaaagtt ttatgctcag ggaacttgtg cctctaggat gggatcagaa     360
   aacaaaactt ttccccggca agttgtatca cagttaggtt t                        401
<210> 178
   <211> 401
   <212> DNA
   <213> homo sapiens
<220>
   <221> variation
   <222> 168
   <223> /replace="t"
<400> 178
   ccatgttgtt gcaaatggca ggatttcttt catttgaagg ctaacattcc attggatgta      60
   tatgccacat tttctttatc cattcatctg ttgataggca tttagattat ttccatatct     120
   tgccttttgt gaacaatgct gcaatgaccg tggaagtact ggtattcctt tgagatctga     180
   tcccaatttc atttccactg gatatatacc cagaagtgag attcctggat tgcaaggtag     240
   ttctagttct ataatggcta caaccaattt acattcccac caacactgtg tggtttcctt     300
   ttctccacat catcaccaga atgttttgtt ttattttatt tttaaaataa tagccaaacc     360
   atttggatat ggtttaaata ttcccaatac tagatcaact a                         401
<210> 179
   <211> 401
   <212> DNA
   <213> homo sapiens
<220>
   <221> variation
   <222> 390
   <223> /replace="t"
<400> 179
   aacttcaatg aggaatgcaa cagagagaga gaaaaaaaga aagatcgggt gattgagtag      60
   acattgaggt ctgaaaaaat gtagaagtca agtcgaacaa ataagcataa acatgtactt     120
   ttaggaagaa gtccatgttg ctggtgccct gctcacattg ctttggccca gccttgaagg     180
   acagctgcag agtggattct ctcatctccc ttcccagagg catcctctgg ccaaataagg     240
   gggcattctc ggccatcaag ctgagcaggc cagtgagggg tgggcactga ggggcacaca     300
   ccagcttgtg tcccactcag aggggcggtt ctgaggcatg ctccctacag tctcccagag     360
   tggccccagg agggctaagc ctcagttgcc catgatggca a                         401
<210> 180
   <211> 401
   <212> DNA
   <213> homo sapiens
<220>
   <221> variation
   <222> 12
   <223> /replace="t"
<220>
   <221> variation
   <222> 320
   <223> /replace="c"
<400> 180
   gagtggattc tctcatctcc cttcccagag gcatcctctg gccaaataag ggggcattct 60
   cggccatcaa gctgagcagg ccagtgaggg gtgggcactg aggggcacac accagcttgt 120
   gtcccactca gaggggcggt tctgaggcat gctccctaca gtctcccaga gtggccccag 180
   gagggctaag cctcagttgc ccatgatggc aatgctcatt attgcaccct cgtggcttcc 240
   ttctctttcc tgcctcattt cgtactccca agctgtgctc ccctgcatca cttcctaaac 300
   taaatccctt catctaagaa cttatcccag ggtctgcttt ggggaagaac aaaagacaaa 360
   gcacttatga tatactcaag actaacagaa gcataagctt c 401
<210> 181
   <211> 401
   <212> DNA
   <213> homo sapiens
<220>
   <221> variation
   <222> 6
   <223> /replace="g"
<220>
   <221> variation
   <222> 348
   <223> /replace="g"
<400> 181
   gttttataaa atttatgatg tgaggttaca atatgccttt ttcttgtgat tcatccttct 60
   tgcaagcttc tatgtagcaa gccggcaaaa ggcagtggaa gggacttctg gggacggtcc 120
   tgtcatttcc tgcagtattt tcgtgagtca ttcagcatcg gtctcttccc tactcctatt 180
   gtctggctgg agagaagcac cctttcatag gtgagctttt cctacaaaca tgagagatta 240
   aaaataagat cttcaaatga agaaataatt tgacatttat tcattgaaaa tcactcatct 300
   tcaactcatc ttccatttac agctctaccc ctttctttta tggatggaga aaatgaggcc 360
   cagaaaaata aatggatttg tccaagataa ccctgattgt t 401
<210> 182
   <211> 401
   <212> DNA
   <213> homo sapiens
<220>
   <221> variation
   <222> 213
   <223> /replace="g"
<400> 182
   tttaaggtat ggatacatct caacttgctt attcattcac ctgttggtgg gcatgtgagt 60
   tgtttaagtt ttgggttatt acaaataaag ctgcttgaat gtttatgtat atgactttgt 120
   gtgtatttat gctttcattt tttctttggt aaaaaccaag aatggaatag ctgcatcata 180
   tggtgattgt gttttttttt aaactttata agaaactgac acattgtttt ccaaaggggc 240
   tgtatcattt tacattctca ccagcagtga atgagattct agttcttcca tatcctcacc 300
   aatacttgct gtggtcagtc tttttagcta ttctaacaga tatctcatta tagttttagt 360
   ttgcttattc ttaatgacta atgatgtcaa gcatctattc a 401
<210> 183
   <211> 401
   <212> DNA
   <213> homo sapiens
<220>
   <221> variation
   <222> 63
   <223> /replace="c"
<220>
   <221> variation
   <222> 328
   <223> /replace="c"
<220>
   <221> variation
   <222> 359
   <223> /replace="g"
<400> 183
   gttggtgaat ttttgtctta gcatctgctt ccggcagaat ccaaacaaag acatgagaat 60
   taaatttttg gtttcattct tgttttattt tcttaacata catgcttata tgtaaaccaa 120
   ttttgaattt tatgtatcaa caatttaagt tctactgctt taagattata tggacattaa 180
   atgtacagaa aaatagaaac caacaaaaga taaataaatt aaatacattt ttaaaaaagt 240
   ctcgcgagtt gtggtggctt aatattgccg tgagctaatg ccttgagggc tactctctct 300
   ggagtgggaa tggagctctc agtaattaga cagttttggc cagtggcttg tcacacggat 360
   tgtgcattca cctggcttta ggaggaagag gaggcatggc a 401
<210> 184
   <211> 401
   <212> DNA
   <213> homo sapiens
<220>
   <221> variation
   <222> 172
   <223> /replace="g"
<400> 184
   taatgtcctt aagatttaag cctcagttct ttgcgctttc tcttacttgc agcttaaagc    60
   attcctaact gatgtttaag aagtttggaa aagaagacta gaagagaaaa acttactcat   120
   tgtccttctt cttttttttg ccctataccc aaaataaaaa ttttcctttc tatgtacaaa   180
   tttgcacata gctatgacca aatggtgtat gtacaactgt ctttgttgtt tttaaatcta   240
   tcttttatct taaaattaaa tcatgtgttc attgcagaaa aactgaagaa tatagaaaat   300
   ggtaagggag aaaataaaat cactcagata cctaccaaga gagaacaaat gttaacatac   360
   taaggtttct ttttgtaatt tttcataaat ttgatgtcct t                       401
<210> 185
   <211> 401
   <212> DNA
   <213> homo sapiens
<220>
   <221> variation
   <222> 142
   <223> /replace="t"
<220>
   <221> variation
   <222> 252
   <223> /replace="t"
<220>
   <221> variation
   <222> 297
   <223> /replace="t"
<220>
   <221> variation
   <222> 308
   <223> /replace="t"
<220>
   <221> variation
   <222> 329
   <223> /replace="t"
<220>
   <221> variation
   <222> 383
   <223> /replace="g"
<400> 185
   ccaccagttt ttcctgagga caaggagcct cttaaatctg aactatggtg aagtgtttta 60
   gtttgagatc ccgggaaata gactctgaga caaagaactg tgagtaggag gtttcatgga 120
   gagtgctctc aggatccaca cctgcgaaat aactataata ataataataa taataataat 180
   aataataata atagtgaagg cctggactgg gtagaggaag aaactgatcc aaaacaaacg 240
   tgcaatgagg cctcagccaa ttcctggggg gcagatctgg ctgtgtacag cagttcctga 300
   ataatgtcgg ttcattcgat gttctttcct tataacgttg atgagaaaac aattgatttg 360
   cacctggggc caccatctgt gtatatttgc acaacctccc c 401
<210> 186
   <211> 401
   <212> DNA
   <213> homo sapiens
<220>
   <221> variation
   <222> 48
   <223> /replace="t"
<220>
   <221> variation
   <222> 93
   <223> /replace="t"
<220>
   <221> variation
   <222> 104
   <223> /replace="t"
<220>
   <221> variation
   <222> 125
   <223> /replace="t"
<220>
   <221> variation
   <222> 179
   <223> /replace="g"
<220>
   <221> variation
   <222> 318
   <223> /replace="g"
<220>
   <221> variation
   <222> 338
   <223> /replace="t"
<220>
   <221> variation
   <222> 362
   <223> /replace="g"
<400> 186
   gactgggtag aggaagaaac tgatccaaaa caaacgtgca atgaggcctc agccaattcc 60
   tggggggcag atctggctgt gtacagcagt tcctgaataa tgtcggttca ttcgatgttc 120
   tttccttata acgttgatga gaaaacaatt gatttgcacc tggggccacc atctgtgtat 180
   atttgcacaa cctccccata gctgcatggg ttttctctga gtactctagt ttgctctctc 240
   attccaaagt catgcacatt aggttaattg gcatgtctac atggactcag tgtgagtgaa 300
   tgtgagcatg ggtgtgaatg tgctctgcca tgagagactg tcctgttcag ggctggttcc 360
   cacctcactc cgtgagctgc tgggatgggc tctggccacc c 401
<210> 187
   <211> 401
   <212> DNA
   <213> homo sapiens
<220>
   <221> variation
   <222> 357
   <223> /replace="c"
<400> 187
<210s 188
   <211> 401
   <212> DNA
   <213> homo sapiens
<400> 188
<210> 189
   <211> 401
   <212> DNA
   <213> homo sapiens
<400> 189
   ttttgagaca aagtcttgct ctgtcactta ggctggagtg caatggcgag atctccgctc      60
   ggtgcaacct ctgcctcctg ggttcaagca attctcctgc ctcagcctcc ctagtagctg     120
   ggattacagg tgcccaccac cactcctagc taattttttg tattttagta gagatggggt      180
   ttcaccatgt tggccttgaa cgcctgatct caagtgatcg gccccccttg gcctcccaaa      240
   gtgctgggat tacagacatg agccaccatg cctggcctgc atgactgact cttattgttt     300
   gtgactataa acattacttc cccagaaact tgcttcctaa cctacaactt aaaatagtgt     360
   ttggtcacta tttgaagagt agacaatcac actttaaata g                         401
<210> 190
   <211> 401
   <212> DNA
   <213> homo sapiens
<220>
   <221> variation
   <222> 392
   <223> /replace="t"
<400> 190
   gggattacag acatgagcca ccatgcctgg cctgcatgac tgactcttat tgtttgtgac      60
   tataaacatt acttccccag aaacttgctt cctaacctac aacttaaaat agtgtttggt     120
   cactatttga agagtagaca atcacacttt aaatagataa tctaatgcta tgccctattg     180
   tattcccctc ataacagtta ctagtgtctt gatattttat ttctatttgt ttattttttt     240
   tgtttgtttc ccatctgagg agaataaaag tctggtgaaa gcagagacca tatttatttt     300
   attcatcatt ctgagaattg gatgggacaa tcagagctca gtaaatattt gtaatgaatc     360
   tgtaaaccaa ggacaacggt aggatccctc tcacaggact t                         401
<210> 191
   <211> 401
   <212> DNA
   <213> homo sapiens
<220>
   <221> variation
   <222> 10
   <223> /replace="t"
<400> 191
   taacagttac tagtgtcttg atattttatt tctatttgtt tatttttttt gtttgtttcc 60
   catctgagga gaataaaagt ctggtgaaag cagagaccat atttatttta ttcatcattc 120
   tgagaattgg atgggacaat cagagctcag taaatatttg taatgaatct gtaaaccaag 180
   gacaacggta ggatccctct cacaggactt tatgagtgtt aaccaagata atgcatgtaa 240
   acaccttcgt acagtgcctg gaacacagtc aggtctcagt ggatgccaaa tcattaggtt 300
   caggtaataa aattgtatat ggagctccat tcttgccctc aagtgcctca aaggccagag 360
   tgaagaagga gatggctcac aaatgaatat gtcatttact a 401
<210> 192
   <211> 401
   <212> DNA
   <213> homo sapiens
<400> 192
   gaataattgg tgtgtctgga ctcagtaaca ccagtgtcag catatgccac tctggtgccc 60
   aaggacagtt cattgatgac accattggtg cttgaggact ggcctatgtg gcatcccagt 120
   tccccacaaa acttcaccac aacctccact aacaactgca ccctaagctg ccaagaaatt 180
   atagacactg ctgatgctgt gtccagctga agaagccaca cacaaactac actactgcac 240
   atgtccagaa tcaaagccaa gatgccgtac acaaccaacg ttatagctac atctccagga 300
   aaatgttttc ctttacaaaa gcaactggaa aaaattggaa gaagtgacca ttacaccagg 360
   tgcaaagata tcagtgtaag gatagaggat acatgaaaaa g 401
<210> 193
   <211> 401
   <212> DNA
   <213> homo sapiens
<220>
   <221> variation
   <222> 48
   <223> /replace="t"
<400> 193
   agtatagata cacaatgaaa tactattcag ttatttaaaa aaaagaacga aatcaagtca 60
   tttgcagcaa caggaatgga actagaggtc attatgttaa gtgaaataag ctggacatga 120
   aaagacagat accatacatt ccactcatct gtgggagcta aaaatgttga tctcatggag 180
   gtagagagta gaatgatgga caccagatgc tggaaagggg gtgtgagtgg gaggtggtga 240
   tgaagagggg ttggtcaata ggtgaaaaca tgcaattatc tagaaggaat aagttccaat 300
   gttcaatagc aaagcagggt gactatggtt atcgacaatg tgttatatac ttcaaagtag 360
   ccagaagaca gaacttgaaa tgttctcaac acatagaaat g 401
<210> 194
   <211> 401
   <212> DNA
   <213> homo sapiens
<220>
   <221> variation
   <222> 122
   <223> /replace="g"
<220>
   <221> variation
   <222> 308
   <223> /replace="t"
<400> 194
   ttctattttt taagagtcca gatttttata tcatttgttt tttatagaaa atgatgctaa     60
   cttggtaaac ttgctccagt cactgttctt caacaggatc ctaaaattaa tttcgatttt    120
   gagagatttt cctctgctaa ggccaagcga atgaattttc aacacaattc tttaagtgaa    180
   atttagaaga gaaatgaagg gtgaaatttg catgtcatgt tcaaatgttg taattctgtc    240
   acacctgata tgctattatc atagaaatta taaaaatatt ttaactgtat tatatagatt    300
   gttatgacgc atacaaatta tcacttcttt tacaataata tcaggatcca tgcagggaag    360
   atatatagtt cctggattct tctttcaaat ttttctgtgc t                        401
<210> 195
   <211> 401
   <212> DNA
   <213> homo sapiens
<220>
   <221> variation
   <222> 15
   <223> /replace="g"
<220>
   <221> variation
   <222> 94
   <223> /replace="t"
<220>
   <221> variation
   <222> 369
   <223> /replace="g"
<400> 195
   taatttcgat tttgagagat tttcctctgc taaggccaag cgaatgaatt ttcaacacaa 60
   ttctttaagt gaaatttaga agagaaatga agggtgaaat ttgcatgtca tgttcaaatg 120
   ttgtaattct gtcacacctg atatgctatt atcatagaaa ttataaaaat attttaactg 180
   tattatatag attgttatga cgcatacaaa ttatcacttc ttttacaata atatcaggat 240
   ccatgcaggg aagatatata gttcctggat tcttctttca aatttttctg tgctaggata 300
   ttatcaagaa aacacaaaat atcatgtctt gtttaattta tttaaatctc tcttcacaca 360
   gcactataat atccataaaa tactctctgc atttattttg g 401
<210> 196
   <211> 401
   <212> DNA
   <213> homo sapiens
<400> 196
   atgttttgga tccattttta ctcccacatt tggcaaataa tttattcatg aacaatgcaa 60
   atgaaaattt tttctcagtt gagctgttgt gaaattatta cacaaaacat tttgaaggtt 120
   atcaattgta atgtgagagc caaaaccacg tgtgtgcagg cccatctttc tggttgggga 180
   ttatactatt tgatataaat cacctcaaga aatagccgta gatgagagaa ataaacattt 240
   taattgttct tataatgaat gataagtgaa gggaactata acctcaattg caaatgttaa 300
   catttacaag atgacctgtt tataaatgta gctctgcctt atacatgtgc tcacatccat 360
   tttgaagtat ccaaccagag cgctgataga ttagtagttt t 401
<210> 197
   <211> 401
   <212> DNA
   <213> homo sapiens
<400> 197
   taaagaattt gcaaacaagg gaccaatcat ggtagtgagt aacttctttg tctcttgctc 60
   cttttgtaca aaatatctga tttttctcct gcacttgtct gtatctgact cactggttgt 120
   cttcttcttt agtttaaggc ctctggaagc ccagagatag gaggcaggga cgagtggagg 180
   gcagttttct ctcgactctt cctgcagtta tttaacttct gtttctaatt tataaggctt 240
   tcttggcata tgtaagccta tgcagtcaaa ctgagttgtg gtccaggaga ggtcatggac 300
   cagtgtcagt catgtgccac atcacagagt gacgtgagat tcggagatga gaaaaatgta 360
   aaaagaccct actttttttt cttggcctat cccaataaaa t 401
<210> 198
   <211> 401
   <212> DNA
   <213> homo sapiens
<400> 198
   aaccaggacc acactgtaac actgccacct ctgcaacata catattagtg caaattgggg 60
   taaagggtgg ggctgataga atgtagagtg taatttagga accagcctat ggtgagatct 120
   tgcaactgct ggcaaagtcc gagccactat cctttgagca atccattcac aatggaacca 180
   gaaaaacaat gtctatttag ccagaaaaaa tttgaatgga caagagtgat gtctctttac 240
   caatacaaga gagataagat tactaggaat agaagagtta catggtttct ctgactgcct 300
   tcacaaagtt cagggtggct ggaggtctct gaagaaaagc ctggacatca gtgatgaatc 360
   aatctctcaa agcctcctgt agcaaacatt gccaagggcc t 401
<210> 199
   <211> 401
   <212> DNA
   <213> homo sapiens
<220>
   <221> variation
   <222> 307
   <223> /replace="t"
<220>
   <221> variation
   <222> 308
   <223> /replace="t"
<400> 199
   tttttttacc aaagatcgaa actctcaaaa cattttcaga ggcttcataa aatcgttatt 60
   tgtgttggaa ttttatatac cacccaaagc tggatgaaat caaagagcca atggctaggc 120
   ttggaggtca ttttaaaaac tgcatttttt caccattata atatttattg caatgggcta 180
   ggccctacgc atcagattgg cgatattatt aaggcatcag aaggccaaga aaatctgaaa 240
   ataaaatggt ttcccaggta aagccatggt aagggtaggc aaaactggca tcaggccaac 300
   cgtttcaaac catgcatcaa acttttgagg tcaggcaaaa ttgaaaacac atttggagaa 360
   gttaattcac cttaattttg aaacccatgt gtatttcaag g 401
<210> 200
   <211> 401
   <212> DNA
   <213> homo sapiens
<400> 200
   ctaagttatc tagtttacca gcctggagtt atctgtagta tgtgatagta tagtatagca      60
   tacattttcc cccttttagt atctacatgg tttgtaatga tttcccctct ttcattcttg     120
   gggtaatctg tcttcctcta ctctcctgac acatactttg ggcagatccc acctctaggt     180
   acagaatgtg attggcaatt cctgtggacc aatctgagca ttgctttcac ttggctgtcc     240
   tgattgcttt gaatggagag agtaaggcgc atcaggcaga tacaaagcat tttgctgaga     300
   ttactggcac tatataaacc ctgcactccc agggagcagt agaaatcttg agaatggatc     360
   cattagagtg taaggcagac gttaaagatg gagaaaccaa c                         401
<210> 201
   <211> 401
   <212> DNA
   <213> homo sapiens
<220>
   <221> variation
   <222> 35
   <223> /replace="t"
<400> 201
   atctccaccc agaaatggag tgctgtagta aggcccactc aagataacga tggcaatggg      60
   aagctggagt gacagatggc acacgatggc aaacagccca tacacgttag tgctttctgt     120
   ggaaaataat tttgtatata tataaatatc ttttataatt taggaatttc cacattatta     180
   agtcaggata gccagtatag cagagataca ggtgtccaat atccctttca tccttcttcc     240
   tttagtaata gtttatctga gtgtatactg agtgtggtcc cttgtccaaa ggaaccttta     300
   aagtggtggt ggtcacatgc taaacttata gtcagaggta tgtgaatggc gatggtggat     360
   atcacctctc agtcatgctc atatctttcc ttctgtccct t                         401
<210> 202
   <211> 401
   <212> DNA
   <213> homo sapiens
<220>
   <221> variation
   <222> 37
   <223> /replace="g"
<220>
   <221> variation
   <222> 102
   <223> /replace="g"
<220>
   <221> variation
   <222> 273
   <223> /replace="c"
<400> 202
   aagtaaacag aaaatgaata agctgtggac tttgaaagat agtttttgag ttagaaatct 60
   gaagggggag aaaaggccac agaaacaggg taaacagtta aagcaaacat tttagaccaa 120
   aatagccagt agacagcagc tgagaatgga cctcactgct ggtaaagagg cccaagtgga 180
   ttaaattgat caaaacagtg cttgggagat agcaaggaat tttatttttc attatttctc 240
   ttcaaaaagg catgatggaa gattagactt ctgtaagaac aaacgtttga aacttttcta 300
   ttgctactgt ttctgtgctt tgcatttgat gttggttatg aaacgtgttt ttctcagtat 360
   agatatatat gtatattttt atacatacac atacatatat a 401
<210> 203
   <211> 401
   <212> DNA
   <213> homo sapiens
<220>
   <221> variation
   <222> 40
   <223> /replace="g"
<220>
   <221> variation
   <222> 110
   <223> /replace="t"
<400> 203
   agtttagtat gttatttaga ctaatatttt ataaatcata ttctggaatt aagctgagta 60
   aggccaggaa gaaagagggt tgaggatgga tgtaatgtag gaaccatgcc taggaagcga 120
   ggaaaagcat cataaaagga cagcaacaat gtagaggaga gaagttaagc agtcagtgag 180
   aggaaggcct ctgaacccac ccaggtactt agctgtagat ggacatcagt taattcgatg 240
   acccaccacc ccatctcacc aataaaaacc aaactctttg ggaactggga ccagtactga 300
   actagtgggt caaaataaag taaaaaagaa gcccacttca agaaatactc ctctaaataa 360
   cttcagagaa aaggaaattt tgagaaacaa gcaatcacac a 401
<210> 204
   <211> 401
   <212> DNA
   <213> homo sapiens
<220>
   <221> variation
   <222> 55
   <223> /replace="g"
<220>
   <221> variation
   <222> 215
   <223> /replace="t"
<220>
   <221> variation
   <222> 224
   <223> /replace="g"
<220> variation
   <221> variation
   <222> 285
   <223> /replace="t"
<400> 204
   ggctatgata accaaaacaa catagtactg gtacaagaac agacacataa accaatgtaa    60
   cagactagag aactctagaa acaagaccac ttgcctacaa ccatctgatc ttcaataaac   120
   ctgacaaaaa caagcaatgg ggaaaggatt tcctatttaa taaatgttgt tgggagaact   180
   ggctggccac atgaagacaa atgaaactgg actccttcct tacaccatgt acaaaagtta   240
   acccaagatg gattaaagac ttaaatgtaa aacaccgaac tataaaaaaa aaaaaaaacc   300
   ctaaaagaaa atctaggcac taccattcag gacacaggca caggcaagga tttcatgatg   360
   aaaatgccaa aagcaattgc aacaaaagca aaaattgaca a                       401
<210> 205
   <211> 401
   <212> DNA
   <213> homo sapiens
<220>
   <221> variation
   <222> 167
   <223> /replace="t"
<220>
   <221> variation
   <222> 190
   <223> /replace="g"
<400> 205
   atatgggtag cttcctagaa acatacaacc taccaagact gaatcattaa gaaatagaaa    60
   atcttaacag acccataatg agtaagaaga ttgaattagt aattaaaaat ctcccaaaaa   120
   gaaaatttcc agacattatg acctcatggt tgaattctac tgaacaagta aagagctaaa   180
   atcaatccta taaactctta caaaaattga acaagaggga atacttccaa actcatttta   240
   ctaggctatt attaccctga taccaaagca agacacaaac actataaaaa aagaaaacta   300
   caggccaata accctgatga acatagataa aaaaatcctc aagaaaatga cacaaactaa   360
   atttaacagc acaataacaa agatttatga accattatag a                       401
<210> 206
   <211> 401
   <212> DNA
   <213> homo sapiens
<220>
   <221> variation
   <222> 71
   <223> /replace="c"
<220>
   <221> variation
   <222> 148
   <223> /replace="g"
<220>
   <221> variation
   <222> 230
   <223> /replace="c"
<220>
   <221> variation
   <222> 396
   <223> /replace="t"
<400> 206
   gcagacccca ctgagggtat aacacacacc aggtgacttc actaaaatcc ccacggtgaa 60
   acacatttgc ggcccttttc cctgcccctg gattttgcta aggatctggg gggttctgtg 120
   gaagtctgaa ggtcagtgga caggacgagt ttggagatgc gcatttggaa actgtacccc 180
   tcaagtgaca aggaaatgag cgtggattta agaagataat gaccgttctg tgaagtttta 240
   atggaatttt taagcaagaa aacccttacc ctgtgaaaac ttcagtctgc acagaagaat 300
   tggtgggaga aggaagactt tgcctaagat cactttatat agcacaaccc agaccgggga 360
   gaaggcagag ttatgcccag catatagcct tggcagcata t 401
<210> 207
   <211> 401
   <212> DNA
   <213> homo sapiens
<220>
   <221> variation
   <222> 134
   <223> /replace="t"
<220>
   <221> variation
   <222> 346
   <223> /replace="t"
<400> 207
   gaaagcagct ttctcccaaa agcagacaga ggatgggata atgacagtga agtgagaatt 60
   gaagagttaa tagtgtccat gttatgctct ttaaagcaga cctgttctgt tgtgtatttt 120
   ggcacatggt tttcagacat attttagtca atctttgcaa agttgaaagt tgattcagag 180
   ccagatgttc agaaatagac agagaaggct attgtaattt ttcatccctc agtttttgta 240
   cagaaacttc attgtaaaaa cccttctaga ctgatggctg cctcattgaa aactgggtca 300
   aaaatatggc tctgttagaa aatctcttgg attacaaaac aatgcctaaa caaaaacaaa 360
   tagaaatgga aaccgcccta cagatgtgag atttgtgcca a 401
<210> 208
   <211> 401
   <212> DNA
   <213> homo sapiens
<220>
   <221> variation
   <222> 360
   <223> /replace="g"
<220>
   <221> variation
   <222> 401
   <223> /replace="g"
<400> 208
   tatacgcata tctataaata tttctatatg gaagcagtgt ctataggaag ctacacatga 60
   gttcatactg cgatcatcaa ctttaatcca ttaccacatg aatcattcta acctgctccc 120
   cttgcctatc tctaaattct tactccaaca gaaaaaaaca ggctctcaac atctgccatc 180
   tacttactta attattcaaa cctagtgcac atgtattgca gtactagaat tgttaaccca 240
   tacccacatg ggaaaaagtt tatcaactag agtacagtgc ttatgtacaa tgttttttgc 300
   aatctgaggc tccactgatt tctaaaatta cttaggtcag catctttttt cttcacccta 360
   ttcagtgaag tgtttcatcc gtttgtaata cagttagatt a 401
<210> 209
   <211> 401
   <212> DNA
   <213> homo sapiens
<400> 209
   tgtatttggg actctgagtt acagcagcct ttttcctact ccaccctcta cttatttggt 60
   cagttgtttc tcacaggctt tgtacagcga attcacccta gtaagtacag caggctgata 120
   cttaatttta ctctgaatgc tggtagatga aaacaacagt gtcattgcct agtaaatgga 180
   actaattctc agacttagtg ccaaggagcc agaaagggca gtgatttcat gacctagtta 240
   aaggcacagt cattactcag ctcccactaa ttttggtcat gtaaaaacac aggcccaatg 300
   atactagata ttatactttc tgcagataac cacggcaaca tttcccattc atatgctttt 360
   ctagaacctt gcctctcacc attaagaaag gaagcgtgtg g 401

<210> 210
   <211> 401
   <212> DNA
   <213> homo sapiens
<400> 210
   ctattattgt ccccatttta caaatgtgga cactgaggca cagaaaaaat aagtaatttt 60
   cctggtgtca tgcagacagt aaatgtggaa gctagggagc ccggctgcca ctgcttctca 120
   gcacagcacc gggctgcctt tgggataatt gtcatattcc ctttcttggc ctcaagggct 180
   tccatatcca gtcttcccca cgaaccccca acagcttcaa cacatgtcaa ccacccccag 240
   tttaaagcaa gttgcccaga ggaatttttt gcgacttttg agtcttttca tttaagaata 300
   tacttttatg ctcaaacata aaatctggta ataaggttat tattatatct atatctcagc 360
   ttcatccaat ttgctgtcgt ctctctttat tatatacttg a 401
<210> 211
   <211> 401
   <212> DNA
   <213> homo sapiens
<400> 211
   tcttgtttcc agagataaat tagtaggaag gggtgaaaag gagaaatatt tgtgaatcat 60
   acagtagtca cagctattgg ttgatgtcta ggatattaaa atgtgaaagt attacgacta 120
   aaaatcctct gctgtctaat ctctcacagc ttatatttga tgttccctaa aatcacggtg 180
   tccaaagaca gaccatgttt ctctagaatt cctgtaatga agatctttgc aagatgccgc 240
   aattttgaat gtcccaaacc acaaagaggt ttcagttctt cctttcaatc cccaaccttg 300
   aactgaagcc tctgcagaaa gctcccagct cactagtgtg gacaggcaac tacttgtgct 360
   tctatgagag agaaaaagtg agcagtttcc aagagacaga a 401
<210> 212
   <211> 401
   <212> DNA
   <213> homo sapiens
<220>
   <221> variation
   <222> 248
   <223> /replace="t"
<400> 212
   ctaacatgta ttatttcatt tttctaataa gaaaaaataa acaatgtgcc aatgtaaata      60
   caaatcacaa aatagttcac ctttctggct gcctgctagc agatttttgt tttattttac     120
   tgcatgtgtg gacaagccat ttcatgtact tttatctctg gtttaagtta tacagaagcc     180
   attctgtttc cctttgtaag agtttcattt aaatgtccag gtgtatgtgg gtgtggtgga     240
   attaggagga agagcgcttc gcccccatca attctgcagt tgccgtggca gcttagccgt   300
   gagtgggtca ggtggccaaa gcagaggccg acccccgcca gccgttgctg tcaggccccc     360
   tctcgctctc tggttcagga gaacgtctct agaagagcgt c                         401
<210> 213
   <211> 401
   <212> DNA
   <213> homo sapiens
<220>
   <221> variation
   <222> 11
   <223> /replace="c"
<220>
   <221> variation
   <222> 26
   <223> /replace="g"
<220>
   <221> variation
   <222> 51
   <223> /replace="t"
<400> 213
   tttgcttcat aacaactgtc ttggaataat ttttatttac ctaaaaataa ggtaagagta      60
   tgtcaaagtt taggcatagt gtttattaaa tgcatagttt gtatcaatgc ttgtaagctg     120
   taattaggaa tgatatgtgg cattatattt tacttttgtt aatgttcact gttaacttcc     180
   tgaaatgtaa gctgtgctca cgtgaaaaaa aaaaatggat cagtttttct gagaagacac     240
   tgtaacacaa aaagacctac aggatagtat ttgaatagtt aattttaatg caaacaaaaa     300
   cttctatttc agcattaaga acacatattc ttgatataag aaatagaaaa tattctagca     360
   catacctgac actggtaaaa tatatgtatt ctttttccct a                         401
<210> 214
   <211> 401
   <212> DNA
   <213> homo sapiens
<400> 214
   ctctcaggtt tgaaactgtg tgtgtgactg tgggagtaaa tagtctagtt tcccactctt 60
   catgtgtaaa tagacacaaa caggtgactt ttcagagcct gggacagaga agacgacccc 120
   ccagtggaga cgactgaaca ggcccctcat gcatacagca tgccaccagt attgcccaga 180
   aatactgttt gtagaaaatc ggaaaggtag ccgggcccgg cattaatgct agggtgtctg 240
   agatgagtcg aaggaacagc tattcttttt ccctttgtta tgtttcttcc atttccaagt 300
   ggatgaagct ctaactcaac ctttcatttg tttcaagagg gaggtatcag aaccacaaca 360
   aatggaatga aacctaccac taccacctgc ctggcacaaa g 401
<210> 215
   <211> 401
   <212> DNA
   <213> homo sapiens
<220>
   <221> variation
   <222> 388
   <223> /replace="g"
<400> 215
   gaaataaaaa agaaaaagat ctttcttttc tcctggcctt taacttggca ggctttatct 60
   gctcttcaga gtcactgaga tgagaaccag gtggcaaact ctgctttcct tctcctccag 120
   gcaacgcaga ggcattctta actcttaacc tgcagacttt gaaactaggc acagaactcc 180
   agaggggata ataacaccag caaatgttta gctaaaaaat ttagccatac atgccacaca 240
   ttgtgctaca cattgagctc tttggtcctc acatcaacgc tgaattagga gctatgatta 300
   tccccattct ttagattagt aagctgggta tcaccaagga gaagtacttg ccccaaagtc 360
   acatggctca tatgtgttgg aagcaagatt tgtgacagat c 401
<210> 216
   <211> 401
   <212> DNA
   <213> homo sapiens
<400> 216
   tttttatttg tgaaacaatc ctaggtcttt gtgttccttt tacctgattc ccaaactgaa 60
   caaacccaag taccaaccat tccccttgga tttttagtca actgaaggtt cagagagctg 120
   ctcccttggg gaagaggctg tggacatgac atttgcacat gcagcatatc aacttggaat 180
   aatttccccc tcatgtgggg gctactccca aggagtttat tctaaaccca aagtacatta 240
   gttgcctgtg gcaccttaga aaaataaact atcctggggt cccctggttt ggaatgtcat 300
   ttcctttatg atatgtgtct ttgaaaggca aggagtggca gtaagcagag tgatataaac 360
   atggggcctg ggctttaatt ctgtctgccc tcatccttta t 401
<210> 217
   <211> 401
   <212> DNA
   <213> homo sapiens
<220> variation
   <221> variation
   <222> 138
   <223> /replace="g"
<220>
   <221> variation
   <222> 335
   <223> /replace="t"
<400> 217
   ctctttcctt ctttctttct ggcttgcttt ccctcctttg ctgctggtga ccacccatgc     60
   ttgagcagtg ttttggctgc tttcccactg atgagttttc caaaccttgt caggagctgg    120
   cagttaaatg ttctgccacc agcttacaga aaatacaagt tggattgcct tgttcctgct    180
   ttctttcaaa cttgcatgtt aatccatctt caccttgatt cctagaagag tcaaacatct    240
   gatcctcagg tcaagcctga aggaaatggt tgtcccagct gaaaataaga tcgatcccaa    300
   tttgctttac agtactcttg ttaaagatca gtgtgtaata ttttctctgt gtgatgcaat    360
   gcttcttaat aaaaacaaaa agggatgttt gaccttaact t                        401
<210> 218
   <211> 401
   <212> DNA
   <213> homo sapiens
<220>
   <221> variation
   <222> 226
   <223> /replace="g"
<220>
   <221> variation
   <222> 294
   <223> /replace="t"
<220>
   <221> variation
   <222> 319
   <223> /replace="t"
<400> 218
   tccatgtcaa cagcaggcgg cttatattcc agaaagttaa gtctgtgcca ttctctgcct 60
   gagccagtat gttacgaaga gcattaccat ggcgatttca cattctgaaa ctgtgcttgt 120
   ctcttaaatg ccacgtgcca atgagtcata gaggtgtttt gttctagagc catgtacccc 180
   ccaaaaaggg agtttgtaca gatttaccaa gaaactatta ttattatcta cagttcatgc 240
   agttacaaaa ctatctttgc ctccttttac tcacaatttg aacccaatct tttctaaggt 300
   gctggcctcc caatattgct gtagataatt cagccttggt ttcttatgat taaagattat 360
   gactcacacc agcctacaag cctggagaat tatactgaag t 401
<210> 219
   <211> 401
   <212> DNA
   <213> homo sapiens
<220>
   <221> variation
   <222> 229
   <223> /replace="t"
<400> 219
   agaattgctg gatgctttta agtcatcctc caacatgaac cagcttctta taaaagatga 60
   gacagttgca tttatatatc caaagctagc tgctatattt tctaagtcat gtctcaagta 120
   gagcatctag aaaaaacctg tatcagtagt gcttaagtct ggcactgcag ggaagctggt 180
   ttaaaattac agagcctctg gaattttcaa atgttcaaaa catccaacct agagcctggg 240
   aatctgtatt tttaatgagc atcgctcatg cattgctatg gaacacacat tgcatatata 300
   gcattttcta acatatagga tcaggccaga ggaagaatgt ctgtgtatga gaactcccta 360
   aatgatgcac agttccactg tctggttaca gttgaaaccg t 401
<210> 220
   <211> 401
   <212> DNA
   <213> homo sapiens
<400> 220
   tgaggcccca ttggtttaaa tatctcattt cttacttgtc aaagtgcatg aatgtccagc 60
   attagaaatg cagttccttg gggtctggag ggcattgagg gctcccagcc ctgcttctta 120
   agtgcatctg ctgctgcata gtatcaggct taagtcttag catccacacc ttgtcagcaa 180
   ttaggtgaca gctaactgtg gtcaaggctt tctggggact gaatgaagaa ctggggtggc 240
   acaggtagtg aaggagggtg agaggctggt ggggccatga cagggggtgt tgggggttgg 300
   cagagcccaa acttccctgc tttccctgga actggctgca ccaagaagtt gaggctcctg 360
   gtttgtttgg aatcaggtat ggtacaatcc attgtcccgt g 401
<210> 221
   <211> 401
   <212> DNA
   <213> homo sapiens
<220>
   <221> variation
   <222> 267
   <223> /replace="t"
<220>
   <221> variation
   <222> 272
   <223> /replace="t"
<220>
   <221> variation
   <222> 291
   <223> /replace="t"
<220>
   <221> variation
   <222> 341
   <223> /replace="t"
<400> 221
   gcctcttctg gacgtgagac aatttgagtg cagtatttct ctagttgctg acccttccca     60
   gctcctcctt tcaggggatg tggtgtcatc ctggcattgt cctgtcctaa cctagccctg    120
   ctggtctcca tggctgcagc tggatatcca aacacccaac acacataatc atggctctgt    180
   gccgtgagga ggagacaaga accccccaaa ccattctgtc gagtttccta tggttgggtt    240
   ctggttgtat ttctcttctt ttctttcctt tccttttctt tttttttttt ctttgagacg    300
   gagttttgct ctttgttgcc caggctggag tgcagtggcg cgatctctgc tcattgctac    360
   aacctctgcc tctcggattc aagtgattct cttgcctcag c                        401
<210> 222
   <211> 401
   <212> DNA
   <213> homo sapiens
<220>
   <221> variation
   <222> 53
   <223> /replace="g"
<220>
   <221> variation
   <222> 137
   <223> /replace="t"
<400> 222
   cacatgtgag agaacagctt tccagaaacc acacttgagt ccacagcaga gcagaagggg    60
   caggaggcat caggaccatt tccagatttc tctctggccc tgattgtcta gatttgcatc   120
   caatcactgc aagagtgtat acaaaagttg ttagaaagat ttgggtttca cacagagtta   180
   tgaaaacaag gtagaagcct gatgtcttca tttcccatga aatcttgtgg gtgtaaatga   240
   aacatgtttt agtctcaaat cctggagctg tggggttcac aatggagaaa tatccaggtc   300
   tcttgaacaa gactggacga taaccaggga cagttccctt tttcccccac attcatttat   360
   gagttggtca aactgttgtc tattcacagg agattcttag a                       401
<210> 223
   <211> 401
   <212> DNA
   <213> homo sapiens
<220>
   <221> variation
   <222> 329
   <223> /replace="t"
<400> 223
   actcttcttg agcagtgaac atcaagagat catggattaa atgggaaaat tagtgtcaag    60
   attacatcag aagaaaggca agtatcgcat catttcactg cccacaaaaa tacattcttt   120
   ccctttccct ccactatttt aagacttcat tgaccttttc taaggtccca atttccattt   180
   aacctctgtg ccagtcaaga gagcagtccc aagtctctaa ctttcttcac ccaatgatca   240
   ctctttgaac tcaccaagca tgctacaaac agaaggaagc cattagcagg gggagttcga   300
   acctatatag ccaaccataa attagaagcc caaggaagca ttttcaacca ctacgaggtc   360
   actcaggctg actgctggca gtggcttctc atcgatgaca g                       401
<210> 224
   <211> 401
   <212> DNA
   <213> homo sapiens
<400> 224
   gaattctgga atcagagagg agaaaccact tacctcaggg gctgaaggag cctttgggct    60
   tcctggctag agtgctgagg aacttggtgc tctcaggctg ctggtgggag gagagacctg   120
   ggtttgagga ggtgactggc caggcttatg gacggggatc cacatgatga atttctccag   180
   cccctgtgca ctcaaactgc ggtaaaatca gttagtgggt ttgcgctttg agcagctcta   240
   gtaattcatt tggtgtgtct ttatagttta caaggcactc ttgcttctga gtttcccttg   300
   gaggctgagg ccactcccag aagcccagta aagagcagtg gccaagtgca caaactgggc   360
   ctgggctcag atcccagctc tgtcgctttc taactgtgtg a                       401
<210> 225
   <211> 401
   <212> DNA
   <213> homo sapiens
<220>
   <221> variation
   <222> 292
   <223> /replace="t"
<220>
   <221> variation
   <222> 341
   <223> /replace="c"
<400> 225
   cattgacctc ccagatctgc ctgtgatttg gggcttatag agatgtgttg attctgtcta 60
   gtcaaagtga gagtcattgc agaacaaaac cagaatctta gtggtgactg tgcaaagcca 120
   tttggtctaa tgattttacc aagagcatct cagaacacta gtattggtag tatgtagcct 180
   tgttaataaa tatatacaag tacatgttga aatgacttgt tattatatgg ctagagcaaa 240
   cccacgtcat cctcatgatg tcttgctgtc acaatctcat tctctcttgg ggtctgacct 300
   gactcctcaa gcaagagaac aagggttggc agagctctcc aggaatgcac agccccaggt 360
   gcccagcaga gggcatcaag tatgacagat attggaatga c 401
<210> 226
   <211> 401
   <212> DNA
   <213> homo sapiens
<220>
   <221> variation
   <222> 61
   <223> /replace="t"
<220>
   <221> variation
   <222> 152
   <223> /replace="t"
<220>
   <221> variation
   <222> 278
   <223> /replace="t"
<400> 226
   aagagcatct cagaacacta gtattggtag tatgtagcct tgttaataaa tatatacaag 60
   cacatgttga aatgacttgt tattatatgg ctagagcaaa cccacgtcat cctcatgatg 120
   tcttgctgtc acaatctcat tctctcttgg ggtctgacct gactcctcaa gcaagagaac 180
   aagggttggc agagctctcc aggaatgcac agccccaggt gcccagcaga gggcatcaag 240
   tatgacagat attggaatga catctgttgt gtgtgcagag cagactgact gagtggaacc 300
   atgaccaaag agaactcagg tagactcaag tgcttctggg aaatttgaga aacttccctg 360
   acagagaatt tacttcaagt cagatacctc cccaataatt g 401
<210> 227
   <211> 401
   <212> DNA
   <213> homo sapiens
<220>
   <221> variation
   <222> 268
   <223> /replace="c"
<220>
   <221> variation
   <222> 328
   <223> /replace="t"
<400> 227
   aaatttagta aagcaatatt ttattgttct agcaacaaaa aaattaagtt attacattaa 60
   ttagaaagtg atctgagaac cttattttga tataggaggg gagcagggaa gtgctgggaa 120
   gggaagggca tggtccctgg ctaggaatcc atccctgggt ttgtgcccat ggacctaggt 180
   gaggacaggg atttctgttt ttgtgtccaa atgttgcatt tcccaagacc gctctggcca 240
   accatgcccc catcctgtgc ctgtaaaaac cctgagacat tagcaggcag agacacaagt 300
   agctggacgt cgagaggaat gtaccggcag aggaggacat caacagacgc ccgcagacac 360
   caggccagta ggccgtcgac aaatggaact atgtggtgat g 401
<210> 228
   <211> 401
   <212> DNA
   <213> homo sapiens
<220>
   <221> variation
   <222> 134
   <223> /replace="t"
<220>
   <221> variation
   <222> 261
   <223> /replace="t"
<400> 228
   gtgatctgag aaccttattt tgatatagga ggggagcagg gaagtgctgg gaagggaagg 60
   gcatggtccc tggctaggaa tccatccctg ggtttgtgcc catggaccta ggtgaggaca 120
   gggatttctg tttctgtgtc caaatgttgc atttcccaag accgctctgg ccaaccatgc 180
   ccccatcctg tgcctgtaaa aaccctgaga cattagcagg cagagacaca agtagctgga 240
   cgtcgagagg aatgtaccgg cagaggagga catcaacaga cgcccgcaga caccaggcca 300
   gtaggccgtc gacaaatgga actatgtggt gatggattgg caagaacctt gaatcctgga 360
   catgaccatt ttacaatgtt attgttaaat ttttttcttg a 401
<210> 229
   <211> 401
   <212> DNA
   <213> homo sapiens
<220>
   <221> variation
   <222> 74
   <223> /replace="t"
<220>
   <221> variation
   <222> 141
   <223> /replace="c"
<400> 229
   gcatggtccc tggctaggaa tccatccctg ggtttgtgcc catggaccta ggtgaggaca 60
   gggatttctg tttctgtgtc caaatgttgc atttcccaag accgctctgg ccaaccatgc 120
   ccccatcctg tgcctgtaaa aaccctgaga cattagcagg cagagacaca agtagctgga 180
   cgtcgagagg aatgtaccgg tagaggagga catcaacaga cgcccgcaga caccaggcca 240
   gtaggccgtc gacaaatgga actatgtggt gatggattgg caagaacctt gaatcctgga 300
   catgaccatt ttacaatgtt attgttaaat ttttttcttg agcaaggtat gtataagtaa 360
   gtaaataagg gcataatgtc aggtaaactg aatggctgga a 401
<210> 230
   <211> 401
   <212> DNA
   <213> homo sapiens
<220>
   <221> variation
   <222> 175
   <223> /replace="t"
<400> 230
   ttagcagtgc ctcccaccac agcagacaga aggtacatct cactctaccc tctgacatca 60
   gctccctcat ggaacttcct ttggccaatg agccgagagc agaggcgtga gttgtgtcta 120
   gccagagggt tcaaatgtgc ttgcgtgatt tggcttggct ttttgagctt cagcattctc 180
   catgggaaaa tcatgccctg agtaggcatt gctccttcaa cctgtgtcct ggaatgaaga 240
   cacatggaac agtctggaac ctaaaacaaa gcctggagtt cagctgagat cagctaaggt 300
   gtggctggga cctgtgcata tggaataaat gtttgctctt ataggtaaat gcgatgtgga 360
   agttgttcat tactcaacat tattgcagca gaagcctggt g 401
<210> 231
   <211> 401
   <212> DNA
   <213> homo sapiens
<220>
   <221> variation
   <222> 127
   <223> /replace="g"
<220>
   <221> variation
   <222> 324
   <223> /replace="t"
<400> 231
   tacatgtacg tgtgcttgta tgcatgggtg tgcattatgt gcgtgtttgc ctgtgtgtgc 60
   atgcatgcgt gtgtgtgtgt gtgtgttctc tgtggaggga ggagactgga gattctcttc 120
   cccaacattc gaggccagtt tgccaaaaat cttggtgcta actgtatgtc caggttacag 180
   atccacacgc aaatgcagac tcaacaacac tgaggctcat cttttgggtt tctccccatc 240
   ttcttcacca tgactcagtt ctccaagaaa aatgttgttg ctactttcag gaacagtcca 300
   ggatgagagc ttaaggtaga gtccggacat gtgcatggaa gcacagccat gattctatct 360
   aattggctca aacaaaagtc ttggacagta ttttggcttt t 401
<210> 232
   <211> 401
   <212> DNA
   <213> homo sapiens
<400> 232
   ggaatctgct ttactcaaag cccatcgagt ccaatgttaa tctcatccaa acacaccctc      60
   acagcaacat ctagactggt gtttgaccaa acagctgttg ggtaccatag cctagccaag     120
   ttgacacata aacttaatca tcatgggatc agtcccacca tgccccatgc ccatctttgg     180
   accagtgttc tagcctcctg actgttcagg tgagttatgg gagaaaactt aggaccccat     240
   gcaaatgcag actttctttt acattgtaaa taccactgac attcttgctc atccaactgg     300
   gacatttttg ccattcagtt tgtcaagcaa atataacgat gcatattttc agcacacact     360
   tccactgttt agcagatggc acatctgaaa catggtacat g                         401
<210> 233
   <211> 401
   <212> DNA
   <213> homo sapiens
<400> 233
   gggggcgcga gagagcagca agccgtaaca gctcgcgtaa aaggcgggag ctggggcttc      60
   ctgggggttc cctagaagcg gacctcgaga caaggtcctg ggtgcaagcg gtttatttgg     120
   gggatggctg caggaggcac agggtaggag cagggctggc agggaaggga gagaagggag     180
   gaagggcagt gaagagcaca aggatggagg ggctactccc gggcctgtgt gcagagcctc     240
   agccctggct ctgtcaggca gaaggagctc ccgacacctc tgtcatctgc tggctgcgga     300
   gaacctgggg ctgacgccgt cctccgggac ctccatccag aaaagggcca cagaggctga     360
   gggcgtcggg gcgggcactc cagggtctta ttctgtttgg g                         401
<210> 234
   <211> 401
   <212> DNA
   <213> homo sapiens
<220>
   <221> variation
   <222> 80
   <223> /replace="g"
<220>
   <221> variation
   <222> 81
   <223> /replace="t"
<220>
   <221> variation
   <222> 227
   <223> /replace="t"
<400> 234
   ggagagcaga gtgacggcct agaaaggagg ttcccgaggg ctctggggag gcggaagagg 60
   ggacgcggtg tgttgagata cggggagaag ttcagagcag catgggcatg caggtgcctg 120
   cagaggggcc catctgggct gcaccccagc aggacagggc catggcttgg tgtgggttcg 180
   gctggctgca cagcttcttg taaagtttgt ccccagggtc cttgggagta gaggtctttc 240
   ggaaggcacc atctccagag cgtgtttctg agtaactctg aggatgggtt ccgggttatc 300
   actgtgtgtt ggaaacagca gcgcccaggc tgccggagga ggctggtctc cctggcaggt 360
   cctggctgtg tctagagttt tgtgggtcct ggagataagg c 401
<210> 235
   <211> 401
   <212> DNA
   <213> homo sapiens
<220>
   <221> variation
   <222> 372
   <223> /replace="g"
<400> 235
   agaggcagag gattgtagtt gtgaaaagtt tgtacattgg agtctggctt tgagtcccag 60
   ttttcccgtg gacaggtcat gtgaccttgg caaaataacc acctcccatg cctcaggtgc 120
   ctcatctgtg aaatggggat aataataaca cctacttcaa ataaaacaat gcaaggaatg 180
   ccctagaaca aacgcctggc acatagtaag ttctacccaa gtatttatta ttataataat 240
   tgtataaaca tttaacgact tccttaaatt gtttcctatt acttgctatt ttgaatagtg 300
   tttcaattaa tatccttgtg atatggtttt gctgtgtccc caaccaaatc ttatcttgag 360
   ctgtaattcc cacagttccc atgtatcgtg ggagaaacct a 401
<210> 236
   <211> 401
   <212> DNA
   <213> homo sapiens
<220>
   <221> variation
   <222> 168
   <223> /replace="t"
<220>
   <221> variation
   <222> 277
   <223> /replace="c"
<220>
   <221> variation
   <222> 359
   <223> /replace="g"
<400> 236
   gatgtgtgtg tgcatatatg tgtgtgtgta tatatatata cacatacata tacagaaaga 60
   gagagagaga gtctcactct gtcttccagg ctgaagtgca gtggcatgat cttggctcac 120
   tgcaacttct gcctcctggg ttcaagcgac ttttgtgcct cagcctcctg agtagctggg 180
   actataggtg caagttacca ggccctgcta atatttttgt atttttaata gagacagggt 240
   ttcaccatgt tggccaggct ggtctcgaat tcctgaactc aagtaatcca cccacctcag 300
   cctccaaaag tcctgggatt acaggcgtga cccactgtgc ccagctccca aaatgtataa 360
   ctaagtgaga agggaaggat gtagaatagg gtgggtgttt t 401
<210> 237
   <211> 401
   <212> DNA
   <213> homo sapiens
<220>
   <221> variation
   <222> 215
   <223> /replace="t"
<220>
   <221> variation
   <222> 360
   <223> /replace="g"
<400> 237
   ctcaggtgat cctcccacct cagcctcctg gctagctggg actatagaca tgtgccacta 60
   tgcttagcta attttttgta tttttttgtt gagacaaggt tttaccatgt tgcccaggct 120
   gttcttgagc tgggctcaag tcatccacct ggcttggcct cccaaagtgt ggggattaca 180
   gcatgagttt ctgggcctgg tctctttgct acaccttgtc agggtctttt gcaggaattc 240
   cttgtggtcg ttggcaggtg tcatgcctta tcactggtag gcaagccttg gaaagctcct 300
   gtgaagattg tcctattagt atcagtttac ttttttgaaa aaatattttt attgatttta 360
   gagacagaat cttgctttgt cacccaaatt ggagtgcagt g 401
<210> 238
   <211> 401
   <212> DNA
   <213> homo sapiens
<220>
   <221> variation
   <222> 296
   <223> /replace="c"
<400> 238
   aattctactt ataatgttct tggcctggaa gtcaaggcct cacatatctt acttcaatcc 60
   actatttctg gcaggacacc tcaccacact tcccttgctc ctgtccacat gagctcttag 120
   gccaggggca tgcttctctg tttgaccaca tgcacctcct ctcctgttat ttctgccatt 180
   tgagatgctc cccttcctac tgctgcatta actgaaagcc acacttcatg atccagaaga 240
   gatgccacct cctctgccaa gctggccctg actttccagg cagaaatgat gtattaccat 300
   atggcattca ttgctttaca gtgaattaaa gctattattt ttagttgtaa aaatacattt 360
   tttaaaacta caagtgtaaa atacatttgt gttgcaaaag t 401
<210> 239
   <211> 401
   <212> DNA
   <213> homo sapiens
<220>
   <221> variation
   <222> 106
   <223> /replace="t"
<400> 239
   tgctcctgtc cacatgagct cttaggccag gggcatgctt ctctgtttga ccacatgcac 60
   ctcctctcct gttatttctg ccatttgaga tgctcccctt cctaccgctg cattaactga 120
   aagccacact tcatgatcca gaagagatgc cacctcctct gccaagctgg ccctgacttt 180
   ccaggcagaa atgatgtatt accatatggc attcattgct ttacagtgaa ttaaagctat 240
   tatttttagt tgtaaaaata cattttttaa aactacaagt gtaaaataca tttgtgttgc 300
   aaaagtccag ataatacagg aaaataaaag cttcccgttc cctcagctct cttctaattt 360
   taattcttgg agctattact aagagtgggg gccgggcgtg g 401
<210> 240
   <211> 401
   <212> DNA
   <213> homo sapiens
<220>
   <221> variation
   <222> 137
   <223> /replace="g"
<220>
   <221> variation
   <222> 334
   <223> /replace="t"
<400> 240
   taagcttcag aagaatctta gtagaaatgc attgtttgga atgcatatgc tttggtctca 60
   ataaaaatat ccggttgcag agatatttca tgtggtttga actaacaggc aaaaaattaa 120
   aaattcattg gtatacacta attcatgcaa ttgtatattt tggaggtaag tgtaatggca 180
   taggtttgct gtaatggaac tgattggaaa atgatctctt ggagaaaatt tattgctggt 240
   cataagattt ctttggttta gaaaaatgct gggtttagta aaatctgagg cactaaatga 300
   aagtctgctt taataaaaat gtggatctga gaactgtggt ttttagtaag tgggcatttg 360
   atccattaaa attgtattgg ttttattctt tacgttggct t 401
<210> 241
   <211> 401
   <212> DNA
   <213> homo sapiens
<220>
   <221> variation
   <222> 262
   <223> /replace="g"
<220>
   <221> variation
   <222> 279
   <223> /replace="g"
<220>
   <221> variation
   <222> 355
   <223> /replace="g"
<400> 241
   agggcaggca agatggatga ggcaaataac tcaatgagca agtgacggca ggcccttcag 60
   gccgccttct catgcaattg atagttgctc cttcctcaca tttggtttgg ttccctaaat 120
   gttaaatcat ctctaggata agcaggtgat agcccatgaa tggagccaga tgctgagatg 180
   ttgttcagct tgagtggcct gcagttcatt cctgaccaag ctgtggtttg ttcctgctgg 240
   ctgttggatc acctccttca aagattcatc actggataag ccactgttgg ccattggagc 300
   tattccacca agactgatct gaactctccc tatgagactt ttctttttcc agtaagctcc 360
   atatctgatt ctatgaaaag ttcagcccaa atgagaggac c 401
<210> 242
   <211> 401
   <212> DNA
   <213> homo sapiens
<220>
   <221> variation
   <222> 198
   <223> /replace="c"
<400> 242
   agacatgcaa aatataaaca cacatttcat tgttattaga ttccacaagc ataaaattat 60
   tctgtcagag tgactatttg gttctttctc atctctatag ttatctcttc gcagacaggt 120
   aacagcctgt ggaggaccac actgggtatc atttgtccgg caggtgctgc gatcctaaga 180
   gggtaacatt ctccccagcc actggaagtg ggtggaatgg agccactata gtaaatgttg 240
   ttggatgcct acttagcagc ctgcctcctg ccccctgcct ccttcctgag agactccaat 300
   ttactcagct gtgcactgga cacaccaccc tgattgtggc tggcctcacc tgtaggttgt 360
   ggcaaacttg actggcctaa gggtaatccc atatgcctta c 401
<210> 243
   <211> 401
   <212> DNA
   <213> homo sapiens
<220>
   <221> variation
   <222> 13
   <223> /replace="c"
<220>
   <221> variation
   <222> 108
   <223> /replace="t"
<400> 243
   atgggaaaaa agagggtctt gataacattc taattagccc caaaccctct attcccatga 60
   gccagtctct tctctttaag ttgatgctag ctgccgtttt gtgttatctg ttacagacta 120
   atacaatttg caataattga agatgcaata tttattgata tattattatc aattgaagtc 180
   catagcttac attaggattc actcttagtt ttgtatgttc tgtggatttt gacatatgta 240
   aaatgacttg tgcccaccat tacagtatca catagaatgg tttcactgcc ccaaaatacc 300
   ctctgttcca tctatttatc cctcttcctt tccatggacc cttgacaacc actgatcttt 360
   ttattgtttc catagttttg ccttttccag aatgtcatgt a 401
<210> 244
   <211> 401
   <212> DNA
   <213> homo sapiens
<400> 244
   agtttcctat gaaggcagtc gctaaagaac attccacttt gcataaaaga cttcatttca 60
   aggctttcag tcaaatgcat ggctagctga tggatcccac tgggcttaac aagcacaatt 120
   aaggtcttca tcaaacctta atagattggt gtcattgagc actgaccagt gcttggaatg 180
   tttttcttct ctgcctatca tccatgtctg aaattttcct caaatattgg caaagagaac 240
   atataagggt ggattagtag ttctccttta ttcatatatt gtgatatttt aaaggcatct 300
   ttcagtaaat gcgttatcat attagcatct aactgactga tccaatgggt tatatcaaaa 360
   tgcaaggtgc gtacacatgg gactcatatc tcaaattcat t 401
<210> 245
   <211> 401
   <212> DNA
   <213> homo sapiens
<220>
   <221> variation
   <222> 313
   <223> /replace="c"
<400> 245
   gcctgtttct aggtccagaa tggtattgcc taggttgcct tctaggagtt ttatagattt 60
   gggttttaca tttaagtctg taatccatac tgagttaatt tttatatata gcataaggaa 120
   agtgtcctgt ttcaatcttc tgcatatggc tagccagtta tcctagcaca atttattgaa 180
   tagggaatcc attcctcatt gcttgttttt tgtcaggttt ttcaaggatc agatagttgt 240
   atgtgtgcag ccttatttct gggctctcta ttctgttcca tcagtctatc tgtctgtttc 300
   tgtatcaata ccatgctgtt ttggttacta tagccctata gcatacttca acgttgagta 360
   gtatgatgcc tccagctttg ttcttttgct taggattgtc t 401
<210> 246
   <211> 401
   <212> DNA
   <213> homo sapiens
<220>
   <221> variation
   <222> 310
   <223> /replace="g"
<400> 246
   tttaaaatag ttttttctag ttttgtggag aaatatcaat aatggtagtt tgataggaat 60
   agcattgaat ctataaattg ctttgggcag tatggccatt ataaagatat tgcttgtcat 120
   tatccatgag catggaatgt ttttccattt gtttgtgtca tctttgtttt gtagttctcc 180
   ttgtagaggt ctttaacttt ttcggttagc tgtattccta agtatttttt tttcttgtgg 240
   caattgtgaa tgggattgtg ttcctggctt ggctcttggc ttgactgttg ttggtatata 300
   gggatgttaa tgatttttgt acattgattt tgtatcctaa gacttttctg aagttttgct 360
   gatgttgttt atcagcttaa ggaaattttg ggcttagact a 401
<210> 247
   <211> 401
   <212> DNA
   <213> homo sapiens
<220>
   <221> variation
   <222> 150
   <223> /replace="t"
<220>
   <221> variation
   <222> 318
   <223> /replace="t"
<220>
   <221> variation
   <222> 343
   <223> /replace="t"
<400> 247
   atctgctgtc ctctgggcct agtttcactc ccatggcagt gttagcatga gggctggatg 60
   ctggtagggg cagggatggc tagttctgtg tctgtccagg ctccatctgt aatggtgata 120
   agaagggaga gggtgggtgg actgcactcc ttgggcaatg gtgggaccag gaaagcaaaa 180
   ctcacctgtg cagacacatg tcagcaaagc aatgtgggga gttgttgtgg gcctggggga 240
   agcagtgctt tggggaggga gtgggagggc tggtgcatgg caactgcctc actggagttc 300
   tctgctgctc aggcatggtc cactgttgca gaagctatgg tgcggtccca cagggcagcc 360
   aagacagccc tgcaagcagg catggccagg ctggggtcct g 401
<210> 248
   <211> 401
   <212> DNA
   <213> homo sapiens
<220>
   <221> variation
   <222> 31
   <223> /replace="g"
<220>
   <221> variation
   <222> 112
   <223> /replace="g"
<400> 248
   ctggcatcct ggagctcaga gcagaggaga agaggtcaga cagtggatct agaggggcag    60
   acaaaaatat ccagaacaag tttcactctc catgggcctg aactagtggt tacaattgct   120
   ggaaggtaga aagtgttagg gggtgttcag gtatgggatc cagatgaagt gggagagaat   180
   tttagtatgc tttgtgaaga tttcccatga tttgaaattg tgtaaggact gtcccaaatt   240
   ttgcaaatga tgctatatca taagataaag aagacagtca tactcatttg gtagctatat   300
   ttatataatg tattcatata aatgtataaa tatgtatatt atgtaagtgg ctgatgttta   360
   catttgctta atcatatgag aatatagcaa aattataact g                        401
<210> 249
   <211> 401
   <212> DNA
   <213> homo sapiens
<220>
   <221> variation
   <222> 234
   <223> /replace="t"
<400> 249
   tatttcactt agcatagtgt ccttcaggtt catccatgtt gttgcaaatg gcaggatttc    60
   tttcatttga aggctaacat tccattggat gtatatgcca cattttcttt atccattcat   120
   ctgttgatag gcatttagat tatttccata tcttgccttt tgtgaacaat gctgcaatga   180
   ccgtggaagt actggtattc ttttgagatc tgatcccaat ttcatttcca ctggatatat   240
   acccagaagt gagattcctg gattgcaagg tagttctagt tctataatgg ctacaaccaa   300
   tttacattcc caccaacact gtgtggtttc cttttctcca catcatcacc agaatgtttt   360
   gttttatttt atttttaaaa taatagccaa accatttgga t                        401
<210> 250
   <211> 401
   <212> DNA
   <213> homo sapiens
<220>
   <221> variation
   <222> 274
   <223> /replace="g"
<400> 250
   tttctgagaa gtgcacgtta aacagcatag catttcagcc ttgaagtgag aagtgtagac 60
   tatttgtgat aatgaatata caactgaggt gttccactgg agcaagtcac ttagaatcac 120
   agaaagcgta ttatcaggcc catgatgtaa atttaaaatg tcttattcct cattttacaa 180
   acttatgtgg gcatctttcc ttctcacagg aagcccattt cagtgtggaa aatcagtctt 240
   gtatgaacca gatttagaac agtgccatga ttcattcata ttcaagagtt aactgcttct 300
   acacagagtt ggtgtcagaa ggcatgttgc tgagtgccag caactccttg ggggttgagc 360
   ttagatggtg tcctctgttc tagcatgtga atgctgtaaa c 401
<210> 251
   <211> 401
   <212> DNA
   <213> homo sapiens
<220>
   <221> variation
   <222> 82
   <223> /replace="t"
<220>
   <221> variation
   <222> 388
   <223> /replace="t"
<400> 251
   tgtcccactc agaggggcgg ttctgaggca tgctccctac agtctcccag agtggcccca 60
   ggagggctaa gcctcagttg cccatgatgg caatgctcat tattgcaccc tcgtggcttc 120
   cttctctttc ctgcctcatt tcgtactccc aagctgtgct cccctgcatc acttcctaaa 180
   ctaaatccct tcatctaaga acttatccca gggtctgctt tggggaagaa caaaagacaa 240
   agcacttatg atatactcaa gactaacaga agcataagct tctgaggatt taaatgcaaa 300
   tagaaacaat gcaatagaaa tagactaaaa ggcctgtgct ctccttaacc cacccagcct 360
   atttcagact gatgggcttg gggtcagttt ctgccagagt g 401
<210> 252
   <211> 401
   <212> DNA
   <213> homo sapiens
<220>
   <221> variation
   <222> 94
   <223> /replace="g"
<400> 252
   ttttactttt tggaaagcca agcaggtccc agagtcgtgg ggcagtactt ggcctcatct 60
   ctgaggccac ccattctgct gctggcagcc acaatgatgt acccttggca ctatgtcgct 120
   ccccctactt ggcatcctca gtttttctaa actcatgggt gagagacgaa tgacgtcaga 180
   ggcccaaacc tttggatgct gatctaaaag catcaaccaa aacccaacac ccgagcttcg 240
   taagttccca tttgaagcag gagcagtttt tatagcaaca gacaaaggat tgctctgtcc 300
   ttgttggaag tgcttgagga ccaagcacag aggtgggcaa agatcgtcat cagggcttct 360
   gctacctgtc ttagccccag cattcttcaa aagagatcct c 401
<210> 253
   <211> 401
   <212> DNA
   <213> homo sapiens
<220> .
   <221> variation
   <222> 69
   <223> /replace="t"
<220>
   <221> variation
   <222> 242
   <223> /replace="t" ,
<220>
   <221> variation
   <222> 310
   <223> /replace="c" /replace="g" /replace="t"
<220> .
   <221> variation
   <222> 334
   <223> /replace="c" /replace="g" /replace="t"
<400> 253
   taaatttgaa gtgttatggt gagatggtat cctggagacg tatgtgtgaa taataaaaat 60
   gtcagcacct gctgcaaaga accactgaat gaaaggaaag gaaaatacca tacatcaaag 120
   aaagaggttt atttagattc aaagtcccct gggttaggac cacacctagt acactgtcac 180
   atccaataaa tgttgagaag agactgtcta gcacatgaga cacagttgtg gagatgtcct 240
   aggcaggctc caccacagaa gcaaaacatg atccagttgc aatggaaatc cactctttgc 300
   tcatgtgcta tttcccaagt acttgcttat taaaatataa ctctgctaat gaaccaaaca 360
   cagggagttg ctttctagag ccgaaaaaaa gggactgtga g 401
<210> 254
   <211> 401
   <212> DNA
   <213> homo sapiens
<220>
   <221> variation
   <222> 35
   <223> /replace="c" /replace="g" /replace="t"
<220>
   <221> variation
   <222> 59
   <223> /replace="c" /replace="g" /replace="t"
<400> 254
   gttgcaatgg aaatccactc tttgctcatg tgctntttcc caagtacttg cttattaana 60
   tataactctg ctaatgaacc aaacacaggg agttgctttc tagagccgaa aaaaagggac 120
   tgtgagaaaa agctagggtg gtctaaggag tgaattagtg acaatgtcag tggatcaact 180
   cccaacacat aggttttcag acagggaatt agtctaggca gaagttcatc agacccagtt 240
   cttagatgtc tttggtcttt gtggaagatg ctgttggtgc cctgttcgtg tactccatgg 300
   gctcttactg gaagcaccat ggctcattca ttatcagggg gctttcttga gcacactttc 360
   ttgcacaagg tggggcacag gctggcattt gtaggggagg t 401
<210> 255
   <211> 401
   <212> DNA
   <213> homo sapiens
<400> 255
   actgtcctct agggtgctaa cacagtaaga ctggatacct ccttagacca gtgcacagca 60
   tggtcaaatg cactgaatcc catccacaaa gacagcctca ttaactattc agaaaattgc 120
   tattttgtgc ttctaatctc tgttaagaca ctgacaagaa gtcattctag tcatgtgtta 180
   cagacatgga gatacaaagc tcagattcgc cttcaaggaa ggacttgtgg cccagatgca 240
   ggggtgtggc tatcaggtgc ctccagggtg tgtctcagct gcagagatca tccttcctag 300
   ggtagcgtgc atctggtggc tgagtgaaat ggagaataaa agtcttgcag ttccccctga 360
   tgcttgatga cttgcgcaat actcactcta gaggtccctg t 401
<210> 256
   <211> 401
   <212> DNA
   <213> homo sapiens
<220>
   <221> variation
   <222> 35
   <223> /replace="t"
<220>
   <221> variation
   <222> 94
   <223> /replace="t"
<220>
   <221> variation
   <222> 145
   <223> /replace="t"
<220>
   <221> variation
   <222> 190
   <223> /replace="t"
<220>
   <221> variation
   <222> 222
   <223> /replace="t"
<220>
   <221> variation
   <222> 276
   <223> /replace="g"
<220>
   <221> variation
   <222> 298
   <223> /replace="c"
<400> 256
   gaggtttcat ggagagtgct ctcaggatcc acacctgcga aataactata ataataataa 60
   taataataat aataataata ataatagtga aggcctggac tgggtagagg aagaaactga 120
   tccaaaacaa acgtgcaatg aggcctcagc caattcctgg ggggcagatc tggctgtgta 180
   cagcagttcc tgaataatgt tggttcattc gatgttcttt ccttataacg ttgatgagaa 240
   aacaattgat ttgcacctgg ggccaccatc tgtgtatatt tgcacaacct ccccataact 300
   gcatgggttt tctctgagta ctctagtttg ctctctcatt ccaaagtcat gcacattagg 360
   ttaattggca tgtctacatg gactcagtgt gagtgaatgt g 401
<210> 257
   <211> 401
   <212> DNA
   <213> homo sapiens
<220>
   <221> variation
   <222> 42
   <223> /replacen"g"
<220>
   <221> variation
   <222> 64
   <223> /replace="c"
<220>
   <221> variation
   <222> 181
   <223> /replace="g"
<220>
   <221> variation
   <222> 225
   <223> /replace="g"
<220>
   <221> variation
   <222> 396
   <223> /replace="t"
<400> 257
   tgagaaaaca attgatttgc acctggggcc accatctgtg tatatttgca caacctcccc      60
   ataactgcat gggttttctc tgagtactct agtttgctct ctcattccaa agtcatgcac     120
   attaggttaa ttggcatgtc tacatggact cagtgtgagt gaatgtgagc atgggtgtga     180
   atgtgctctg ccatgagaga ttgtcctgtt cagggctggt tcccacctca ctccgtgagc     240
   tgctgggatg ggctctggcc acccatgacc atgaactgga aataagcagg taaagaatta     300
   tcttacttgg ttttattaat ctttcttaaa tgtatgtata gttcacattt atttcaatgt     360
   ttaatattag aaatgttttg cctttattta gaagtgtggt a                         401
<210> 258
   <211> 401
   <212> DNA
   <213> homo sapiens
<220>
   <221> variation
   <222> 81
   <223> /replace="g"
<220>
   <221> variation
   <222> 108
   <223> /replace="t"
<400> 258
   attgattttt atgtctatgc ttacaccagg agcacactgt tttcattttt gtagcttttt      60
   agagagtttt gaaatcaata agtgtctttc aattttgttc accttttcaa aaataatttt     120
   ggttattctt gagtttgcac ataaaatttg gaatttctga aaaaacaaaa actgagattt     180
   ttatagggct tgtattgaat ttttatatca gttttgggaa tattgtcatc ttaacaatat     240
   tgagtcttcc aatccatgta tatggatgtc ttttcattta tttatgtctt taaaattttt     300
   tttaaataat gtcctgtagt tttcattaaa tatcttacac ttctttggtc aaatttattc     360
   ttaaatatat tataattttt gatattactg taaatgatat t                         401
<210> 259
   <211> 401
   <212> DNA
   <213> homo sapiens
<400> 259
   aaattgggac aacttagtgt tcgagaaata gagaaatagc tcaaggagct attagatacc 60
   aacccatacc atgttgtttt ctgagggtgg gagaaactag aagaaagttt gacattactc 120
   tcattccatg agacaacctc aagaacacaa acccaaacta tccaggtagg acttttagtt 180
   tagggagacc accaaaagaa atggagccca tttcctctgg tgattcaaca agattaacag 240
   cccctcctga agtttttgtc cagtggccta gaatctaaat atgtgctcat acccagttct 300
   ttgtcttcaa ttcccgagtc ttctgtgtga ctcattctga aagtccaggt gtggaaggga 360
   gaggcgcatg gaggaaaaag ccaggctcag aatttatatt t 401
<210> 260
   <211> 401
   <212> DNA
   <213> homo sapiens
<220>
   <221> variation
   <222> 41
   <223> /replace="g"
<220>
   <221> variation
   <222> 139
   <223> /replace="g"
<220>
   <221> variation
   <222> 399
   <223> /replace="g"
<400> 260
   atgtatctat ggttgagaaa ttcccattaa atattggggc aaggttcaga tgctgtagat 60
   atttgtttac attttatcaa atgctttctt acatgtgaac ccctcaatct ctgagaaatc 120
   acctaaggga agccatggat ctgcctcctc caggtttcat tattgcatgg aatgtgaggg 180
   gatttctgtc ttaattaatg tataagccag ctcttctgag tttgggtgga tatccttgag 240
   gagaaaggta gctaaaatta gcagcctcta ctctcttctg tgttccttat aaaaaaaaag 300
   agaaactgtc tcatctttaa gaatggacca gttatgaact tccaaccttt ccattaatgc 360
   tttgaagcaa tgatggtgct ggctgatgtg cttaagtaag a 401
<210> 261
   <211> 401
   <212> DNA
   <213> homo sapiens
<220>
   <221> variation
   <222> 156
   <223> /replace="t"
<220>
   <221> variation
   <222> 290
   <223> /replace="c"
<220>
   <221> variation
   <222> 388
   <223> /replace="g"
<400> 261
   gatttaacaa gaactgcatg tattccctgt tatgtgcatg taggtatgag tgtatgtgtg 60
   ttatgagtgt gtatgtgtct atatgtatgt gtgtgtttgt gtctgttggg gagagaaatg 120
   ggataaaaag gatcagaatc tcacgtagac ttttcctaag tcattttccc cccaaagtct 180
   catttgtcca cctctatgaa acttacttgg ctgagaactg ctgccaaatg atccattttc 240
   taatgtgtta gtaatcacta catgaatcct cttgagaaag agctgggcca ggagaactta 300
   atgacttcca aggtcacaca gcaaatccag ggccgagcct ggttccccgc tcctgttcca 360
   ggctcctccc atgccaccac atcatcaagc ttgacatatg t 401
<210> 262
   <211> 401
   <212> DNA
   <213> homo sapiens
<400> 262
   aaagcatctt ccaacaaaaa aataattcag gaccaggtgg cttcatggct gaattctact 60
   gtactttaaa agaacaccta actctaattc tcctgaaact attccaaaaa ttgaagagga 120
   gggaattctt cctaattcat tccgtgaggg cagtattgcc ctgatattaa agccagacaa 180
   tgatgcaaca aaaaagaaaa tggatgaaca tagatacaaa aattctcaac aaaatactag 240
   caaactgaat ccaagggcac ataaaaaaat acaccacgct caagtggaat ttatcccatg 300
   gacacaaaga tgattaaacg tatgcaaatc aataaatgtg atacatctta tcaacataat 360
   aaatacaaaa accatgtgat catctcaata gatgtggaaa a 401
<210> 263
   <211> 401
   <212> DNA
   <213> homo sapiens
<400> 263
   acgtaagtgt ggcagcatct aaacaaatct gtcttcattt caaggaagag aacgctgata 60
   atgcacatgg atgttgcaac aatgtcaaat tgctatgaaa atttctaaat gaattttctg 120
   ctcaattgcc taccttgttg caaattaata acaggcagtt aacataactg gtagtgttct 180
   gcagaccaca ttctgagtaa tactacacaa tttactgcct gcatccctag aggggatggg 240
   ggaagaaata ggctaaatcc taaaatgcca tgatgccata ttcaaatagt gttttatgaa 300
   gaattgttca tttggcattc agcactgggc agtgattgaa cttgagggag gcaaggagtt 360
   ttagaacctg taatatacca ggtaagaatt tataaaaagc t 401
<210> 264
   <211> 401
   <212> DNA
   <213> homo sapiens
<220>
   <221> variation
   <222> 153
   <223> /replace="t"
<400> 264
   tcatttggca ttcagcactg ggcagtgatt gaacttgagg gaggcaagga gttttagaac 60
   ctgtaatata ccaggtaaga atttataaaa agctaacaga acaggttttc ataaaagtgg 120
   gactgagagt tttagggaca tgcagaaaaa atggggatat tgttaattat cccttatcct 180
   tatttattac acttgtcatt agttattata attaattatt agtgatcctt aattaatctt 240
   aaatatttta tgattattta aacatgaatt ttatgaatat ttaaataatt ttgtaaatat 300
   cataaaatat tttatgatat ttatcaattc agttatttca gataaaagca ctgaattagt 360
   agattctgaa caatcagtta agactgatca aatactaaat c 401
<210> 265
   <211> 401
   <212> DNA
   <213> homo sapiens
<220>
   <221> variation
   <222> 129
   <223> /replace="g"
<400> 265
   ttgggggaat cctttccatg cctttctctt taactgatct caggcaaaat gtttgtcctg 60
   gatgtacaat acttaaaaga gttgatttaa aatttcctga aaataagttc atctcagctt 120
   ttgtgtgaaa aaggaagttc tatgttggct ggtgaccatt tcaaagctac cctcagtctc 180
   acaattagtg tgcttggcaa tgctattgga cattggaatg caaatgttca catcccacct 240
   tgagcttgca ggattttgat gcatttcctt tttgttggac aaaatagtga gtattcccta 300
   agattacttc cagttgcaac ctgaacaaag agaaatgcat ctaaatatcc tgctccaaag 360
   cctttgaact tcaccaatgc ataactcatg ggacacttat c 401
<210> 266
   <211> 401
   <212> DNA
   <213> homo sapiens
<220>
   <221> variation
   <222> 88
   <223> /replace="t"
<220>
   <221> variation
   <222> 161
   <223> /replace="g"
<400> 266
   ggctgtttct aagactgatg tggcttaaca aggaattttc ccagaccatc tctactctcc 60
   acaattttca aacaggatta ttactttgtc tttcacctca gtgcccatgt gtttgtggat 120
   ccctctagat acttgctttg ctatttctta catctctaat atgaagaatt aatgtcccag 180
   ttataagaac tttatgtcct gtacgtagat gttctctagt cacacccctg acatgtgtga 240
   ggcctgggaa gaggcccaca tcccatgggc acaatattta aaagttgtaa atcaagtggg 300
   aaagttgtta aataaaacag attctattct tctacattgt cagatattcc accttcatca 360
   tgataaaata gaaaaatatg tgcaaacagg tttttgtgtg a 401
<210> 267
   <211> 401
   <212> DNA
   <213> homo sapiens
<220>
   <221> variation
   <222> 13
   <223> /replace="g"
<220>
   <221> variation
   <222> 26
   <223> /replace="t"
<220>
   <221> variation
   <222> 247
   <223> /replace="t"
<220>
   <221> variation
   <222> 275
   <223> /replace="g"
<400> 267
   ctatccaatc taatctaatc taatgcaatc taatctaatc taatctaatc tctatctagt 60
   ctatcatcta tctatctaat ctatcatcta tctatgtatc tatctaatct atcatctata 120
   tctacatatt tttcaagtgt ttacatgaag acagatatta tgggttggaa ttaatgggac 180
   agctgagact atggtttgag tcaatcagaa gagcttggat gctaaatctt gctaggtaag 240
   agaaaccttg agaaagaagg gtaaaaggaa gtggacaaag ttacataatt tatttaattt 300
   gtaatgaaac acagtggata gtattaatta taattttaca tataatttct gggataagaa 360
   acaaaagcag ttttattagg taaaatatta gtctgaaaca t 401
<210> 268
   <211> 401
   <212> DNA
   <213> homo sapiens
<220>
   <221> variation
   <222> 214
   <223> /replace="t"
<220>
   <221> variation
   <222> 348
   <223> /replace="c"
<220>
   <221> variation
   <222> 381
   <223> /replace="g"
<400> 268
   cacgtaagaa gtgccttttg cctcctgcta tgattctgag gcctccccag ccctgtggaa 60
   ctgtaagtcc agttaaagct ctttttgttc ccagttttgg tcatgtcttt atcagcagtg 120
   tgaaaatgaa ctaatacgga tggatagaag ggtggaggga tagatggata gaatgggtgg 180
   actcaatctt gttgcccttc tttttcttca ccccgtttct ctaccctctg caggaagagc 240
   ccaccttact tctcagtcta agaaagtggc aagcactgct gggctctttc cacacctgta 300
   gttcctcagt tttctgaaac ataggttgtg tgtttctaat ctggcaaaga cttgaaggaa 360
   aaaaaatcct ttttaaagtc attgtaaaca gatattactt t 401
<210> 269
   <211> 401
   <212> DNA
   <213> homo sapiens
<220>
   <221> variation
   <222> 39
   <223> /replace="g"
<220>
   <221> variation
   <222> 54
   <223> /replace="t"
<220>
   <221> variation
   <222> 55
   <223> /replace="t"
<220>
   <221> variation
   <222> 164
   <223> /replace="g"
<220>
   <221> variation
   <222> 395
   <223> /replace="t"
<400> 269
   tttgtgtact ttttgattgg ttgtctcagt aggcatcaaa tcacttgttg cggcgtgttg 60
   gggggagctt gttgatgcac agatagtggg gttccatcgc cagggtttct ggttcagaag 120
   gtctggtgtg gagactgaga atttgcattt ctaacaagtt cctaggtgaa ctgcagaatt 180
   acattttaag aacaactgtt tggagcaccc ttgccttaat aatgctgcag agactttctg 240
   ggctggtcca ggtgtcttgt ggctccgctt ctgctgtgtc aatttttgaa cagcatgtgg 300
   aggaaagatg ccctctccag ggtgtggtga ggagtgagga caatgtgggc atgtctgagc 360
   tgtagtgggc atgggtgatt cagcaaggac tgaccggaca a 401
<210> 270
   <211> 401
   <212> DNA
   <213> homo sapiens
<220>
   <221> variation
   <222> 321
   <223> /replace="g"
<220>
   <221> variation
   <222> 386
   <223> /replace="g"
<400> 270
   tgcagttcag acacagtggg tttcctgcaa ctcacttttg ctatggcgac ttttgttatg     60
   cacagtgaac aggaagtaga aatttgcaga atataagcct tcttcacagg aaacgtattt    120
   tggagctcac cacaccatac aggctgctga tttaatacaa ccaggatatg tgcaaatctc    180
   cgaggaatgg caaatgccat tcaccttcac ttttttcacc ttccttctcc tcctcctcct     240
   caccttcatt tttcaataac ccaattattt gctggtgcat ataaaagtaa acagaaaatg    300
   aataagctgt ggactttgaa agatagtttt tgagttagaa atctgaaggg ggagaaaagg    360
   ccacagaaac agggtaaaca gttaaagcaa acattttaga c                        401
<210> 271
   <211> 401
   <212> DNA
   <213> homo sapiens
<220>
   <221> variation
   <222> 305
   <223> /replace="t"
<220>
   <221> variation
   <222> 324
   <223> /replace="g"
<400> 271
   ttttgtagca gaggaatcat tcttgaaggt cccaagatcc tttctgctag acttcctatt     60
   cttatagttg tttctaaact taattctttc tggaaaacag aataactgca agaaaaaatc    120
   tacatcctga aagggttgaa cccatcttga aaatggcaaa atctccatcc tggtgatcac    180
   tggcaccatc actcccagta tagccatcac tctatgggat cctgggcact gcttagcact    240
   aggaaggcga atggctttaa attatgattc ctgtcctcaa gagataagtc acattgttga    300
   aaacctgaca tcaacgtaca aagattcata atcataaaag gtaactcttg gtaagtcatt    360
   ataatcttat taattgcttc taatggtaag tcacatcaat g                        401
<210> 272
   <211> 401
   <212> DNA
   <213> homo sapiens
<220>
   <221> variation
   <222> 38
   <223> /replace="c"
<220>
   <221> variation
   <222> 63
   <223> /replace="c"
<220>
   <221> variation
   <222> 279
   <223> /replace="g"
<400> 272
   tattatcttt gatgcaatgt tatcattata ctttatgact tttatcatgc tttctcttag      60
   ttatttgaac ataattataa tggctatttt gaagtctttt tctcttaaat ctgatgtttt     120
   gtcattctca cagacaaatt tatgttttct gctttttctg gtatatggct catatttttc     180
   tgtttcattg catgcctcat tactttcttt tggaagttgg acaatttaga tcatatgtat     240
   tgcagcaact gtgggtagta gcccttcccc aatgcttaac ttgtattgtt atttgcttct     300
   ttatgtttta gtgagtggct ggattatttt agcgatgtct gtttacctcc aacagtggta     360
   aacctctgag gtcgtttctc agggaggtgc agttttgagt a                         401
<210> 273
   <211> 401
   <212> DNA
   <213> homo sapiens
<220>
   <221> variation
   <222> 4
   <223> /replace="t"
<220>
   <221> variation
   <222> 382
   <223> /replace="g"
<400> 273
   gaagtctgac agagagtgtg tgtggttata gtggactgta tcagttcaaa gacccaaaga      60
   ttagctattc gtttgtgctt gggttttaaa gtggagtttt ctgagctttc tttgttcagt     120
   attcactaca attgagtaga ttttggggaa gaaatctgta ctgtatttgt gtaaaattac     180
   aaaataacac tggtttatgt gtgatgttat atgttttccc caattttctt tttattatcc     240
   tttgtaaaat tgcaaggcaa tacagccttc caattacagg gaccactgac aaacgtaaac     300
   agccatcaag taccagttgg cacacatctc ctgtagtgca tgccattgcc atcgcaccac     360
   tcctcaaaaa tgcacccaca aacttattca caattacgga t                         401
<210> 274
   <211> 401
   <212> DNA
   <213> homo sapiens
<400> 274
   gactgggcaa tttacaaaag aaagatattt aagggactta tagttccaca tggctaggga 60
   ggtctcacaa tcatggcaga aggcaaggag gagcaagtca tgttttccat tgatggtggc 120
   agtcaaagag agagctgtgc aaggaaactc ccatttttaa agccatcaga tctcatgaga 180
   ctcgttaact atgaggggaa gagtgcagga aaaacccaac cccataattc aatcacctcc 240
   caccaggttc ttcccgtcat acatgggaat tgtgggagtt acacttcaag atgagatttc 300
   agtggggaca cagtgaaacc atatcattcc accccagacc cctcccaaat ctcatgtcct 360
   cacatttcaa aatcaatcat gccttaccaa cagtccccca a 401
<210> 275
   <211> 401
   <212> DNA
   <213> homo sapiens
<220>
   <221> variation
   <222> 38
   <223> /replace="t"
<220>
   <221> variation
   <222> 45
   <223> /replace="t"
<220>
   <221> variation
   <222> 118
   <223> /replace="g"
<220>
   <221> variation
   <222> 144
   <223> /replace="g"
<220>
   <221> variation
   <222> 349
   <223> /replace="g"
<400> 275
   tattcattaa actattccac aaataaatgt catatgagaa cttacagctt taataagagg 60
   atgaaacaac tctggaaaat cagaaaagaa tgaatgggaa tcaactcact gatgtgcaat 120
   gatggaggat gctgagggct ttaagctgca gcaacttact taataagttc ccaagcagga 180
   gagaacttgg cttctacagg gaattaaaaa gtttgccaga acaagttggt ctggagacac 240
   aggcagctca gagtcttatt ggtcatgcta aggagatctg agtcttagtt acatgaacaa 300
   tcaaaaactc ctgaagagtt ctaattctgt tgacaaatta tcagatttat cttctgaaag 360
   gaacactctg acagtcttgt ggaaaagaaa ttggaaatgc t 401
<210> 276
   <211> 401
   <212> DNA
   <213> homo sapiens
<220>
   <221> variation
   <222> 45
   <223> /replace="t"
<400> 276
   ttggacacat tgcattgcat acatttattt atcaagtcat tccacaagaa tgtactgatt      60
   gtcttctgtg tgtcaggcat tgttgcaggc tctggggatt cagcaataca taatttgatg     120
   atcgaatttg atgtatcttc acaacatcca agaggaacta tcagataggc ctttggacat     180
   atttggtctg gacctcagaa tgtaggtgtg aattctgagt tgcaactata catgtatgtg     240
   tcatttgtgt gtaggtgaca attgaagctt ggggcaaaga tgagatgctc tgattcttag     300
   attctaaggt gagatcaata aataagaaga aaaaaaggag tcatctctgg gactacacct     360
   tgagaatctc aagttttaat ggatggctta ggaattactc a                         401
<210> 277
   <211> 401
   <212> DNA
   <213> homo sapiens
<220>
   <221> variation
   <222> 214
   <223> /replace="t"
<400> 277
   aactcaggat tttgctcact attagaactt agaggaggtc ccagacacca gatgctatga      60
   cagtaccaca gaaaagcatc aaataaatgg cttaaataaa ttatctgtca ctcaaggaaa     120
   tactcttacc atctgcagag gtaagaactc ctggtctcac ctctgcatgt caaaaactcc     180
   aaagagctac aagaagaaaa gcttgaaact catgttctcc caatgttcag tcttcttctc     240
   aacacaaggc atatcagatt ggaagctgac ccctgtgtcc tgacaccatg gattctataa     300
   acttgctgaa tgactgatgg agactgatgc catcttccta aaaaatgagc cattgtggtc     360
   cttagcagtg ggcaataaaa aggagagcta tctactgcat t                         401

<210> 278
   <211> 401
   <212> DNA
   <213> homo sapiens
<220>
   <221> variation
   <222> 273
   <223> /replace="g"
<400> 278
   aatccatttt gtctcttctg ctattttgga gactgctctg aaaatgtttt tcatggtttt 60
   gcatgactct tgggatcacg atttgcatct taaaaaaagg agtcatggaa aatattgcaa 120
   gatatgcatt tggtggagag agaatgcttt tcagcttgaa gctatttggg ggtctgatga 180
   tctgatgatc tttctgttgt tggagctttg agcaggcaca tgctgtagag atgctcatgt 240
   gtcactgggg agctggtttc tgatgcgact ggaacttata gatatttgat aagtcatctt 300
   ttccttctga tactgccgtc aattcagtga attgaataca tacagagtct atggtaggag 360
   agcaaaagat caaaaggccc actatgttac ctccccacac t 401
<210> 279
   <211> 401
   <212> DNA
   <213> homo sapiens
<220>
   <221> variation
   <222> 292
   <223> /replace="t"
<220>
   <221> variation
   <222> 356
   <223> /replace="t"
<220>
   <221> variation
   <222> 361
   <223> /replace="t"
<400> 279
   ggaaatgccc ttgaagctga ggctggaagg acacggagga gctgcccaga gggaacagcc 60
   agtgctattt catgatggga cagagcatgg caggtatgag caagagaaag aggccagtgt 120
   ggctggagcg taatgcaaga aagagagtgt gacaaggtgc agctgagcag gtgggagggg 180
   tggcatcaaa cggggcctag gatttacgat gtgacttgca gaacaatagg aagcaccaaa 240
   gattgaaagc ctagtagtga catgagttta tgatgtttaa ggattcttcc acctgcaatt 300
   caaggtggat tggggggtca gatggggcaa tagtgggtga gattttaatt aggaagctcc 360
   ggtaacccag ccaataagga tggcagcatt agctatggga t 401
<210> 280
   <211> 401
   <212> DNA
   <213> homo sapiens
<400> 280
   tggaaagtaa ataggagagg agaaactcaa tggcacattt tcttctcttt caaacacatg      60
   ttcttattta ataaattctc tctcaaataa aataagcctg tagttattgg tggtcagttt     120
   catgtaatag gagctactac agttttccca ttaaaaaaat aatacaagac tgcaataaag     180
   catatagtga gtcactttat tgggtacgaa gtaataaacc agtggttaca atgataacgt     240
   ctatgatgtg tcttagaaga aacaaccttg attgttttgt ttggtttggg gtggggcttt     300
   ttggtttagt ggcacagaaa accaatatgg gatcagaccc cacccttcaa atcagtgttc    360
   ttatagcaac tggaaaattt cactctcagc aatgatcaca a                         401
<210> 281
   <211> 401
   <212> DNA
   <213> homo sapiens
<400> 281
   agagggttaa gtttacaagc atcctatctt catagcactc aataatagat atggctttat      60
   tgtgtggtca catccattca tctaggctgg tgatataaat tagtggtcaa agcattcgct     120
   ttggaatcat acaagcctta atttaaatta tagccccacc acttaagaga taggagacct     180
   tgggaattca tgtatgttct tcgagactta atttccttac ctgcaaaatt aggataataa     240
   tattaatagt tacattataa aattgtgaag attaactgat atatataata agcttagcac     300
   aatgtctgac acagagcata agctcaagaa atgatagctg atgttactac ttttagaggt     360
   ccatgtccat aaaacccttt atagagaaat tggaagtagg a                         401
<210> 282
   <211> 401
   <212> DNA
   <213> homo sapiens
<220>
   <221> variation
   <222> 41
   <223> /replace="t"
<220>
   <221> variation
   <222> 231
   <223> /replace="g"
<400> 282
   ttaactgtgc cagagaagga aatgtacctg caattgctca ctggggtaat atttaagtca      60
   aggctggatt tcaagctcaa tttaagtgct acattttgat tttttttaag aagaaagatt     120
   tgcctgtaaa aatgctactt ttcttataca agatctataa acatgctttg acaacctttg     180
   aaattgatta aaagtgggct tatatttcta aatggagatg ccttgagtca agatgagtaa     240
   gctaagaaaa tctattcatt ttagatatat tttaactctc cttactgtgc attgaaatta     300
   tcaataattt ccagttattc tttgtttata ttttggcacc attttattcc cctcccctca     360
   aaaaaaaact ccaaacacta aggtgaaatg taagcaaata g                         401
<210> 283
   <211> 401
   <212> DNA .
   <213> homo sapiens
<220>
   <221> variation
   <222> 76
   <223> /replace="g"
<220>
   <221> variation
   <222> 296
   <223> /replace="g"
<400> 283
   gtaagcaaat agtactggaa attttataaa ttttatacat agcaatcaga agtttataaa      60
   gtagatcatt ctgaaagcag attatgttaa tcagtcaaac tatttaaaag caggagtgcc     120
   aatgtggggg taaactcaac tctttcgaag agctcatttg ttaacacatt tatcgatatc     180
   cccagttgcc atctccttaa tcttttgctc actcggagga aaattcctaa tagcaagata     240
   cacatgaaga cagcagaaca tttacaagaa agtcagccag acatgtggca gccaaagtct     300
   gtcaaagtgt agagaatttt ttgtatgtgg caaaatacac ataacataaa attgaccatt     360
   ttaaccattc ttaaatgtac agttaagtgg cattaagcac a                         401
<210> 284
   <211> 401
   <212> DNA
   <213> homo sapiens
<220>
   <221> variation
   <222> 357
   <223> /replace="c"
<220>
   <221> variation
   <222> 371
   <223> /replace="t"
<400> 284
   ttggtgagtt ttcccatttc tcctcttcca acaaccttat tcagaagaag ctgtagctac      60
   ctgtttttgg cagaggccag agagtttgag caccttttca ccagctcacc aactcaatgg     120
   tggactttag caaagcatta tctaatttta gtacccacct gcttacccac tatactataa     180
   actacaaaaa gcagaacagc gtagcatttt tgcataaatg gtggatcaac agaccagaac     240
   tgggagccct ggaatgaatg tgtgttagag gaaccattgc ctgtcaagga atatgatatt     300
   attcaaagat gacttaccta tttggaaaaa taaaacaaag cattctcata ttatatgaca     360
   tattaaaata cattccacct ggattaagga gttatcgaaa a                          401
<210> 285
   <211> 401
   <212> DNA
   <213> homo sapiens
<220>
   <221> variation
   <222> 36
   <223> /replace="g"
<220>
   <221> variation
   <222> 37
   <223> /replace="t"
<220>
   <221> variation
   <222> 188
   <223> /replace="g"
<220>
   <221> variation
   <222> 283
   <223> /replace="g"
<220>
   <221> variation
   <222> 293
   <223> /replace="g"
<400> 285
   aacgggatgg tattctcaaa cttttctgtg cataaaggtt atggagagct tttaaatatc      60
   cccatggcct ggacacatcc aagaccaata aatcgcaatc tcagttattc aaatttgcag     120
   gtaatttcaa tgggtggtag ccaagtttga gattaaagta aagaagcaag gttctgtaaa     180
   cttttcaagt gatcccatct tgcaaacgag gaaaactgca atctgctttt tcacagccat     240
   cgtgtgtttc agcaattgca aaatattctc agactatctc taagagtccc taagactctg     300
   gtcacatcac ctcctgcagc aagttgacgg aaggccacta tacttggtga cttttctctc     360
   atgcattcac tttcacctcc tagagctttc ttttggaccc a                          401
<210> 286
   <211> 401
   <212> DNA
   <213> homo sapiens
<220>
   <221> variation
   <222> 199
   <223> /replace="g"
<400> 286
   ggtagcatgg gttatctatg ttattattat acctcttcaa gcctgtctta aaaccgcccc 60
   atccaggaag ttccaggtta ggaaagcgtc ttctttttaa actaagcctt tgggcttaag 120
   atattgtcga gatctgcgaa ctgtgagtaa tggctcattc ttctccatgt gtatcttgat 180
   tttttgacac aggcttcaaa tcctacctaa actgcagacc aatctctgaa agcaactttg 240
   ttcaattgca gcctgactag gtaaggcttt gagaatttgt ttctgtttta aatcctacag 300
   gataaaagag gtggagggtg gtctataata ggatgaagtg gtatataata agtatttaac 360
   ttaaaagaaa atctaccatt taaaactgta actagattat t 401
<210> 287
   <211> 401
   <212> DNA
   <213> homo sapiens
<220>
   <221> variation
   <222> 248
   <223> /replace="g"
<400> 287
   tattcttttt ccctttgtta tgtttcttcc atttccaagt ggatgaagct ctaactcaac 60
   ctttcatttg tttcaagagg gaggtatcag aaccacaaca aatggaatga aacctaccac 120
   taccacctgc ctggcacaaa gaaataattt acaattcacg gggaatttcc tgtgtttcat 180
   tttgtctgaa gaactgtaac tggacttgaa tgaactaagt gtctaattgt tgcattattt 240
   ttcttgtaga gattttagtg ttttattact ttctccagtt tacttcaata gtgaattagg 300
   ttccctctac catctgaaat gttacagttc gataaaatag gtaaattaat aaagtctaaa 360
   gtgcttccaa gctgagcaga gacctttctg tgcgctgtgc t 401
<210> 288
   <211> 401
   <212> DNA
   <213> homo sapiens
<220>
   <221> variation
   <222> 9
   <223> /replace="g"
<220>
   <221> variation
   <222> 217
   <223> /replace="t"
<220>
   <221> variation
   <222> 324
   <223> /replace="c"
<400> 288
   tttttaccat aactattttt attaggttcc taagtgataa gatcaggaca aagacaggtt      60
   cattatgcta gttagacatt ttcctaaagt atgataaaat aaatgaaaag gaaaacaata     120
   cctgtttatc tgagatagaa gaagtgttta caagttgtta tgctgcattc tagttgtgct     180
   aggggttaca gggatctata tgtgtgttta tgctcacggg atgtacacca aaaagaagtc     240
   aattttactg tatgttaatt acaaaaagaa agctattaaa tcttcctcaa atcctagaaa     300
   aatataagta cacattattt cccacacaaa ctcttgtgaa gggttaacaa aactcatctg     360
   gctttatttc atgcaagact tagaaaggct aatagtacgg a                         401
<210> 289
   <211> 401
   <212> DNA
   <213> homo sapiens
<220>
   <221> variation
   <222> 185
   <223> /replace="t"
<220>
   <221> variation
   <222> 308
   <223> /replace="c"
<400> 289
   ttttattagg ttcctaagtg ataagatcag gacaaagaca ggttcattat gctagttaga      60
   cattttccta aagtatgata aaataaatga aaaggaaaac aatacctgtt tatctgagat     120
   agaagaagtg tttacaagtt gttatgctgc attctagttg tgctaggggt tacagggatc     180
   tatacgtgtg tttatgctca tgggatgtac accaaaaaga agtcaatttt actgtatgtt     240
   aattacaaaa agaaagctat taaatcttcc tcaaatccta gaaaaatata agtacacatt     300
   atttcccaca caaactcttg tgaagggtta acaaaactca tctggcttta tttcatgcaa     360
   gacttagaaa ggctaatagt acggaactgc agatcaaacc a                         401
<210> 290
   <211> 401
   <212> DNA
   <213> homo sapiens
<220>
   <221> variation
   <222> 281
   <223> /replace="g"
<400> 290
   cctccctcaa aatacttgca tctggaattt ggctcctttt ctccctgctt aaatggtata      60
   gtgcaggtga taactgcgta gataaacctt tatagttaga aagagtctaa atgttggtgt     120
   ctgtaagcac aataagtaaa tgagtttggt ttttccttcc aaaataactg aacgttcact     180
   ttaaatataa tgttagccag ttttgaatcc agaatattta catcttgcag actgtgtctt     240
   gcagcctctg aatcaactga ctgggagcat ccgtctctgc atgcagggct gctgaaaggt     300
   gggagcagag ataaaagaca ggagatggga agaaacaaag ggagccaaga gatttcatag     360
   aatgctgcaa gagacattct tttgatctat ttggactcct c                         401
<210> 291
   <211> 401
   <212> DNA
   <213> homo sapiens
<220>
   <221> variation
   <222> 291
   <223> /replace="t"
<400> 291
   ttgaaaagat agtgtcaaaa ttgtaccaca atctggcagc tcccaaatta ttgttgataa      60
   gccaatttca gacttgtcta catatcaaaa gttatttttt tctcagggaa aagaagagcg     120
   tgcatgtgca aacgcctgcc actcaacaac cgtaagacta tgagagggcg ggactcactg     180
   gaaggcagag agccttttta tttctctttc tctgacctat gatgcttagc catgtgcttg     240
   ttattagtgg acaatcaaca aatattttct gaattaaaac ccagatttga atttttgaaa     300
   cataagattt attttattaa aataagaaaa tatacctagc catatacctc cccaaattga     360
   aaaaaggtat tcagacaaag acttatacac aatattcata g                         401
<210> 292
   <211> 401
   <212> DNA
   <213> homo sapiens
<220>
   <221> variation
   <222> 38
   <223> /replace="t"
<400> 292
   ccaaggtcct gaggtaacag atgtccccat taactcagcc aggaggggct gaaaggcaca 60
   tcagactaaa gagagagaga gaggtaaaag gtcctagctc ctcaaactga gtgccagtgg 120
   atagtgttgc acagagtggg aagagggctc atggcaggag ccaaacccaa gtgtcccacg 180
   ggatgtgtgt cttgagacaa tatggccagc cccacccctc ccctcatccc tcagtgaaaa 240
   tgacatccaa gtgggacagc ccagtgccat ggcagcaaga cagctggagc tggaagcaca 300
   gaggggtgga gagatctctg tgggctggtg gtggggccag ggctggtggt ggggctgctg 360
   gtctatgtgg tcacccccag gcacctgttg tgccatggga t 401
<210> 293
   <211> 440
   <212> PRT
   <213> homo sapiens
<400> 293
   Met Glu Gln Thr Tyr Gly Glu Val Asn Gln Leu Gly Gly Val Phe Val
   1 5 10 15
   Asn Gly Arg Pro Leu Pro Asn Ala Ile Arg Leu Arg Ile Val Glu Leu
   20 25 30
   Ala Gln Leu Gly Ile Arg Pro Cys Asp Ile Ser Arg Gln Leu Arg Val
   35 40 45
   Ser His Gly Cys Val Ser Lys Ile Leu Ala Arg Tyr Asn Glu Thr Gly
   50 55 60
   Ser Ile Leu Pro Gly Ala Ile Gly Gly Ser Lys Pro Arg Val Thr Thr
   65 70 75 80
   Pro Asn Val Val Lys His Ile Arg Asp Tyr Lys Gln Gly Asp Pro Gly
   85 90 95
   Ile Phe Ala Trp Glu Ile Arg Asp Arg Leu Leu Ala Asp Gly Val Cys
   100 105 110
   Asp Lys Tyr Asn Val Pro Ser Val Ser Ser Ile Ser Arg Ile Leu Arg
   115 120 125
   Asn Lys Ile Gly Ser Leu Ala Gln Pro Gly Pro Tyr Glu Ala Ser Lys
   130 135 140
   Gln Pro Pro Ser Gln Pro Thr Leu Pro Tyr Asn His Ile Tyr Gln Tyr
   145 150 155 160
   Pro Tyr Pro Ser Pro Val Ser Pro Thr Gly Ala Lys Met Gly Ser His
   165 170 175
   Pro Gly Val Pro Gly Thr Ala Gly His Val Ser Ile Pro Arg Ser Trp
   180 185 190
   Pro Ser Ala His Ser Val Ser Asn Ile Leu Gly Ile Arg Thr Phe Met
   195 200 205
   Glu Gln Thr Gly Ala Leu Ala Gly Ser Glu Gly Thr Ala Tyr Ser Pro
   210 215 220
   Lys Met Glu Asp Trp Ala Gly Val Asn Arg Thr Ala Phe Pro Ala Thr
   225 230 235 240
   Pro Ala Val Asn Gly Leu Glu Lys Pro Ala Leu Glu Ala Asp Ile Lys
   245 250 255
   Tyr Thr Gln Ser Ala Ser Thr Leu Ser Ala Val Gly Gly Phe Leu Pro
   260 265 270
   Ala Cys Ala Tyr Pro Ala Ser Asn Gln His Gly Val Tyr Ser Ala Pro
   275 280 285
   Gly Gly Gly Tyr Leu Ala Pro Gly Pro Pro Trp Pro Pro Ala Gln Gly
   290 295 300
   Pro Pro Leu Ala Pro Pro Gly Ala Gly Val Ala Val His Gly Gly Glu
   305 310 315 320
   Leu Ala Ala Ala Met Thr Phe Lys His Pro Ser Arg Glu Gly Ser Leu
   325 330 335
   Pro Ala Pro Ala Ala Arg Pro Arg Thr Pro Ser Val Ala Tyr Thr Asp
   340 345 350
   Cys Pro Ser Arg Pro Arg Pro Pro Arg Gly Ser Ser Pro Arg Thr Arg
   355 360 365
   Ala Arg Arg Glu Arg Gln Ala Asp Pro Gly Ala Gln Val Cys Ala Ala
   370 375 380
   Ala Pro Ala Ile Gly Thr Gly Arg Ile Gly Gly Leu Ala Glu Glu Glu
   385 390 395 400
   Ala Ser Ala Gly Pro Arg Gly Ala Arg Pro Ala Ser Pro Gln Ala Gln
   405 410 415
   Pro Cys Leu Trp Pro Asp Pro Pro His Phe Leu Tyr Trp Ser Gly Phe
   420 425 430
   Leu Gly Phe Ser Glu Leu Gly Phe
   435 440
<210> 294
   <211> 2838
   <212> DNA
   <213> homo sapiens
<400> 294
   aaattgattc ccgcacgctg cagcctcccg gtcagacgaa tttctcccaa tcggatgaag      60
   ttcaccctgg gcctggggtc gcgggcgtgg agagtgtcct gggagggggc agcagcggcg     120
   gcggcaggcc ctggagcggg cggcagcgcg ctccgctgcc gcgcacagcg cgtctccagc     180
   ccgcggctgg gccgccgcgg ctctcggctc tcgggcgccc tccctctatg cctctcacgc     240
   ggcggcggcg gcgcccaagc tctcccggac tgcgccgggc ccagccccgg ccaccccggc     300
   caccccggcg ccaggcagct ggccggcccg ctcgctatgg agcagacgta tggcgaggtg     360
   aaccagctgg gcggtgtgtt cgtcaacggc cgccccctgc ccaacgccat ccgcttgcgc     420
   attgtggagc tggcgcagct gggcatccga ccctgtgaca tcagtcggca gctccgcgta     480
   tcccacggct gcgtgagcaa gatcctggcg cgctacaacg agaccggctc cattctgccc     540
   ggggccatcg gggggagcaa gccccgcgtc accactccca acgtggtcaa gcacatccgg     600
   gactacaagc aaggagaccc tggcatcttt gcctgggaga tccgcgaccg gctgctggcc     660
   gacggcgtct gtgacaagta caatgtgcct tcggtgagct ccatcagccg catcctgcgc     720
   aacaagatcg gcagcctggc gcagcccgga ccgtacgagg caagtaagca gccgccgtcg     780
   cagcctacgc tgccctacaa ccacatctac cagtacccct accccagtcc cgtgtcgccc     840
   acgggcgcca agatgggcag ccaccccggg gtcccgggca cggcgggcca cgtcagcatc     900
   ccgcgctcat ggccctcggc acactcggtc agcaacatcc tgggcatccg gacgtttatg     960
   gagcaaacag gggccctggc tgggagcgaa ggcaccgctt actctcccaa gatggaagac    1020
   tgggccggcg tgaaccgcac ggccttcccc gccacccccg cagtgaatgg gctagagaaa    1080
   cctgccttag aggcagacat taaatacact cagtcggcct ccaccctctc tgccgtgggc    1140
   ggctttctcc ccgcctgcgc ctacccggcc tccaaccagc acggcgtgta cagcgccccg    1200
   ggcggcggct acctcgcccc gggcccgccg tggccgcctg cgcaaggtcc tcctctggcg    1260
   ccccccgggg ccggcgtagc tgtgcatggc ggggaactcg cggcagcaat gaccttcaag    1320
   catcccagcc gagaaggaag cctcccagct ccggcagcaa ggccccggac gccctcagta    1380
   gcttacacgg actgcccatc ccggcctcga cctcctaggg gcagctctcc ccggacccga 1440
   gcccggaggg aacggcaggc ggacccgggc gcacaggtct gcgcggcggc cccggcaatc 1500
   ggcacgggca ggatcggagg actcgcggag gaggaagcca gtgccggccc gcggggtgca 1560
   cgcccagcca gcccccaggc ccagccctgc ctctggccgg acccaccaca cttcctttat 1620
   tggtctgggt ttttaggctt ctctgaactt gggttttaga ctgccgtacc ctcctcacaa 1680
   tccttgctct gacgtggcct ccttcgctct gccagcttca aatttctttt ttgtcactcc 1740
   cttgtccgtc tccgtctcgc ctctctccct gtttccttcc cccctctttc tttctcactc 1800
   tccctccctt ccctttctct ttcccttcct ccctaccttc caccccggct ttccccgacc 1860
   ttccagggct ccctctgccc ttccactctc ttttccttgc tccgacctct gctccagtcc 1920
   cgcttcttcc cccgtctcct ctttctagtc ctctatatgc tatcagccct ttttcctggt 1980
   ccatccctgt cgttccttct ctctctgtct ctggttctac tgtcaagggc cctagttccc 2040
   tttgttttac tgcttcccca accctccctc agtccctgag agccctagag ccatctcggt 2100
   gacagtccct tctctggcac ctgggacttt gtagccttca ctcctctgag cccaccagcc 2160
   ctaatggagg ggggctcaaa cctaggaagt ggacagaaaa gccacagcct ttattttgta 2220
   ttttttttgt aataacaaac cctaaaaaac cctccatgaa ctgtcctctc ccgtcgatcc 2280
   ccgcagcata ttgtctggaa atctgttttc tttctcagag cccagtggcc gccgggcttc 2340
   tcttcccttc tgagatgatg tcaccccaaa gaagactggg gtaggagtag ggagcacagg 2400
   ggttgttggt gttttcagtg cttagaaaac tgggttgtcc tgcaggtgtc tcctcccacc 2460
   cctccactcg caacacttgc tagggaatcc gaccagtggt aaacacatca gcactttttg 2520
   gtgttcaggg gtcagcacat ttgttaattt aaaccacaga ctgcttccag gtacaaaggt 2580
   tcatttggac ttctggtttg caagggatga gggttaaaaa aaacaaaaca aaacaaacaa 2640
   caacaacaac aaaaacaacc cagaaagtat cttctagagc cagccgcaag agctgtgagc 2700
   tgcttccaag ttaggcgagc tggtggtggt accctggaca taatgcaatt ctgtagtcag 2760
   agcaatatcc ctggcgagag cctgacatcg cctgttaaag gtggctgcct ctgaataaag 2820
   gaaagcgtct gaaaggtg 2838